# EUROPEAN PATENT SPECIFICATION

(11) **EP 4 028 094 B1**
(45) Date of publication and mention of the grant of the patent: **15.04.2026**
(21) Application number: 20862487.4
(22) Date of filing: 10.09.2020
(51) Int. Cl.: A61M 16/06, A61M 16/08, A61M 16/20, A61M 16/00

(54) **PATIENT INTERFACE AND POSITIONING AND STABILISING STRUCTURE FOR THE PATIENT INTERFACE**
PATIENTENSCHNITTSTELLE UND POSITIONIERUNGS- UND STABILISIERUNGSSTRUKTUR FÜR DIE PATIENTENSCHNITTSTELLE
INTERFACE PATIENT ET STRUCTURE DE POSITIONNEMENT ET DE STABILISATION POUR L'INTERFACE PATIENT

(30) Priority: 10.09.2019 AU 2019903367; 28.02.2020 AU 2020900590; 29.05.2020 AU 2020901773
(43) Date of publication of application: 20.07.2022
(73) Proprietor: ResMed Pty Ltd, Bella Vista, New South Wales 2153 (AU)
(72) Inventor: BARLOW, Adam Francis, Bella Vista, New South Wales 2153 (AU); STANISLAS, Luke Andrew, Bella Vista, New South Wales 2153 (AU); FINLAY, Iain McNicol, Bella Vista, New South Wales 2153 (AU); PARKER, Christopher Daniel, Bella Vista, New South Wales 2153 (AU); YU, Hongjiang, Bella Vista, New South Wales 2153 (AU); KLINKENBERG, Luke Emmanuel, Bella Vista, New South Wales 2153 (AU); THOMAS, Hugh Francis Stewart, Bella Vista, New South Wales 2153 (AU); KOOIJ, Michiel, Bella Vista, New South Wales 2153 (AU); WATSON, Paul Derrick, Bella Vista, New South Wales 2153 (AU); KHERA, Rahul, Bella Vista, New South Wales 2153 (AU); GREIG, Nigel Paul, Bella Vista, New South Wales 2153 (AU); DAY, Shannon, Bella Vista, New South Wales 2153 (AU); MANJUNATH, Vinay, Bella Vista, New South Wales 2153 (AU)
(74) Representative: Vossius & Partner Patentanwälte Rechtsanwälte mbB
(86) International application number: PCT/AU2020/050959
(87) International publication number: WO 2021/046605

(56) References cited:
- EP-A1- 4 051 354
- EP-B1- 3 906 078
- WO-A1-2011/022779
- WO-A1-2016/193859
- WO-A1-2019/003094
- WO-A1-2019/123348
- WO-A2-2010/023590
- AU-A1- 2018 201 283
- AU-A4- 2005 100 738
- US-A1- 2015 059 759
- US-A1- 2015 352 308
- US-A1- 2016 082 214
- US-A1- 2016 158 475
- US-A1- 2016 158 475
- US-A1- 2017 035 979
- US-A1- 2017 035 979
- US-A1- 2017 312 468
- US-A1- 2017 312 468

## Description

### 1 CROSS-REFERENCE TO RELATED APPLICATIONS

This application claims the benefit of Australian Provisional Application No. 2019903367 filed on September 10, 2019; Australian Provisional Application No. 2020901773, filed May 29, 2020; and Australian Provisional Application No. 2020900590, filed February 28, 2020.

### 2 BACKGROUND OF THE TECHNOLOGY

### 2.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 2.2 DESCRIPTION OF THE RELATED ART

### 2.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"*Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient CO₂ to meet the patient's needs. Respiratory failure may encompass some or all of the following disorders.

A patient with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

### 2.2.2 Therapy

Various therapies, such as Continuous Positive Airway Pressure (CPAP) therapy, Non-invasive ventilation (NIV) and Invasive ventilation (IV) have been used to treat one or more of the above respiratory disorders.

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

### 2.2.3 Treatment Systems

These therapies may be provided by a treatment system or device. Such systems and devices may also be used to screen, diagnose, or monitor a condition without treating it.

A treatment system may comprise a Respiratory Pressure Therapy Device (RPT device), an air circuit, a humidifier, a patient interface, and data management.

Another form of treatment system is a mandibular repositioning device.

### 2.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O.

Certain other patient interface systems may be functionally unsuitable for the present field. For example, purely ornamental patient interface may be unable to maintain a suitable pressure. Patient interface systems used for underwater swimming or diving may be configured to guard against ingress of water from an external higher pressure, but not to maintain air internally at a higher pressure than ambient.

Certain patient interfaces may be clinically unfavourable for the present technology e.g. if they block airflow via the nose and only allow it via the mouth.

Certain patient interfaces may be uncomfortable or impractical for the present technology if they require a patient to insert a portion of a mask structure in their mouth to create and maintain a seal via their lips.

Certain patient interfaces may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some patient interfaces suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Patient interfaces designed solely for aviators, masks designed as part of personal protection equipment (e.g. filter masks), SCUBA masks, or for the administration of anaesthetics may be tolerable for their original application, but nevertheless such patient interfaces may be undesirably uncomfortable to be worn for extended periods of time, e.g., several hours. This discomfort may lead to a reduction in patient compliance with therapy. This is even more so if the patient interface is to be worn during sleep.

CPAP therapy is highly effective to treat certain respiratory disorders, provided patients comply with therapy. If a patient interfaces is uncomfortable, or difficult to use a patient may not comply with therapy. Since it is often recommended that a patient regularly wash their patient interfaces, if a patient interfaces is difficult to clean (e.g., difficult to assemble or disassemble), patients may not clean their patient interfaces and this may impact on patient compliance.

While a patient interfaces for other applications (e.g. aviators) may not be suitable for use in treating sleep disordered breathing, a patient interfaces designed for use in treating sleep disordered breathing may be suitable for other applications.

For these reasons, patient interfaces for delivery of CPAP during sleep form a distinct field.

### 2.2.3.1.1 Seal-forming structure

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal patient interfaces, full-face patient interfaces, nasal pillows, nasal puffs and oro-nasal patient interfaces.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the patient interface so as to provide a self-sealing action against the face of the patient when positive pressure is applied within the patient interface. Like the previous style of seal forming portion, if the match between the face and the patient interface is not good, additional force may be required to achieve a seal, or the patient interface may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFT^{™} nasal pillows mask, SWIFT^{™} II nasal pillows mask, SWIFT^{™} LT nasal pillows mask, SWIFT^{™} FX nasal pillows mask and MIRAGE LIBERTY^{™} full-face mask. The following patent applications, assigned to ResMed Limited, describe examples of nasal pillows masks: International Patent Application WO2004/073,778 (describing amongst other things aspects of the ResMed Limited SWIFT^{™} nasal pillows), US Patent Application 2009/0044808 (describing amongst other things aspects of the ResMed Limited SWIFT^{™} LT nasal pillows); International Patent Applications WO 2005/063,328 and WO 2006/130,903 (describing amongst other things aspects of the ResMed Limited MIRAGE LIBERTY^{™} full-face mask); International Patent Application WO 2009/052,560 (describing amongst other things aspects of the ResMed Limited SWIFT^{™} FX nasal pillows).

### 2.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 2.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressurised. Examples of RPT devices include a CPAP device and a ventilator.

### 2.2.3.3 Humidifier

Delivery of a flow of air without humidification may cause drying of airways. The use of a humidifier with an RPT device and the patient interface produces humidified gas that minimizes drying of the nasal mucosa and increases patient airway comfort. In addition in cooler climates, warm air applied generally to the face area in and about the patient interface is more comfortable than cold air.

### 2.2.3.4 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system.

Existing processes to communicate and manage such data can be one or more of costly, time-consuming, and error-prone.

### 2.2.3.5 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the patient interface. Many such vents are noisy. Others may become blocked in use and thus provide insufficient washout. Some vents may be disruptive of the sleep of a bed partner of the patient 1000, e.g. through noise or focussed airflow.

ResMed Limited has developed a number of improved patient interface vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

US 2017/312468 A1 relates to a mask system for delivery of respiratory therapy to a patient which includes a nares portion and a mouth portion, and an inlet conduit connected to at least one of the nares portion and the mouth portion to deliver the pressurized, breathable gas. The mask system is adapted to selectively utilize the nares portion and/or the mouth portion in a first mode utilizing both the nares portion and the mouth portion, and in a second mode utilizing the nares portion and not utilizing the mouth portion.

US 2017/035979 A1 relates to a gas ventilation mask which includes an anesthesia nasal mask and a mouth mask defining respectively a nasal chamber and an oral chamber, detachably connected to one another so that the nasal mask and the mouth mask may be used either separately as a nasal mask or as a mouth mask, or as a combination nasal-mouth mask. Also provided is an anesthesia mask strap system having a first expandable strap portion having the ability to extend; second and third non-expandable strap sections fixed to ends of the first expandable strap section; and an adhesion section for fixing a length of the strap system when the second and third non-expandable strap sections are pulled to tension the expandable strap section.

WO 2010/023590 A2 relates to respiratory patient interfaces that may be used to treat a variety of disorders involving upper airway obstruction, such as, without limitation, obstructive sleep apnea, obstructive sleep hypopnea, and upper airway resistance syndrome. A number of the embodiments employ a nasal pillow assembly including a frame that supports a nasal pillow sleeve and a clip that is slid over a flange of the fame to couple the nasal pillow sleeve to the frame.

WO 2011/022779 A1 relates to systems, devices and methods of use adapted for treatment of respiratory disease or sleep disordered breathing include a patient interface adapted to be secured to and sealed against a portion of a patient's face, in use. A flow generator is adapted to be connected to the patient interface and to be secured by a portion of the patient's body. The flow generator includes a blower adapted to provide pressurised breathable gas to a patient through the patient interface. The blower is adapted to be at least partially vibrationally isolated from the patient's body by at least one dampening system or device. The dampening device or system is adapted to reduce the amount of transmitted vibration received by the patient. A PAP system includes a patient interface including sealing arrangement adapted to form a seal with the patient's nose and/or mouth and headgear to support the sealing arrangement in position on the patient's head. A blower is structured to generate a supply of pressurized air. The blower is supported by the patient interface on the patient's head and in communication with the patient interface. The headgear forms one or more ducts (42) to communicate pressurized air from the blower to a breathing cavity defined by the sealing arrangement.

EP 3 906 078 B1 which constitutes a prior art document within the meaning of Article 54(3) EPC, relates to a patient interface for treating sleep disorder breathing which includes a headgear tube that provides support for the seal forming structure. The headgear tube includes a patient-contacting portion and a non-patient contacting portion that are joined along seams to form a gas passageway. The headgear tube may comprise a textile material and/or a foam material. Portions of the headgear tube may be imparted with greater rigidity than other portions.

EP 4 051 354 A1 which constitutes a prior art document within the meaning of Article 54(3) EPC, relates, relates to a patient interface kit to deliver a flow of air at a positive pressure with respect to ambient air pressure to an entrance to a patient's airways which includes at least the entrance of a patient's nares while the patient is sleeping. The patient interface comprises a nasal cushion having a nasal cushion opening, a mouth cushion having a mouth cushion opening, the mouth cushion including a flexible joint, positioned above the mouth opening, to selectively connect the nasal cushion to the mouth cushion, and a positioning and stabilizing structure to provide a force to hold the nasal and/or mouth seal-forming structures in a therapeutically effective position on a patient's head. The positioning and stabilizing structure includes a nasal headgear including upper straps or conduits and a mouth headgear including lower straps, the mouth headgear being selectively connected to the nasal headgear.

### 3 BRIEF SUMMARY OF THE TECHNOLOGY

The invention is defined in the appended claims. Aspects, embodiments and examples disclosed herein which do not fall within the scope of the appended claims do not form part of the invention and are merely provided for illustrative purposes.

The present technology is directed towards providing medical devices used in the screening, diagnosis, monitoring, amelioration, treatment, or prevention of respiratory disorders having one or more of improved comfort, cost, efficacy, ease of use and manufacturability.

One aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

An aspect of the present technology is directed to a patient interface system that is able to be used as either a nasal only type patient interface or an oro-nasal patient interface.

Another aspect of the present technology relates to an oro-nasal patient interface that is substantially comprised of flexible components.

Another aspect of the present technology relates to an oro-nasal patient interface that is stabilised at the nose sealing portion separately to the mouth sealing portion.

Another aspect of the present technology relates to an oro-nasal patient interface having at least a first headgear connection on the nasal portion of the patient interface and at least a second headgear connection on the mouth portion of the patient interface.

Another aspect of the present technology relates to a modular patient interface system that is adapted to be used in a nares only configuration, or in a nares and mouth configuration.

Another aspect of the present technology relates to a modular patient interface system that is adapted to be used in a nares only configuration, in a mouth only configuration, or in a nares and mouth configuration.

An aspect of the present technology is directed to a patient interface comprising: at least one plenum chamber for a patient interface, the plenum chamber being pressurisable to a therapeutic pressure of at least 6 cmH2O above ambient air pressure throughout a patient's respiratory cycle in use.

In one form of the present technology, the patient interface system is provided with a nasal portion and a mouth portion that are useable at the option of the patient with the nasal portion and the mouth portion, or with just the nasal portion. The mouth portion when used with the nasal portion may be used to deliver respiratory therapy to the patient's mouth, or may be used as a mouth seal.

In one form of the present technology, the patient interface system is provided with a nasal portion and a mouth portion that are useable at the option of the patient with the nasal portion and the mouth portion, with just the nasal portion, or with just the mouth portion.

In one form of the present technology, the patient interface system is provided with a nasal portion and a mouth portion, where the nasal portion includes a nares sealing portion adapted to form a seal with the patient's nares, where the nares sealing portion is structured to extend or curve outwardly from a supporting wall defining an air path into the nares sealing portion.

Another aspect of the present technology relates to a patient interface system that includes a nasal portion and a mouth portion, and the mouth portion is adapted to be used in both a first configuration where at least a portion of the pressurized, breathable gas is delivered to the patient's mouth and in a second configuration where the mouth portion functions as a mouth seal that prevents any of the pressurized, breathable gas from being delivered to the patient's mouth.

Another aspect of the present technology relates to a method of providing a patient interface kit for delivering respiratory therapy to a patient, where a nasal portion and a mouth portion are useable at the option of the patient with the nasal portion and the mouth portion, with just the nasal portion, or with just the mouth portion.

Another aspect of the present technology relates to a method of providing a patient interface kit for delivering respiratory therapy to a patient, where a nasal portion and a mouth portion are useable at the option of the patient with the nasal portion and the mouth portion, with just the nasal portion, or with just the mouth portion, where the when the nasal portion and the mouth portion are both utilized, the nasal portion and the mouth portion may both be utilized to deliver pressurized air to the nares and mouth of the patient, respectively, or one of the nasal portion and the mouth portion may be utilized to deliver pressurized air to the nares or mouth of the patient, while the other of the nasal portion and the mouth portion is utilized as a nares or mouth seal.

Another aspect of the present technology relates to a method of converting a patient interface system having a nasal portion and a mouth portion between first and second modes, where in the first mode the patient interface system is useable with the nasal portion and the mouth portion, and in the second mode the patient interface system is useable with only the nasal portion.

Another aspect of the present technology relates to a kit providing a nares only portion for delivering respiratory therapy to a patient's nares, the nasal portion adapted to function with a mouth portion.

Another aspect of the present technology relates to a kit providing a mouth portion for delivering respiratory therapy to a patient's mouth, the mouth portion adapted to function with a nasal portion.

Another aspect of the present technology relates to a method of providing respiratory therapy to one of a patient's nares only, a patient's mouth only, or to both a patient's nares and mouth, and periodically changing the respiratory therapy to another of the patient's nares only, the patient's mouth only, or to both the patient's nares and mouth.

Another aspect of the present technology relates to a method of respiratory therapy treatment, where a first patient interface with a first footprint is applied to a patient for a period of time, then the first patient interface is removed, and a second patient interface with a second footprint different from the first footprint is applied to the patient for a second period of time.

Another aspect of the present technology relates to a mouth portion for delivering respiratory therapy to a patient's mouth, the mouth portion including a docking station adapted to receive a nasal portion, wherein the nasal portion is capable of functioning as a nares only device for delivering pressurized gas to the nares of a patient.

Another aspect of the present technology relates to a patient interface system for delivery of respiratory therapy to a patient, the patient interface system including a nasal portion adapted to form a seal with the patient's nares, a mouth portion including a mouth plenum chamber and a mouth sealing portion adapted to form a seal with the patient's mouth, the mouth plenum chamber including an opening adapted to selectively receive pressurized, breathable gas, an inlet conduit connected to at least one of the nasal portion and the mouth portion to deliver the pressurized, breathable gas, wherein the patient interface system is adapted to selectively utilize the nares portion and/or the mouth portion in a first mode utilizing both the nasal portion and the mouth portion, and in a second mode utilizing the nasal portion and not utilizing the mouth portion.

Another aspect of the present technology relates to a patient interface system for delivery of respiratory therapy to a patient, the patient interface system including a nasal portion adapted to form a seal with the patient's nares, a mouth portion including a mouth plenum chamber and a mouth sealing portion adapted to form a seal with the patient's mouth, an inlet conduit connected to at least one of the nasal portion and the mouth portion to deliver pressurized, breathable gas to the patient, wherein the nasal portion and the mouth portion are adapted to connect to each other, and the mouth portion is adapted to selectively function in both a first configuration where at least a portion of the pressurized, breathable gas is delivered to the patient's mouth and in a second configuration where the mouth portion functions as a mouth seal that prevents any of the pressurized, breathable gas from being delivered to the patient's mouth.

Another aspect of the present technology relates to a patient interface system for delivery of respiratory therapy to a patient, the patient interface including a sealing portion including a nares sealing portion and a mouth sealing portion, the nares sealing portion adapted to form a seal with the patient's nares, the nares sealing portion structured to extend or curve outwardly from a supporting wall defining an air path into the nares sealing portion, the mouth sealing portion adapted to form a seal with the patient's mouth, the sealing portion adapted to connect to at least one inlet conduit to receive a supply of pressurized, breathable gas and headgear for retaining the sealing assembly in position on the head of the patient.

Another aspect of the present technology relates to a method of providing a patient interface system kit for delivering respiratory therapy to a patient, the method including providing a nasal portion adapted to form a seal with the patient's nares, providing a mouth portion including a mouth plenum chamber and a mouth sealing portion adapted to form a seal with the patient's mouth, the mouth plenum chamber including an opening adapted to selectively receive pressurized, breathable gas, and providing an inlet conduit connectable to at least one of the nasal portion and the mouth plenum chamber to deliver the pressurized, breathable gas, wherein, at the option of the patient, the patient interface system is adapted to selectively utilize the nasal portion and/or the mouth portion in at least first and second modes, the first mode utilizing the nasal portion and the mouth portion to provide the respiratory therapy to the patient's nares, wherein the mouth portion is configured to provide the respiratory therapy to the patient's mouth or to seal the mouth portion, and in the second mode utilizing the nasal portion to provide the respiratory therapy to the patient's nares and not utilizing the mouth portion as a seal or to deliver respiratory therapy.

Another aspect of the present technology relates to a method of converting a patient interface system between first and second modes, the patient interface system for delivery of respiratory therapy to a patient, the method including providing a nasal portion adapted to form a seal with the patient's nares, providing a mouth portion including a mouth plenum chamber and a mouth sealing portion adapted to form a seal with the patient's mouth, the mouth plenum chamber including an opening adapted to selectively receive pressurized, breathable gas, assembling the patient interface system in the first mode utilizing the nasal portion and the mouth portion to provide the respiratory therapy to the patient's nares, utilizing the mouth portion to provide the respiratory therapy to the patient's mouth or utilizing the mouth portion as a mouth seal, and converting the patient interface system in the second mode utilizing the nasal portion to provide the respiratory therapy to the patient's nares and not utilizing the mouth portion. The nasal portion may comprise a lip superior portion configured to seal against the lip superior of the patient in use; the lip superior portion has a stiffness similar to a stiffness of the central portion; the lip superior portion has a wall thickness substantially equal to that of the central portion; the lip superior portion comprises a wall thickness lesser than a wall thickness of the intermediate portions.

Another aspect of the present technology relates to a patient interface system for delivery of respiratory therapy to a patient, the patient interface system including a mouth portion including a mouth plenum chamber and a mouth sealing portion adapted to form a seal with the patient's mouth, the mouth plenum chamber including an opening adapted to selectively receive pressurized, breathable gas, and a nasal portion docking station formed on the mouth portion, the nasal portion docking station adapted to receive a nasal portion, wherein the nasal portion is capable of functioning as a nares only device for delivering pressurized gas to the nares of the patient.

Another aspect of the present technology relates to a medical package including a kit for converting a CPAP device comprising a nares only portion to a CPAP device having a nares and a mouth portion, the medical package including a mouth portion including a mouth plenum chamber and a mouth sealing portion adapted to form a seal with the patient's mouth, the mouth plenum chamber including an opening adapted to selectively receive pressurized, breathable gas, a vent plug adapted to plug an aperture in the nares only portion, and headgear adapted to secure at least the mouth portion to a patient's head.

Another aspect of the present technology relates to a system kit adapted to convert a nares only device for delivering respiratory therapy to a patient into a nares and mouth device for delivering respiratory therapy to a patient, the system kit including a mouth portion including a mouth plenum chamber and a mouth sealing portion adapted to form a seal with the patient's mouth, the mouth plenum chamber including an opening adapted to selectively receive pressurized, breathable gas, the mouth portion adapted to function with the nares only device as a nares and mouth device, a plug adapted to connect to either the opening in the mouth plenum chamber or an opening in the nares only device, and headgear adapted to secure at least the mouth portion to the patient's head.

Another aspect of the present technology relates to a patient interface system for delivery of respiratory therapy to a patient, the patient interface system including a nasal portion adapted to form a seal with the patient's nares, a mouth portion including a mouth plenum chamber and a mouth sealing portion adapted to form a seal with the patient's mouth, and a double elbow, e.g., having one branch connected to the nasal portion and another branch connected to the mouth portion to deliver the pressurized breathable gas to the nasal portion and the mouth portion.

Another aspect of the present technology relates to a patient interface system for delivery of respiratory therapy to a patient, the patient interface system including a nasal portion adapted to form a seal with the patient's nares, a mouth portion including a mouth plenum chamber and a mouth sealing portion adapted to form a seal with the patient's mouth, and a covering flap extending from the mouth portion to cover the nasal portion and/or the patient's nares in use.

Another aspect of the present technology relates to a patient interface system for delivery of respiratory therapy to a patient, the patient interface system including a nasal portion including a nares sealing portion adapted to form a seal with the patient's nares, a mouth portion including a mouth sealing portion adapted to form a seal with the patient's mouth, the mouth sealing portion being oriented generally horizontally and the nares sealing portion being oriented generally vertically.

Another aspect of the present technology relates to a patient interface system for delivery of respiratory therapy to a patient, the patient interface system including a nasal portion including a nares sealing portion and a nares supporting portion, the nares sealing portion adapted to form a seal with the patient's nares, a mouth portion including a mouth sealing portion adapted to form a seal with the patient's mouth, the nares supporting portion being a semi-rigid material and the nares sealing portion being a flexible material.

Another aspect of the present technology relates to a patient interface system for delivery of respiratory therapy to a patient, the patient interface system including a nasal portion including a nares sealing portion and a nares supporting portion, the nares sealing portion adapted to form a seal with the patient's nares, a mouth portion including a mouth sealing portion adapted to form a seal with the patient's mouth, the mouth portion and/or the nasal portion including a magnet to retain the mouth portion and/or the nasal portion in position.

Another aspect of the present technology relates to a patient interface system for delivery of respiratory therapy to a patient, the patient interface system including a nasal portion adapted to form a seal with the patient's nares, a mouth portion adapted to form a seal with the patient's mouth, and a foam portion adapted to clip onto at least one of the nasal portion and the mouth portion to form a seal between the patient interface system and the patient.

Another aspect of the present technology relates to a patient interface system for delivery of respiratory therapy to a patient, the patient interface system including a nasal portion including a nares sealing portion and a nares supporting portion, the nares sealing portion adapted to form a seal with the patient's nares, a mouth portion including a mouth sealing portion adapted to form a seal with the patient's mouth, and a connecting ring adapted to connect the mouth portion to the nasal portion, the connecting ring having a first channel adapted to receive the mouth portion and a second channel adapted to receive the nasal portion.

Another aspect of the present technology relates to a patient interface system for delivery of respiratory therapy to a patient, the patient interface system including a nasal portion including a nares sealing portion and a nares supporting portion, the nares sealing portion adapted to form a seal with the patient's nares, a mouth portion including a mouth sealing portion adapted to form a seal with the patient's mouth, wherein the nares sealing portion has indentations adapted to form an interference fit with the mouth portion.

Another aspect of the present technology relates to a patient interface system for delivery of respiratory therapy to a patient, the patient interface system including a nasal portion including a nares sealing portion and a nares supporting portion, the nares sealing portion adapted to form a seal with the patient's nares, a mouth portion including a mouth sealing portion adapted to form a seal with the patient's mouth, upper headgear adapted to connect to the nasal portion, the upper headgear including a back strap adapted to wrap around a back of the patient's head, and lower headgear adapted to connect to the mouth portion, the lower headgear having a loop adapted to receive the back strap of the upper headgear.

Another aspect of the present technology relates to a patient interface system for delivery of respiratory therapy to a patient, the patient interface system including a nasal portion including a nares sealing portion adapted to form a seal with the patient's nares, an elbow connected to the nasal portion to deliver pressurized gas, the elbow including a lug portion, a mouth portion including a mouth sealing portion adapted to form a seal with the patient's mouth, the mouth portion including an aperture selectively connectable to the lug portion of the elbow to connect the mouth portion to the nasal portion.

Another aspect of the present technology relates to a patient interface system for delivery of respiratory therapy to a patient, the patient interface system including a nasal portion including a nares sealing portion adapted to form a seal with the patient's nares, a mouth portion including a mouth sealing portion adapted to form a seal with the patient's mouth, the mouth sealing portion including a cushion having a pocket or lower stiffness region, shaped and adapted to receive the nasal portion.

Another aspect of the present technology relates to a patient interface system for delivery of respiratory therapy to a patient, the patient interface system including a nasal portion including a nares sealing portion and a nares supporting portion, the nares sealing portion adapted to form a seal with the patient's nares, a mouth portion including a mouth sealing portion adapted to form a seal with the patient's mouth, the mouth portion including a cushion and a cushion fascia portion, the cushion fascia portion adapted to connect to the cushion, the cushion having a connecting portion with an aperture, the aperture adapted to receive a ring to connect the nasal portion to the mouth portion.

Another aspect of the present technology relates to a patient interface system for delivery of respiratory therapy to a patient, the patient interface system including a mouth portion including a mouth sealing portion adapted to form a seal with the patient's mouth, the mouth sealing portion not comprising a pocket.

Another aspect of the present technology relates to a patient interface system for delivery of respiratory therapy to a patient, comprising: a nasal portion including a nasal chamber and a nasal sealing portion adapted to form a seal with the patient's face surrounding the entrance to a patient's nares, the nasal chamber including an opening; an inlet conduit connected to the nasal portion to deliver the pressurized, breathable gas to the nasal chamber; and a nasal chamber coupling component adapted to form part of a releasable fluid coupling to enable the nasal portion to be fluidly coupled to a mouth portion to communicate a portion of the pressurized, breathable gas through the nasal chamber opening to the mouth portion.

In some forms: (a) the nasal portion comprises a nasal cushion that forms at least part of the nasal chamber and wherein the nasal chamber opening and the coupling component are formed in the nasal cushion; the nasal cushion is a pillow cushion; (b) the nasal cushion is a cradle cushion; (c) the coupling component comprises a clip mechanism that extends about the nasal chamber opening and arranged to form part of a resilient clip connection; (d) the clip mechanism includes a resilient hinge; (e) the coupling component comprises an at least partial peripheral rim extending about the nasal chamber opening; (f) the peripheral rim includes at least one of projections or recesses that allow for an interlocking connection; (g) the peripheral rim includes a raked surface to provide a lead in surface to a resilient connection; and/or (g) the coupling component forms part of a latch connection.

In some forms the patient interface further comprises a removable vent module adapted to be attached to the nasal chamber to vent gas exhaled by the patient; the vent module being arranged to be releasable connectable with the nasal chamber coupling component such that the patient interface system is configurable in a first mode where the vent module is fluidly coupled to the nasal chamber to enable the patient interface system to operate as a nasal only patient interface and a second mode where the vent module is removed to enable the nasal portion to be fluidly coupled to a mouth portion to communicate a portion of the pressurized, breathable gas through the nasal chamber opening to the mouth portion.

In some forms: (a) the patient interface further includes at least one guide vane providing an obstruction to the fluid coupling to direct the follow of gas from the nasal chamber through the vent module; (b) the at least one guide vane forms part of the vent module; (c) the vent module is an anti-asphyxiation vent (AAV); and/or (d) the AAV includes a flap and the at least one guide vane is arranged to direct gas from the nasal chamber onto the flap.

In some forms the patient interface further comprising a mouth portion including a mouth chamber and a mouth sealing portion adapted to form a seal with the patient's mouth, the mouth chamber including an opening adapted to receive pressurized, breathable gas, and wherein when fluidly coupled, the nasal portion is adapted to communicate the portion of the pressurized, breathable gas to the mouth portion through the nasal chamber opening and the mouth chamber opening.

In some forms: (a) the mouth chamber includes a vent opening adapted to receive a vent adapted to vent gas exhaled by the patient; (b) the vent opening is configured to receive the removable vent module; (c) the mouth portion comprises a mouth cushion that forms at least part of the mouth chamber, and wherein the opening is formed in the mouth cushion; (d) the mouth portion further comprises a coupling component arranged to form part of the fluid coupling to connect the nasal portion and the mouth portion; (e) the mouth portion coupling component extends around the mouth chamber opening; (f) the mouth portion coupling component comprises a clip mechanism that extends about the mouth chamber opening and arranged to form part of a resilient clip connection; (g) the clip mechanism includes a resilient hinge; (h) the mouth portion coupling component comprises an at least partial peripheral rim extending about the mouth chamber opening; (i) the peripheral rim includes at least one of projections or recesses that allow for an interlocking connection; (j) the peripheral rim includes a raked surface to provide a lead in surface to a resilient connection; (k) the mouth portion coupling component forms part of a latch connection; (l) the mouth portion coupling component is formed as an overmoulding incorporating a silicone overlay and a rigid backing; (m) the mouth chamber coupling component is adapted to connect directly with the nasal chamber coupling component to form the fluid coupling; (n) the patient interface further including a connector adapted to form part of the fluid coupling and having a nasal portion end adapted to be attachable to the nasal chamber coupling component, an opposite mouth portion end attachable to the mouth portion coupling component, and a passage extending between the nasal portion end and the mouth portion end; (o) the connector further comprises at least one vane disposed in the connector passage; (p) at least one of the nasal portion end or the mouth portion end includes a clipping mechanism to engage with the coupling component of the mouth portion; (q) at least one of the nasal portion end or the mouth portion end is resiliently deformable to connect with the coupling component of the nasal portion or mouth portion; (r) at least one of the nasal portion end or the mouth portion end include at least one of projections or recesses that allow for an interlocking connection with the coupling component of the nasal portion or mouth portion; (s) at least one of the nasal portion end or the mouth portion end include a part latching formation that allow for a latch connection with the coupling component of the nasal portion or mouth portion; (t) the connector incorporates a connector chamber intermediate the nasal and mouth end portions; (u) the connector is formed as a unitary structure; (v) the connector is formed from multiple components; (w) an anti-asphyxiation vent (AAV) is integrally formed with the mouth portion coupling component; (x) the connector incorporates an anti-asphyxiation vent (AAV) disposed between the nares portion end and mouth portion end; (y) the AAV includes a flap that extends across an interior of the connector passage; (z) the connector includes one or more guide vanes which extend across at least part of the interior of the connection to direct the flow of gas in the connector passage; (aa) the one or more guide vanes is arranged to direct gas from the nasal chamber onto the flap; and/or (ab) the patient interface further including a locking member to form part of the connection formed at least one of the nasal portion or mouth portion coupling components in fluidly coupling the nasal portion to the mouth portion;

In some forms the mouth portion further comprises a coupling component arranged to form part of the fluid coupling to connect the nasal portion and the mouth portion.

In some forms, the mouth chamber coupling component is adapted to connect directly with the nasal chamber coupling component to form the fluid coupling.

In some forms, the patient interface further comprises a connector adapted to form part of the fluid coupling and having a nasal portion end adapted to be attachable to the nasal chamber coupling component, an opposite mouth portion end attachable to the mouth portion coupling component, and a passage extending between the nasal portion end and the mouth portion end.

In some forms, the connector incorporates an anti-asphyxiation vent (AAV) disposed between the nares portion end and mouth portion end.

Another aspect of the technology relates to A patient interface system for delivery of respiratory therapy to a patient, comprising: a nasal portion including a nasal chamber and a nasal sealing portion adapted to form a seal with the patient's face surrounding the entrance to a patient's nares, the nasal chamber including an opening; an inlet conduit connected to the nasal portion to deliver the pressurized, breathable gas to the nasal chamber; a vent fluidly coupled to an opening in the nasal chamber to vent gas exhaled by the patient; and at least one guide vane providing an obstruction to the fluid coupling to direct the follow of gas from the nasal chamber through the vent.

In some forms: (a) the nasal portion comprises a nasal cushion that forms at least part of the nasal chamber and wherein the nasal chamber opening is formed in the nasal cushion; (b) the nasal cushion is a pillow cushion; (c) the nasal cushion is a cradle cushion; (d) the vent forms part of a vent module that is fitted to the nasal portion and incorporates the at least one guide vane; (e) the patient interface further including a connector adapted to form part of a fluid coupling between the nasal chamber and the vent, the connector having a nasal portion end adapted to be fitted to the nasal chamber, an opposite vent end adapted to be fitted to the vent, and a passage extending between the nasal portion end and the vent end; (f) the connector further comprises the at least one guide vane disposed in the connector passage; (g) the vent is an anti-asphyxiation vent (AAV); and/or (h), the AAV includes a flap and the at least one guide vane is arranged to direct gas from the nasal chamber onto the flap.

Another aspect of the technology relates to a patient interface system for delivery of respiratory therapy to a patient, comprising: a mouth portion including a mouth chamber and a mouth sealing portion adapted to form a seal with the patient's mouth, the mouth chamber including an opening adapted to receive pressurized, breathable gas, and a mouth chamber coupling component adapted to form part of a releasable fluid coupling to enable the mouth portion to be fluidly coupled to a nasal portion to receive a portion of the pressurized, breathable gas from the nasal portion through the mouth portion opening.

In some forms: (a) the mouth portion comprises a mouth cushion that forms at least part of the mouth chamber, and wherein the opening is formed in the mouth cushion; (b) the mouth portion coupling component extends around the mouth chamber opening; (c) the mouth portion coupling component comprises a clip mechanism that extends about the mouth chamber opening and arranged to form part of a resilient clip connection; (d) the clip mechanism includes a resilient hinge; (e) the mouth portion coupling component comprises an at least partial peripheral rim extending about the mouth chamber opening; (f) the peripheral rim includes at least one of projections or recesses that allow for an interlocking connection; (g) the peripheral rim includes a raked surface to provide a lead in surface to a resilient connection; (h) the mouth portion coupling component forms part of a latch connection; (i) the coupling component is formed as an overmoulding incorporating a silicone overlay and a rigid backing; and/or (j) an anti-asphyxiation vent (AAV) is integrally formed with the mouth portion coupling component.

Another aspect of the technology relates to a patient interface system for delivery of respiratory therapy to a patient, comprising: a mouth portion including a mouth chamber and a mouth sealing portion adapted to form a seal with the patient's mouth, the mouth chamber including a coupling component adapted to receive pressurized, breathable gas; and an anti-asphyxiation vent (AAV) forming part of the mouth chamber coupling component.

In some forms: (a) the coupling component is formed as an overmoulding incorporating a silicone overlay and a rigid backing; (b) the patient interface further comprising at least one guide vane to direct the follow of gas to the AAV; and/or (c) the AAV includes a flap and the at least one guide vane is arranged to direct the flow of gas onto the flap.

Another aspect of the technology relates to patient interface system for delivery of respiratory therapy to a patient, comprising: a nasal portion including a nasal chamber and a nasal sealing portion adapted to form a seal with the patient's nares, the nasal chamber including an opening; a mouth portion including a mouth chamber and a mouth sealing portion adapted to form a seal with the patient's mouth, the mouth chamber including an opening adapted to receive pressurized, breathable gas; and an inlet conduit connected to at least one of the nasal portion or the mouth portion to deliver the pressurized, breathable gas, a connector enabling the mouth portion to be coupled to the nasal portion to provide a sealed connection between the opening in the nasal chamber and the opening in the mouth chamber, the connector including an anti- asphyxiation vent (AAV); the patient interface system being configurable so that the nasal portion is coupled to the mouth portion through the connector to communicate a portion of the pressurized, breathable gas between the nasal chamber and the mouth chamber with the AAV disposed between the nasal portion and mouth portion.

In some forms: (a) the AAV includes a flap that extends across an interior passage of the connector; (b) the connector includes one or more vanes which extend across at least part of the interior passage to direct the flow of gas onto the flap; (c) at least one of the nasal portion and the mouth portion includes headgear connectors adapted to connect to the headgear; (d) both the nasal portion and the mouth portion include headgear connectors to connect to the headgear; and/or (e) the headgear further comprises complementary patient interface connectors adapted to connect the headgear to either or both the nasal portion and the mouth portion.

According to another aspect of the present technology there is provided a method of providing a patient interface system kit for delivering respiratory therapy to a patient. The method comprising: providing a nasal portion including a nasal chamber and a nasal sealing portion adapted to form a seal with the patient's nares, the nasal chamber including an opening; providing an inlet conduit connected to the nasal portion to deliver the pressurized, breathable gas to the nasal chamber; providing a mouth portion including a mouth chamber and a mouth sealing portion adapted to form a seal with the patient's mouth, the mouth chamber including an opening adapted to receive pressurized, breathable gas; providing fluid coupling to enable a sealed connection between the opening in the nasal chamber and the opening in the mouth chamber; and providing a removable nasal vent module adapted to be fitted to the opening in the nasal chamber, wherein at the option of the patient, the patient system is adapted to selectively utilize the patient interface in at least first and second modes, the first mode having the nasal portion coupled to the mouth portion through the fluid coupling to communicate a portion of the pressurized, breathable gas between the nares chamber and the mouth chamber to deliver respiratory therapy through both the nasal portion and mouth portion; and in the second mode where the mouth portion is not coupled to the nasal portion via the fluid coupling and the nasal vent module is fitted to the opening in the nasal chamber to enable the patient interface system to delivery respiratory therapy as a nasal only patient interface.

According to another aspect of the present technology there is provided a method of providing a patient interface system kit for delivering respiratory therapy to a patient. The method comprising: providing a nasal portion including a nasal chamber and a nasal sealing portion adapted to form a seal with the patient's nares, the nares chamber including an opening; providing an inlet conduit connected to at least one of the nasal portion or the mouth portion to deliver the pressurized, breathable gas to the nasal chamber; providing a mouth portion including a mouth chamber and a mouth sealing portion adapted to form a seal with the patient's mouth, the mouth chamber including an opening adapted to receive pressurized, breathable gas; and providing a connector enabling the mouth portion to be coupled to the nasal portion to provide a sealed connection between the opening in the nasal chamber and the opening in the mouth chamber, the connector including an anti-asphyxiation vent (AAV); wherein patient interface system being configurable where the nasal portion is coupled to the mouth portion through the connector to communicate a portion of the pressurized, breathable gas between the nasal chamber and the mouth chamber with the AAV disposed between the nasal portion and mouth portion.

According to another aspect of the present technology there is provided a method of converting a patient interface system between first and second modes, the mask system for delivery of respiratory therapy to a patient. The method comprising: providing a nasal portion including a nasal chamber and a nasal sealing portion adapted to form a seal with the patient's nares, the nasal chamber including an opening; providing a mouth portion including a mouth chamber and a mouth sealing portion adapted to form a seal with the patient's mouth, the mouth chamber including an opening adapted to selectively receive pressurized, breathable gas; assembling the mask system in the first mode utilizing the nasal portion and the mouth portion to provide the respiratory therapy to the patient's nares, utilizing the mouth portion to provide the respiratory therapy to the patient's mouth; and converting the mask system in the second mode utilizing the nasal portion to provide the respiratory therapy to the patient's nares by disconnecting the mouth portion from the nasal portion and incorporating a vent in the nasal chamber opening.

According to another aspect of the present technology there is provided a positioning and stabilising structure to hold a seal-forming structure in a therapeutically effective position on a head of a patient, the seal-forming structure being constructed and arranged to form a seal with a region of the patient's face surrounding an entrance to the patient's airways for sealed delivery of a flow of air at a therapeutic pressure of at least 4 cmH₂O with respect to ambient air pressure throughout the patient's respiratory cycle in use. The positioning and stabilising structure comprising: a first strap configured, in use, to pass around the back of the patient's head; and an attachment arrangement disposed on the first strap and forming part of a coupling for releasably attaching a second strap to the first strap, the second strap being connectable to an additional component of the seal-forming structure that is, at the option of the patient, selectively combined with the seal forming structure.

According to another aspect of the present technology there is provided a positioning and stabilising structure to hold a patient interface in a therapeutically effective position on a head of a patient, the patient interface comprising a nasal portion including a nasal chamber and a nasal sealing portion adapted to form a seal with the patient's nares for sealed delivery of a flow of air at a therapeutic pressure of at least 4 cmH₂O with respect to ambient air pressure throughout the patient's respiratory cycle in use, the positioning and stabilising structure comprising: a first strap configured, in use, to pass around the back of the patient's head; and an attachment arrangement disposed on the first strap and forming part of a coupling for releasably attaching a second strap to the first strap, the second strap being connectable to a mouth portion that is, at the option of the patient, selectively combined with the nasal portion to allow the patient interface to deliver respiratory therapy through both the nasal portion and the mouth portion.

In some forms: (a) the attachment arrangement comprises an attachment region that allows the position of attachment of the second strap to vary within the attachment region; (b) the attachment arrangement is formed from multiple components; (c) the coupling comprises one or more of a hook or loop fastening mechanism; (d) the coupling comprises at least one snap fastener; (e) the attachment arrangement includes a guide portion to facilitate correct positioning of the second strap with the first strap; (f) the guide portion is in the form of indicia disposed on the first strap; (g) the guide portion comprises at least one of projections or recesses, the at least one projection or recess allowing for an interlocking connection with a corresponding at least one projection or recess of the second strap; (h) the attachment arrangement includes two or more guide portions that are located in a spaced manner along the first strap; (i) the attachment arrangement includes at least one slit along a portion of the first strap; (j) the positioning and stabilising structure further comprising the second strap, the second strap comprising a complementary attachment arrangement for forming the coupling with the attachment arrangement of the first strap; (k) the complementary attachment arrangement comprises a flap extending laterally from the second strap, the flap able to pass through the slit, in use, for releasably attaching the second strap to the first strap; (l) the flap, once passed through the slit in use, is releasably attached to the first strap to form at least part of the coupling; (m) the flap, once passed through the slit in use, is releasably attached to the second strap whereby the second strap is releasably attached to the first strap to forma at least part of the coupling; and/or (n) coupling assists in the dynamic stability of the positioning and stabilising structure to hold the seal-forming structure in a therapeutically effective position on a head of a patient.

According to another aspect of the present technology there is provided a patient interface system for delivery of respiratory therapy to a patient, comprising: a nasal portion including a nasal chamber and a nasal sealing portion adapted to form a seal with the patient's face surrounding the entrance to a patient's nares, the nasal chamber including an opening; an inlet conduit connected to the nasal portion to deliver the pressurized, breathable gas to the nasal chamber; and a positioning and stabilising structure to hold the patient interface in a therapeutically effective position on a head of a patient, the structure comprising: a first strap configured, in use, to pass around the back of the patient's head; and an attachment arrangement disposed on the first strap and forming part of a coupling for releasably attaching a second strap to the first strap, the second strap being connectable to a mouth portion that is, at the option of the patient, selectively combined with the nasal portion to allow the patient interface to deliver respiratory therapy through both the nasal portion and the mouth portion.

In some forms: (a) the patient interface system further comprises the mouth portion that is, at the option of the patient, selectively combined with the nasal portion, and the second strap connected to the mouth portion, the second strap comprising a complementary attachment arrangement for forming the coupling with the attachment arrangement of the first strap; (b) the patient interface system further comprises a nasal chamber coupling component adapted to form part of a releasable fluid coupling to enable the nasal portion to be fluidly coupled to the mouth portion to communicate a portion of the pressurized, breathable gas through the nasal chamber opening to the mouth portion; and/or (c) the coupling assists in the dynamic stability of the positioning and stabilising structure to hold the seal-forming structure in a therapeutically effective position on a head of a patient.

According to another aspect of the present technology there is provided a plenum chamber for a patient interface, the plenum chamber pressurisable to a therapeutic pressure of at least 6 cmH₂O above ambient air pressure, the plenum chamber comprising: one or more plenum chamber inlet ports sized and structured to receive a flow of air at the therapeutic pressure for breathing by a patient; a seal-forming structure constructed and arranged to form a seal with a region of the patient's face surrounding an entrance to the patient's airways, said seal-forming structure having one or more holes therein such that the flow of air at said therapeutic pressure is delivered to an entrance to the patient's nares and to the patient's mouth via the one or more holes, the seal-forming structure constructed and arranged to maintain said therapeutic pressure in the plenum chamber throughout the patient's respiratory cycle in use; wherein the seal-forming structure comprises a nasal portion configured to form a seal to the patient's face at or proximate the patient nose, and an oral portion configured to form a seal to the patient's face around the patient's mouth, the seal-forming structure having a first surface finish in the nasal portion and a second surface finish in the oral portion different from the first surface finish.

In examples: the first surface finish and the second surface finish differ in such a way that a coefficient of friction between the seal-forming structure and the patient's face is greater in the oral portion than in the nasal portion; the first surface finish is configured to provide the nasal portion with a smooth feel on the patient's face (e.g. for comfort); the second surface finish is configured to provide the oral portion with grippy contact on the patient's face (e.g. for a more robust seal); the first surface finish may be a frosted surface finish; the second surface finish may be a polished surface finish; a boundary between the first surface finish and the second surface finish may be located on a part of the seal-forming structure in contact with the patient's cheeks in use; the nasal portion may comprise a lip superior portion having the first surface finish; non-patient contacting surfaces of the nasal portion may comprise a surface finish other than the first surface finish; and/or the non-patient contacting surfaces of the nasal portion may comprise the second surface finish.

According to another aspect of the present technology, there is provided a patient interface comprising: a plenum chamber according to an aspect of the present technology described above; a positioning and stabilising structure to provide a force to hold the seal-forming structure in a therapeutically effective position on the patient's head, the positioning and stabilising structure comprising a tie, the tie being constructed and arranged so that at least a portion overlies a region of the patient's head superior to an otobasion superior of the patient's head in use; and a vent structure to allow a continuous flow of gases exhaled by the patient from an interior of the plenum chamber to ambient, said vent structure being sized and shaped to maintain the therapeutic pressure in the plenum chamber in use; wherein the patient interface is configured to allow the patient to breath from ambient through their mouth in the absence of a flow of pressurised air through the plenum chamber inlet port.

Another aspect of one form of the present technology is a patient interface that is moulded or otherwise constructed with a perimeter shape which is complementary to that of an intended wearer.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

In one aspect of the present technology is disclosed a positioning and stabilising structure to hold a seal-forming structure in a therapeutically effective position on a head of a patient. The seal-forming structure is constructed and arranged to form a seal with a region of the patient's face surrounding an entrance to the patient's airways for sealed delivery of a flow of air at a therapeutic pressure of at least 4 cmH2O with respect to ambient air pressure throughout the patient's respiratory cycle in use. The positioning and stabilising structure comprises a first strap and an attachment arrangement. The first strap is configured, in use, to pass around the back of the patient's head. The attachment arrangement is disposed on the first strap and forms part of a coupling for releasably attaching a second strap to the first strap. The second strap is connectable to an additional component of the seal-forming structure that is, at the option of the patient, selectively combined with the seal forming structure.

In a further aspect of the present technology is disclosed positioning and stabilising structure to hold a patient interface in a therapeutically effective position on a head of a patient. The patient interface comprising a nasal portion including a nasal chamber and a nasal sealing portion adapted to form a seal with the patient's nares for sealed delivery of a flow of air at a therapeutic pressure of at least 4 cmH2O with respect to ambient air pressure throughout the patient's respiratory cycle in use. The positioning and stabilising structure comprises a first strap and an attachment arrangement. The first strap is configured, in use, to pass around the back of the patient's head. The attachment arrangement is disposed on the first strap and forms part of a coupling for releasably attaching a second strap to the first strap. The second strap is connectable to a mouth portion that is, at the option of the patient, selectively combined with the nasal portion to allow the patient interface to deliver respiratory therapy through both the nasal portion and the mouth portion.

In some forms, the attachment arrangement may comprise an attachment region that allows the position of attachment of the second strap to vary within the attachment region.

In some forms, the attachment arrangement may be formed from multiple components.

In some forms, the coupling may comprise one or more of a hook or loop fastening mechanism.

In some forms, the coupling may comprise at least one snap fastener.

In some forms, the attachment arrangement may include a guide portion to facilitate correct positioning of the second strap with the first strap. In some forms, the guide portion may be in the form of indicia disposed on the first strap. In some forms, the guide portion may comprise at least one of projections or recesses, the at least one projection or recess allowing for an interlocking connection with a corresponding at least one projection or recess of the second strap. In some forms, the attachment arrangement may include two or more guide portions that are located in a spaced manner along the first strap.

In some forms, the attachment arrangement may include at least one slit along a portion of the first strap.

In some forms, the positioning and stabilising structure may further comprise the second strap. The second strap may comprise a complementary attachment arrangement for forming the coupling with the attachment arrangement of the first strap.

In some forms, the complementary attachment arrangement may comprise a flap extending laterally from the second strap, the flap able to pass through the slit, in use, for releasably attaching the second strap to the first strap. In some forms, the flap, once passed through the slit in use, may be releasably attached to the first strap to form at least part of the coupling. In some forms, the flap, once passed through the slit in use, may be releasably attached to the second strap whereby the second strap is releasably attached to the first strap to forma at least part of the coupling.

In some forms, coupling may assist in the dynamic stability of the positioning and stabilising structure to hold the seal-forming structure in a therapeutically effective position on a head of a patient.

In a further aspect of the present technology is disclosed a patient interface system for delivery of respiratory therapy to a patient. The patient interface system comprises a nasal portion, an inlet conduit, a positioning and a stabilising structure. The nasal portion includes a nasal chamber and a nasal sealing portion that are adapted to form a seal with the patient's face surrounding the entrance to a patient's nares. The nasal chamber includes an opening. The inlet conduit is connected to the nasal portion to deliver the pressurized, breathable gas to the nasal chamber. The positioning and stabilising structure holds the patient interface in a therapeutically effective position on a head of a patient. The positioning and a stabilising structure comprises a first strap and an attachment arrangement. The first strap is configured, in use, to pass around the back of the patient's head. The attachment arrangement is disposed on the first strap and forms part of a coupling for releasably attaching a second strap to the first strap. The second strap is connectable to a mouth portion that is, at the option of the patient, selectively combined with the nasal portion to allow the patient interface to deliver respiratory therapy through both the nasal portion and the mouth portion.

In some forms, the patient interface system may further comprise the mouth portion that is, at the option of the patient, selectively combined with the nasal portion, and the second strap connected to the mouth portion. The second strap comprises a complementary attachment arrangement for forming the coupling with the attachment arrangement of the first strap.

In some forms, the patient interface system may further comprise a nasal chamber coupling component adapted to form part of a releasable fluid coupling to enable the nasal portion to be fluidly coupled to the mouth portion to communicate a portion of the pressurized, breathable gas through the nasal chamber opening to the mouth portion.

In some forms, the coupling may assist in the dynamic stability of the positioning and stabilising structure to hold the seal-forming structure in a therapeutically effective position on a head of a patient.

In a further aspect of the present technology is disclosed a retaining assembly for connecting a patient interface to a positioning and stabilising structure configured to generate a force to hold the patient interface in a therapeutically effective position on the patient's head. The patient interface comprises: a plenum chamber pressurisable to a therapeutic pressure of at least 6 cmH2O above ambient air pressure throughout a patient's respiratory cycle in use; one or more plenum chamber inlet ports sized and structured to receive the flow of air at the therapeutic pressure for breathing by a patient throughout the patient's respiratory cycle in use; a seal-forming structure defining at least a portion of the plenum chamber, the seal-forming structure constructed and arranged to form a seal with a region of the patient's face surrounding an entrance to the patient's airways, the seal-forming structure constructed and arranged to maintain said therapeutic pressure in the plenum chamber throughout the patient's respiratory cycle in use; and a shell defining at least a portion of the plenum chamber, the shell supporting the seal-forming structure. The retaining assembly comprising a connector and a patient interface engagement portion. The connector comprises: a connecting portion configured to engage the positioning and stabilising structure and a retaining portion configured to retain the connector with respect to the patient interface. The patient interface engagement portion located on the patient interface and the patient interface engagement portion and the retaining portion being configured to removably engage one another through a first retention arrangement and a second retention arrangement.

In some forms, the first retention arrangement is a mechanical arrangement.

In some forms, the first retention arrangement includes complementary protrusions adapted to retain the retaining portion with respect to the patient interface engagement portion in a direction along a surface of the patient interface.

In some forms, the second retention arrangement is magnetic.

In some forms, the second retention arrangement is adapted to retain the retaining portion with respect to the patient interface in a direction away from the patient interface. In some forms, the patient interface engagement portion projects from a surface of the patient interface. In some forms, the engagement portion projects from the shell. In some forms, the positioning and stabilising structure comprises a strap configured to extend around the patient's head and hold the seal-forming structure in a therapeutically effective position on the patient's head.

In some forms, the retaining assembly further comprises a hinge element intermediate the connecting portion and the retaining portion and configured such that the connecting portion and the retaining portion are hinged with respect to one another.

In some forms, the hinge comprises a living hinge.

In a further aspect of the present technology is disclosed a retaining assembly for connecting a patient interface to a positioning and stabilising structure configured to generate a force to hold the patient interface in a therapeutically effective position on the patient's head. The patient interface comprises: a plenum chamber pressurisable to a therapeutic pressure of at least 6 cmH2O above ambient air pressure throughout a patient's respiratory cycle in use; one or more plenum chamber inlet ports sized and structured to receive the flow of air at the therapeutic pressure for breathing by a patient throughout the patient's respiratory cycle in use; a seal-forming structure defining at least a portion of the plenum chamber, the seal-forming structure constructed and arranged to form a seal with a region of the patient's face surrounding an entrance to the patient's airways, the seal-forming structure constructed and arranged to maintain said therapeutic pressure in the plenum chamber throughout the patient's respiratory cycle in use; and a shell defining at least a portion of the plenum chamber, the shell supporting the seal-forming structure. The retaining assembly comprises a connector and a patient interface engagement portion. The connector comprises: a connecting portion configured to engage the positioning and stabilising structure, a retaining portion configured to retain the connector with respect to the patient interface, and a hinge element intermediate the connecting portion and the retaining portion and configured such that the connecting portion and the retaining portion are hinged with respect to one another. The patient interface engagement portion is located on the patient interface. The patient interface engagement portion and the retaining portion being configured to removably engage one another through a magnetic retention arrangement.

In a further aspect of the present technology is disclosed a patient interface comprising a plenum chamber pressurisable to a therapeutic pressure of at least 6 cmH2O above ambient air pressure throughout a patient's respiratory cycle in use; one or more plenum chamber inlet ports sized and structured to receive the flow of air at the therapeutic pressure for breathing by a patient throughout the patient's respiratory cycle in use; a seal-forming structure defining at least a portion of the plenum chamber, the seal-forming structure constructed and arranged to form a seal with a region of the patient's face surrounding an entrance to the patient's airways, the seal-forming structure constructed and arranged to maintain said therapeutic pressure in the plenum chamber throughout the patient's respiratory cycle in use; a shell defining at least a portion of the plenum chamber, the shell supporting the seal-forming structure; and a patient interface engagement portion projecting from the shell and being configured to attach with a connector for a positioning and stabilising structure configured to generate a force to hold the patient interface in a therapeutically effective position on the patient's head, such that the connector and the patient interface engagement portion are engaged through a first mechanical retention arrangement and a second magnetic retention arrangement.

In some forms, the patient interface engagement portion comprises a projection projecting from the shell and including a magnet configured to magnetically engage the connector and a shoulder configured to mechanically engage the connector.

In some forms, the magnet is configured to resist movement of the connector away from the patient interface and the shoulder is configured to resist movement of the connector in a direction along a surface of the patient interface.

In a further aspect of the present technology is disclosed a patient interface system comprising a plenum chamber pressurisable to a therapeutic pressure of at least 6 cmH2O above ambient air pressure throughout a patient's respiratory cycle in use; one or more plenum chamber inlet ports sized and structured to receive the flow of air at the therapeutic pressure for breathing by a patient throughout the patient's respiratory cycle in use; a seal-forming structure defining at least a portion of the plenum chamber, the seal-forming structure constructed and arranged to form a seal with a region of the patient's face surrounding an entrance to the patient's airways, the seal-forming structure constructed and arranged to maintain said therapeutic pressure in the plenum chamber throughout the patient's respiratory cycle in use; a shell defining at least a portion of the plenum chamber, the shell supporting the seal-forming structure; a positioning and stabilising structure configured to generate a force to hold the seal-forming structure in a therapeutically effective position on the patient's head; and a retaining assembly comprising a connector and a patient interface engagement portion. The connector comprises: a connecting portion configured to engage the positioning and stabilising structure, a retaining portion configured to retain the connector with respect to the patient interface, and a hinge element intermediate the connecting portion and the retaining portion configured such that the connecting portion and the retaining portion are hinged with respect to one another. The patient interface engagement portion being located on the patient interface. The patient interface engagement portion and the retaining portion being configured to removably engage one another through a first retention arrangement and a second retention arrangement.

In some forms, (a) the first retention arrangement is a mechanical arrangement; (b) the first retention arrangement includes complementary protrusions adapted to retain the retaining portion with respect to the patient interface engagement portion in a direction along a surface of the patient interface; (c) the second retention arrangement is magnetic; (d) the second retention arrangement is adapted to retain the retaining portion with respect to the patient interface in a direction away from the patient interface; (e) the patient interface engagement portion projects from a surface of the patient interface; (f) the engagement portion projects from the shell; (g) the positioning and stabilising structure comprises a strap configured to extend around the patient's head and hold the seal-forming structure in a therapeutically effective position on the patient's head; and/or (h) the hinge comprises a living hinge.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### 4 BRIEF DESCRIPTION OF THE DRAWINGS

The present technology is illustrated by way of example, and not by way of limitation, in the figures of the accompanying drawings, in which like reference numerals refer to similar elements including:

### 4.1 TREATMENT SYSTEMS

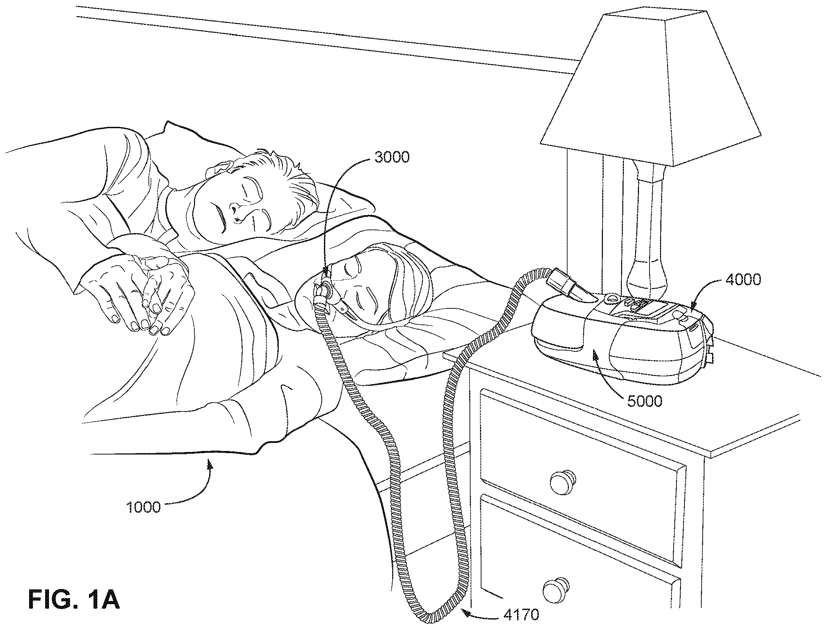

Fig. 1A shows a system including a patient 1000 wearing a patient interface 3000, in the form of nasal pillows, receiving a supply of air at positive pressure from an RPT device 4000. Air from the RPT device 4000 is humidified in a humidifier 5000, and passes along an air circuit 4170 to the patient 1000. A bed partner is also shown. The patient is sleeping in a supine sleeping position.

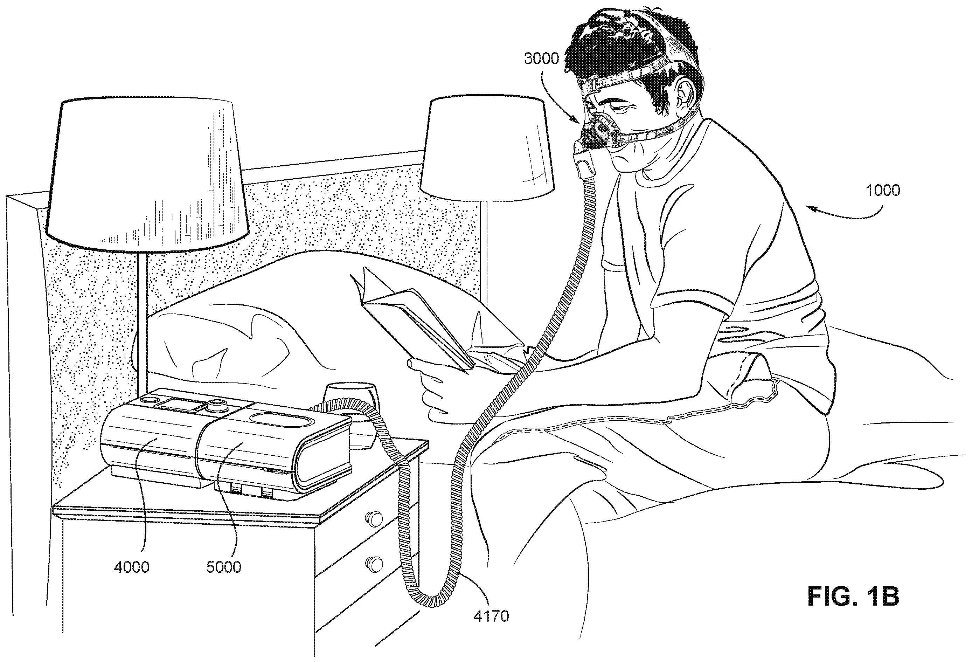

Fig. 1B shows a system including a patient 1000 wearing a patient interface 3000, in the form of a nasal patient interface, receiving a supply of air at positive pressure from an RPT device 4000. Air from the RPT device is humidified in a humidifier 5000, and passes along an air circuit 4170 to the patient 1000.

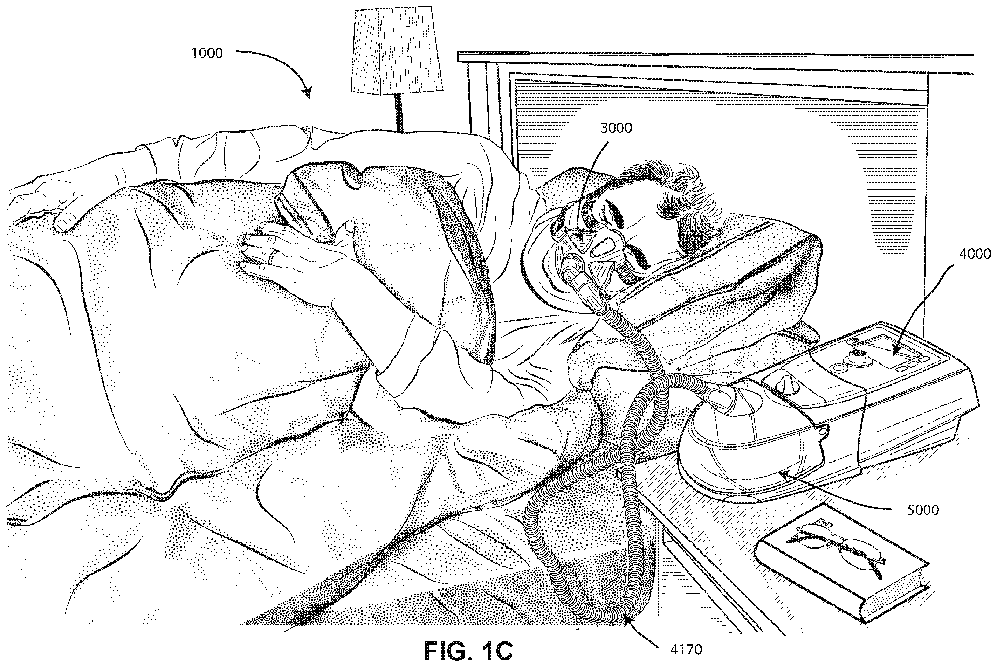

Fig. 1C shows a system including a patient 1000 wearing a patient interface 3000, in the form of a full-face patient interface, receiving a supply of air at positive pressure from an RPT device 4000. Air from the RPT device is humidified in a humidifier 5000, and passes along an air circuit 4170 to the patient 1000. The patient is sleeping in a side sleeping position.

### 4.2 RESPIRATORY SYSTEM AND FACIAL ANATOMY

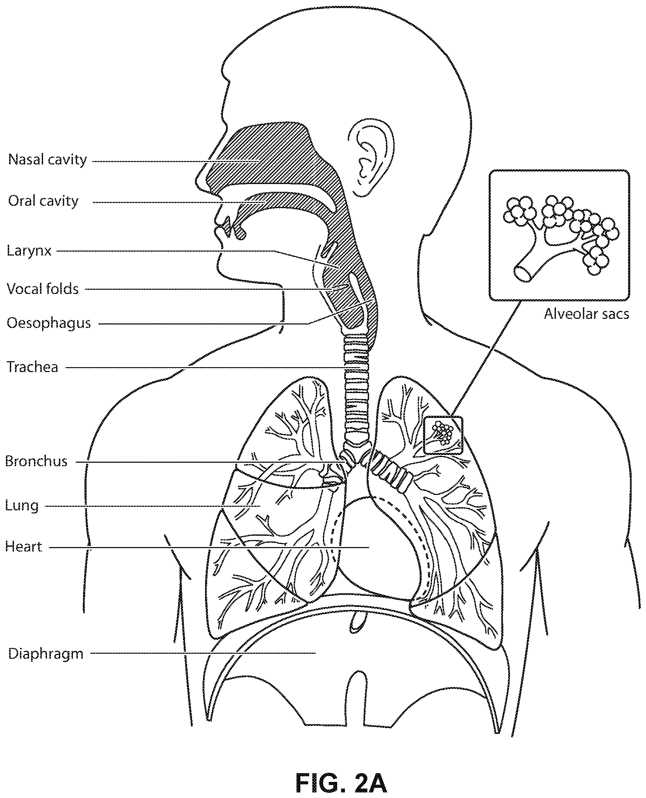

Fig. 2A shows an overview of a human respiratory system including the nasal and oral cavities, the larynx, vocal folds, oesophagus, trachea, bronchus, lung, alveolar sacs, heart and diaphragm.

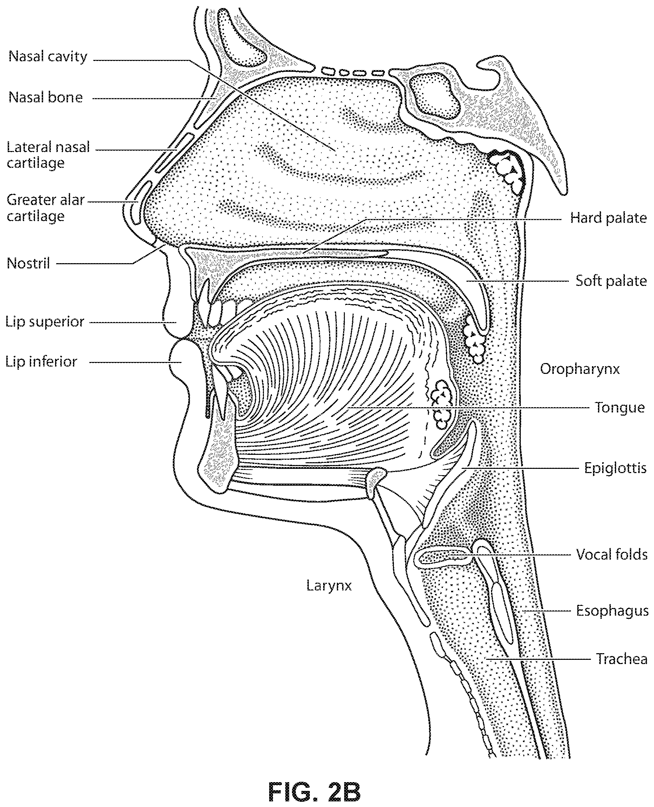

Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

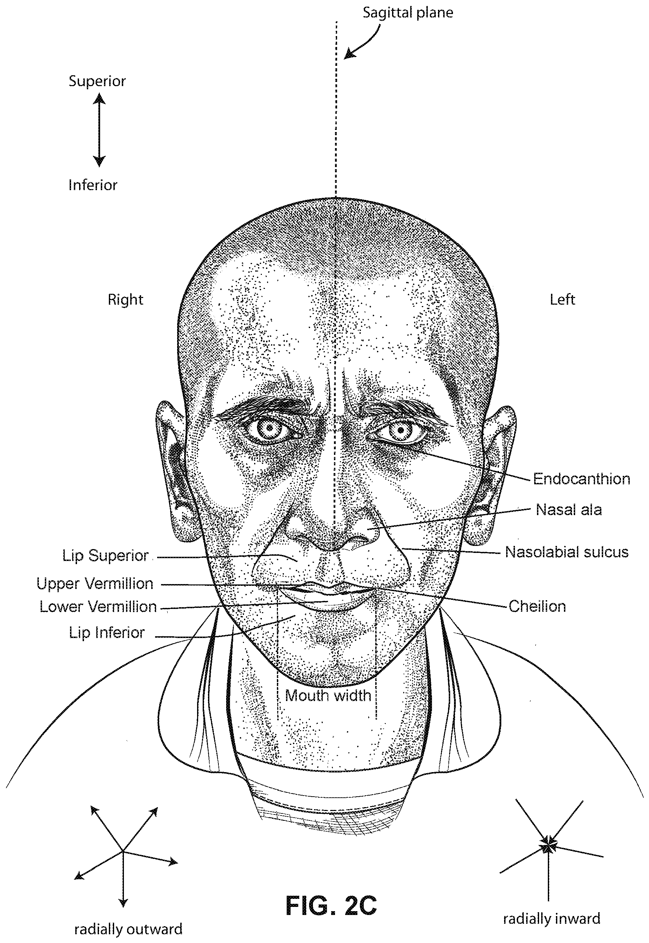

Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.

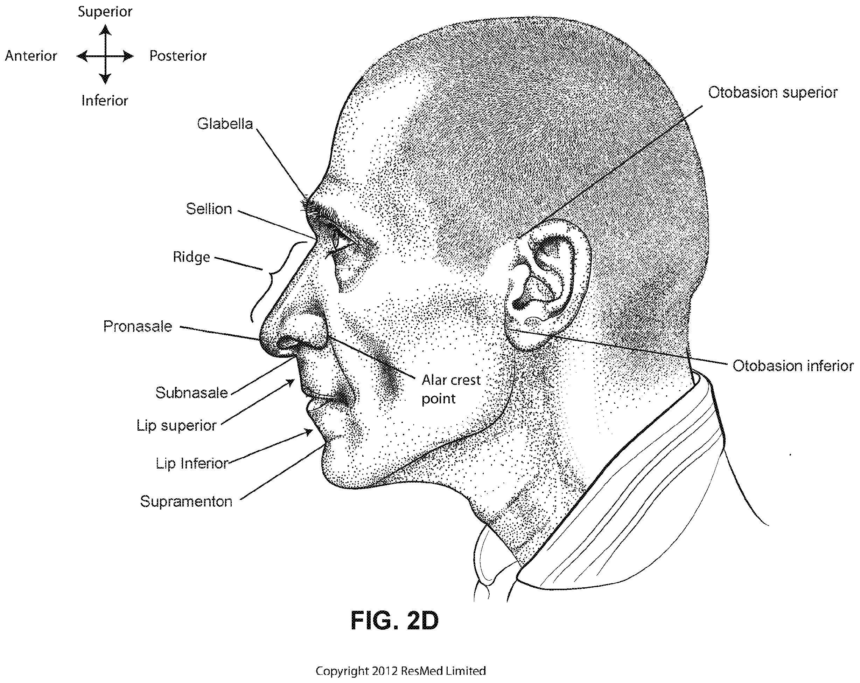

Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.

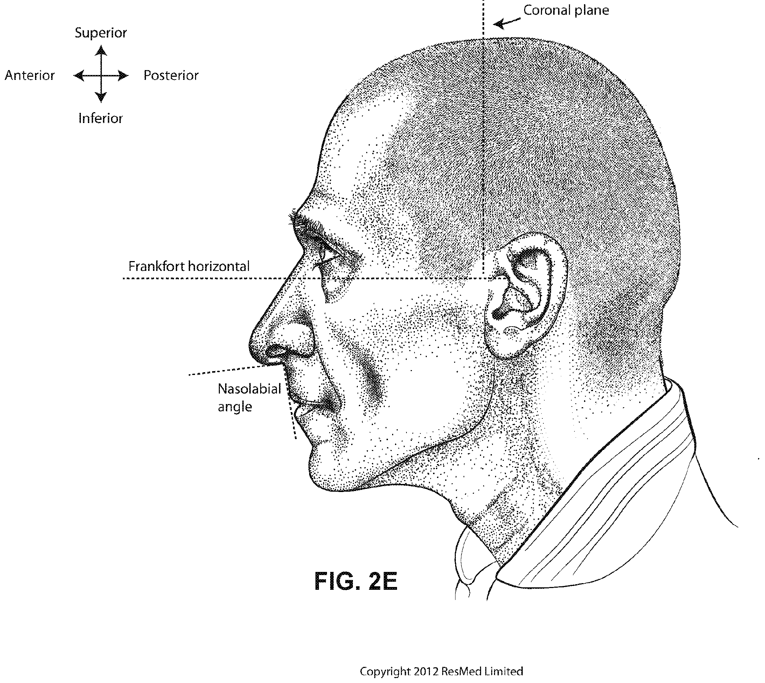

Fig. 2E is a further side view of a head. The approximate locations of the Frankfort horizontal and nasolabial angle are indicated. The coronal plane is also indicated.

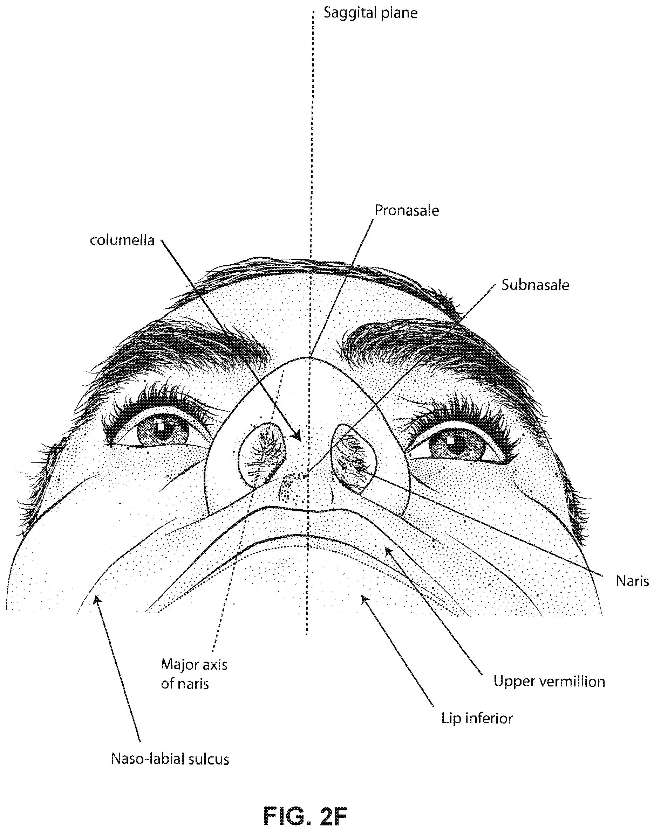

Fig. 2F shows a base view of a nose with several features identified including naso-labial sulcus, lip inferior, upper Vermilion, naris, subnasale, columella, pronasale, the major axis of a naris and the midsagittal plane.

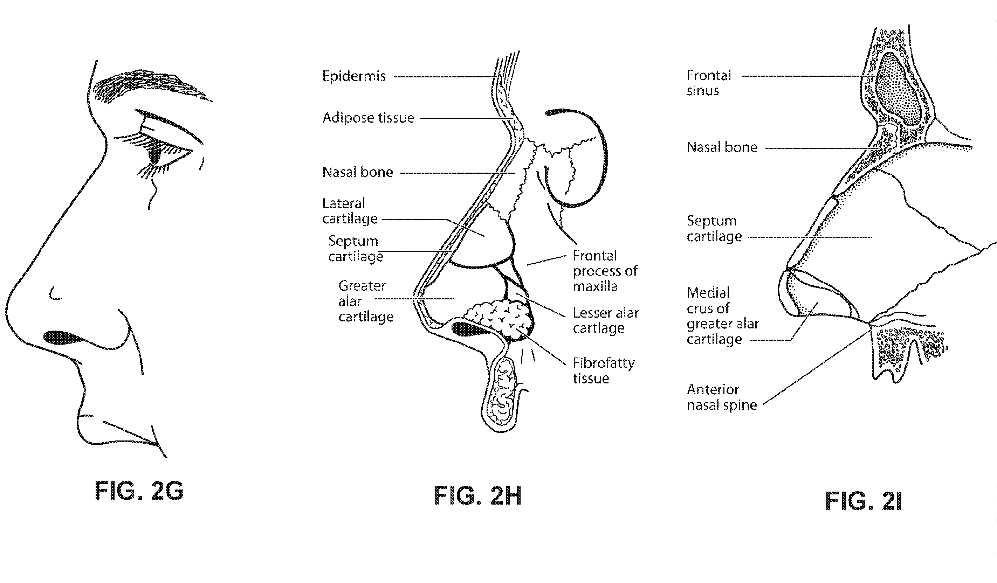

Fig. 2G shows a side view of the superficial features of a nose.
Fig. 2H shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.
Fig. 2I shows a medial dissection of a nose, approximately several millimeters from the midsagittal plane, amongst other things showing the septum cartilage and medial crus of greater alar cartilage.

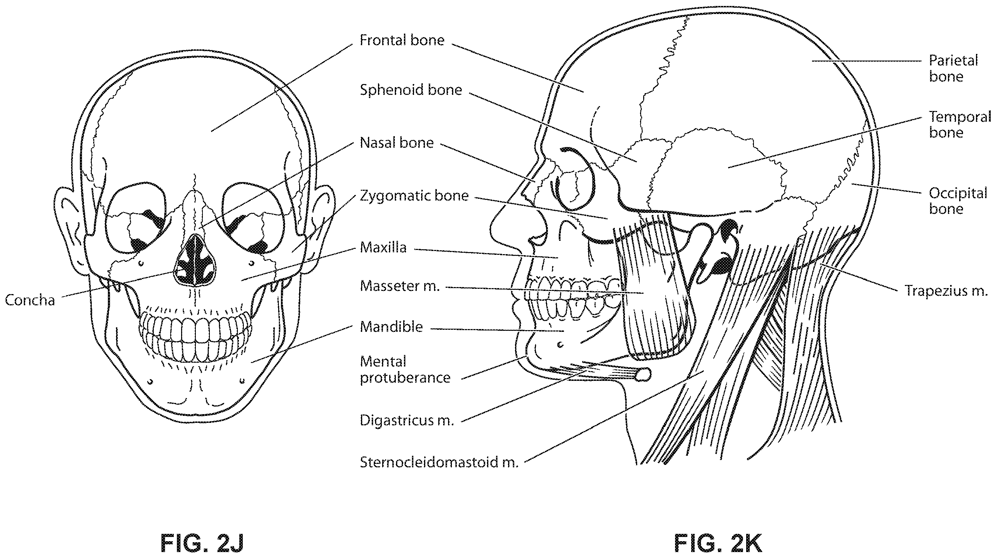

Fig. 2J shows a front view of the bones of a skull including the frontal, nasal and zygomatic bones. Nasal concha are indicated, as are the maxilla, and mandible.
Fig. 2K shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.

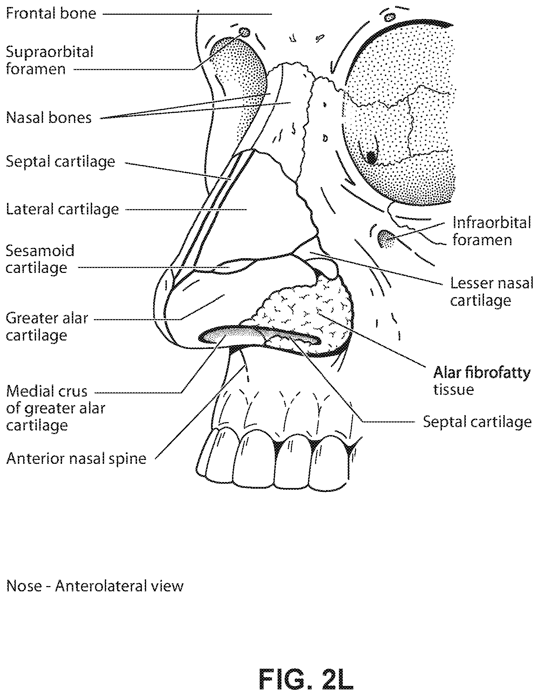

Fig. 2L shows an anterolateral view of a nose.

### 4.3 PATIENT INTERFACE

Fig. 3A shows a patient interface in the form of a nasal patient interface in accordance with one form of the present technology.
Fig. 3B shows a schematic of a cross-section through a structure at a point. An outward normal at the point is indicated. The curvature at the point has a positive sign, and a relatively large magnitude when compared to the magnitude of the curvature shown in Fig. 3C.
Fig. 3C shows a schematic of a cross-section through a structure at a point. An outward normal at the point is indicated. The curvature at the point has a positive sign, and a relatively small magnitude when compared to the magnitude of the curvature shown in Fig. 3B.
Fig. 3D shows a schematic of a cross-section through a structure at a point. An outward normal at the point is indicated. The curvature at the point has a value of zero.
Fig. 3E shows a schematic of a cross-section through a structure at a point. An outward normal at the point is indicated. The curvature at the point has a negative sign, and a relatively small magnitude when compared to the magnitude of the curvature shown in Fig. 3F.
Fig. 3F shows a schematic of a cross-section through a structure at a point. An outward normal at the point is indicated. The curvature at the point has a negative sign, and a relatively large magnitude when compared to the magnitude of the curvature shown in Fig. 3E.
Fig. 3G shows a cushion for a patient interface that includes two pillows. An exterior surface of the cushion is indicated. An edge of the surface is indicated. Dome and saddle regions are indicated.
Fig. 3H shows a cushion for a patient interface. An exterior surface of the cushion is indicated. An edge of the surface is indicated. A path on the surface between points A and B is indicated. A straight line distance between A and B is indicated. Two saddle regions and a dome region are indicated.
Fig. 3I shows the surface of a structure, with a one dimensional hole in the surface. The illustrated plane curve forms the boundary of a one dimensional hole.
Fig. 3J shows a cross-section through the structure of Fig.3I. The illustrated surface bounds a two dimensional hole in the structure of Fig. 3I.
Fig. 3K shows a perspective view of the structure of Fig. 3I, including the two dimensional hole and the one dimensional hole. Also shown is the surface that bounds a two dimensional hole in the structure of Fig. 3I.
Fig. 3L shows a patient interface having an inflatable bladder as a cushion.
Fig. 3M shows a cross-section through the patient interface of Fig. 3L, and shows the interior surface of the bladder. The interior surface bounds the two dimensional hole in the mask.
Fig. 3N shows a further cross-section through the patient interface of Fig. 3L. The interior surface is also indicated.
Fig. 3O illustrates a left-hand rule.
Fig. 3P illustrates a right-hand rule.
Fig. 3Q shows a left ear, including the left ear helix.
Fig. 3R shows a right ear, including the right ear helix.
Fig. 3S shows a right-hand helix.
Fig. 3T shows a view of a patient interface, including the sign of the torsion of the space curve defined by the edge of the sealing membrane in different regions of the patient interface.
Fig. 3U shows a view of a plenum chamber 3200 showing a sagittal plane and a mid-contact plane.
Fig. 3V shows a view of a posterior of the plenum chamber of Fig. 3U. The direction of the view is normal to the mid-contact plane. The sagittal plane in Fig. 3V bisects the plenum chamber into left-hand and right-hand sides.

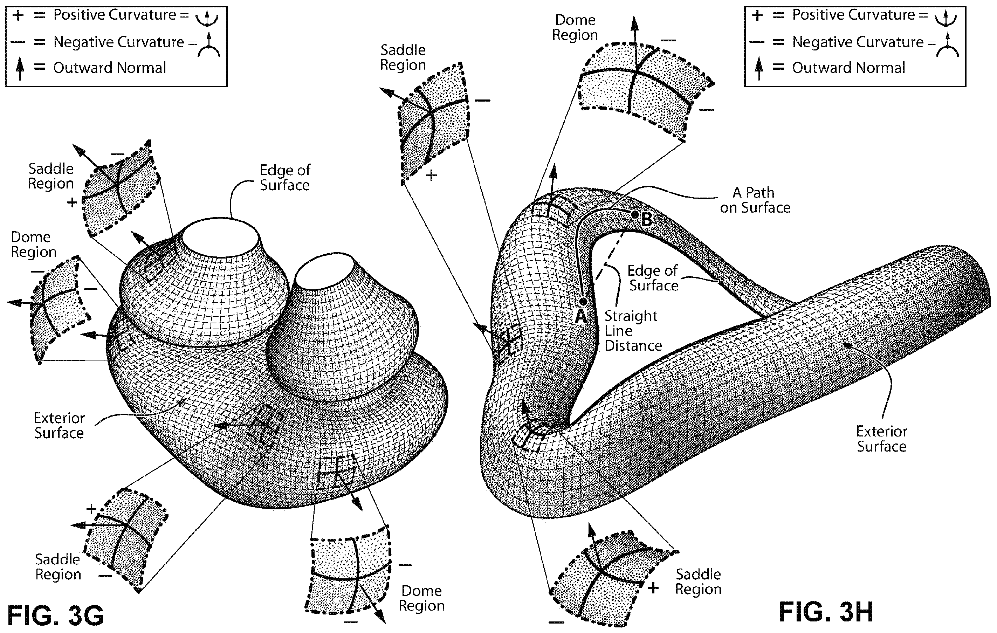

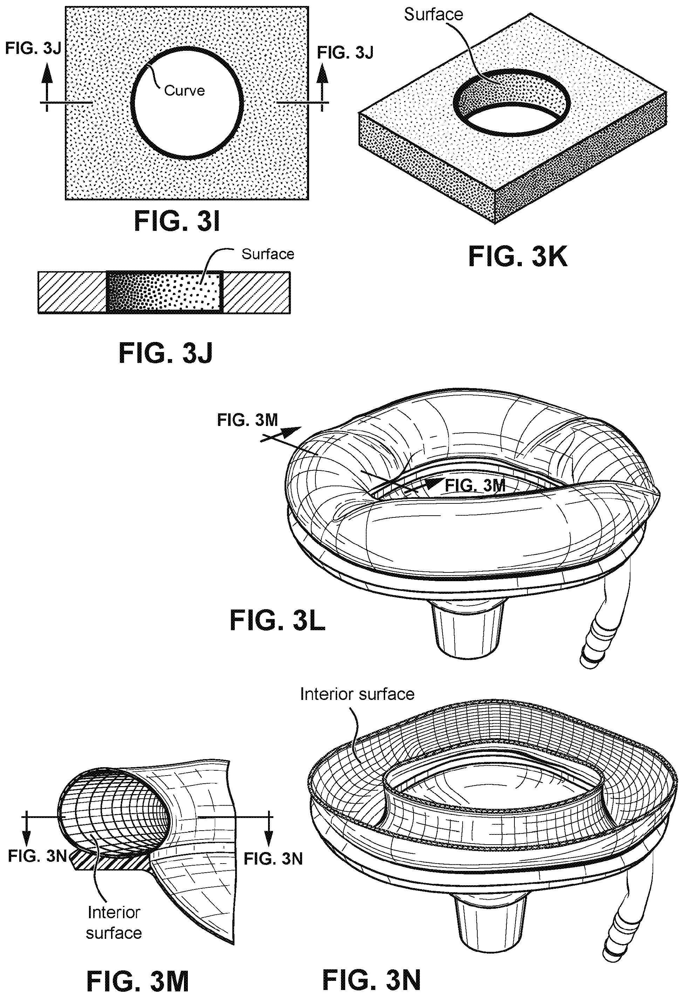

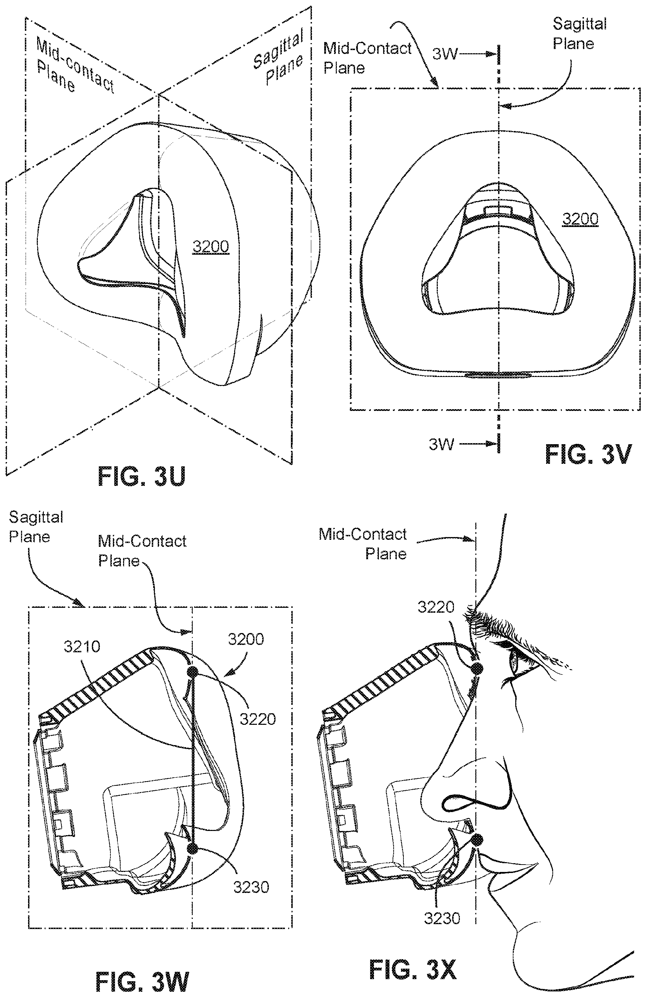

Fig. 3W shows a cross-section through the plenum chamber of Fig. 3V, the cross-section being taken at the sagittal plane shown in Fig. 3V. A 'mid-contact' plane is shown. The mid-contact plane is perpendicular to the sagittal plane. The orientation of the mid-contact plane corresponds to the orientation of a chord 3209 which lies on the sagittal plane and just touches the cushion of the plenum chamber at two points on the sagittal plane: a superior point 3220 and an inferior point 3229. Depending on the geometry of the cushion in this region, the mid-contact plane may be a tangent at both the superior and inferior points.
Fig. 3X shows the plenum chamber 3200 of Fig. 3U in position for use on a face. The sagittal plane of the plenum chamber 3200 generally coincides with the midsagittal plane of the face when the plenum chamber is in position for use. The mid-contact plane corresponds generally to the 'plane of the face' when the plenum chamber is in position for use. In Fig. 3X the plenum chamber 3200 is that of a nasal patient interface, and the superior point 3220 sits approximately on the sellion, while the inferior point 3229 sits on the lip superior.

### 4.4 RPT DEVICE

Fig. 4 shows an RPT device in accordance with one form of the present technology.
Fig. 5 is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

### 4.5 BREATHING WAVEFORMS

Fig. 6 shows a model typical breath waveform of a person while sleeping.

### 4.6 PATIENT INTERFACE EXAMPLES OF THE PRESENT TECHNOLOGY

Fig. 7-1 depicts a front view of a patient interface system in a nares only mode on a model patient's head according to an embodiment of the present technology.
Fig. 7-2 depicts a front view of a patient interface system on a model patient's head in a nares and mouth mode according to an embodiment of the present technology.
Fig. 7-3 depicts a front view of a patient interface system on a model patient's head in a nares and mouth mode according to an embodiment of the present technology.
Fig. 7-4 depicts a front view of a patient interface system on a model patient's head in a mouth only mode according to an embodiment of the present technology.
Fig. 7-5 depicts a front exploded view of a patient interface system illustrating how the nasal portion may connect to the mouth portion according to an embodiment of the present technology.
Fig. 8 depicts a front view of the patient interface system of Figs. 7-1 to 7-5 without the elbow connector according to an embodiment of the present technology.
Fig. 9 depicts a rear view of the patient interface system of Figs. 7-1 to 7-5 according to an embodiment of the present technology.
Fig. 10 depicts a rear isometric view of the patient interface system of Figs. 7-1 to 7-5 according to an embodiment of the present technology.
Fig. 11-1 depicts a front isometric view of a patient interface system on a model patient's head according to another embodiment of the present technology.
Fig. 11-2 depicts a front isometric view of the patient interface system of Fig 11-1 on a model patient's head in a nares only mode.
Fig. 12 depicts a side view of the patient interface system of Fig. 11 in use according to an embodiment of the present technology.
Fig. 13 depicts a rear isometric view of a headgear utilized with the patient interface system of Fig. 11-1 according to an embodiment of the present technology.
Fig. 14 depicts a front isometric view of the patient interface system of Fig. 11-1 without the elbow connector according to an embodiment of the present technology.
Fig. 15 depicts a front view of the patient interface system of Fig. 11-1 without the elbow connector according to an embodiment of the present technology.
Fig. 16 depicts a top view of the patient interface system of Fig. 11-1 without the nasal portion according to an embodiment of the present technology.
Fig. 17 depicts a rear isometric view of the patient interface system of Fig. 11-1 according to an embodiment of the present technology.
Fig. 18 depicts a rear view of the patient interface system of Fig. 11-1 according to an embodiment of the present technology.
Fig. 19 depicts a front view of a portion of the patient interface system of Fig. 11-1 without the nasal portion and without the elbow connector and including a headgear according to an embodiment of the present technology.
Fig. 20 depicts a front view of a patient interface system on a model patient's head according to another embodiment of the present technology.
Fig. 21 depicts a side view of the patient interface system of Fig. 20 on a model patient's head according to an embodiment of the present technology.
Fig. 22 depicts a rear view of a patient interface system with a decoupling portion and connecting portion removed according to another embodiment of the present technology.
Fig. 23 depicts a front isometric view of the patient interface system of Fig. 22 according to an embodiment of the present technology.
Fig. 24 depicts a front isometric view of the patient interface system of Fig. 22 according to an embodiment of the present technology.
Fig. 25 depicts a rear isometric view of a nasal portion that may be used with patient interface systems according to an embodiment of the present technology.
Fig. 26 depicts a front isometric view of the nasal portion of Fig. 25 according to an embodiment of the present technology.
Fig. 27 depicts a front isometric view of a patient interface system on a model patient's head in a nares only mode according to another embodiment of the present technology.
Fig. 28 depicts a front isometric view the patient interface system of Fig. 27 on a model patient's head in a nares and mouth configuration according to an embodiment of the present technology.
Fig. 29 depicts a front isometric view of a patient interface system on a model patient's head according to another embodiment of the present technology.
Fig. 30 depicts a side view of a patient interface system on a model patient's head according to another embodiment of the present technology.
Fig. 31 depicts a side view of a patient interface system on a model patient's head according to another embodiment of the present technology.
Fig. 32 depicts a schematic cross-sectional view of a modular patient interface system on a model patient's head according to an embodiment of the present technology.
Fig. 33 depicts a schematic cross-sectional view of an integrated patient interface system on a model patient's head according to an embodiment of the present technology.
Fig. 34 depicts a schematic cross-sectional view of an integrated patient interface system on a model patient's head according to an embodiment of the present technology.
Fig. 35-1 depicts a schematic cross-sectional view of a modular patient interface system on a model patient's head according to an embodiment of the present technology.
Fig. 35-2 depicts a schematic top view of a mouth sealing portion of the patient interface system of Fig. 35-1 according to an embodiment of the present technology.
Fig. 35-3 depicts a schematic cross-sectional view of a modular patient interface system on a model patient's head according to an embodiment of the present technology.
Fig. 36-1 depicts a schematic cross-sectional view of a modular patient interface on a model patient's head according to an embodiment of the present technology.
Fig. 36-2 depicts a schematic cross-sectional view of a modular patient interface system on a model patient's head according to an embodiment of the present technology.
Fig. 37 depicts a cross-sectional view of an integrated patient interface system on a model patient's head according to an embodiment of the present technology.
Figs. 38(a)-(k) are schematic views the flow of pressurized gas through the patient interface system various configurations according to embodiments of the present technology.
Fig. 39 depicts a schematic view of a nares and mouth kit according to embodiments of the present technology.
Fig. 40 depicts a schematic view of a modular patient interface system on a model patient's head according to an embodiment of the present technology.
Fig. 41 depicts a front view of a nares sealing portion according to an embodiment of the present technology.
Fig. 42 depicts a front perspective view of the nares sealing portion of Fig. 41 according to an embodiment of the present technology.
Fig. 43 depicts a prior art nares only CPAP device on a model patient's head.
Fig. 44 depicts a retrofit kit used to convert the nares only CPAP device of Fig. 43 into a nares and mouth CPAP device according to an embodiment of the present technology.
Fig. 45 depicts a retrofit kit used to convert the nares only CPAP device of Fig. 43 into a nares and mouth CPAP device according to an embodiment of the present technology.
Fig. 46 depicts a prior art nares only CPAP device.
Fig. 47 depicts a retrofit kit to convert the nares only CPAP device of Fig. 46 into a nares and mouth CPAP device illustrated on a model patient's head according to an embodiment of the present technology.
Fig. 48 depicts a schematic cross-sectional view of a modular patient interface system on a model patient's head according to an embodiment of the present technology.
Fig. 49 depicts a schematic cross-sectional view of a modular patient interface system on a model patient's head according to an embodiment of the present technology.
Fig. 50 depicts a schematic cross-sectional view of a modular patient interface system on a model patient's head according to an embodiment of the present technology.
Fig. 50A is a cross-sectional view of a nasal portion according to an embodiment of the present technology.
Fig. 50B is a cross-sectional view of a nasal portion according to an embodiment of the present technology.
Fig. 51 depicts a schematic cross-sectional view of a modular patient interface system on a model patient's head according to an embodiment of the present technology.
Fig. 52 depicts a schematic cross-sectional view of a modular patient interface system on a model patient's head according to an embodiment of the present technology.
Fig. 53 depicts a schematic cross-sectional view of a modular patient interface system on a model patient's head according to an embodiment of the present technology.
Fig. 54 depicts a schematic partial cross-sectional view of a modular patient interface system according to an embodiment of the present technology.
Fig. 55 is an exploded view of a modular patient interface system on a model patient's head according to an embodiment of the present technology.
Fig. 56 depicts a schematic cross-sectional view of an integrated (or modular) patient interface system on a model patient's head according to an embodiment of the present technology.
Fig. 57 depicts a perspective view of the integrated (or modular) patient interface system of Fig. 56 on a model patient's head.
Fig. 58 depicts a side view of the integrated (or modular) patient interface system of Fig. 57.
Fig. 59 depicts schematic cross-sectional view of a modular patient interface system on a model patient's head according to an embodiment of the present technology.
Fig. 60 depicts a schematic cross-sectional view of a modular patient interface system on a model patient's head according to an embodiment of the present technology.
Fig. 61 depicts a rear view of a nasal portion of a patient interface system according to an embodiment of the present technology.
Fig. 61A is a cross-sectional view through line 61A-61A of Fig. 61.
Fig. 62 depicts a rear view of a nasal portion of a patient interface system according to an embodiment of the present technology.
Fig. 62A is a cross-sectional view through line 62A-62A of Fig. 62.
Fig. 63 depicts a schematic cross-sectional view of a modular patient interface system on a model patient's head according to an embodiment of the present technology.
Fig. 64 depicts a side view of an integrated or modular patient interface system on a model patient's head according to an embodiment of the present technology.
Fig. 65 depicts a perspective view of an integrated or modular patient interface system on a model patient's head according to an embodiment of the present technology.
Fig. 66 depicts a rear view of a modular patient interface system according to an embodiment of the present technology.
Fig. 67 depicts a schematic cross-sectional view of a modular patient interface system on a model patient's head according to an embodiment of the present technology.
Fig. 68 depicts a schematic cross-sectional view of a modular patient interface system on a model patient's head according to an embodiment of the present technology.
Fig. 69 depicts an exploded view of a modular patient interface system according to an embodiment of the present technology.
Fig. 70 depicts a schematic cross-sectional view of a modular patient interface system on a model patient's head according to an embodiment of the present technology.
Fig. 71 depicts a schematic cross-sectional view of a modular patient interface system on a model patient's head according to an embodiment of the present technology.
Fig. 72 depicts an exploded view of a modular patient interface system according to an embodiment of the present technology.
Fig. 73 depicts an exploded view of a modular patient interface system according to an embodiment of the present technology.
Fig. 74 depicts a perspective view of a modular patient interface system according to an embodiment of the present technology.
Fig. 75 depicts a front view of the modular patient interface system of Fig. 74.
Fig. 76 depicts a top view of the modular patient interface system of Fig. 74.
Fig. 77 depicts a bottom view of the modular patient interface system of Fig. 74.
Fig. 78 depicts a side view of the modular patient interface system of Fig. 74.
Fig. 79 depicts a cross sectional view along lines 75-75 from Fig. 75.
Fig. 80 depicts a perspective view of a nasal portion of the patient interface system of Fig. 74.
Fig. 81 depicts a perspective view of a mouth portion of the patient interface system of Fig. 74.
Fig. 82 depicts a perspective view of the cushion portion of the patient interface system of Fig. 74.
Fig. 83 depicts a front view of the cushion portion of the patient interface system of Fig. 74.
Fig. 84 depicts a top view of the cushion portion of the patient interface system of Fig. 74.
Fig. 85 depicts a bottom view of the cushion portion of the patient interface system of Fig. 74.
Fig. 86 depicts a cross sectional view along lines 80-80 from Fig. 83.
Fig. 87 depicts a cross sectional view along lines 81-81 from Fig. 83.
Fig. 88 depicts a cross sectional view along lines 82-82 from Fig. 83.
Fig. 89 depicts a cross sectional view along lines 83-83 from Fig. 83.
Fig. 90 depicts a cross sectional view along lines 84-84 from Fig. 83.
Fig. 91 depicts a cross sectional view along lines 85-85 from Fig. 77.
Fig. 92 depicts a rear view of the cushion portion of the patient interface system of Fig. 74.
Fig. 93 depicts a right side view of the cushion portion of the patient interface system of Fig. 74.
Fig. 94 depicts a left side view of the cushion portion of the patient interface system of Fig. 74.
Fig. 95 depicts a perspective view of the cushion portion of the patient interface system of Fig. 74.
Figs. 96, 97A, and 97 B illustrate a headgear system that is convertible between two point and four point support in accordance with a sample of the current technology.
Figs. 98A to 98E illustrate a mouth cushion according to a variant of the current technology.
Figs. 99A to 99I illustrate a mouth cushion according to a variant of the current technology.
Figs. 100-108 illustrate a cushion-to-fascia connection according to an example of the present technology.
Fig. 109 illustrates a lip seal according to an example of the present technology.
Fig. 110 is an exploded assembly view of a patient interface system according to an embodiment of the present technology.
Fig. 111 is a rear view of the patient interface system of Fig. 110 in the assembled state.
Fig. 112 is a top view of the patient interface system of Fig. 110 in the assembled state.
Fig. 113 is a bottom view of the patient interface system of Fig. 110 in the assembled state.
Fig. 114 is a left side view of the patient interface system of Fig. 110 in the assembled state.
Fig. 115 is a cross section view along the line 109-109 in Fig. 111.
Fig. 116 is a cross section view along the line 110-110 in Fig. 111.
Fig. 117 is a detailed view of a portion of Fig. 116.
Fig. 118 is a rear isometric view of the mouth cushion of the patient interface system of Fig. 110.
Fig. 119 is a front isometric view of the mouth cushion of the patient interface system of Fig. 110.
Fig. 120 is front view of the mouth cushion of the patient interface system of Fig. 110.
Fig. 121 is a top view of the mouth cushion of the patient interface system of Fig. 110.
Fig. 122 is a bottom view of the mouth cushion of the patient interface of Fig. 110.
Fig. 123 is a right side view of the mouth cushion of the patient interface system of Fig. 110.
Fig. 124 is a front view of the mouth cushion of the patient interface system of Fig. 110.
Fig. 125 is a cross section view of the mouth cushion along the line 119-119 in Fig. 124.
Fig. 126 is a cross section view of the mouth cushion along the line 120-120 in Fig. 124.
Fig. 127 is a cross section view of the mouth cushion along the line 121-121 in Fig. 124.
Fig. 128 is a cross section view of the mouth cushion along the line 122-122 in Fig. 124.
Fig. 129 is a cross section view of the mouth cushion along the line 123-123 in Fig. 124.
Fig. 130 is a cross section view of the mouth cushion along the line 124-120 in Fig. 124.
Fig. 131 is a rear isometric view of the cushion clip of the patient interface system of Fig. 110.
Fig. 132 is a front isometric view of the cushion clip of Fig. 131.
Fig. 133 is a top view of the cushion clip of Fig. 131.
Fig. 134 is a bottom view of the cushion clip of Fig. 131.
Fig. 135 is a left side view of the cushion clip of Fig. 131.
Fig. 136 is a rear view of the cushion clip of Fig. 131.
Fig. 137 is a cross section view along lines 131-131 in Fig. 136.
Fig. 138 is a front isometric view of a fascia of the patient interface system of Fig. 110.
Fig. 139 is a front view of the fascia of Fig. 138.
Fig. 140 is a rear view of the fascia of Fig. 138.
Fig. 141 is a top view of the fascia of Fig. 138.
Fig. 142 is a bottom view of the fascia of Fig. 138.
Fig. 143 is a right side view of the fascia of Fig. 138.
Fig. 144 is a cross section view along the line 138-138 in Fig. 139.
Fig. 145 is a front isometric view of a cushion clip according to an embodiment of the present technology.
Fig. 146 is a rear isometric view of the cushion clip of Fig. 145.
Fig. 147 is a front view of the cushion clip of Fig. 145.
Fig. 148 is a top view of the cushion clip of Fig. 145.
Fig. 149 is a bottom view of the cushion clip of Fig. 145.
Fig. 150 is a left side view of the cushion clip of Fig. 145.
Fig. 151 is a cross section view along lines 145-145 in Fig. 147.
Fig. 152 depicts a perspective view of a modular patient interface system according to an exemplary embodiment of the present invention.
Fig 153 is an exploded side view of the modular patient interface system of Fig. 152.
Fig. 154 is a perspective view of the modular patient interface system of Fig. 152 in a nares only mode.
Fig 155a is a perspective view of the mouth portion of the modular patient interface system of Fig. 152;
Fig. 155B is a left side view of the mouth portion of Fig. 155A.
Fig. 155C is a rear view of the mouth portion of Fig. 155A.
Fig. 155D is a rear perspective view of the mouth portion of Fig. 155A connected to a nasal portion.
Fig. 155E is a front perspective view of the mouth portion of Fig. 155A connected to a nasal portion.
Fig. 155F is a top view of a nasal portion including a pillow cushion.
Fig. 155G is a top view of a nasal portion including a prongs.
Fig. 156A illustrates an embodiment of a coupling arrangement for the components of the modular patient interface system of Fig. 152.
Fig. 156B is a schematic view of an embodiment of a coupling arrangement for a vent or cover of the modular patient interface system of Fig. 152.
Fig. 156C is a schematic view of an embodiment of a coupling arrangement for a mouth portion of the modular patient interface system of Fig. 152.
Fig. 157 is a schematic view of an embodiment of a coupling arrangement for the components of the modular patient interface system of Fig. 152.
Fig. 158A is a schematic view of an embodiment of a coupling arrangement for the components of the modular patient interface system of Fig. 152.
Fig. 158B is a schematic view of an embodiment of a coupling arrangement for the components of the modular patient interface system of Fig. 152.Fig. 159 is a schematic view of an embodiment of a coupling arrangement for the components of the modular patient interface system of Fig. 152.
Fig. 160 is a schematic view of an embodiment of a coupling arrangement for the components of the modular patient interface system of Fig. 152.
Fig. 161 is a schematic view of an embodiment of a coupling arrangement for the components of the modular patient interface system of Fig. 152.
Fig. 162 is a schematic view of an embodiment of a coupling arrangement for the components of the modular patient interface system of Fig. 152.
Fig. 163 is a schematic view of an embodiment of a coupling arrangement for the components of the modular patient interface system of Fig. 152.
Fig. 164 is a schematic view of an embodiment of a coupling arrangement for the components of the modular patient interface system of Fig. 152.
Fig. 165 is a schematic view of an embodiment of a coupling arrangement for the components of the modular patient interface system of Fig. 152.
Fig. 166 is a schematic view of an embodiment of a variation of the modular patient interface system of Fig. 152 incorporating an intermediate chamber.
Fig. 167 is a schematic view of an embodiment of a variation the connector of the modular patient interface system of Fig. 152.
Fig 168A is a section left side view of a nasal patient interface with AAV according to an embodiment of the present technology.
Fig 168B is a top perspective view of a nasal patient interface with AAV of Fig. 168A.
Fig. 169 is rear side view of a AAV connector module for the patient interface of Fig. 168A.
Fig. 170 is a section side view of the AAV of the patient interface of Fig. 168A.
Fig. 171A is a front perspective view of a connector of the patient interface of Fig. 152.
Fig. 171B is a top perspective view of the connector of the patient interface of Fig. 171A.
Figs. 172A-C are close-up views of a section of the nasal and mouth portion headgears when joined by various embodiments of a connector comprising one or more portions of Velcro, the mouth portion headgear being V-shaped and having two portions of Velcro, U-shaped and having 2 portions of Velcro, or one piece that is attachable to two Velcro portions on the nasal portion headgear, respectively.
Figs. 173A-C are close-up views of a section of the nasal and mouth portion headgears configured for being joined by various embodiments of a connector comprising a plurality of snap connectors, the nasal portion headgear comprising two male snap connectors, or alternating spaced male/female snap connectors, respectively.
Fig. 174 is a close-up view of a section of the nasal and mouth portion headgears configured for being joined by an embodiment of a connector comprising a plurality of tabs and latches.
Figs. 175A and 175B are close-up views of a section of the nasal and mouth portion headgears configured for being joined by various embodiments of a connector, where the nasal and mouth portion headgears also comprise an embodiment of an alignment guide comprising one or more indicia, shapes or patterns.
Figs. 176A, 176B and 175C are close-up views of a section of the nasal and mouth portion headgears configured for being joined by various embodiments of a connector, where the nasal and mouth portion headgears also comprise an embodiment of an alignment guide, where the nasal portion comprises a single recess groove, a plurality of recesses, or a recessed loop, respectively, to engage a correspondingly shaped projection of the mouth portion headgear.
Fig. 177 is a close-up view of a section of the nasal and mouth portion headgears configured for being joined by an embodiment of a connector having hook tabs and corresponding latches, and where the nasal portion headgear also comprises a recessed portion around the hook tabs and corresponding latches of the connector.
Figs. 178A-C are close up views of a section of the nasal and mouth portion headgears configured for being joined by an embodiment of a connector that is configured on a flap of the mouth portion headgear, the flap being passed through a slit in the nasal portion headgear, wherein the flap is narrower than the slit, the flap is sized to correspond to the slit, or a plurality of flaps being passed through a single slit, respectively.
Fig. 179 illustrates a connector for coupling a headgear to a patient interface according to an example of the present technology.
Fig. 180 illustrates a chassis including chassis connectors according to an example of the present technology.
Fig. 181 illustrates a connector of Fig. 179 connected to the chassis illustrated in Fig. 180 according to an example of the present technology.
Fig. 182 is a front view of a plenum chamber 3200 in accordance with one form of the present technology.
Fig. 183 is a rear view of the plenum chamber 3200 of Fig. 182.
Fig. 184 is a side view of the plenum chamber 3200 of Fig. 182.
Fig. 185 is a top view of the plenum chamber 3200 of Fig. 182.
Fig. 186 is a bottom view of the plenum chamber 3200 of Fig. 182.
Fig. 187 is a perspective view of the plenum chamber 3200 of Fig. 182.
Fig. 188 is another perspective view of the plenum chamber 3200 of Fig. 182.
Fig. 189 is another perspective view of the plenum chamber 3200 of Fig. 182.

### 5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

Where anatomical directional terms are used in describing aspects and examples of the present technology, such as "anterior", "posterior", "superior", "inferior", "lateral", "medial" and the like, the directions are to be applied in the context of the present technology during use by a patient. For example, an anterior side of a patient interface refers to the side of the patient interface which is anterior with respect to the patient when the patient has donned the patient interface in the intended manner.

Where surfaces or portions are described as facing a direction, e.g. "superior-facing", "anterior-facing" and the like, unless the context clearly requires otherwise the surfaces or portions are to be understood as at least partially facing in the particular direction. A portion may be "superior-facing" if the portion generally faces a superior direction, even if it partially also faces another direction.

### 5.1 THERAPY

In one form, the present technology comprises a method for treating a respiratory disorder comprising the step of applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 5.2 TREATMENT SYSTEMS

In one form, the present technology comprises an apparatus or device for treating a respiratory disorder. The apparatus or device may comprise an RPT device 4000 for supplying pressurised air to the patient 1000 via an air circuit 4170 to a patient interface 3000.

### 5.3 PATIENT INTERFACE

The patient interface system of the present technology delivers pressurized breathable gas to the patient and includes a nasal portion, a mouth portion, a positioning and stabilizing structure, and an air delivery system. A sealing portion may be included with the nasal portion and/or the mouth portion to form a seal or substantially seal with the nose and/or mouth of the patient. One or more vents may also be included to vent gas exhaled by the patient on the nasal portion and/or the mouth portion. However, the patient interface may be non-vented and be used in a hospital/ventilation scenario. The patient interface system may also be provided with an anti-asphyxia valve (AAV).

The sealing portion is arranged to form a seal or substantially seal with the nose and/or mouth of the patient to deliver pressurized gas to the patient. Preferably, the sealing portion has a nares sealing portion and a mouth sealing portion. The nares sealing portion and the mouth sealing portion may be integrally formed, or they may be in the form of separate elements. The nares sealing portion and mouth sealing portion may be connected or otherwise positioned so as to independently or discretely seal with the nose and mouth of the patient, respectively.

A positioning and stabilizing structure may be connected to the sealing portion and is adapted to engage the sealing portion with the patient. The positioning and stabilizing structure may have an upper portion and a lower portion that is constructed and arranged to avoid the patient's eyes and ears in use. The upper and lower portions may be integrated with one another, in separate pieces that do not connect with one another, or in separate pieces that are coupled to one another. The positioning and stabilizing structure may have a rear portion adapted to engage with the back of a patient's head in use. The rear portion may interconnect the upper and lower portions.

The air delivery system may connect the sealing portion to a flow generator. The air delivery system may include an elbow and/or a tube.

The patient interface system provides patients with options for therapy (nares only, mouth only or nares and mouth therapy) without the system becoming too expensive and also improving intuitive use of the patient interface system. This allows the patient to use the patient interface system without too much or any instruction.

The patient interface system may be a modular patient interface system having the nasal portion and the mouth portion, where the nasal portion is adapted to be utilized without the mouth portion to provide respiratory therapy to a patient's nares, or the nasal portion may be adapted to be utilized with the mouth portion to provide respiratory therapy to the patient's nares and mouth, or to provide respiratory therapy to the patient's nares and to utilize the mouth portion as a mouth seal.

The patient interface system may be a modular patient interface system having the nasal portion and the mouth portion, where the mouth portion is adapted to be utilized without the nasal portion to provide respiratory therapy to a patient's mouth, or the mouth portion is adapted to be utilized with the nasal portion to provide respiratory therapy to the patient's nares and mouth, or to provide respiratory therapy to the patient's mouth and to utilize the nasal portion as a nares seal.

The patient interface system may be an integrated patient interface system having a nasal portion and a mouth portion, where the nasal portion and the mouth portion are integrated and formed in a single piece. Pressurized gas may be provided to the nares and mouth portions for delivery to the nose and mouth, or pressurized gas may be delivered only to the nares or mouth portion, with the other portion being blocked off from the pressurized gas.

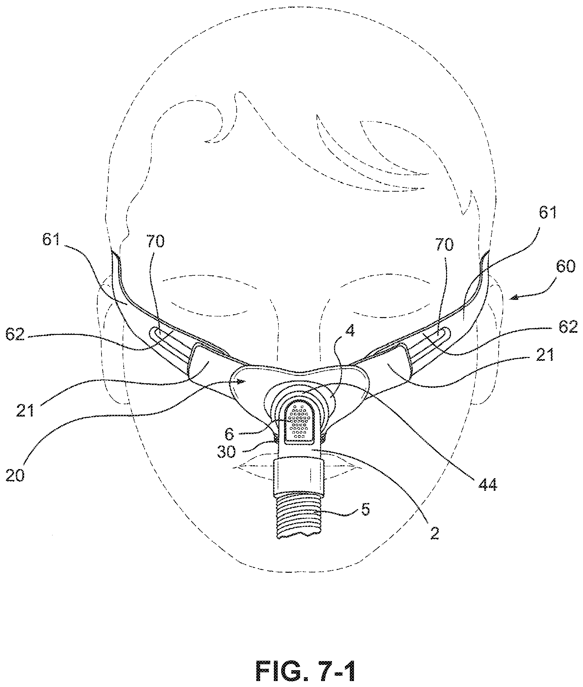

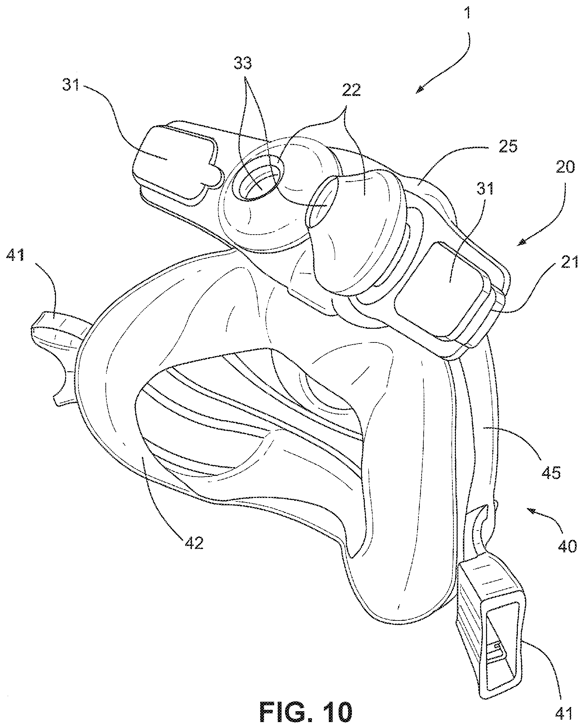

As illustrated in Figs 7-1 to 10, for example, a patient interface system 1 may include a nasal portion 20, a mouth portion 40 and positioning and stabilizing structure, which may include headgear 60. An air delivery system may deliver air to the patient interface system 1, such as through flexible tube 5. The patient interface system of Figs 7-1 to 10 is a patient interface system in which the nasal portion 20 and the mouth portion 40 are both adapted to be used without each other (in a nares only mode or in a mouth only mode), or the nasal portion 20 and the mouth portion 40 may be utilized together (in a nares and mouth mode). When utilized in the nares and mouth mode, one of the nasal portion 20 and the mouth portion 40 may be utilized to deliver pressurized gas, while the other of the nasal portion 20 and the mouth portion 40 may be utilized as a nares or mouth seal. In another form, when utilized in the nares and mouth mode, both the nasal portion 20 and the mouth portion 40 may be utilized to deliver pressurized gas to the patient's airways. Alternatively, the patient interface system 1 may be formed as an integrated patient interface system, where the nasal portion 20 and the mouth portion 40 are formed as a single element.

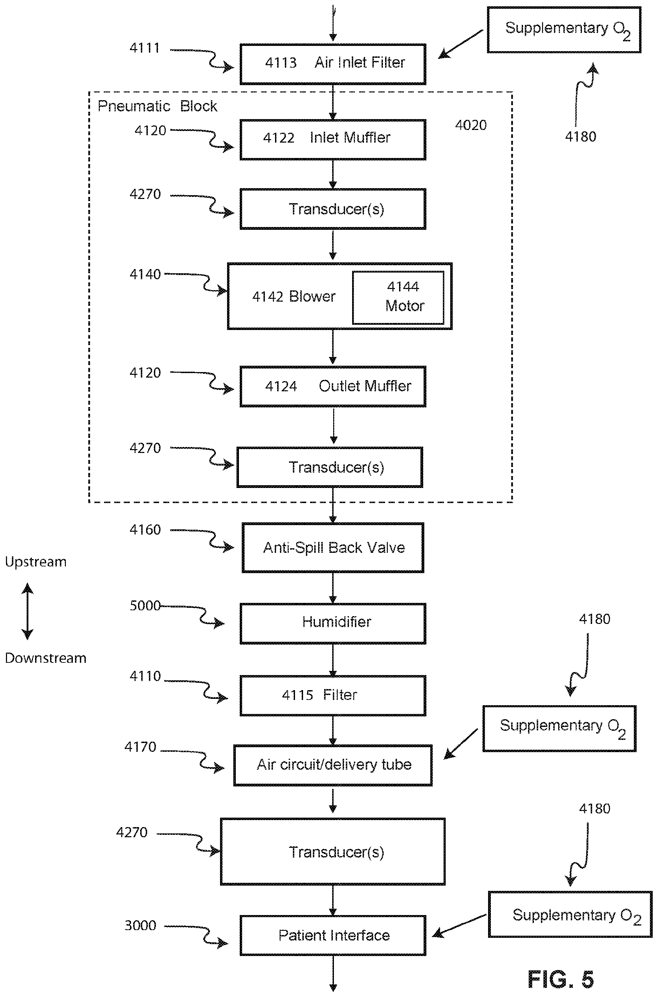

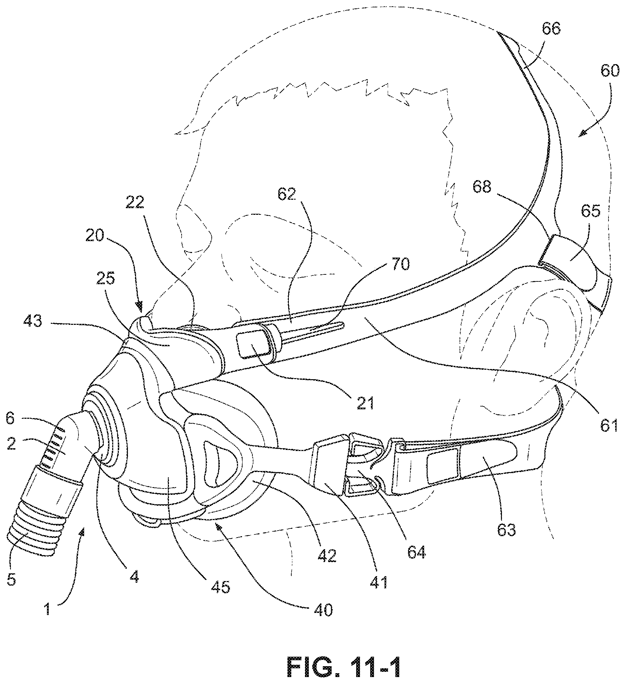

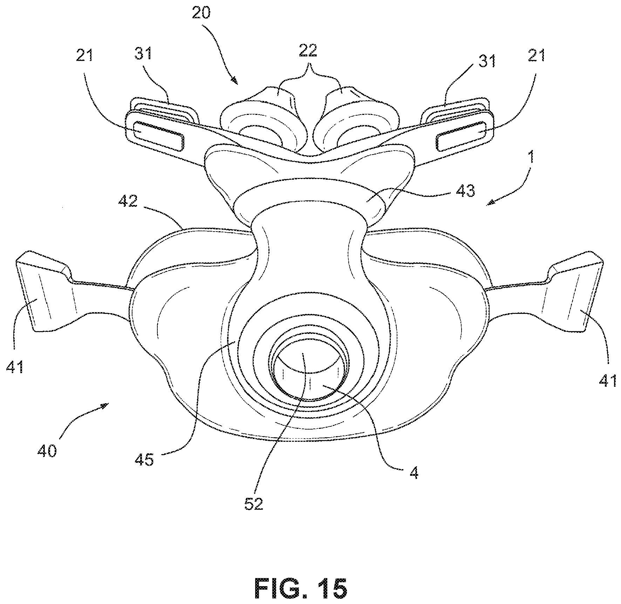

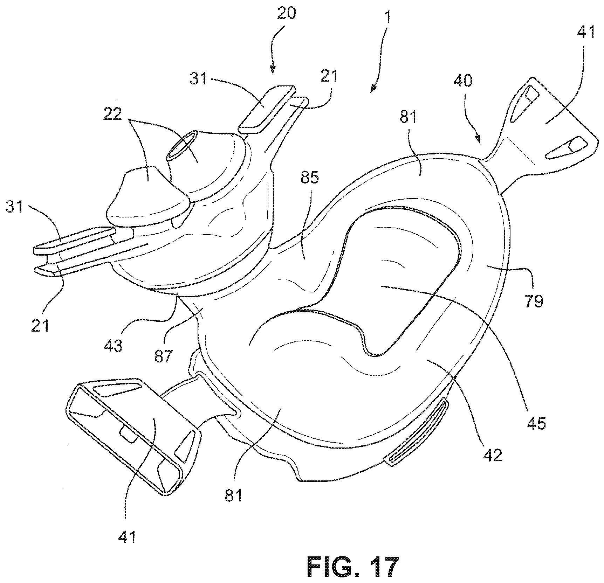

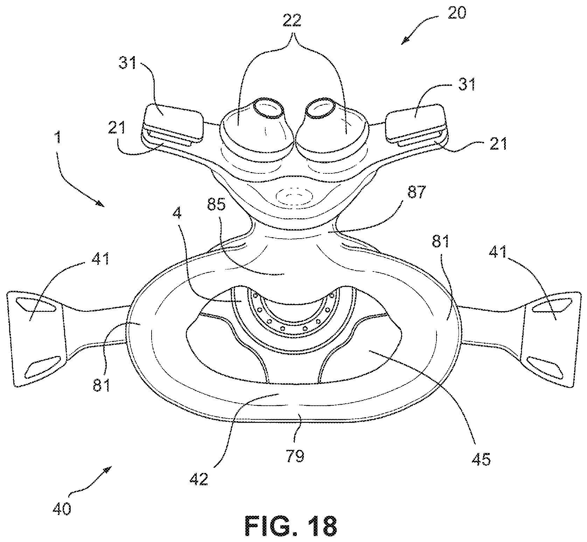

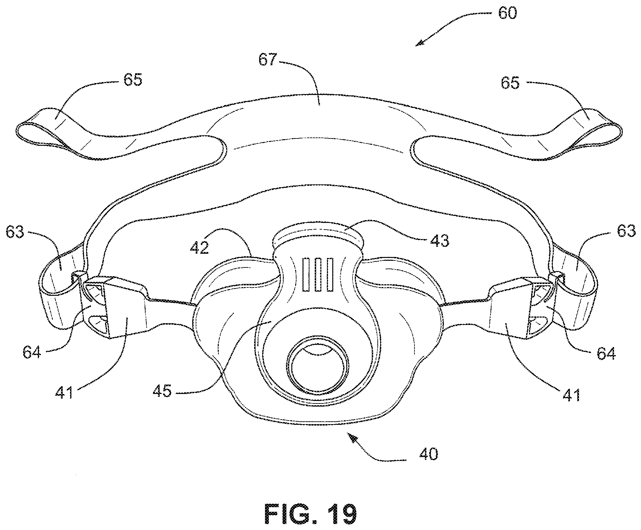

Figs. 11-1 to 19 illustrate another patient interface system 1 that may be in the form of a modular patient interface, where the nasal portion 20 and the mouth portion 40 may be adapted to be used without each other, or the nasal portion 20 and the mouth portion 40 may be adapted to function together to deliver the breathable, pressurized gas to the patient's nares and mouth. In Figs. 11-1 and 12 to 19, the flexible tube 5 is connected to the swivel ring 4 via the elbow 2, to direct the pressurized, breathable gas to the chamber of the mouth portion 40 and to the nasal portion 20. Fig. 11-1 and Figs. 12 to 18 illustrate the modular patient interface system in a nares and mouth mode, where the nasal portion 20 and the mouth portion 40 are both utilized. Fig. 5-2 illustrates the patient interface system 1 in a nares only mode, where the nasal portion 20 is used without the mouth portion 40. In the nares only mode, the elbow 2, flexible tube 5 and the swivel ring 4 are connected to an opening in the nasal portion 20.

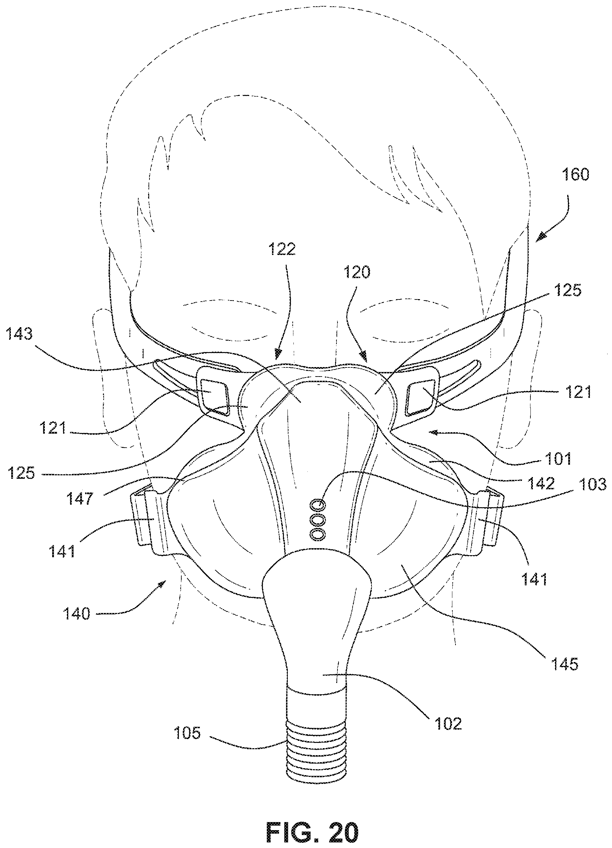

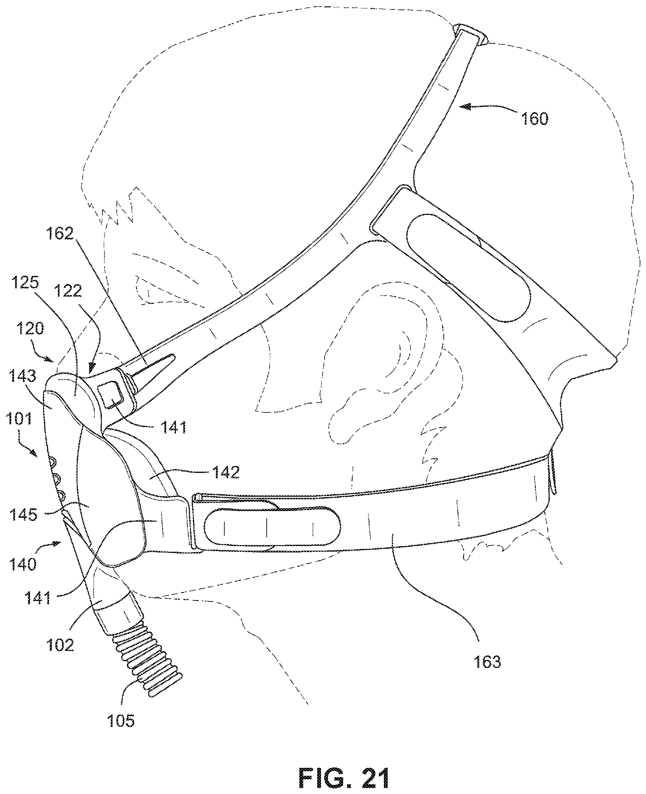

Figs. 20 and 21 illustrate an integrated patient interface system 101, which includes a nasal portion 120, a mouth portion 140, and positioning and stabilizing structure, which may include headgear 160. In the integrated patient interface system, the nasal portion 120 and the mouth portion 140 are not separable, and are typically formed together in a single piece. An air delivery system may deliver air to the patient interface system 101, such as through and a flexible tube 105 connected to the mouth portion 140 via an elbow 102.

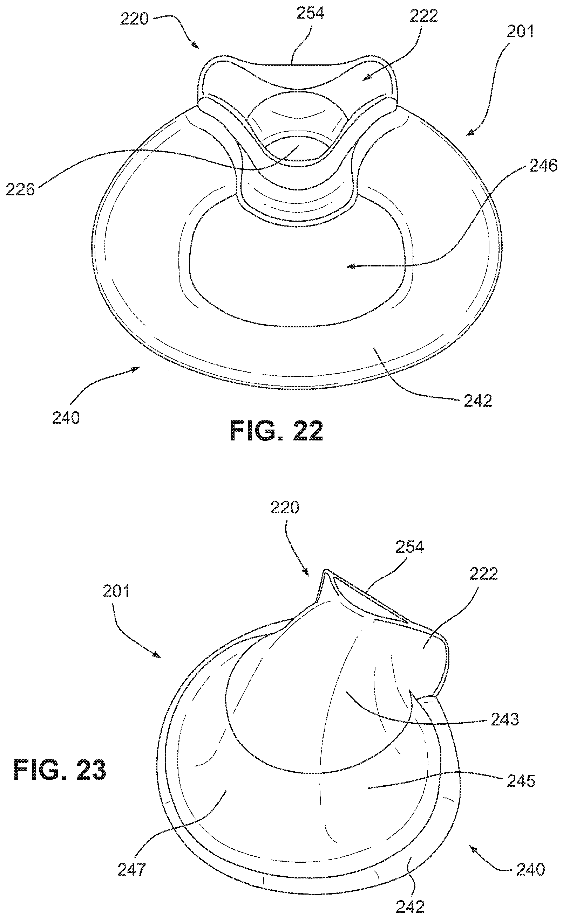

Figs. 22-24 illustrate another patient interface system 201, which may be an integrated patient interface system, and which includes a nasal portion 220 and a mouth portion 240. An air delivery system may deliver air to the patient interface system 201.

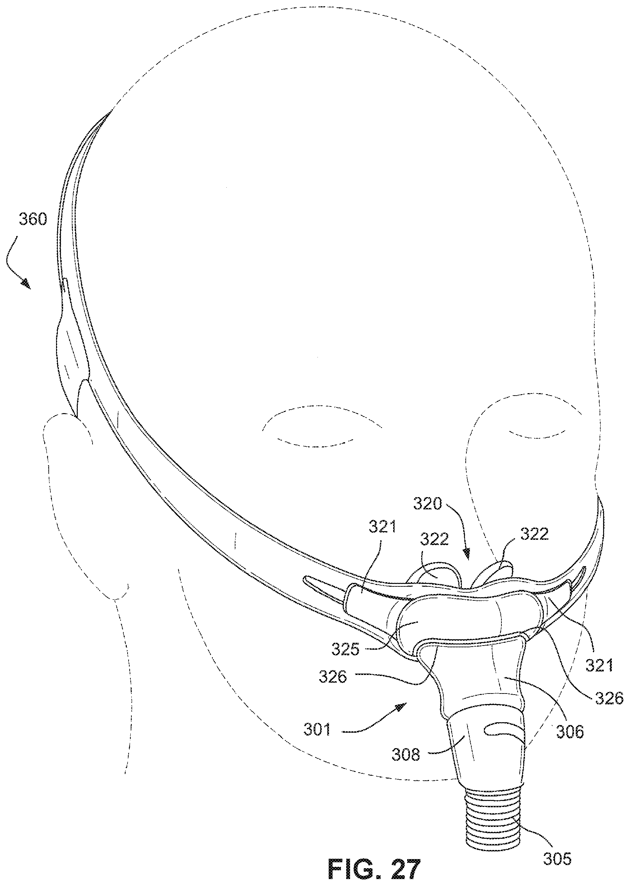

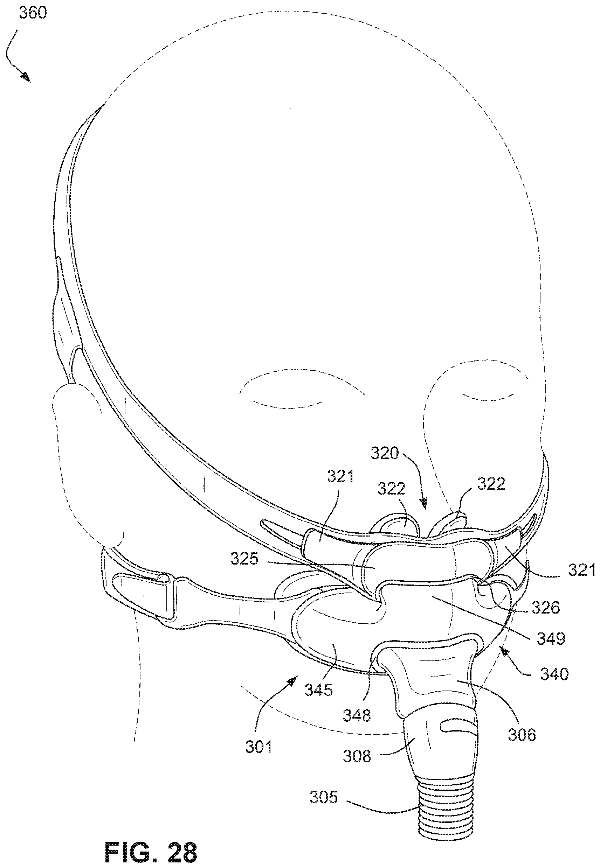

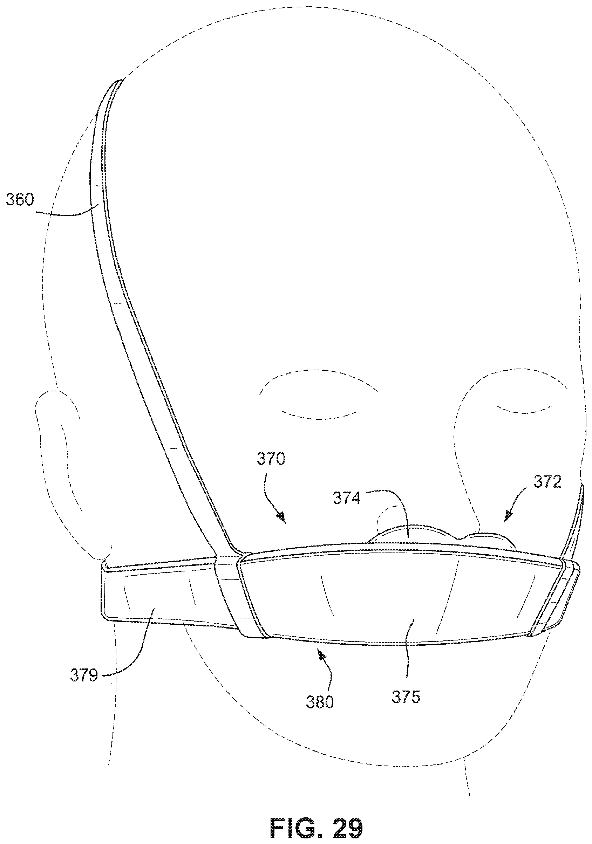

Figs. 27 and 29 illustrate a modular patient interface system 301 in accordance with an embodiment of the present technology. The patient interface system 301 includes a nasal portion 320, a mouth portion 340 and headgear 360. An air delivery system may deliver air to the patient interface system 301, such as through flexible tube 305 connected to the mouth portion 340 or nasal portion 320 via connector 306 and optional swivel connector 308. The connector 306 may be in the form of an elbow or other type of connector. The connector 306 may be a flexible portion that is able to absorb external forces that may otherwise dislodge the seal of nasal portion 320 and/or mouth portion 340 with the patient, for example as may be caused by tube drag forces. Fig. 27 illustrates the patient interface system 301 in the nares only mode, and Fig. 28 illustrates the patient interface system 301 in the nares and mouth mode.

Fig. 29 illustrates a modular patient interface system 370 in accordance with an embodiment of the present technology. The patient interface 370 includes a nasal portion 372, a mouth portion 380, and headgear 360. An air delivery system including an air delivery tube or conduit 379 may deliver air to the patient interface system 370. Further, tube 379 may function as headgear in addition to being an air delivery tube or conduit.

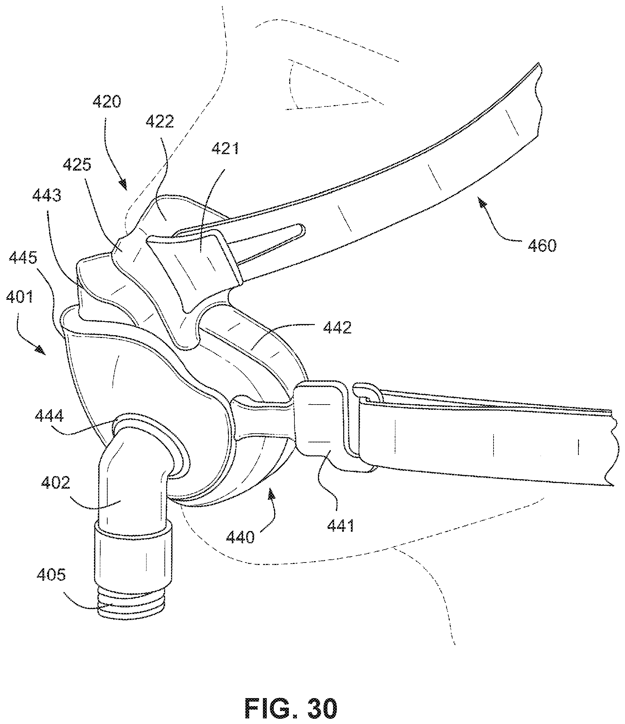

Fig. 30 illustrates a modular patient interface 401 in accordance with an embodiment of the present technology. The patient interface 401 includes a nasal portion 420, a mouth portion 440, and headgear 460. An air delivery system may deliver air to the patient interface system 401, such as through a flexible tube 405 connected to the mouth portion 440 via connector such as an elbow 402 and optional swivel connector 444.

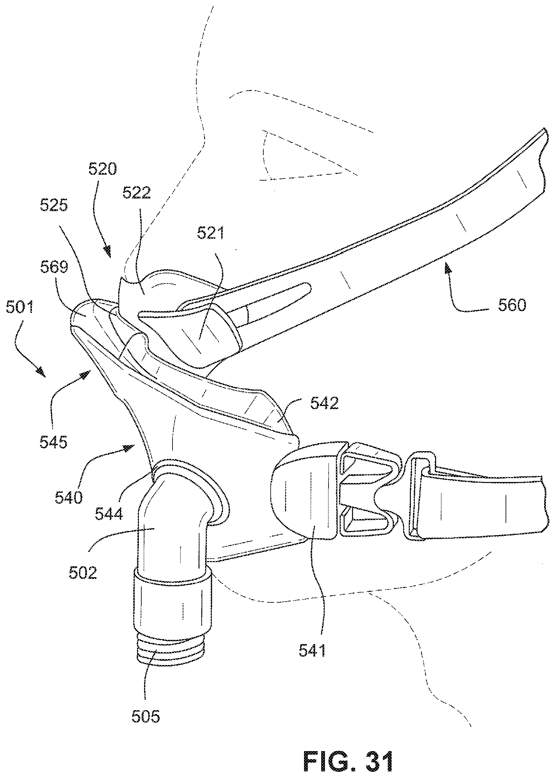

Fig. 31 illustrates an integrated patient interface 501 in accordance with an embodiment of the present technology. The patient interface 501 includes a nasal portion 520, a mouth portion 540, and headgear 560. An air delivery system may deliver air to the patient interface system 501, such as through a flexible tube 505 connected to the mouth portion 540 via connector such as an elbow 502 and optional swivel connector 544.

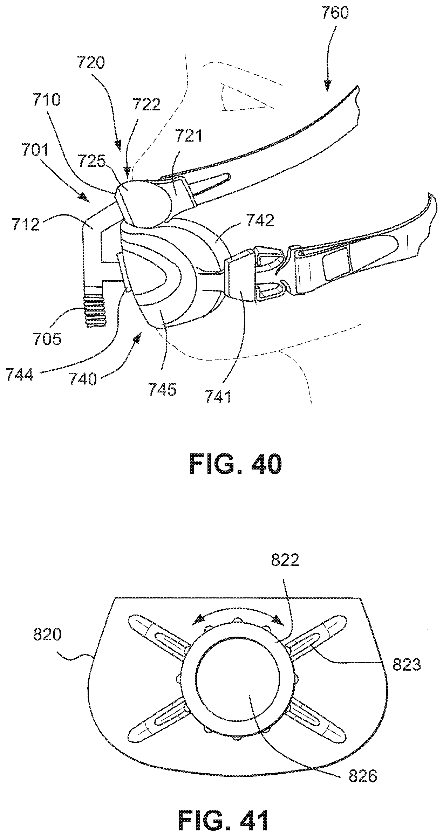

Fig. 40 illustrates patient interface 701 that may be in the form of a modular patient interface, where the nasal portion 720 and the mouth portion 740 may be adapted to be used without each other, or the nasal portion 720 and the mouth portion 740 function together to deliver the breathable, pressurized gas to the patient's nares and mouth. Fig. 40 illustrates the patient interface system 701 utilized in the nares and mouth mode, with the flexible tube 705 connected to the swivel rings 710 and 744 via the double elbow 712, to direct the pressurized, breathable gas to the chamber of the mouth portion 740 and to the nasal portion 720.

In a nares only mode, only the nasal portion 720 is utilized and the mouth portion 740 is not utilized. In this mode, the double elbow 712 is removed and the mouth portion 740 is removed. The patient utilizes the nasal portion 720 only and connects the tube 705 to the swivel ring or other connector 710. An elbow such as elbow 2 may be utilized to connect the tube 705 to the swivel ring or other connector 710.

In a mouth only mode, only the mouth portion 740 is utilized and the nasal portion 720 is not utilized. In this mode, the double elbow 712 is removed and the nasal portion 720 is removed. The patient utilizes the mouth portion 740 only and connects the tube 705 to the swivel ring or other connector 744. An elbow such as elbow 2 may be utilized to connect the tube 705 to the swivel ring or other connector 744.

### 5.3.1 Nasal portion

The nasal portion is intended to form a seal with the patient's nasal airway in use. The nasal portion could be a seal that is disposed on the outside of the patient's nose, for example a cradle type patient interface or a typical nasal patient interface. Alternatively, it could be an around the nares type seal like a pillows type patient interface, or it could be an in the nose type seal like nasal plugs. A nares seal may be an appropriate choice for patient's who have upper airway obstructions. Breathing through the nose may also have other benefits like a natural filtration of the inhaled air.

As illustrated in Figs. 7-1 to 10, the modular patient interface system 1 may include a nasal portion 20. The nasal portion 20 may include nares sealing portion 22, a decoupling portion 25, headgear connectors 21, a swivel ring 4, and a plug 3. The headgear connectors 21 may include headgear tabs 31 adapted to connect to headgear.

The decoupling portion 25 may function to decouple forces applied to the nasal portion 20 from the nares sealing portion 22, such as tube drag forces applied at the swivel ring 4 by movement of flexible tube 5. The swivel ring 4 fits into an opening in the decoupling portion 25 on a front of the nasal portion 20. The decoupling portion 25 may comprise a thin walled section, for example less than 1mm to enable flexibility of this portion.

The plug 3 is adapted to fit into the swivel ring. The plug 3 may include a plurality of optional vent holes 6 that allow air exhaled by the patient's nose to be vented out of the nasal portion 20. Preferably, vent holes 6 are arranged in a diffuse array to prevent jetting of air from the vent. In some embodiments, the plug 3 may be removed and replaced with a connection to the tube 5.

Fig. 7.1 illustrates the nasal portion 20 of patient interface system 1 utilized in a nares only mode. Nasal portion 20 is utilized without mouth portion 40, and the elbow 2 and flexible tube 5 are connected to the swivel ring 4 on the nasal portion 20.

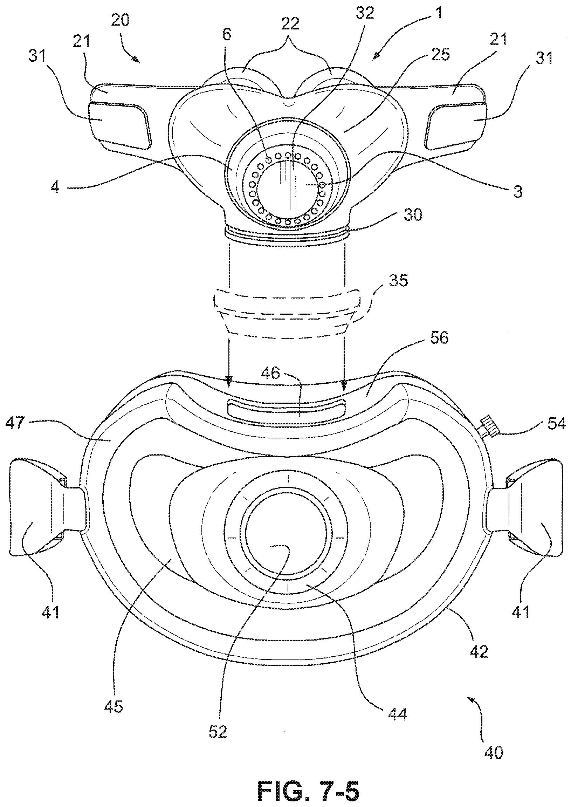

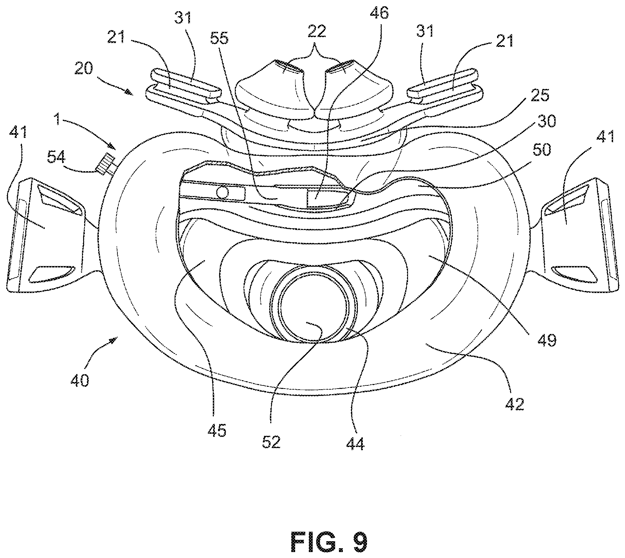

The nasal portion 20 illustrated in Figs. 7-1 to 10 may be a modified version of the commercially sold Swift^{™} FX by ResMed Ltd, (e.g., as described in WO 2009/052560 A1 and WO 2010/139014 A1, for example, although other nasal portions may be utilized. The nasal portion 20 may be modified from the commercially sold Swift^{™} FX by ResMed Ltd. to include a connector 30, as illustrated in Figs. 7-1, 7-5 and 9, the connector 30 adapted to connect a chamber 49 of the nasal portion 20 at an opening 46 in mouth portion 40, so that air is pneumatically allowed to flow between the nasal portion 20 and the mouth portion 40. When used in the nares only configuration as illustrated Fig. 7-1, the connector 30 is removed from the opening 46 as shown in the exploded view of Fig. 7-5, and the connector 30 is engaged with plug 35. The plug 35 is shaped so as to fit into either the opening 46 or the connector 30 as needed. The plug 3 is removed from the nasal portion 20, and the elbow 2 and flexible tube 5 may be connected via the swivel ring 4 to the nasal portion 20 to deliver the pressurized breathable gas to the nasal portion 20.

Figs. 11-1 to 19 illustrate a patient interface system 1 that includes a nasal portion 20. The nasal portion 20 may include nares sealing portion 22, a decoupling portion 25, headgear connectors 21, and a swivel ring 4. The headgear connectors 21 may include headgear tabs 31 adapted to connect to headgear 60.

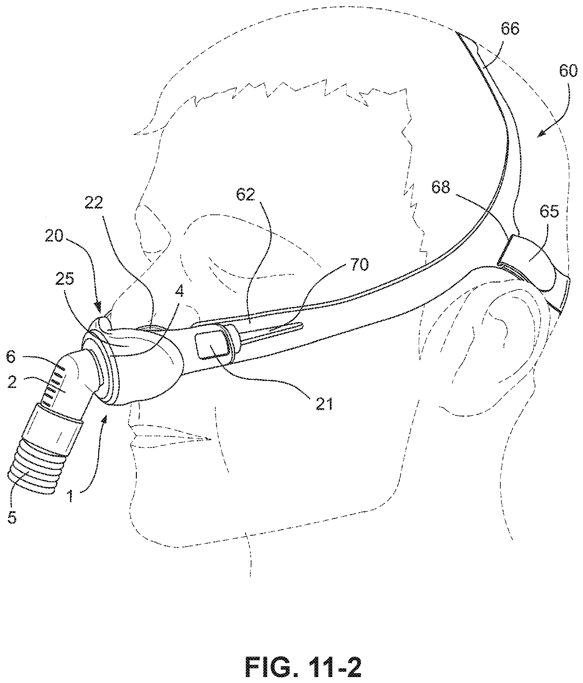

The nasal portion 20 illustrated in Figs. 11 to 19 may be the commercially sold Swift^{™} FX by ResMed Ltd, for example, although other nasal portions may be utilized. Further details of such a nasal portion 20 are disclosed in WO 2009/05256 A1.

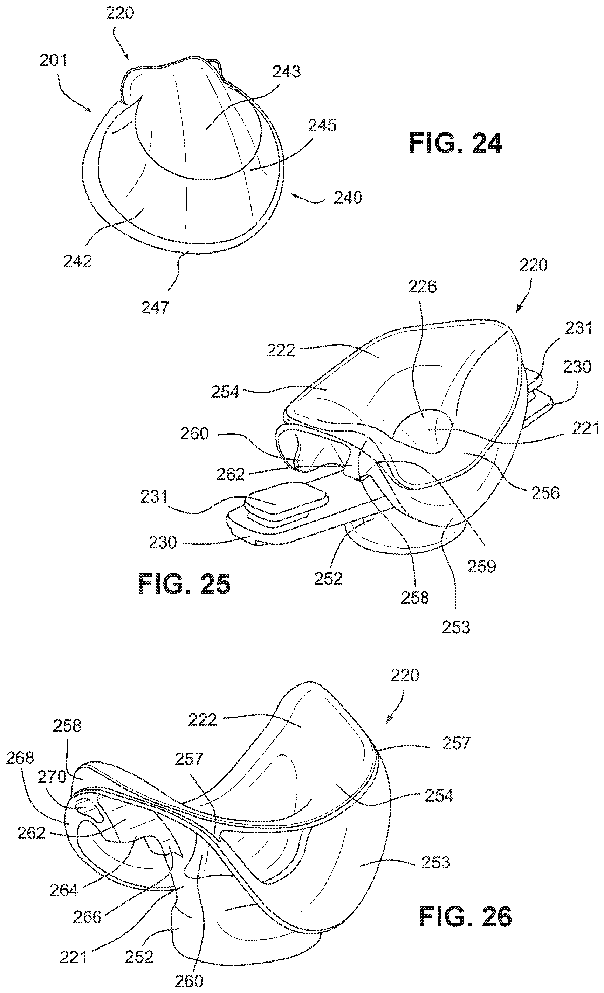

Figs. 25 and 26 illustrate another nasal portion 220, which may be utilized with any of the embodiments disclosed herein. The nasal portion 220 may include nares sealing portion 222, a support membrane 252, a supporting portion 253, and headgear connectors 230 with headgear tabs 231 for connecting to headgear. The support membrane 252 may be adapted to connect to a swivel ring or other connector for connection to a supply of pressurized, breathable gas. Further details of the nasal portion 220 are disclosed in WO 2010/139014 A1.

Figs. 20 and 21 illustrate an integrated patient interface system 1, which includes the nasal portion 120. The nasal portion 120 may include nares sealing portion 122, decoupling portion 125 and headgear connectors 121 adapted to connect to headgear 160. A nasal portion such as the commercially sold Swift^{™} FX nasal portion, the modified version of the commercially sold Swift^{™} FX nasal portion, or the nasal portion of Figs. 25 and 26 may be utilized and integrated with the embodiment of Figs 20 and 21.

Figs. 22-24 illustrate a patient interface system 201 that may include nasal portion 220. The nasal portion 220 may include nares sealing portion 222 and an orifice 226 for receiving pressurized, breathable gas. The patient interface system 201 is illustrated with a nasal portion 220 such as illustrated in Figs. 25 and 26, although other nasal portions could be utilized, such as the commercially sold Swift^{™} FX nasal portion or the modified version of the commercially sold Swift^{™} FX nasal portion.

Figs. 27 and 28 illustrate a modular patient interface system 301, which includes nasal portion 320. The nasal portion 320 may include nares sealing portion 322, decoupling portion 325 and headgear connectors 321 for connecting to headgear 360. Nasal portion 320 may be adapted to connect to connector 306 by including opening 326 adapted to receive connector 306.

Fig. 29 illustrates an integrated patient interface system that includes a nasal portion 372 connected to a mouth portion 380 and headgear 360. The nasal portion 372 includes nares sealing portion 374 adapted to form a seal with the patient's nares.

Fig. 30 illustrates an integrated patient interface system 401 that includes a nasal portion 420 connected to a mouth portion 440, and headgear 460. The nasal portion 420 includes nares sealing portion 422 adapted to form a seal with the patient's nares, decoupling portion 425, and headgear connectors 421 adapted to connect to headgear 460. The nasal portion 420 is illustrated as the membrane type illustrated in Figs. 25and 26, although other nasal portions could be utilized, such as the commercially sold Swift^{™} FX nasal portion or the modified version of the commercially sold Swift^{™} FX nasal portion.

Fig. 31 illustrates an integrated patient interface system 501 that includes a nasal portion 520 connected to a mouth portion 540, and headgear 560. The nasal portion 520 includes nares sealing portion 522 adapted to form a seal with the patient's nares, decoupling portion 525, and headgear connectors 521 adapted to connect to headgear 560. The nasal portion 520 is illustrated as the membrane type illustrated in Figs. 25 and 26, although other nasal portions could be utilized, such as the commercially sold Swift^{™} FX nasal portion or the modified version of the commercially sold Swift^{™} FX nasal portion.

Fig. 40 illustrates a modular patient interface system 701 that includes a nasal portion 720 connected to a mouth portion 740, and headgear 760. The nasal portion 720 includes nares sealing portion 722 adapted to form a seal with the patient's nares, decoupling portion 725, and headgear connectors 721 adapted to connect to headgear 760. The nasal portion 720 may be the commercially sold Swift^{™} FX nasal portion, although other nasal portions could be utilized, such as the commercially sold Swift^{™} FX nasal portion or the nasal portion illustrated in Figs. 25 and 26.

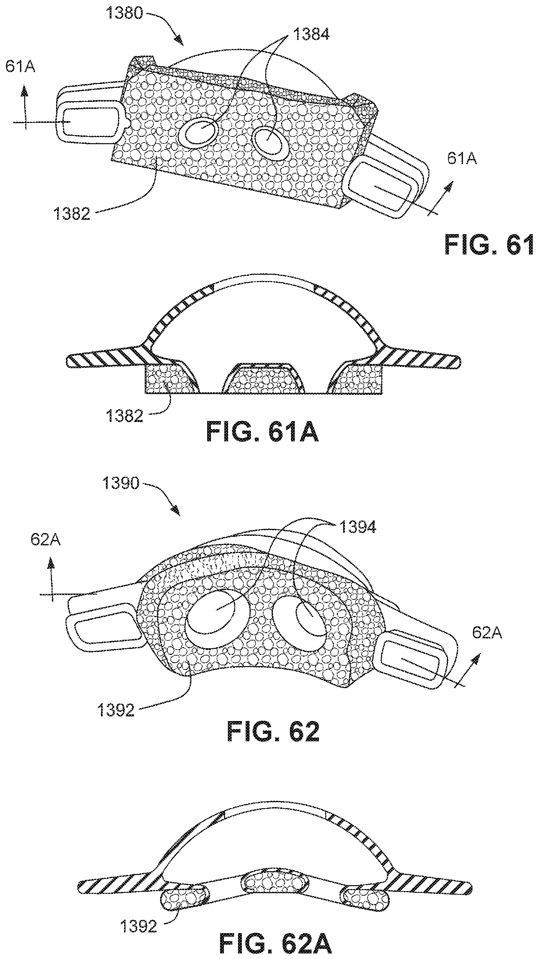

Figs. 61, 61A, 62, and 62A illustrate nasal portions 1380, 1390, each including foam portions 1382, 1392. The foam portions 1382, 1392 have holes 1384, 1394 allowing passage of air to the nares of a patient. As shown Fig. 61A, the foam portion 1382 is compliant and will reveal the nasal prong once compressed. As shown in Fig. 62A, the nasal prong rolls back to hold the foam portion 1392 in place and provide a relatively flat surface to contact with the nose.

### 5.3.2 Mouth Portion

The mouth portion may be adapted to surround the patient's mouth and form a seal with the patient's airway at the mouth. The seal of the mouth portion could be a flap type seal like a membrane type seal, or it could be a compression seal utilizing materials such as foam, gel, fabric, etc.

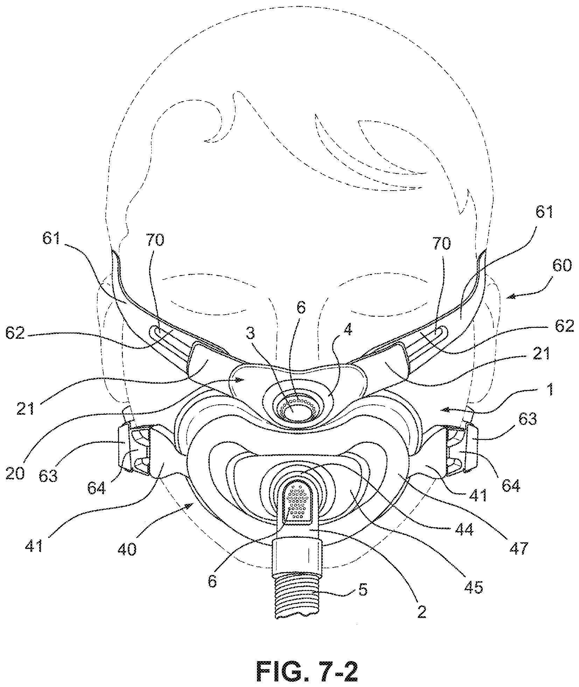

A mouth portion 40 as illustrated in Figs. 7-2 to 10 may include mouth sealing portion 42, a decoupling portion 45 to decouple forces applied to the mouth portion 40 from the mouth sealing portion 42, lower headgear connectors 41, and a swivel connector 44. The mouth portion 40 forms a chamber 49 into which air may be delivered and directed to the patient's mouth.

A swivel connector 44 may be disposed in a front aperture 52 of the mouth portion 40. The swivel connector 44 may be a swivel ring adapted to connect to a tube connector, such as elbow 2, for connecting to flexible tube 5, which may deliver breathable gas to the mouth portion 40. The swivel connector 44 may be removed from the front aperture of the mouth portion 40. Alternatively, the tube 5 may connect directly to the mouth portion 40.

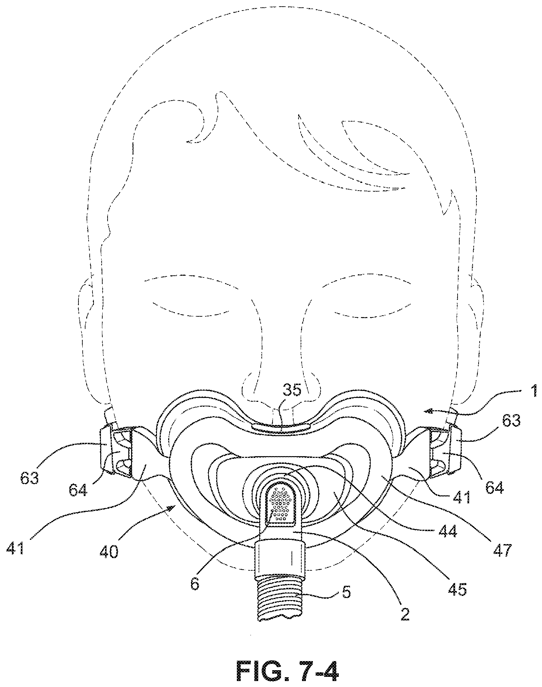

The mouth portion 40 illustrated in Figs. 7-2 to 10 is also adapted to connect to the nasal portion 20. Specifically, the mouth portion 40, as illustrated in Figs. 7-4 and 7-5, may include an aperture or opening 46 adapted to receive and seal with the connector 30 of the nasal portion 20, to pneumatically connect the nasal portion 20 to the chamber 49 of the mouth portion 40. A plug 35 may be included with the patient interface system 1, the plug 35 being adapted to fit within and plug the connector 30 or the aperture 46. Also, the nasal portion 20 may function in a nares mode only without the mouth portion 40.

The mouth portion 40 may function as a docking station, where the mouth portion 40 is adapted to provide docking (the mouth portion is adapted to removably receive the nasal portion) with respect to the nasal portion 20. As illustrated in Fig. 7-5, the mouth portion may include a nasal portion docking station 56 formed on the mouth portion 40, where the nasal portion docking station 56 is adapted to receive a nasal portion 20. The received nasal portion 20 is capable of functioning as a nares only device for delivering pressurized breathable gas to the nares of a patient.

The nasal portion docking station 56 may be adapted to receive the nasal portion 20 by having a shape selected to mate with a shape of the nasal portion. For example, as shown in Fig. 7-5, the nasal portion docking station may have a curved upper surface to match a curved lower of the mouth portion 40. The nasal portion docking station 56 may be adapted to receive the nasal portion 20 by having an opening or aperture, such as aperture 46, adapted to receive a protrusion or connector in the nasal portion 20, such as connector 30. Alternative connections of the docking station 56 to the nasal portion 20 are possible, such as utilizing undercuts or grooves to hold the nasal portion 20 in place. A plug 35 may be provided, the plug 35 adapted to plug either the connector 30 or the opening 46.

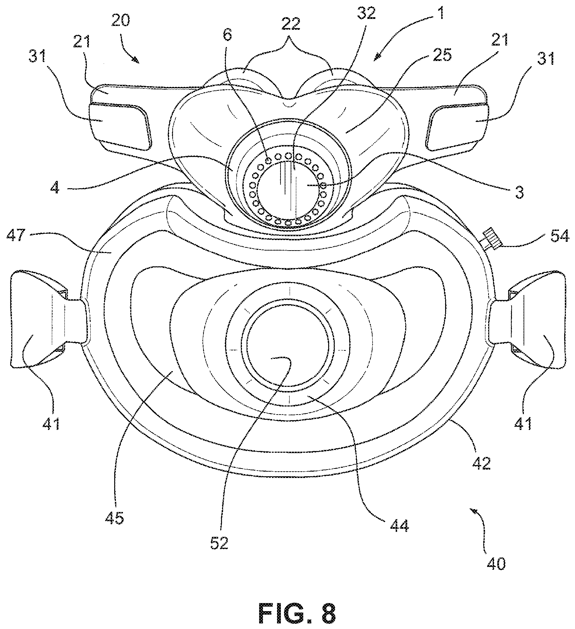

When the connector 30 of the nasal portion 20 is connected to the aperture 46 of the mouth portion, the plug 35 may be placed in the connector 30 to prevent air from pneumatically flowing between the nasal portion 20 and the mouth portion 40. The mouth portion 40 may alternatively be adapted to prevent air from pneumatically flowing between the nasal portion 20 and the mouth portion 40, by including a valve 55 as illustrated in Fig. 9, the valve 55 selectively opening and closing the opening 46. The patient can selectively operate the valve 55, such as by turning a knob 54 illustrated in Figs. 8 and 9.

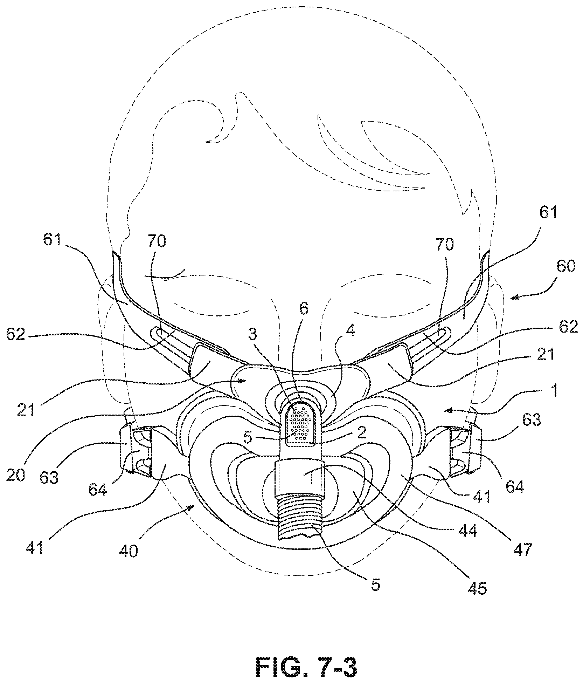

In some embodiments, such as illustrated in Fig. 7-3, the elbow 2 and flexible tube 5 may not be connected to the swivel connector 44, but may instead be replaced with plug 3, which may or may not include the optional vent holes 6.

Figs. 11-1 to 19 illustrate a modular patient interface system 1 which includes a mouth portion 40 that includes an alternative connection to nasal portion 20. In particular, in the embodiments of Figs. 11-1 to 19, a nasal portion connector 43 is utilized to connect the mouth portion 40 to the nasal portion 20. The nasal portion connector 43 is adapted to fit over and seal with the nasal portion 20, so that the air from the flexible tube 5 may be delivered into the mouth portion 40, and through nasal portion connector 43 to the nasal portion. The decoupling portion 45 may decouple forces at swivel ring 4, such as tube drag forces, from both the sealing portion 22 of the nasal portion 20 and from the sealing portion 42 of the mouth portion 40.

The sealing portion 42 of the mouth portion 40 may include a bottom portion 79, side portions 81, a top portion 85 and a neck portion 87. The bottom portion 79 may be adapted to form a seal with a patient's lower lip or chin area. The top portion 85 may be adapted to form a seal with the patient's upper lip area. The side portions may be adapted to form a seal with the patient's face on side areas of the patient's mouth. The side portions 81 extend between the bottom portion 79 and the top portion 85. The neck portion 87 connects the top portion 85 to the nasal portion connector 43. The sealing portion 42 transitions from the top portion 85 sweeping into the neck portion 87, with the neck portion 87 being narrower than the top portion 85 to conform to the size and shape of the nasal portion connector 43. By having the narrower neck portion 87, the pressure and flow of air into the sealing portion 42 may be improved. Figs. 20 and 21 illustrate a patient interface system 101 that includes the mouth portion 140. The mouth portion 140 may include a structural portion 147, a mouth sealing portion 142, decoupling portion 145, headgear connectors 141 for connecting to headgear, and vent 103, which may include one or more vent holes or slots for venting gas exhaled by the patient. A nasal portion connector 143 for connecting to the nasal portion 120 may be utilized when the nasal portion 120 and the mouth portion 140 are not formed as a unitary element. Flexible tube 105 may be connected to the mouth portion 140 via an elbow 102. Structural portion 147 may be constructed and arranged to stiffen the outer perimeter of the sealing portion to the shape of the sealing portion is maintained and sufficient sealing force is applied to the patient's face.

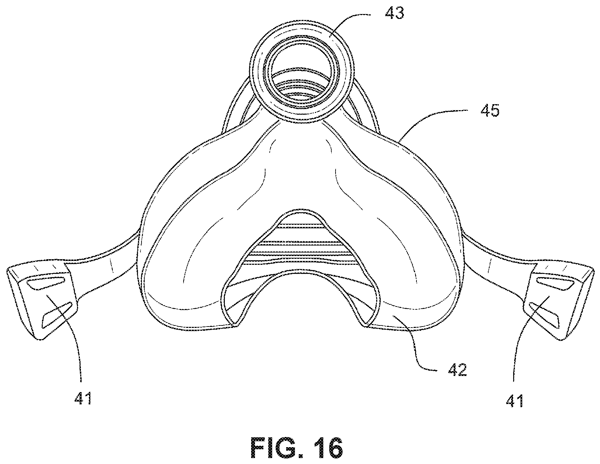

Figs. 22 to 24 illustrate a patient interface system 201 that includes the mouth portion 240. The mouth portion 240 includes a mouth seal portion 242 which may be in the form of a cushion for sealing with the mouth of the patient, a frame 247, a mouth orifice 246 for receiving the pressurized, breathable air, a decoupling portion 245, and a nares connecting portion 243 for connecting with the nasal portion. Fig. 16 is illustrated with the frame 247, the decoupling portion 245 and the connecting portion 243 removed. Although not illustrated, the mouth portion may include connection to a flexible tube for delivery of the pressurized, breathable gas, such as the swivel connector, elbow and flexible tube illustrated in other embodiments.

Figs. 27 and 28 illustrate a modular patient interface system 301 that includes a mouth portion 340. The mouth portion 340 includes decoupling portion 345, opening 348 and connector 349. The connector 306 is adapted to connect to either opening 326 in the nasal portion 320 as illustrated in Fig. 27 or to opening 348 in the mouth portion 340 as illustrated in Fig. 28. The connector 349 of the mouth portion is adapted to connect to opening 326 in nasal portion 320, when the patient interface 301 is utilized in a nares and mouth mode. A plug may be provided to plug the connector 349 when the patient interface 301 is utilized in a mouth only mode.

The patient interface system illustrated in Fig. 29 may include a mouth portion 380. The mouth portion 380 may include a mouth sealing portion 375, and may be adapted to connect to headgear 360, and to connect to nasal portion 372. The patient interface system may include air delivery conduit or tube 379 which may be integrated into the headgear. The air delivery tube 379 thus eliminates the need for an elbow connected to the mouth portion 380, providing a "ducted" air delivery and giving the patient interface system 375 a more streamlined look.

The patient interface system 401 illustrated in Fig. 30 includes a mouth portion 440. The mouth portion 440 includes a mouth sealing portion 442, headgear connectors 441 for connecting to headgear 460 and a decoupling portion 445. The swivel connector 444 may connect the mouth portion 440 to elbow 402 and flexible hose 405, which delivers pressurized, breathable gas to the patient interface system 401.

The patient interface system 501 illustrated in Fig. 31 includes a mouth portion 540. The mouth portion 540 includes a mouth sealing portion 542, headgear connectors 541 for connecting to headgear 560, swivel connector 544 and foam patient contacting portion 569. The foam may be placed around a perimeter of the mouth sealing portion 542, but optionally could be placed only at selected portions, e.g., just at a top lip portion for comfort. Silicone or another sealing means could be placed in the areas around the perimeter of the mouth sealing portion 542 not containing the foam. The mouth portion 540 may also include an optional decoupling portion 545. The swivel connector 544 may connect to elbow 502 and flexible hose 505, which delivers pressurized, breathable gas to the patient interface system 501. The foam patient contacting portion 569 may contact with and form a seal with the upper lip area of the patient, and thus form part of the mouth seal.

The patient interface system 701 illustrated in Fig. 40 includes a mouth portion 740. The mouth portion 740 may include a mouth sealing portion 742, a decoupling portion 745 and headgear connectors 741 for connecting to headgear 760. A swivel ring or other connector 744 may also be used to connect to flexible hose 705 via double elbow 712.

### 5.3.3 Sealing Portions

The sealing portion may consist of at least two portions: a nares sealing portion and a mouth sealing portion. By providing at least a nares sealing portion and a mouth sealing portion, the patient may receive pressurized breathable gas selectively through their nose and/or mouth.

The nares sealing portion may function independently of the mouth sealing portion. Therefore, in some embodiments, the patient may remove the mouth sealing portion by removing the mouth portion and use only the nasal portion with the nares sealing portion if desired. The mouth sealing portion may function independently of the nares sealing portion. Thus, in some embodiments, the patient may remove the nasal portion and the nares sealing portion and use only the mouth portion and the mouth sealing portion.

The nares sealing portion and the mouth sealing portion may both need to form a seal with the upper lip of the patient. However, due to the limited amount of surface area in which to position the nares sealing portion and the mouth sealing portion in the upper lip region, the various embodiments utilize structural elements to accomplish sealing of both the nares sealing portion and the mouth sealing portion in the upper lip region of the patient.

The mouth sealing portion may fit a wide range of the population. The mouth sealing portion may have a single size that fits most people. However, the mouth sealing portion may be provided in various sizes to accommodate a wide range of the population. The mouth seal may be lengthwise adjustable to accommodate the varying anthropometric range of the population.

The nares sealing portion may fit a wide range of the population. Preferably, the nares sealing portion may be provided in one or two sizes to accommodate a wide range of the population.

### 5.3.3.1 Nares Sealing Portion

The nares sealing portion is adapted to provide a comfortable and effective seal with the nares of a patient.

The nares sealing portion may be in the form of pillows, prongs, a membrane seal such as a nasal cradle, and/or a nasal chamber.

The nares sealing portion may be the pillows seal such as disclosed in PCT Application No. WO 2009/052560 A1.

Preferably the nares sealing portion may be flexible and compliant so as to conform to the shape of the patient's nose and/or nares in use.

Preferably the nares sealing portion may be constructed from a polymer such as silicone, thermoplastic elastomer, thermoplastic urethane. Alternatively the nares seal portion may be constructed of foam, gel, fabric or other compliant material. Alternatively, the nares sealing portion may be constructed of a combination of materials such as a foam and fabric lamination, fabric and polymer combination or any other combination of the afore mentioned materials.

As illustrated in Figs. 7-1 to 10, embodiments of the present technology may include a nares sealing portion 22 in the form of a pillows or prongs type nares seal. The nares sealing portion 22 may alternatively be a membrane type nares seal or a nasal chamber. Nares sealing portion 22 is adapted to form a seal with the nares of a patient in use. Nares sealing portion 22 may be positioned to sit underneath the patient's nose, and be shaped to form an effective seal.

As illustrated in Figs. 25 and 26, a nasal portion 220 may include nares sealing portion 222, a support membrane 252, a supporting portion 253, and headgear connectors 230 with headgear tabs 231 for connecting to headgear. All of the embodiments disclosed herein may use a nares sealing portion in the form of the nares sealing portion 222 when utilized with a membrane type nasal portion.

The nares sealing portion 222 may include a nose tip engagement portion 254, an upper lip engagement portion 256, and thickened corner regions 258. The supporting portion 253 may include rear thickened portions 262 and front thickened portions 260. A higher stiffness material in portions of the supporting portion 253 may be used instead of or in addition to the thickened portions 260 and 262 to provide additional support in these areas. The front thickened portions 260 are positioned adjacent to an area of the sealing portion that contacts with sides of the patient's nose in use, and transfer headgear load into a pinch force on the sides of a patient's nose to provide an effective seal. The front thickened portion 260 may have a thickness that increases from a top to a bottom. The rear thickened portions 262 may include a lower portion 266 having a first thickness and an upper portion 264 having a second thickness greater than the first thickness.

The nose tip engagement portion 254 is formed as a hanging, flexible membrane. The sides of the sealing portion 222 are connected to or bonded to the supporting portion 253, while there is a front gap between a central portion of the sealing portion 222 and the supporting portion 253 between front anchor points 257. By utilizing this hanging, flexible membrane, the nose tip engagement portion 254 provides a flexible surface that remains in tensile contact with the nose during patient interface movement, and better accommodates varying nose geometries. The nose tip engagement portion 254 engages with and seals with the patient's nose, and stretches towards the supporting portion 253. The sides of the sealing portion 222 engage with and seal with the sides of the patient's nose.

The sealing portion 222 includes an upper lip engagement portion 256 that engages with a patient's upper lip in use. The upper lip engagement portion 256 is formed as a hanging, flexible membrane, with a rear gap between the upper lip engagement portion 256 of the sealing portion 222 and the supporting portion 253. The rear gap is positioned between rear anchor points 259 that anchor the sealing portion 222 to the supporting portion 253. The flexible, hanging membrane provides a flexible surface that remains in tensile contact with the upper lip of the patient during patient interface movement, and can stretch to accommodate varying facial geometries by allowing movement of the upper lip engagement portion 256.

The rear thickened portions 262 may have a curved portion 268. The rear thickened portions 262 may include a cored out portion 270 to reduce a bulk of the silicone and to reduce a curing time. The rear thickened portions 262 are positioned directly below the thickened corner regions 258 of the sealing portion 222, as may be seen in Fig. 20. When utilized with headgear connectors such as illustrated in Figs. 24 and 25, the rear thickened portions 262 transfer a load from the headgear connectors to the thickened corner regions 258 and to the lower corners of the patient's nose to aid in providing an effective seal, and when the headgear is tensioned, the transfer of load to the lower corners of the patient's nose is increased. The bending force from the headgear connectors is transferred in use by the rear thickened portions 262 to the thickened corner regions 258 of the sealing portion 222 to apply a sealing force as an anchor force to regions of the patient's nose adjacent the nasal labial creases.

The nares sealing portion 222 is structured to extend or curve outwardly from a supporting wall 221 formed in aperture 226 which defines an air path through the nasal portion 220.

Further details of the nares sealing portion 222 are disclosed in WO 2010/139014 A1.

All of the embodiments disclosed herein may use a nares sealing portion in the form of a pillows or prongs type nares seal, a membrane type nares seal such as illustrated in Figs. 25 and 26, or a nasal chamber.

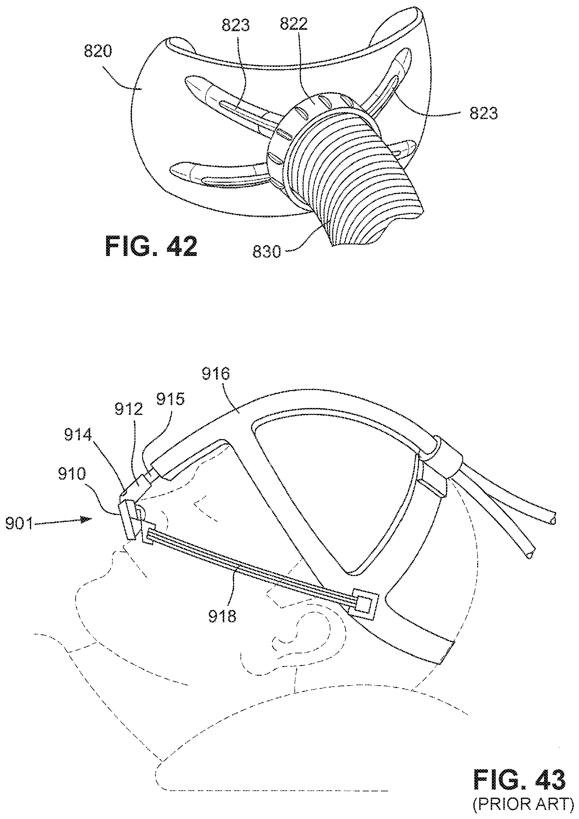

Figs. 41 and 42 illustrate a nares sealing portion 820 that may be utilized to form a seal with a patient's nares to deliver pressurized, breathable gas to the patient's nares via opening 826 and inlet tube 830 in a patient interface system. The nares sealing portion 820 may be a membrane type nares seal, such as the sealing portion 222 illustrated in Figs. 25 and 26.

A dial 822 and rigid elements 823 are disposed on a bottom surface of the nares sealing portion 820. There may be four such rigid elements 823, although any number could be used. The rigid elements 823 may be disposed to extend radially from the dial 822, and the dial 822 may be disposed around the opening 826. Turning the dial 822 rotates a beveled gear, causing the rigid elements 823 to move upwards or downwards, in turn causing the sides of the nares sealing portion 820 to move upwards or downwards, increasing or decreasing a pinching force on the sides of the patient's nares in use.

### 5.3.3.2 Mouth Sealing Portion

The mouth sealing portion may be adapted to seal with the mouth of a patient. Preferably, the mouth sealing portion may be flexible and compliant so as to conform to the shape of the patient's nose and/or nares in use. The mouth sealing portion may include a membrane that can stretch over the upper lip and chin regions of the patient, i.e. be more flexible than the side regions of the membrane that interface with the cheeks of the patient. The cheeks or side of the mouth region are less sensitive to pressure from the force of the patient interface system so more force can be applied in the cheeks or side region thereby anchoring the mouth portion over the mouth of the patient. Further, the flexibility of the upper lip and chin regions means that the mouth sealing portion is able to flex from a generally planar position to fit flat/pancake faces, and can also fit pointy/angular faces by flexing into a concave position.

Preferably the mouth sealing portion may be constructed from a polymer such as silicone, thermoplastic elastomer, or thermoplastic urethane. Alternatively, the mouth seal portion may be constructed of a foam, gel, fabric or other compliant material. Alternatively, the mouth seal portion may be constructed of a combination of materials such as a foam and fabric lamination, fabric and polymer combination or any other combination of the afore mentioned materials.

As illustrated in Figs. 7-1 to 10, embodiments of the present technology may include a mouth sealing portion 42. Mouth sealing portion 42 is adapted to form a seal with the mouth of a patient in use. Mouth sealing portion 42 may be positioned to sit around the patient's mouth, and be shaped to form an effective seal. The mouth sealing portion 42 may be in the form of a cushion, which forms a rear opening 50 for insertion of the patient's mouth. The cushion may be a soft silicone cushion, which contacts with the patient's mouth area and forms a seal. All of the embodiments disclosed herein may include a mouth sealing portion as described above.

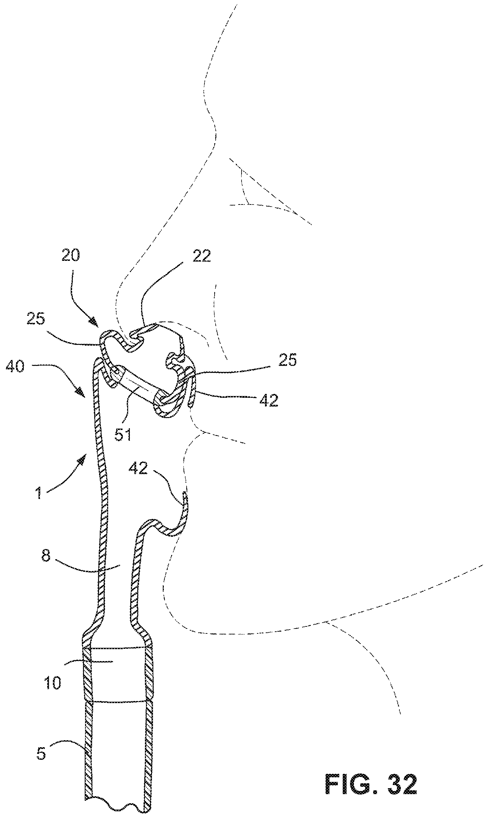

As illustrated in the schematic cross-sectional view of Fig. 32, the mouth sealing portion 42 may seal with the patient around the patient's mouth, in the areas of the patient's upper lip and an area between the patient's lower lip and the patient's chin. As further illustrated in Fig. 32, the mouth sealing portion 42 may be disposed between the decoupling portion 25 and the patient's upper lip.

### 5.3.3.3 Decoupling

There may be a decoupling or force absorbing element positioned as part of the nasal portion or the mouth portion. A decoupling element may be positioned between a first portion of the nasal portion and a second portion of the nasal portion, and/or between a first portion of the mouth portion and a second portion of the mouth portion, to prevent movement or forces acting on a the nares or mouth portions from disrupting the seal of the respective sealing portions.

In an embodiment, the decoupling element may be a spring.

In another embodiment, the decoupling element may be a gusset or flexible chamber.

As illustrated in Figs. 7-4, a decoupling portion 25 may be disposed between the nares seal portion 22 and the swivel ring 4. The decoupling portion 25 may be a thin, flexible region of elastic material adapted to absorb force and/or movement by flexing and/or altering its shape. For example, the decoupling portion 25 decouples forces or movement acting on the swivel ring 4 from being transferred to the nares sealing portion 22.

Mouth sealing portion 40 may also include a decoupling portion 45 that may be disposed between the frame 47 and swivel connector 44. The decoupling portion 45 may be a thin, flexible region adapted to absorb force and/or movement by flexing and/or altering its shape. For example, the decoupling portion 45 decouples forces or movement acting on the flexible tube 5, swivel connector 44, and/or the elbow 2 from being transferred to the mouth sealing portion 42, such as tube drag forces, providing a more effective seal with the patient. All of the embodiments disclosed herein may include one or more decoupling portions as described that may be part of the nasal portion and/or the mouth portion.

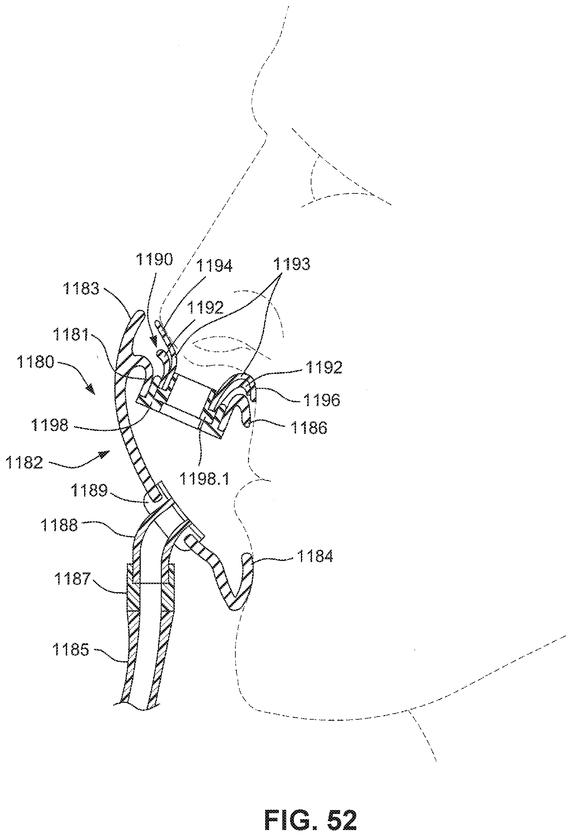

In the patient interface system 1180 of Fig. 52, the nasal portion 1190 is decoupled from movement in the mouth portion 1182 by decoupling portion 1181. The decoupling portion 1181 allows the nasal portion 1190 to remain in sealing engagement with the patient's nares if the air hose 1185 and/or mouth portion 1182 move. The nasal portion 1190 may rotate relative to the mouth portion 1182. The covering flap 1183 will move with the air hose 1185 and/or mouth portion 1182.

### 5.3.3.4 Relationship between Nares and Mouth Sealing Portions

The sealing portion may be constructed in one piece, with the one piece including the nares sealing portion and the mouth sealing portion.

The sealing portion may be constructed in more than one piece and then may be joined together to operate as a nose and mouth patient interface. For example, the nares sealing portion may be constructed in a first piece and the mouth sealing portion may be constructed in a second piece, and the first and second pieces may then be joined together.

In a further preferred embodiment, the sealing portions may be constructed in more than one piece, where each piece can be used independently of the other pieces or joined to the other pieces, each arrangement still providing treatment to the patient. For example, the nasal portion including the nares sealing portion may be separate from the mouth portion including the mouth sealing portion, and the nasal portion including the nares sealing portion can be used without the mouth portion including the mouth sealing portion attached yet still be able to deliver treatment to the patient's nares.

### 5.3.3.5 Connection of Nares and Mouth Portions to Headgear

The headgear 60 can connect to each of the nasal portion 20 and the mouth portion 40. This is unlike many prior art arrangements where the headgear connects to the mouth portion only and the mouth portion is structured and arranged to adapt the nasal portion into sealing engagement with the patient's nose.

As illustrated in Figs. 7-1 to 19, the nasal portion 20 may include headgear connectors 21. The headgear connectors 21 may include headgear tabs 31 that may interface or connect with apertures 70 which may be in the form of a slot or loop on the headgear strap 61.

The mouth portion 40 may include a lower headgear connector 41 for receiving a locking device 64 from lower headgear strap 63.

Alternative connecting devices are possible for the nasal portion 20 and the mouth portion 40. For example, hook and loop type connections, push fit connections, interference fits, ball and socket joints, etc. may be used.

### 5.3.4 Positioning and Stabilizing Structure

The positioning and stabilizing structure in the form of headgear 60 may be adapted to maintain the patient interface system in sealing engagement with the nares and mouth of a patient. The headgear 60 may include straps adapted to secure the position of the headgear 60 on the head of the patient. The straps may include a side strap headgear strap 61, a lower headgear strap 63, a rear headgear portion 65 and a crown headgear portion 66. The straps may also direct a tension force on the patient interface adapted to seal the patient interface with the face of the patient.

### 5.3.4.1 Headgear Side Portion

Figs. 7-1 to 7-3 and 11-1 to 13 show a headgear 60 in use. Side headgear straps 61 may be adapted to be positioned over the cheeks, underneath the eyes and above the ears of the patient. Side headgear straps 61 may be connected to the headgear connectors 21 of the nasal portion 20.

Side headgear straps 61 may include side headgear connectors 62 for connection with the headgear connectors 21 of the nasal portion 20. Side headgear connectors 62 may be in the form of a loop for capturing on the headgear tabs 31 on the nasal portion 20. Any connection method may be possible, for example hook and loop connection, clips, push fit tabs. Alternatively, side headgear straps 61 may be integrally formed with nasal portion 20.

Side headgear straps 61 may preferably be constructed of a flexible material that may be lengthwise stretchable. Side strap 61 may be constructed of a fabric, foam, foam and fabric composition, silicone, nylon, elastic, or any other flexible material.

Side headgear straps 61 may provide an upwards vector to the patient interface system 1 to assist in maintaining the patient interface system 1 in the desired position. The upwards vector may be angled upwards with respect to a vector applied by the lower headgear strap 63.

### 5.3.4.2 Headgear Crown Portion

Headgear 60 may further include a crown strap portion or top strap 66 that may be adapted to be positioned in use over the top or crown of the patient's head. Crown strap portion 66 may provide an upwards vector to the patient interface system to assist in maintaining the patient interface system in the desired position.

Crown strap portion 66 may be continuous with or otherwise connected to side headgear strap 61. Crown strap portion 66 and/or side headgear strap 61 may further include strap loop 68. Strap loop 68 may be structured to receive a rear portion 65.

Crown strap portion 66 may further include adjustment mechanism 69. Adjustment mechanism 69 may be that described in WO 2009/052560 A1.

Crown strap portion 66 may preferably be constructed of a flexible material that may be lengthwise stretchable. Crown strap portion 66 may be constructed of a fabric, foam, foam and fabric composition, silicone, nylon, elastic, or any other flexible material.

### 5.3.4.3 Headgear Lower Portion

Headgear 60 may further include lower headgear strap 63, which may connect to the mouth portion 40 of the patient interface system 1. Lower headgear strap 63 may direct a tension force on the mouth portion 40 to provide a vector force normal to the patient's face and hence sealing of the mouth portion 40 on the patient's face.

Lower headgear strap 63 may be a strap that is directed substantially under the ears of the patient.

Lower headgear strap 63 may connect or be otherwise formed with headgear rear portion 65.

### 5.3.4.4 Headgear Rear Portion

Headgear 60 may further include a rear portion or strap 65. Rear portion 65 may be continuous with or otherwise attached to side headgear strap 61 or crown strap portion 66. Rear portion 65 may be threaded through strap loop 68, and positioned above the ears of the patient. Rear portion 65 may travel down the sides of the patient's head in use and capture the occiput to anchor the headgear 60 in position. Rear portion 65 may further attach to a lower headgear strap 63 at connection region 67.

Rear portion 65 may provide a downwards and rearwards (i.e. away from the patient's face) vector to balance the upwards vector of the crown strap portion 66, and assist in pulling the patient interface system 1 into sealing engagement with the patient's face.

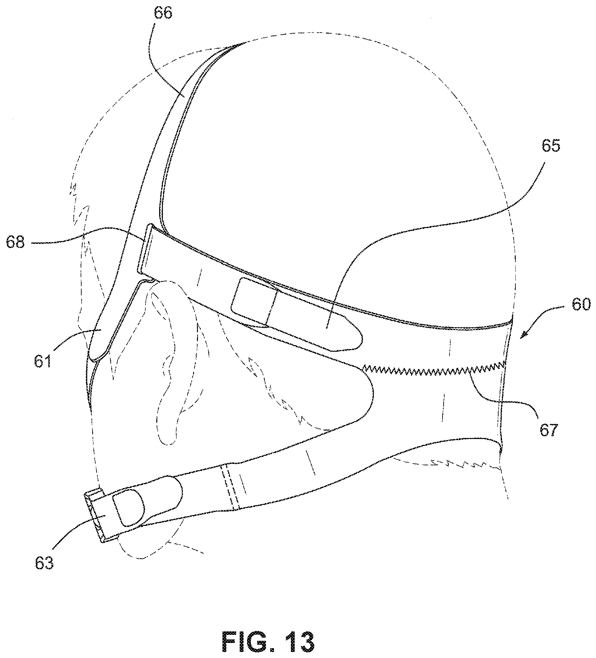

Rear portion 65 may be secured in position under the patient's occiput by attachment to a lower strap 63 (see Fig. 13). Attachment may be at connection region 67. Connection region 67 may comprise a stitched join, hook and loop join or any other join. Connection region 67 may also be a permanent join such that rear portion 65 and lower strap 63 are formed in one piece.

Rear portion 65 may preferably be constructed of a flexible material that may be lengthwise stretchable. Rear portion 65 may be constructed of a fabric, foam, foam and fabric composition, silicone, nylon, elastic, or any other flexible material.

### 5.3.4.5 Headgear Options

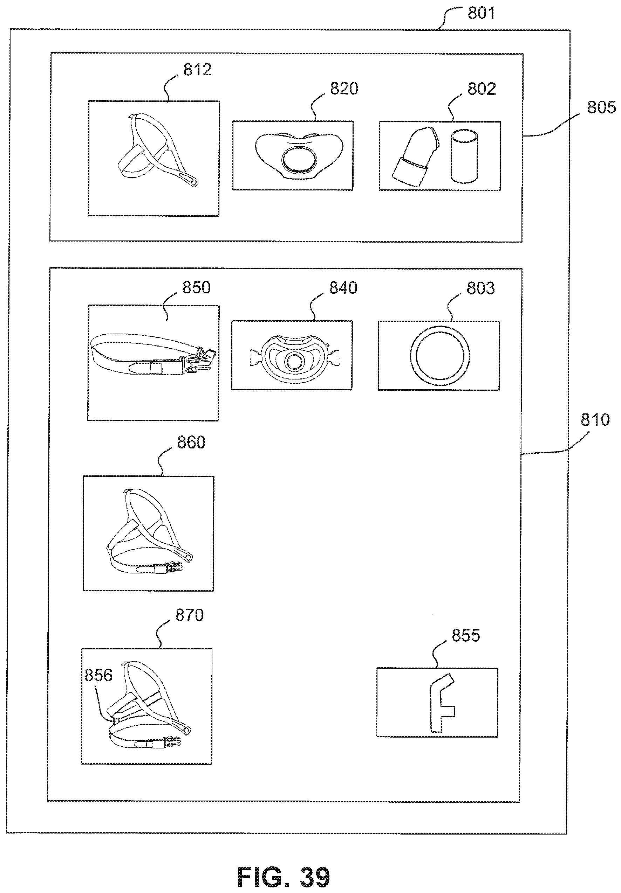

The various patient interface system and kit embodiments may include various headgear options such as illustrated in Fig. 39. For example, when utilized in a nares only mode, nasal portion headgear 812 adapted to secure the nasal portion to the patient's head may be utilized. When utilized in a mouth only mode, mouth portion headgear 850 adapted to secure the mouth portion to the patient's head may be utilized.

The nasal portion headgear 812 utilizes headgear vectors that are angled upwards to align the nares sealing force with the patient's nares. The mouth portion headgear 850 utilizes headgear vectors that are generally oriented in the horizontal plane to align the mouth sealing force with the patient's mouth. The headgear vectors are angled relative to one another in the examples shown in Fig. 39 and Figs. 43-44. However, in the examples of Figs. 40-41, the headgear vectors are generally parallel to one another due to the nature of the cushion and the sealing forces involved.

When utilized in a nares and mouth mode, nasal portion headgear 812 and mouth portion headgear 850 may be utilized together to secure the nasal portion and the mouth portion to the patient's head. Alternatively, nares and mouth portion headgear 860 may be utilized, which includes a connection region in the back portion, such as connection region 67 illustrated in Fig. 13. The nares and mouth portion headgear 860 creates headgear vectors that go back and up along the patient's head to secure the nasal portion 820 to the patient's nares, and that go back along the patient's head to secure the mouth portion 840 to the patient's head.

Each of the nasal portion headgear 812 and mouth portion headgear 850 can be connected at two points to a respective one of the nasal portion or mouth portions of the patient interface system of the present technology. In some forms, when both the nasal portion and mouth portions of the patient interface system are used together as one system, both of the nasal portion headgear 812 and the mouth portion headgear 850 can be used, thus providing together a positioning and stabilizing structure having four points of connection. The conversion from a two-point to a four-point positioning and stabilizing structure may enable a user to optimise, or at least improve, the force vectors of the reconfigured patient interface system. The improved vectors of the reconfigured four-point positioning and stabilizing structure may thus improve the effectiveness of the seal forming structure and may in some embodiments also result in improved overall comfort for the user. In some forms, the addition of a mouth portion headgear 850 can provide improved stability to the patient interface system. This may be particularly useful in forms comprising a patient interface system that has an overall seal forming structure that engages both the oral and nasal air passages of a patient.

For example, when reconfiguring a nasal interfacing portion to become a dual nasal and mouth interface (i.e. oro-nasal), or similarly, reconfiguring a mouth interfacing portion to become a dual nasal and mouth interface, by providing the patient interface system as separable nasal and mouth portions, with respective removable and adjustable accompanying headgear systems, the patient interface system may be modularly configurable by a user to suit their individual treatment requirements and desired comfort levels. A modularly configurable patient interface system may also improve the cost efficiency of manufacturing a patient interface system.

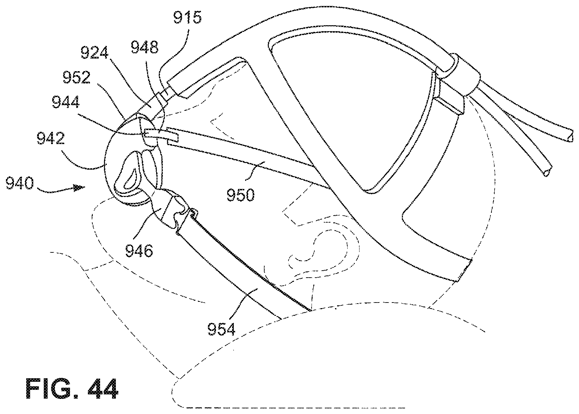

In some forms, the nasal portion headgear and mouth portion headgear of the disclosed patient interface system can be configured to be independent of one another (e.g. Fig. 44). In some forms, a connector 856 can be used to facilitate the connection of the nares and mouth portion headgear 870. For example, as illustrated in Fig. 39. The connector 856 can be used to facilitate the connection of the nasal portion headgear and the mouth portion headgear at one or more positions along the respective headgears. For example, the connector 856 can be positioned towards the centre rear of the user's head, adjacent the occipital bone.

As would be understood by one skilled in the art, the connector, or plurality of connectors used to join the nasal portion headgear and the mouth portion headgear can take many forms. Functionally, the joined nasal portion headgear and the mouth portion headgear provide together a positioning and stabilizing structure that has four points of connection to the treatment providing facial interface structure.

In some forms, each of the nasal portion headgear and the mouth portion headgear can comprise one or more connectors, the connector/s of the nasal portion headgear corresponding, in opposing engagement, to the connector/s of the mouth portion headgear. The connector 856 may be a Velcro connector, a hook or loop material connector, or the like. Any of the above described headgear options may be utilized with the various embodiments.

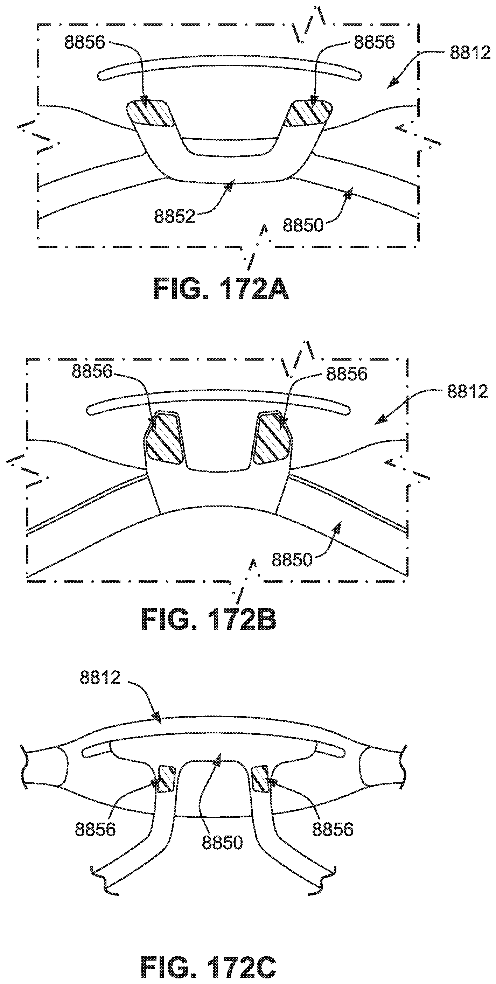

For example, in some forms, the nasal portion headgear 8812 can be formed from a material that is able to removably engage a connector 8856 comprising Velcro that is adapted on the mouth portion headgear 8850 (e.g. Figs. 172A and 172B). Alternatively, in some forms, the nasal portion headgear 8812 can comprise one or more connectors 8856 comprising a layer of Velcro (or similar materials) that are adapted to removably engage the material of the mouth portion headgear 8850 (e.g. Figs. 172C).

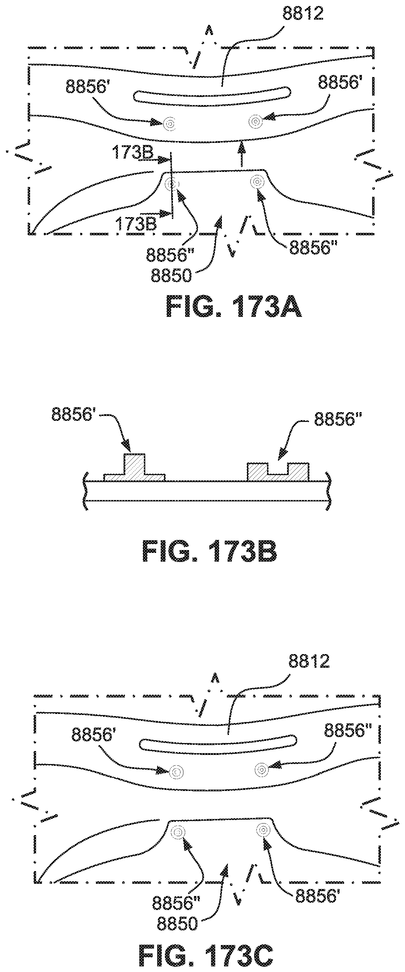

In a further example, in some forms, the connector 8856 can comprise one or more snap connections, with one or more male snap connectors 8856' adapted on the nasal portion headgear 8812 and one or more female snap connectors 8856" adapted on the mouth portion headgear 8850, or vice versa (e.g. Fig 173A and 173B). The plurality of male snap connectors 8856' can be spaced along the nasal portion headgear 8812 so as to correspond to, and removably engage with, similarly spaced plurality of female snap connectors 8856" on the mouth portion headgear 8850. In some forms, the male and female snap connectors 8856', 8856" can be thermoformed into the material of each respective headgear portion 8812, 8850. In some alternative forms, the male and female snap connectors 8856', 8856" can be countersunk by a riveting process into the material of each respective headgear portion 8812,8850. It may be advantageous to reduce the profile of the connector 8856. For example, by reducing the profile of the connector 8856 user comfort may be improved in some forms.

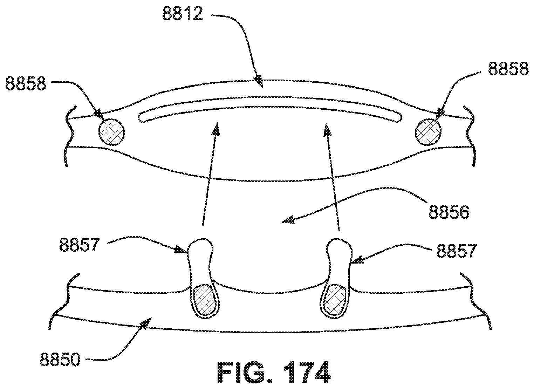

In a further example, in some forms, the connector 8856 can comprise one or more tabs 8857 that extend laterally from the mouth portion headgear 8850 to hook into, and thus removably engage with, one or more latches 8858 adapted on the nasal conduit system headgear strap to receive one or more of the one or more tabs 8857, or vice versa (e.g. Fig. 174). In some forms, each of the one or more tabs 8857 can be formed as a fixed portion of material. In some forms, each of the one or more tabs 8857 can be formed from a material that is elastically stretchable, thus allowing the one or more tabs 8857 to be stretched to reach a corresponding one of the one or more latches 8858.

In forms comprising a corresponding pair of connectors 856, the location of the one or more connectors on one headgear portion 8812, 8850 can limit the variance with which the corresponding connectors 856 and other of the headgear portions 8812, 8850 can be located in relative space. This may improve the ease of conversion from a two-point to a four-point positioning and stabilizing structure, as the connector/s 856 can guide the user, and may allow the connection to be more intuitive.

For example, in some forms, a plurality of spaced connectors 856 can be located along the nasal portion headgear 8812. The spaced connectors 856 thus can act as a guide to the user, by limiting the allowable variance of locations with which the corresponding connectors 856 of the mouth portion headgear 8850 can engage (e.g. Figs. 172A-173C).

In a further example, with reference to Fig 172A, the mouth portion headgear 8850 comprises a U-shaped segment 8852, with the flanges at each distal end of the U-shape comprising a layer of Velcro. Whilst in some forms, the entire nasal portion headgear 8812 can be formed from a material that engages with Velcro, in other forms, the material of the nasal portion headgear 8812 can be formed from a material that does not engage with Velcro, and instead two segments of Velcro engaging material can be adapted as a layer on the nasal portion headgear 8812 so as to guide the user when locating the Velcro of the at each distal end of the U-shaped segment of the mouth portion headgear 8850.

In a further example, with reference to Fig 173A, the distinct male and female snap connectors 8856', 8856", can be spaced along each respective headgear portion 8812, 8850 thus limiting the scope of variance allowed when connecting the nasal portion headgear 8812 and mouth portion headgear 8850. In some forms, with reference to Fig 173C, the male and female snap connectors 8856', 8856" can be alternated, such that a male snap connector 8856' is spaced from a female snap connector 8856" on the same headgear portion. This may assist in guiding a user to correctly connect the headgear portions 8812, 8850 relative to one another.

In some further forms, at least one of the nasal portion headgear 8812 and mouth portion headgear 8850 can comprise alignment guides 8860, so as to provide a guide to a user when engaging the connector 856. The alignment guides 8860 can assist with at least one of locating and/or orientating the connector 856 such that the vectors of the reconfigured four-point positioning and stabilizing structure may thus be improved.

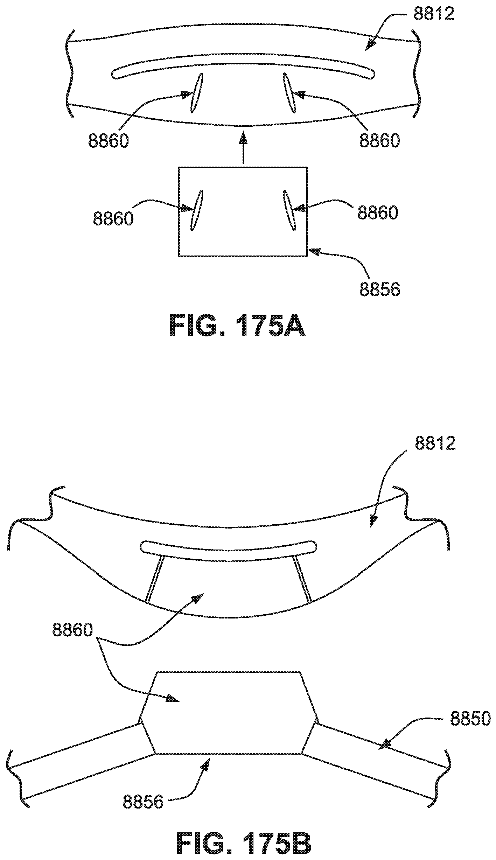

For example, with reference to Figures 175A and 175B, the connector 8856 and the alignment guides 8860 may comprise an identical pattern, plurality of visible lines (e.g. Fig 175A), or shape (e.g. Fig 175B). The alignment guides 8860 can thus indicate a location and/or orientation on the headgear portion. The user can be directed to simply place the connector 8856 over the matching pattern, lines or shape of the alignment guides 8860. This may be intuitive for a user to understand where, and at what angle, the connector should be engaged.

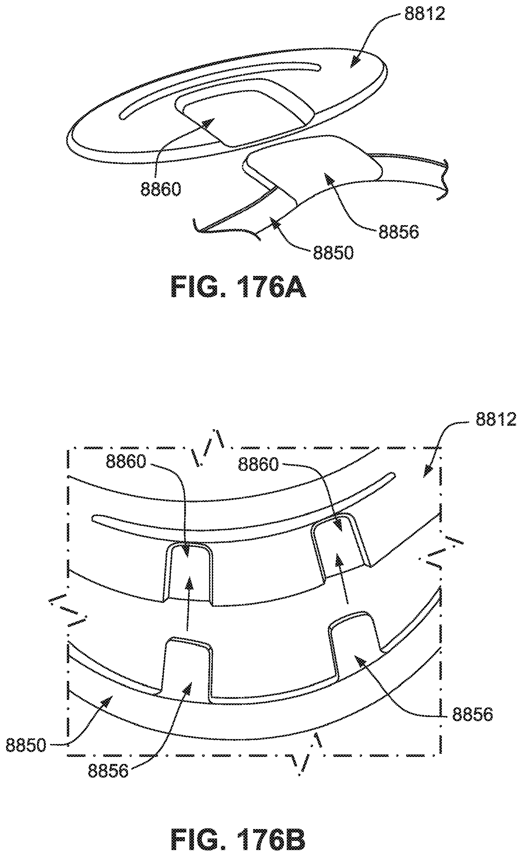

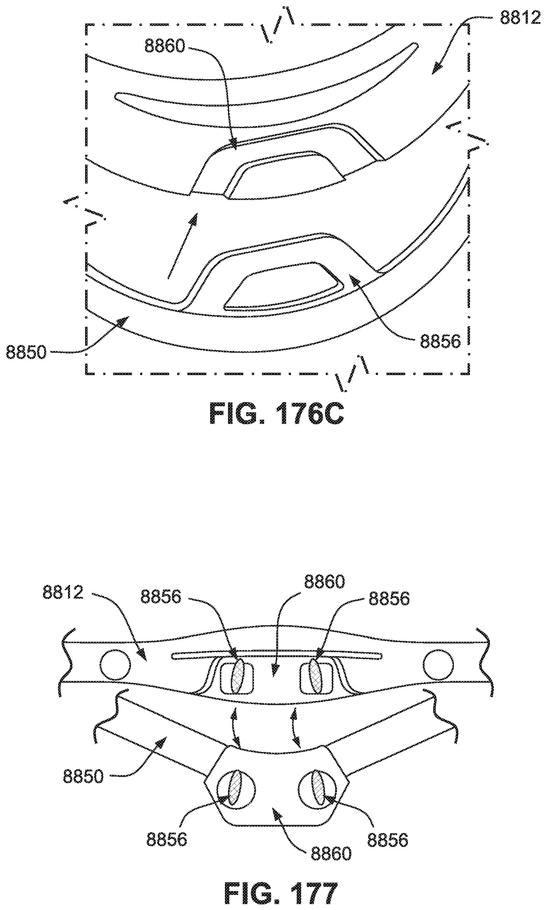

In a further example, with reference to Figures 176A to 176C, the alignment guides 8860 can take the form of one or more protrusions and/or one or more recessed portions in at least one of the nasal portion headgear 8812 and mouth portion headgear 8850. In some forms, the one or more protruding and/or recessed portions of the alignment guides 8860 can be thermoformed onto each headgear strap. The one or more protruding and/or recessed portions of the alignment guides 8860 can be formed as a single protrusion or recess (e.g. Fig 176A), as a plurality of protrusions or recesses (e.g. Fig 176B), or as a protrusion or recess that is shaped as a loop (e.g. Fig 176C) so as to correspond to the number and shape of the corresponding protrusion or recess connector/s 8856 that are adapted to fit within the alignment guides 8860 so as to be engaged thereat.

In some forms, the nasal portion headgear 8812 and mouth portion headgear 8850 can comprise a combination of a plurality of types of connectors 8856 and alignment guides 8860. For example, with reference to Fig 177, the connector 8856 can comprise a plurality of corresponding hooks and tabs that are raised on island-like embossments within an otherwise recessed alignment guide 8860 region of the nasal portion headgear 8812.

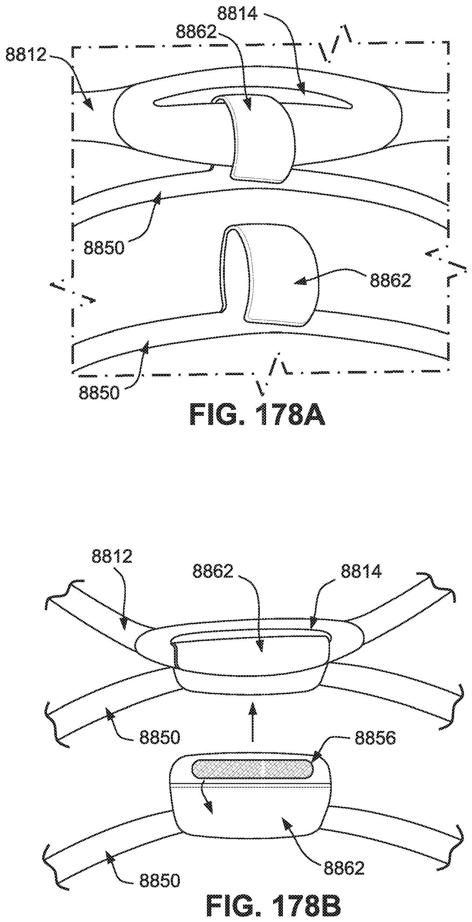

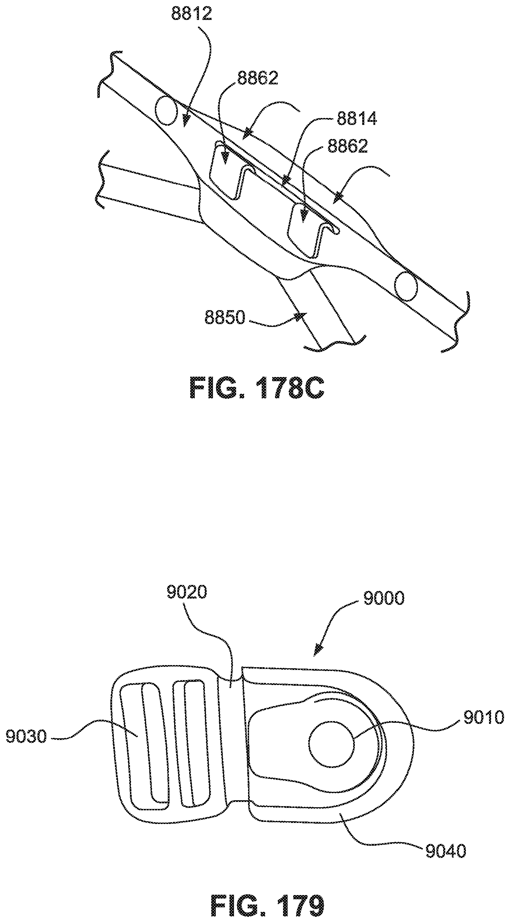

In some forms, the mouth portion headgear 8850 can comprise a connector 8856 comprising one or more flaps 8862 that extend laterally therefrom, and are adapted to be passed through a corresponding configured at, or through, the nasal portion headgear 8812. The one or more flaps 8862, once passed through the slit 8814 can be folded and secured so as to retain the nasal portion headgear 8812 and mouth portion headgear 8850 together (e.g. Fig 178A). In some forms, the folded flap 8862 can be secured to itself, or to the nasal portion headgear 8812 via the connector 8856. The one or more flaps 8862 can comprise a connector 8856 layer of Velcro, snap connectors, or other similar corresponding fastening means (e.g. Fig 178B). In some forms the flap 8862 can have a width that partially extends along the extent of the width of the slit 8814 (e.g. Fig 178A). In other forms, the flap 8862 and slit 8814 can be correspondingly sized (e.g. Fig 178B). In some other forms, a plurality of flaps 8862 can be passed through a singular slit 8814 (e.g. Fig 178C).

Also, connected nares and mouth portion headgear 870 may be utilized, which includes a connector 856 for connecting the nares only headgear and the mouth only headgear at a back portion. The connector 856 may be a Velcro connector, a hook or loop material connector, or the like. Any of the above described headgear options may be utilized with the various embodiments.

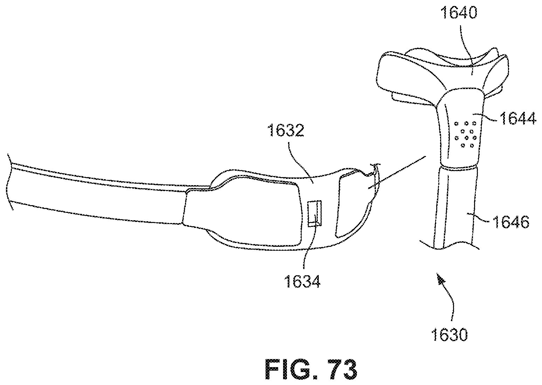

Other alternatives for allowing selective attachment and detachment are possible, see, e.g., Fig. 73 described below, which includes a back loop 1622 through which the back strap 1619 extends.

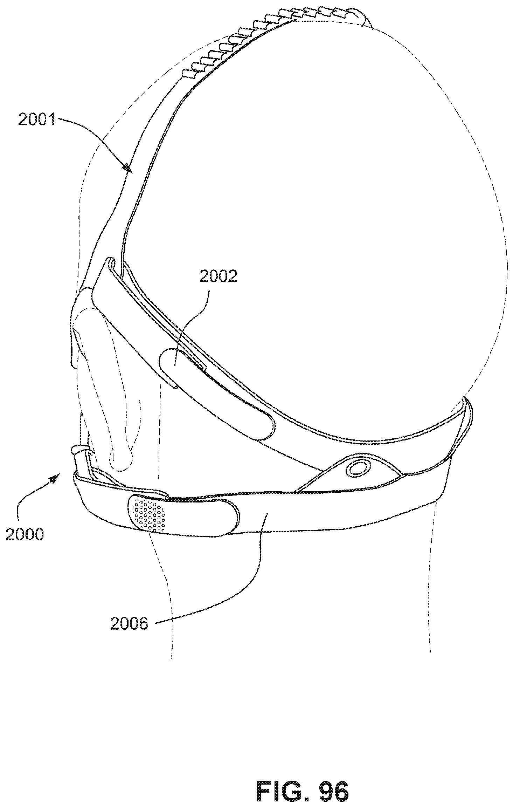

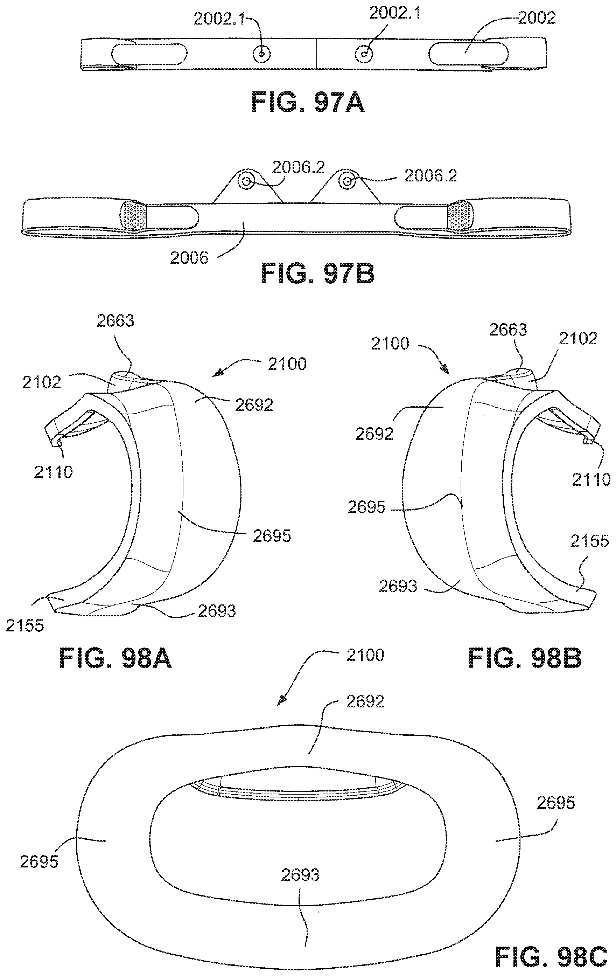

Another variant is shown in Figures 96 and 97 which show another option for converting from a two point headgear to a four point headgear. The headgear 2000 includes the two point headgear 2001, as shown for example in Figure 39 and Figure 73, which include a pair of front straps to support the patient interface. In addition, the rear of the headgear 2000 includes the first rear strap 2002 connected to the pair of front straps and a second rear strap 2006 that may be connected to the first rear strap, e.g., by a press stud arrangement or snaps.

In particular, the first rear strap 2002 may have a male part 2002.1, and the second rear strap 2006 may have a complementary/female part 2006.2, to allow selected attachment and detachment.

### 5.3.5 Vent

Embodiments of the present technology may utilize one or more vents in the patient interface system to vent gas exhaled by the patient. The vent or vents may be disposed to vent exhaled gas from the nasal portion 20, from the mouth portion 40 or from the nasal portion 20 and the mouth portion 40.

For example, a plug 3 is included on nasal portion 20 and may include a vent 6 to vent exhaled gas from the patient's nares. Another vent 6 may optionally be included on the elbow 2 connected to the mouth portion 40, to vent exhaled gas from the patient's mouth. Either of the vents 6 may be excluded, particularly if one of the nasal portion 20 or the mouth portion 40 is to be used as a sealed portion, e.g., a portion not delivering breathable gas to the patient.

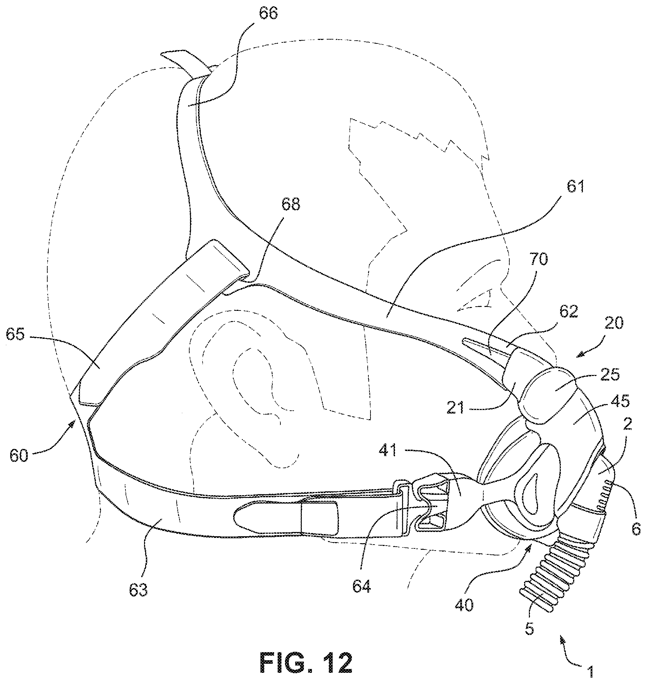

The vents 6 may be in the form of a series or array of individual vent holes. Alternatively, the vent 6 may be in the form of one or a series of slots, such as illustrated in Figs 11 and 12, or another aperture or apertures.

### 5.3.6 Air Delivery System

Air delivery in the form of pressurized, breathable gas is delivered to the patient interface system 1 through the flexible tube 5. The flexible tube 5 is coupled to a blower/humidifier or other air delivery device, which is adapted to deliver the pressurized, breathable gas to the patient to provide therapy.

### 5.3.7 Modular Patient Interface System

The patient interface system embodiments disclosed herein may be modular patient interface systems, including a nasal portion, a mouth portion and headgear. The modular patient interface systems may be adapted to allow a patient to selectively utilize the nasal portion and/or the mouth portion in a first mode and in a second mode. In the first mode, the patient may utilize both the nasal portion and the mouth portion. In the second mode, the patient may utilize only the nasal portion without utilizing the mouth portion.

Some of the embodiments may also allow the modular patient interface system to be utilized in a third mode, where the mouth portion is utilized, and the nasal portion is not utilized. In embodiments that provide the third mode, the mouth portion is utilized to provide air to the patient's mouth, while the nasal portion is not utilized and not worn by the user. In addition, in some embodiments, the nasal portion and the mouth portion may be adapted to allow air to freely flow between the chambers of the nasal portion and the mouth portion, such as an opening and/or a connector between the chambers. Some embodiments may include a plug or a selectively operated valve that may be used to plug the opening or connector to prevent air from flowing between the nasal portion and the mouth portion. The plug may also be used to plug the opening or connector when the nasal portion or the mouth portions are used alone. All of the modular patient interface systems may alternatively be formed as integrated patient interface systems, where the nasal portions are not separable and individually useable from the mouth portions.

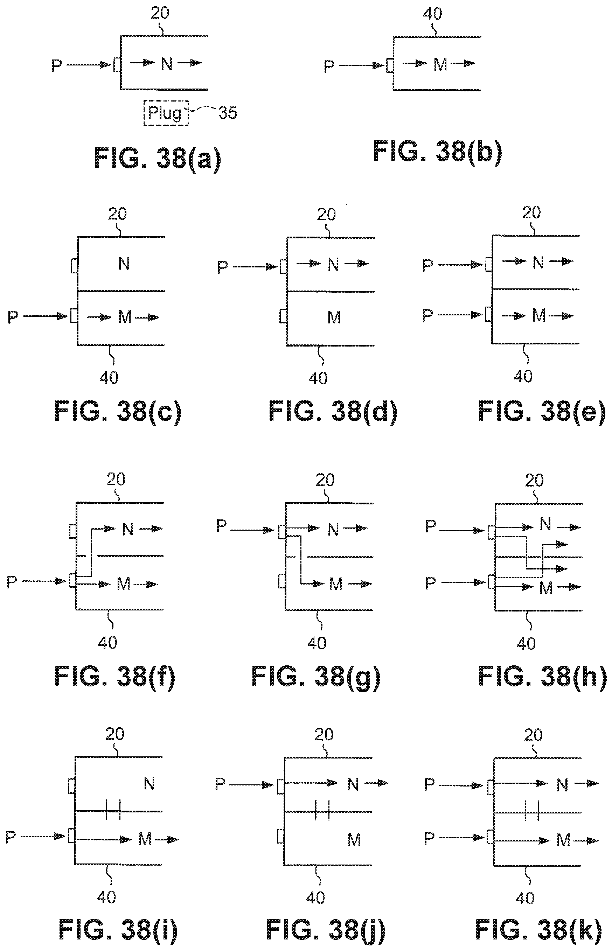

Figs. 38(a) to 38(k) are schematic illustrations of a nasal portion 20 and a mouth portion 40 utilized with the various modes. In the first mode, the patient may utilize both the nasal portion and the mouth portion, as illustrated in Figs. 38(e), 38(f), 38(g), 38(h) and 38(k), by utilizing the nasal portion 20 to deliver air to the patient's nares while utilizing the mouth portion 40 to deliver air to the patient's mouth. In Fig. 38(e), the nasal portion 20 and the mouth portion 40 do not include a connection between the chambers of the nasal portion and the mouth portion, and the pressurized air is applied separately to both the nasal portion 20, which delivers the air to the patient's nares, and to the mouth portion 40, which delivers the air to the patient's mouth. In fig. 38(f), there is a connection between the chambers of the nasal portion 20 and the mouth portion 40. The pressurized air is applied to the chamber of the mouth portion 40, and flows through the mouth portion 40 to the patient's mouth, and flows from the mouth portion 40 through the connection to the nasal portion 20 and to the patient's nares.

In Fig. 38(g), there is a connection between the chambers of the nasal portion 20 and the mouth portion 40. The pressurized air is applied to the chamber of the nasal portion 20, and flows through the nasal portion 20 to the patient's nares, and flows from the nasal portion 20 through the connection to the mouth portion 40 and to the patient's mouth. In Fig. 38(h), there is a connection between the chambers of the nasal portion 20 and the mouth portion 40. The pressurized air is applied to the chamber of the nasal portion 20 and to the chamber of the mouth portion 40, and may flow through the connection between the nasal portion 20 and the mouth portion 40. In Fig. 38(k), there is a connection between the chambers of the nasal portion 20 and the mouth portion 40, but the connection is blocked by a plug, a valve or the like. The pressurized air is applied to the chamber of the nasal portion 20 and to the chamber of the mouth portion 40, and flows through the nasal portion 20 to the patient's nares and through the mouth portion 40 to the patient's mouth.

In the first mode, the patient may also utilize both the nasal portion 20 and the mouth portion 40, as illustrated in Figs. 38(c) and 38(i) by utilizing the nasal portion 20 as a nares seal while utilizing the mouth portion 40 to deliver air to the patient's mouth. In Fig. 38(c), there is no connection between the nasal portion 20 and the mouth portion 40, and the pressurized air is delivered through the mouth portion 40 to the patient's mouth, while the nasal portion is utilized as a nares seal. In Fig. 38(i), there is a connection between the nasal portion 20 and the mouth portion 40, but the connection is blocked by a plug, a valve or the like. The pressurized air is delivered through the mouth portion 40 to the patient's mouth, and the nasal portion 20 is utilized as a nares seal.

In the first mode, the patient may utilize both the nasal portion 20 and the mouth portion 40, as illustrated in Fig 38(d) and 38(j), by utilizing the nasal portion 20 to deliver air to the patient's nares while utilizing the mouth portion 40 as a mouth seal. In Fig. 38(d), there is no connection between the nasal portion 20 and the mouth portion 40, and the pressurized air is delivered through the nasal portion 20 to the patient's mouth, while the mouth portion 40 is utilized as a mouth seal.

In the second mode, as illustrated in Fig. 38(a), the nasal portion 20 is used alone to deliver air to the patient's nares, while the mouth portion is not used and is not worn by the patient. In embodiments that utilize a connection allowing air to flow between the nasal portion and the mouth portion, when the nasal portion 20 is utilized alone, a plug, a valve or the like may be utilized to plug the opening.

In the third mode, as illustrated in Fig. 38(b), the mouth portion 40 is used alone to deliver air to the patient's mouth, while the nasal portion 20 is not used and is not worn by the patient. In embodiments that utilize a connection allowing air to flow between the nasal portion 20 and the mouth portion 40, when the mouth portion 40 is utilized alone, a plug, a valve or the like may be utilized to plug the opening.

The embodiment of Figs. 7-1 to 10 is a modular patient interface system 1, where the nasal portion 20 may be used by a patient in a nares only mode without the mouth portion 40, the mouth portion 40 may be utilized by a patient in a mouth only mode without the nasal portion 20, and the nasal portion 20 may be utilized with the mouth portion 40 in a nares and mouth mode. Figs. 7-2 and Figs. 8 to 10 illustrate the patient interface system 1 in a nares and mouth mode where the nasal portion 20 is utilized with the mouth portion 40. When utilized in the nares and mouth mode, the patient interface system 1 may utilize the nasal portion 20 to deliver air to the nares of a patient and the mouth portion 40 to deliver air to the mouth of the patient, the patient interface system 1 may utilize the nasal portion 20 to deliver air to the nares of a patient and the mouth portion 40 may be utilized as a mouth seal that does not deliver air to the mouth of the patient, or the patient interface system 1 may utilize the mouth portion 40 to deliver air to the mouth of the patient and the nasal portion 20 may be utilized as a nares seal that does not deliver air to the nares of the patient.

Both the nasal portion 20 and the mouth portion 40 are adapted to connect to a source of the pressurized, breathable gas. In particular, in the embodiments of Figs. 7-2 to 10, both the nasal portion 20 and the mouth portion 40 are adapted to connect to a source of the pressurized, breathable gas, such as flexible tube 5 via elbow 2, although only one of the portions may be connected. The flexible tube 5 may be connected to either or both of the nasal portion 20 and the mouth portion 40. A connection to both the nasal portion 20 and the mouth portion 40 may be made by utilizing a connector such as double elbow 712 illustrated in Fig.40, for example.

In the embodiments of Figs. 7-2 and Figs. 8 to 10, the nasal portion 20 and mouth portion 40 are internally connected allowing air to flow therebetween, such as by utilizing the connector 30 in the nasal portion connected to the opening 46 in the mouth portion 40, although other connections could be used. If it is desired to prevent the air flowing between the nasal portion 20 and the mouth portion 40, the connection may be block by using the plug 35 or the valve 55 activated by the knob 54.

The patient interface system 1 may be utilized where the pressurized, breathable gas is received by the patient's nares and mouth by connecting the elbow 2 and flexible tube 5 to either of the swivel connector 4 of the nasal portion 20 or the swivel connector 44 of the mouth portion 40, and the breathable gas can flow through the connector 30 and opening 46. The patient interface system 1 can also function as a mouth seal or nares seal, where the connector 30 and/or opening 46 would be blocked, by utilizing plug 35, or by activating the valve with the knob 54, and connecting only one of the nasal portion 20 or the mouth portion 40 to the elbow 2 and flexible tube 5. For example, if the elbow 2 and flexible tube 5 are connected to the nasal portion 20, and the connector 30 and/or opening 46 are blocked by utilizing plug 35 or by activating the valve 55 with the knob 54, then the pressurized air is delivered to the nasal portion 20 and the mouth portion 40 acts as a mouth seal that does not deliver the pressurized air to the patient's mouth. Alternatively, if the elbow 2 and flexible tube 5 are connected to the mouth portion 40, and the connector 30 and/or opening 46 are blocked by utilizing plug 35 or by activating the valve 55 with the knob 54, then the pressurized air is delivered to the mouth portion 40 and the nasal portion 20 acts as a nares seal that does not deliver the pressurized air to the patient's nares.

The patient interface system 1 is also adapted to function in a nares only mode utilizing the nasal portion 20 without the mouth portion 40, as illustrated in Fig. 7-1. In this mode, the mouth portion 40 and lower headgear strap 63 are not utilized, the plug 3 is removed from the nasal portion 20, and the elbow 2 and the flexible tube 5 are connected to the nasal portion 20 to deliver the pressurized, breathable gas to the patient's nares. The connector 30 is plugged with plug 35.

The patient interface system 1 is also adapted to function in a mouth only mode utilizing the mouth portion 40 without the nasal portion 20, as illustrated in Fig. 7-4. In this mode, the nasal portion 20 along with the headgear strap 61 are not utilized, and the elbow 2 and the flexible tube 5 are connected via the swivel connector 44 to the mouth portion 40 to deliver the pressurized, breathable gas to the patient's mouth. The opening 46 on the mouth portion is plugged with the plug 35.

Figs. 11-1 to 19 illustrate another embodiment of a patient interface system 1 that may be a modular patient interface system. Figs. 11-1 and 12-19 illustrate the modular patient interface system 1 in a nares and mouth mode. The flexible tube 5 is connected via the swivel ring 4 to the elbow 2, to direct the pressurized, breathable gas to the chamber of the mouth portion 40 and to the nasal portion 20 via the nasal portion connector 43.

The decoupling portion 45 is connected between the swivel ring 4 and both the nasal portion 20 and the mouth portion 40. In addition, the nasal portion 20 also may include the decoupling portion 25, such that the nasal portion benefits from two decoupling portions to decouple forces or movements applied to the flexible tube 5, the elbow 2, and/or the swivel ring 4 from being transferred to the sealing portion 22 of the nasal portion 20.

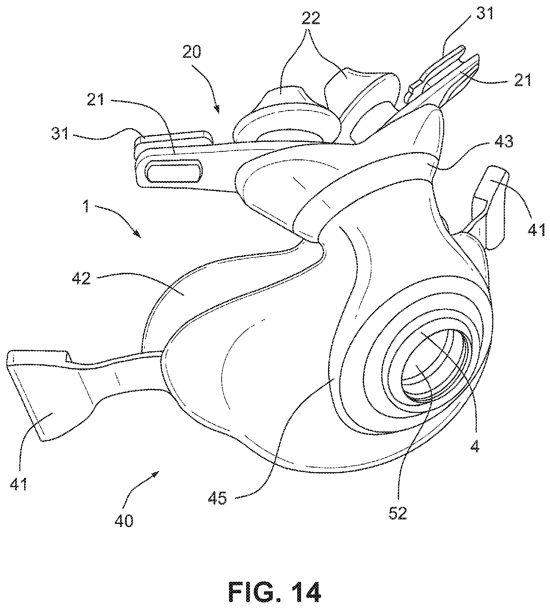

In the embodiments of Figs. 11-1 to 19, the nares sealing portion 22 may be in the form of nasal pillows such as illustrated in Figs. 14, 15, 17 and 18, or may be in the form of prongs, a membrane seal such as a nasal cradle, and/or a nasal chamber. For example, the nasal portion of Figs. 11-19 may be in the form of the nasal portion 220 illustrated in Figs. 25 and 26. The supporting portion 252 may or may not be included. When utilizing the nasal portion 220, the sealing portion 222 sealing with the patient's nares is structured to extend or curve outwardly from a supporting wall 221 defining an air path through the sealing portion 222, and forms a seal with the nose tip, upper lip and nares of the patient.

As illustrated in Fig. 11-2, the patient interface system may be utilized in a nares only mode. In this mode, the patient interface system is utilized with the nasal portion 20, and the mouth portion 40 is not utilized by the patient. To convert the patient interface system from the nares and mouth mode to the nares only mode, the swivel ring 4, the elbow 2 and the flexible tube 5 are removed from mouth portion 40, the mouth portion 40 is removed from connection with the nasal portion 20 by disconnecting the nasal portion connector 43 from the nasal portion 20, and the swivel ring 4, the elbow 2 and the flexible tube 5 are connected to the nasal portion. While the headgear 60 illustrated is shown with a connection region 67, the connection region 67 may be omitted to facilitate usage of the patient interface system 1 in the nares only configuration without the lower headgear strap 63.

Figs. 27 and 28 illustrate a patient interface system 301 in accordance with an embodiment of the present technology. The patient interface system 301 is adapted to be utilized in a nares only mode, in a mouth only mode, or in a nares and mouth mode. The patient interface system 301 includes a nasal portion 320, a mouth portion 340 and headgear 360, connector 306, optional swivel connector 308 and flexible tube 305. The connector 306 may be in the form of an elbow or other type of connector.

The nasal portion 320 includes nares sealing portion 322, headgear connectors 321 for connecting to headgear 360, decoupling portion 325 and opening 326. The nares sealing portion 322 may be in the form of pillows, prongs, a nasal chamber, or a membrane seal, and may be in the form of nares sealing portion 222 illustrated in Figs. 25 and 26. The mouth portion 340 includes decoupling portion 345, opening 348 and connector 349. The connector 306 is adapted to connect to either opening 326 in the nasal portion 320 or opening 348 in the mouth portion 340.

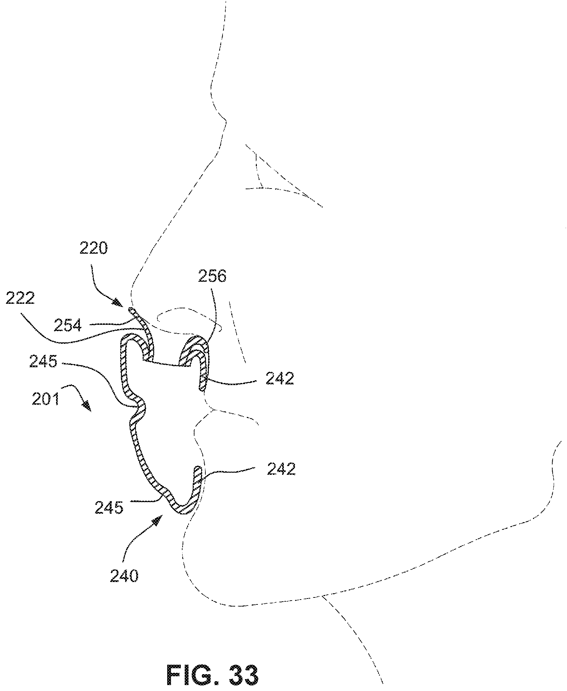

As illustrated in Fig. 33, the patient interface system 301 is adapted to be used by a patient in a nares only mode without the mouth portion 340. Connector 306 is connected to the opening 326 in the nasal portion 320. The optional swivel connector 308 may be utilized between the connector 306 and the flexible tube 305, which delivers the pressurized, breathable gas to the nasal portion 320.

As illustrated in Fig. 28, the nares only configuration of Fig. 27 may be converted into a nares and mouth configuration by removing the connector 306 from opening 326 in the nasal portion 320, connecting connector 349 of the mouth portion to opening 326, and connecting connector 306 to opening 348 in the mouth portion 340. In the nares and mouth configuration, the nasal portion may receive the pressurized, breathable gas through the connector 349 and the opening 326. Alternatively, the nasal portion 320 may be used as a nares seal by placing a plug in the connector 349, so that the pressurized, breathable air is not delivered to the nasal portion 320.

The mouth portion 340 may also be utilized without the nasal portion 320 in a mouth only configuration. In the mouth only configuration, the nasal portion 320 is not utilized, and a plug is placed in connector 349 to provide a seal.

Fig. 30 illustrates a modular patient interface system 401 in accordance with an embodiment of the present technology. The patient interface system 401 includes a nasal portion 420, a mouth portion 440, and headgear 460.

The nasal portion 420 includes a nares sealing portion 422 for forming a seal with the patient's nares, and headgear connectors 421 adapted to connect to headgear 460. The nasal portion 420 is illustrated with a nares sealing portion 422 such as the type illustrated in Figs. 25 and 26, although other nares sealing portions could be utilized, such as pillows, prongs, a nasal chamber, etc. An optional decoupling portion 425 may be included for decoupling any forces applied to the nasal portion 420. The headgear 460 may be the same as the headgear 60 illustrated in Figs. 7-1 to 10.

The mouth portion 440 includes a mouth sealing portion 442, headgear connectors 441 for connecting to headgear 460, and swivel connector 444. The mouth portion may also include an optional decoupling portion 445. The swivel connector 444 may connect to elbow 402 and flexible hose 405, which delivers pressurized, breathable gas to the patient interface system 401. The patient interface system 401 could alternatively be formed as a modular patient interface system, where the mouth portion 440 is separable from the nasal portion 420 and both portion are individually useable, and the flexible tube 405 is connectable via the swivel connector 444 or the like to the nasal portion 420. A flexible portion 443 may be disposed between the decoupling portion 425 and the decoupling portion 445. The flexible portion 443 may be a gusset or collapsible portion between the mouth portion 440 and the nasal portion 420, which allows the nasal portion 420 to adopt the nasolabial angle (the angle between the septum of the nose and the top lip of the patient).

Fig. 31 illustrates a modular patient interface system 501 in accordance with an embodiment of the present technology. The patient interface system 501 includes a nasal portion 520, a mouth portion 540, and headgear 560.

The nasal portion 520 includes nares sealing portion 522 adapted to form a seal with the nares of the patient, and headgear connectors 521 for connecting to headgear 560. The nasal portion 520 is illustrated with a nares sealing portion 522 such as the type illustrated in Figs. 25 and 26, although other nares sealing portions could be utilized, such as pillows, prongs, a nasal chamber, etc. An optional decoupling portion 525 may be included for decoupling any forces applied to the nasal portion 520. The headgear 560 may be the same as the headgear 60 illustrated in Figs. 7-1 to 11.

The mouth portion 540 includes a mouth sealing portion 542, headgear connectors 541 for connecting to headgear 560, swivel connector 544 and foam sealing or patient contacting portion 569. The foam patient contacting portion 569 may be a wedge utilized as at least a part of a portion of the mouth seal. The mouth portion may 540 also include an optional decoupling portion 545. The swivel connector 544 may connect to elbow 502 and flexible hose 505, which delivers pressurized, breathable gas to the patient interface system 501. The patient interface system 501 could alternatively be formed as an integrated patient interface system, where the mouth portion 540 is separable from the nasal portion 520 and both portions are individually useable, and the flexible tube 505 is connectable via the swivel connector 544 or the like to the nasal portion 420.

Fig. 40 illustrates another modular patient interface system 701. The patient interface system 701 includes a nasal portion 720, a mouth portion 740, and headgear 760. In this embodiment, the nasal portion 720 is in a modular connection with the mouth portion 740, so that the nasal portion 720 may be utilized without the mouth portion 740 in a first nares only mode, the mouth portion 740 may be utilized without the nasal portion in a second mouth only mode, or the nasal portion 720 may be utilized with the mouth portion 740 in a third nares and mouth mode.

The nasal portion 720 may include a sealing portion 722 adapted to form a seal with the patient's nares, an optional decoupling portion 725, and headgear connectors 721 for connecting to headgear 760. A swivel ring or other connector 710 may also be utilized. The sealing portion 722 may be in the form of pillows, prongs, a nasal chamber or a nares sealing portion, such as the membrane type illustrated in Figs. 25 and 26.

The mouth portion 740 may include a mouth sealing portion 742, a decoupling portion 745 and headgear connectors 741 for connecting to headgear 760. A swivel ring or other connector 744 may also be used.

In a mouth and nares mode such as illustrated in Fig. 40, the nasal portion 720 and the mouth portion 740 are both utilized and connected to flexible tube 705 through double elbow 712. Double elbow 712 includes two elbow joints so that it may connect to both the nasal portion 720 and the mouth portion 740 to deliver the pressurized, breathable air to the patient's nares and mouth. The nasal portion 720 and the mouth portion 740 may or may not be connected pneumatically.

In a nares only mode, only the nasal portion 720 is utilized and the mouth portion 740 is not utilized. In this mode, the double elbow 712 is removed and the mouth portion 740 is removed. The patient utilizes the nasal portion 720 only, and connects the tube 705 to the swivel ring or other connector 710. An elbow such as elbow 2 may be utilized to connect the tube 705 to the swivel ring or other connector 710.

In a mouth only mode, only the mouth portion 740 is utilized and the nasal portion 720 is not utilized. In this mode, the double elbow 712 is removed and the nasal portion 720 is removed. The patient utilizes the mouth portion 740 only, and connects the tube 705 to the swivel ring or other connector 744. An elbow such as elbow 2 may be utilized to connect the tube 705 to the swivel ring or other connector 744.

Fig. 32 illustrates a schematic cross-section view of a patient interface system 1 utilized in a nares and mouth mode. In this embodiment, the mouth portion 40 may include a sealing ring 51 adapted to connect and seal the mouth portion 40 to the nasal portion 20. Alternatively, the sealing ring 51 may be formed as part of the nasal portion 20 and connect and seal with the mouth portion 40. Further, other types of connectors could be utilized or the nasal portion 20 and the mouth portion 40 could be formed as a unitary element so that a sealing ring or other type of connector would not be needed.

In Fig. 32, the flexible tube 5 is connected to the mouth portion 40 via a gusset 8 and an optional inline AAV (anti-asphyxia valve) adaptor 10, and does not utilize the elbow 2 and swivel connector 4. This embodiment presents a very streamlined appearance by moving the flexible tube 5 away from the front of the patient's face.

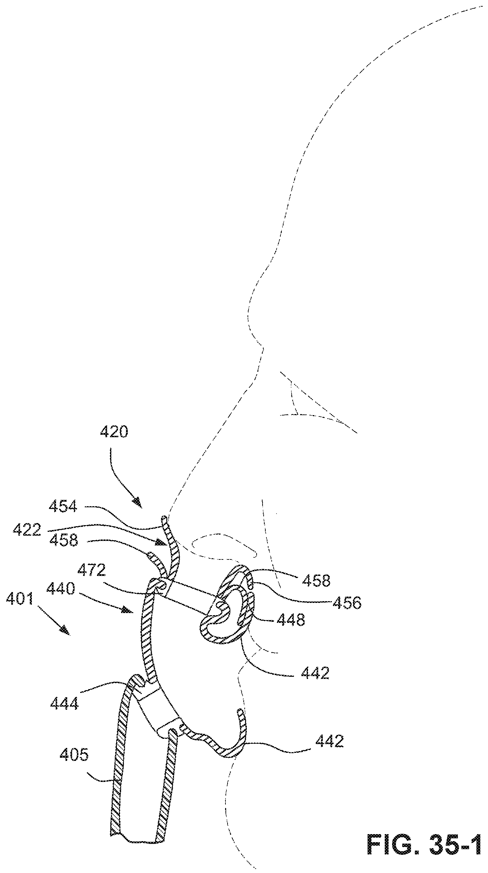

Fig. 35-1 illustrates a schematic cross-section view of a patient interface system 401 utilized in a nares and mouth mode. The nasal portion 420 may include a supporting portion 458 and a sealing portion 422. The sealing portion 422 includes a nose tip engagement portion 454 that engages and seals with the patient's nose tip, an upper lip engagement portion 456 that engages and seals with the patient's upper lip area, and a sealing ring 472 that engages and seals with the mouth portion 440. The sealing ring 472 may optionally be part of the mouth portion 440, instead of being part of the nasal portion 420.

Mouth portion 440 may include swivel ring 444, which connects to flexible tube 405. The flexible tube 405 may connect directly to the swivel ring 444, or may be connected through an elbow, such as elbow 402. The mouth sealing portion 442 forms a seal around the patient's mouth and includes an upper lip engagement portion 448 that forms a seal with the patient's upper lip area.

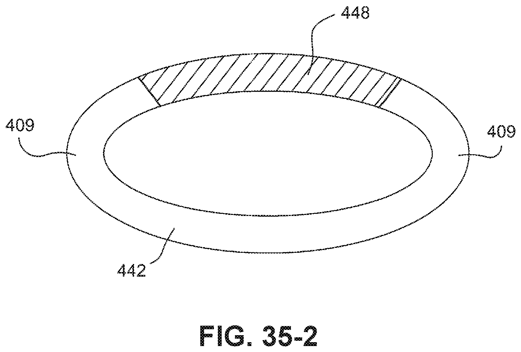

There is a limited amount of room on the patient's upper lip for the upper lip engagement portion 456 of the nasal portion 420 and for the upper lip engagement portion 448 of the mouth portion. Accordingly, the upper lip engagement portion 448 may be disposed between the support portion 458 of the nasal portion 420 and the patient's upper lip. Fig 35-2 illustrates the mouth sealing portion 442, with the upper lip engagement portion 448. The upper lip engagement portion 448 may be an ultra thin flat membrane having a thickness of about 0.1 to 0.3mm, preferably about 0.2mm, as compared to the rest of the mouth sealing portion 442, which may have a thickness of about 0.3 to 2mm, preferably about 0.5mm. However, portions of the mouth sealing portion 442 could include thickened portions 409 around the sides of the mouth region corresponding to a cheek area of a patient for added support. These thickened regions 409 could have a thickness of about 1.5mm, for example.

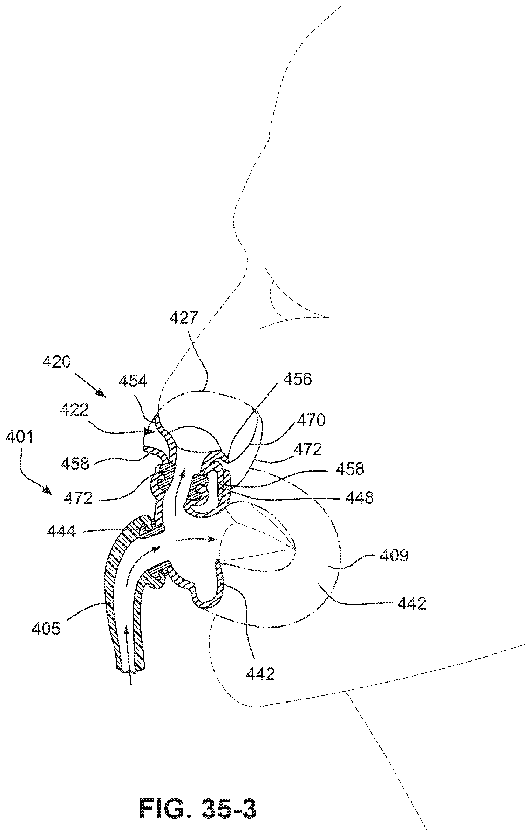

Fig. 35-3 is a schematic cross-sectional view that illustrates further details of the patient interface system 401. In particular, Fig. 35-3 illustrates how the mouth sealing portion 442 extends around the mouth of the patient and how the nares flare engagement portion 427 engages with the flares of the patient's nares. Additionally, the back edge of the nasal sealing area 470 is illustrated along with an edge of the nares sealing portion 422.

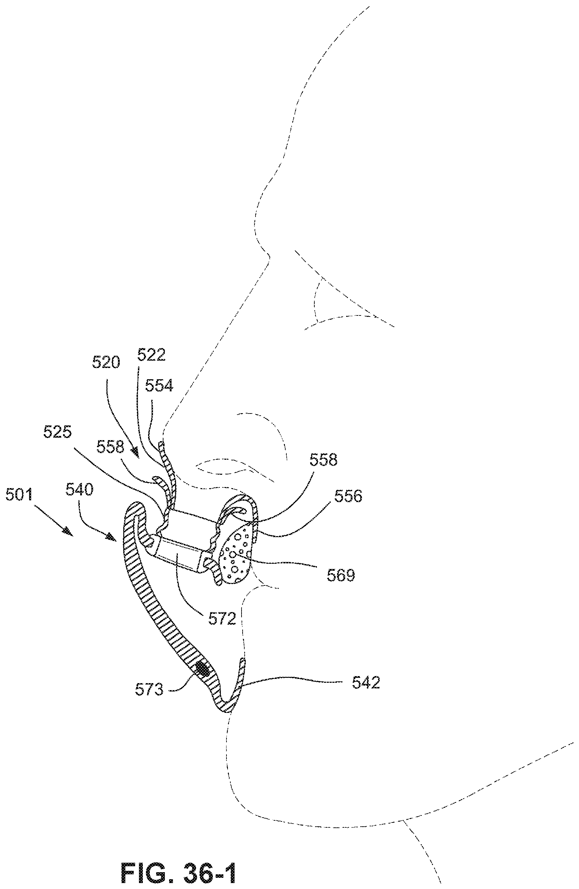

Fig. 36-1 illustrates a schematic cross-sectional view of a patient interface system 501 utilized in a nares and mouth mode. The nasal portion 520 may include a supporting portion 558, a sealing portion 522 and an optional decoupling portion 525. The sealing portion 522 includes a nose tip engagement portion 554 that engages and seals with the patient's nose tip, and an upper lip engagement portion 556 that engages and seals with the patient's upper lip area. A sealing ring 572 that engages and seals with the mouth portion 540 may be attached to the decoupling portion. The sealing ring 572 may optionally be part of the mouth portion 540 instead of being part of the nasal portion 520.

The mouth portion 540 may include an optional cushion rigidizer 573 to increase the rigidity of the sealing portion 522. The cushion rigidizer 573 may be made from a polymer such as polycarbonate, nylon, polypropylene, high density foam, a fabric having low stretch properties like a 3D weave, or a higher shore hardness silicone or gel, e.g. 70 Shore A. There is a limited amount of room on the patient's upper lip for the upper lip engagement portion 556 of the nasal portion and for an upper lip engagement portion of the mouth portion. Accordingly, the foam patient contacting 569 may be disposed in a gap between the cushion on the mouth portion 540 and the upper lip engagement portion 556. The foam patient contacting 569 may contact with and form a seal with the upper lip area of the patient, and thus form part of the mouth seal.

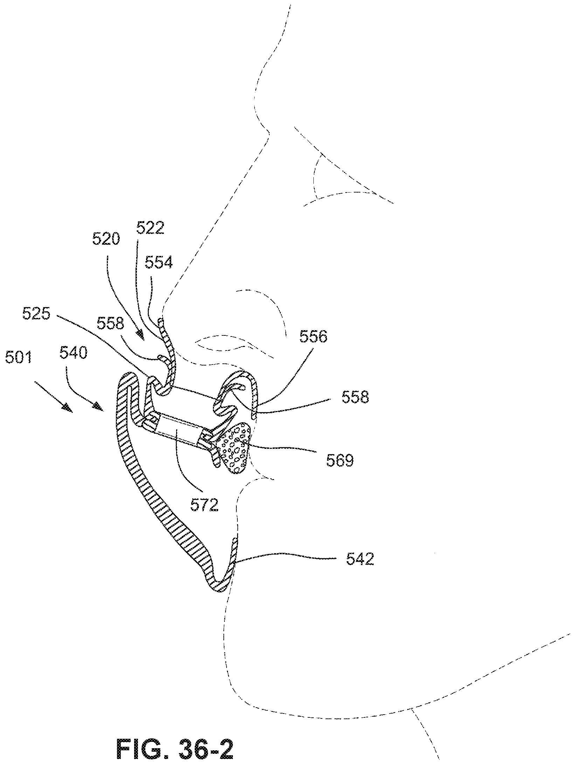

Fig. 36-2 illustrates a schematic cross-sectional view of the patient interface system 501 utilized in the second nares and mouth mode. The sealing ring 572 is formed as part of the mouth portion 540 and is adapted to engage and seal with the decoupling portion 525 of the nasal portion 520. There is a limited amount of room on the patient's upper lip for the upper lip engagement portion 556 of the nasal portion and for an upper lip engagement portion of the mouth portion. In this embodiment, the foam patient contacting portion 569 is disposed against the decupling portion 525 and the cushion on the mouth portion 540, and contacts with and forms a seal with the upper lip area of the patient. The foam patient contacting portion 569 may form part of the mouth seal. The cushion rigidizer 573 of Fig. 36-1 may also be used in the embodiment of Fig. 36-2 and in any of the other embodiments disclosed herein.

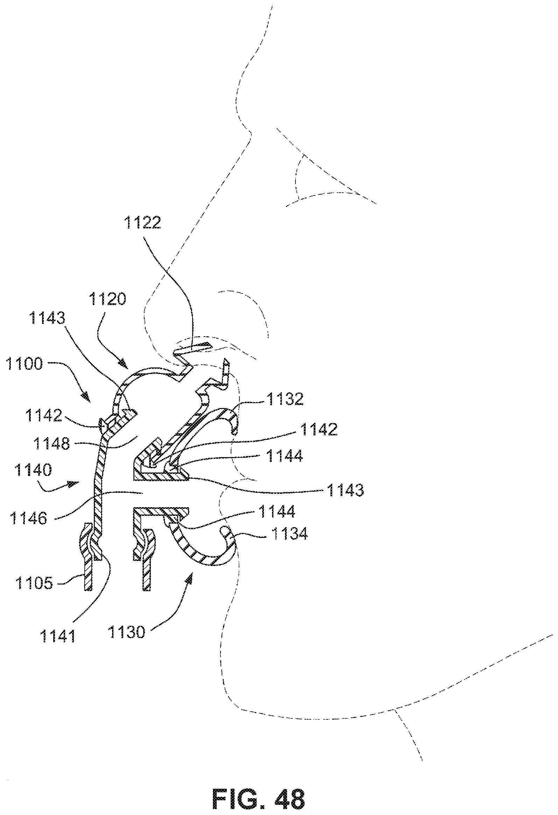

Fig. 48 illustrates a schematic cross-section view of a patient interface system 1100 utilized in a nares and mouth mode. The patient interface system 1100 may be a variation of the patient interface system 701 illustrated in Fig. 40. The patient interface system 1100 includes a nasal portion 1120, a mouth portion 1130, and a double elbow 1140. A lower end 1141 of the double elbow 1140 is adapted to connect to swivel 1105, which may be connected to a flexible tube to deliver air to the patient interface system 1100. Lower end 1141 may connect to swivel 1105 with a ball and socket arrangement (as shown) adapted to provide a greater degree of movement of the swivel and therefore the air delivery tube. Alternatively, lower end 1141 may connect to swivel 1105 in any arrangement for example an interference fit, over mold, etc.

A first air passage or branch 1146 of the double elbow 1140 is adapted to communicate with an aperture in mouth portion 1130, and a second air passage or branch 1148 of the double elbow 1140 is adapted to communicate with an aperture in nasal portion 1120. A first elbow ring 1142 and a second elbow ring 1144 are used to connect the double elbow 1140 to the nasal portion 1120 and the mouth portion 1130, respectively, although other types of connectors may be utilized. The double elbow may include barbed end portions 1143 on the ends of the first branch 1146 and the second branch 1148 to engage with the mouth portion 1130 and the nasal portion 1120.

The mouth portion 1130 includes a lower lip engagement portion 1134 adapted to form a seal below the patient's lower lip, and an upper lip engagement portion 1132 adapted to form a seal just above the patient's upper lip. The nasal portion 1120 includes nozzles 1122 adapted to locate the nasal portion 1120 proximal to the patient's nares and ensure the foam portion 1154 is located in a position to sealingly engage the patient's nares, although pillows, a nasal chamber, a nasal cradle (nares seal) or a membrane seal could also be used to form a seal with the patient's nares.

The mouth portion 1130 may be a single wall or fabric/foam composition mouth portion with a relatively low Shore hardness. The nasal portion 1120 may have a hardness of e.g., 40 Shore A.

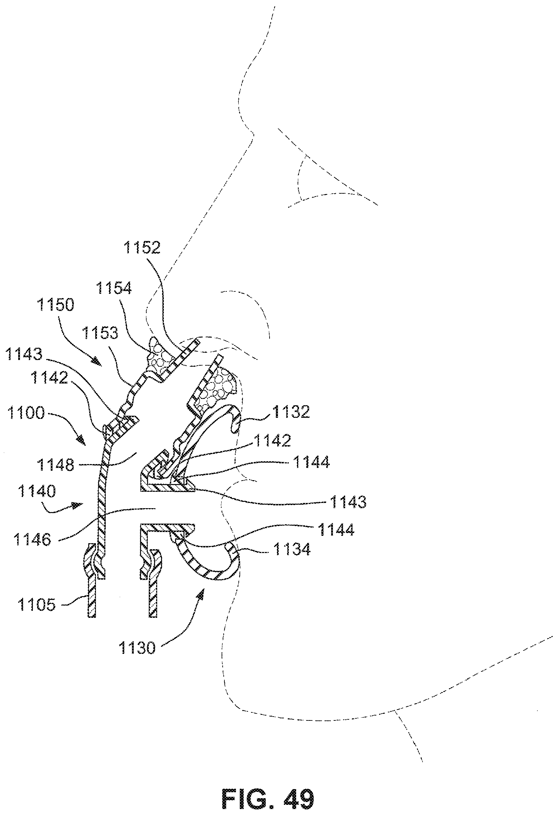

Fig. 49 illustrates a schematic cross-section view of the patient interface system 1100 utilized in a nares and mouth mode, and is the same as the patient interface system 1100 of Fig. 48 with a different nasal portion1150. Nasal portion 1150 includes a supporting portion 1153 that supports nozzles 1152, and foam portion 1154. The nozzles 1152 and foam portion 1154 together form a sealing portion adapted to form a seal with the patient's nares. The nozzles 1152 may have an air passage that gradually decreases in size as the air approaches the patient's nares. The supporting portion 1153 may have a hardness of e.g., 40 Shore A. The nozzles 1152 aid in supporting the foam portion 1154 in position proximate to the patient's nares. The foam portion 1154 enables a larger fit range as the foam is able to conform to various nares sizes due to the compressibility of the foam. The foam portion 1154 also provides comfort to the patient.

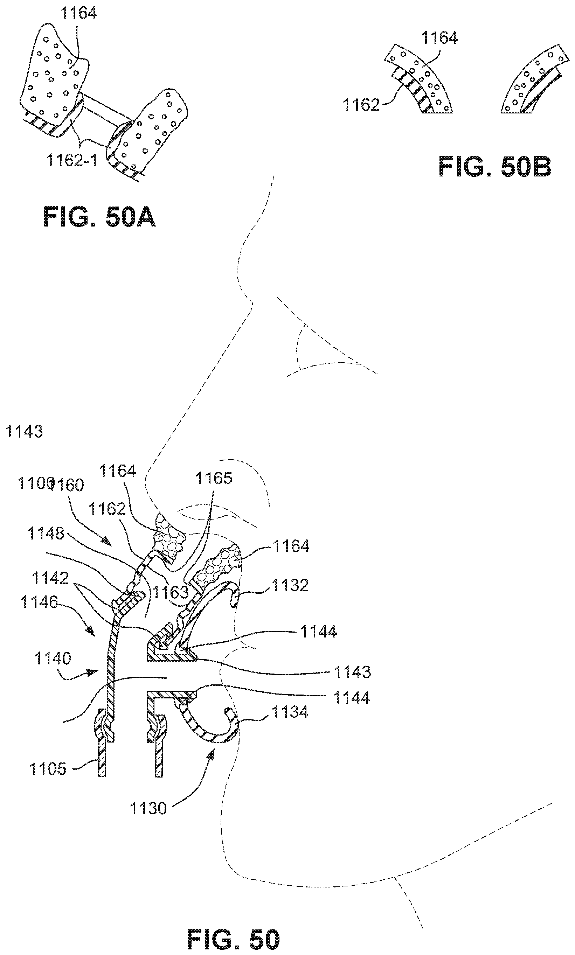

Fig. 50 illustrates a schematic cross-section view of the patient interface system 1100 utilized in a nares and mouth mode, with a nasal portion 1160. Nasal portion 1160 includes a supporting portion 1162 and a sealing portion 1164. The supporting portion 1162 may be constructed of silicone and acts as an intermediate or connecting portion between the double elbow 1140 and the sealing portion 1164.

The supporting portion 1162 positions the sealing portion 1164 underneath the nares of the user and maintains the sealing portion 1164 in this position. The supporting portion 1162 may include substantially parallel side portions and end portions 1165 formed substantially perpendicular to the side portions. The end portions 1165 provide an interface surface for the sealing portions 1164. The sealing portion 1164 may be constructed of foam, gel or other conformable material. Preferably the sealing portion 1164 is more flexible than the supporting portion 1162 so as to aid in the comfort of the seal and the fit range. The sealing portion 1164 may be foam with an aperture, the foam forming a seal with the patient's nose tip, upper lip and nares. In an example, as shown in Fig. 50A, the supporting portion may include support walls 1162-1 (e.g., constructed of silicone or a material with higher stiffness than the foam sealing portion) to prevent the foam from compressing and occluding the nares. In another example, as shown in Fig. 50B, the foam sealing portion 1164 may have a trumpet shape (e.g., flare outwards) with a silicone support wall underneath to ensure structural stability of the foam and also ensure sealing engagement with the patient.

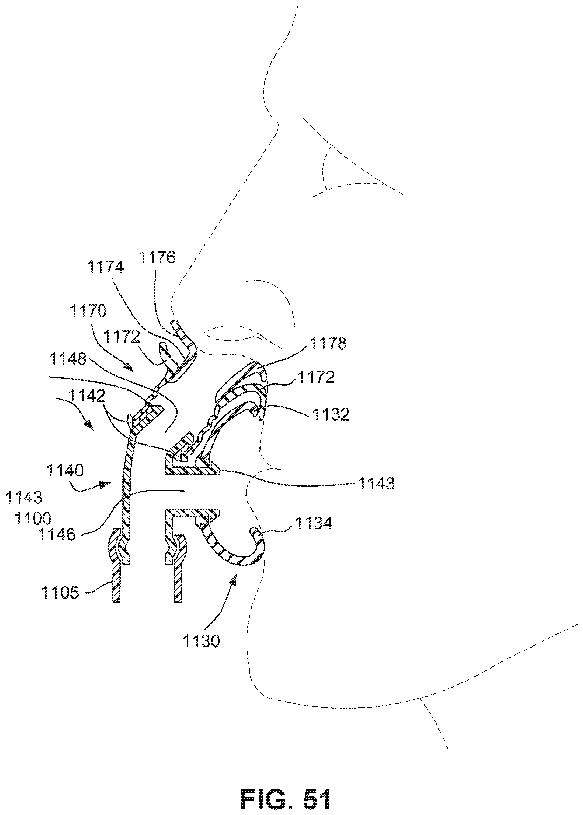

Fig. 51 illustrates a schematic cross-section view of the patient interface system 1100 utilized in a nares and mouth mode, with a nasal portion 1170. The nasal portion 1170 includes a supporting portion 1172 and a sealing portion 1174. The sealing portion 1174 includes a nose tip engagement portion 1176 and an upper lip engagement portion 1178. The supporting portion 1172 may form a seal just above the patient's upper lip, and may be positioned between the patient's upper lip and the upper lip engagement portion 1132 of the mouth portion 1130.

The supporting portion 1172 may have a higher Shore hardness than the sealing portion 1174. For example, the supporting portion may have a Shore A hardness of, e.g., 20-80, preferably 30-60, or about 40 while the sealing portion 1174 has a Shore A hardness of about 5-40 or about 20. The hardness of the sealing portion is generally lower than the supporting portion.

Fig. 52 illustrates a schematic cross-section view of a patient interface system 1180 utilized in a nares and mouth mode, with mouth portion 1182 and a nasal portion 1190. The nasal portion 1190 may be removable from the patient interface system 1180 and replaceable with a plug, so that the patient interface system 1180 may be used in a mouth only mode.

The mouth portion 1182 includes an aperture for connection of an air supply to deliver air to the patient interface system 1180. Elbow 1188 connects to the aperture via sealing ring 1189. A swivel 1187 may be connected between the elbow 1188 and the air hose 1185. A swivel cuff (not shown) may be connected between the swivel 1187 and air hose 1185.

The mouth portion 1182 includes a lower lip engagement portion 1184 adapted to form a seal below the patient's lower lip, an upper lip engagement portion 1186 adapted to form a seal just above the patient's upper lip, and a vanity flap to cover nares and/or nose tip - primarily to cover nose tip - or covering flap 1183 adapted to cover the patient's nares and the nasal portion 1190, making the patient interface system 1180 more visually appealing. The covering flap 1183 extends upward a sufficient height to cover the patient's nares and the nasal portion 1190.

The nasal portion 1190 may include supporting portion 1192 and sealing portion 1193. The sealing portion includes nose tip engagement portion 1194 and upper lip engagement portion 1196. The nasal portion 1190 may be connected to an upper aperture in the mouth portion 1182 via a mouth clip 1198 and a nares clip 1198.1, into which the nasal portion 1190 may be placed. When the nasal portion 1190 is not to be used with the patient interface system 1180, the nasal portion 1190 is removed from the mouth clip 1198, and a plug may be put in the mouth clip 1198 or in the aperture to provide a seal. The mouth clip 1198 may be silicone or another material, e.g., nylon, polypropylene, etc.

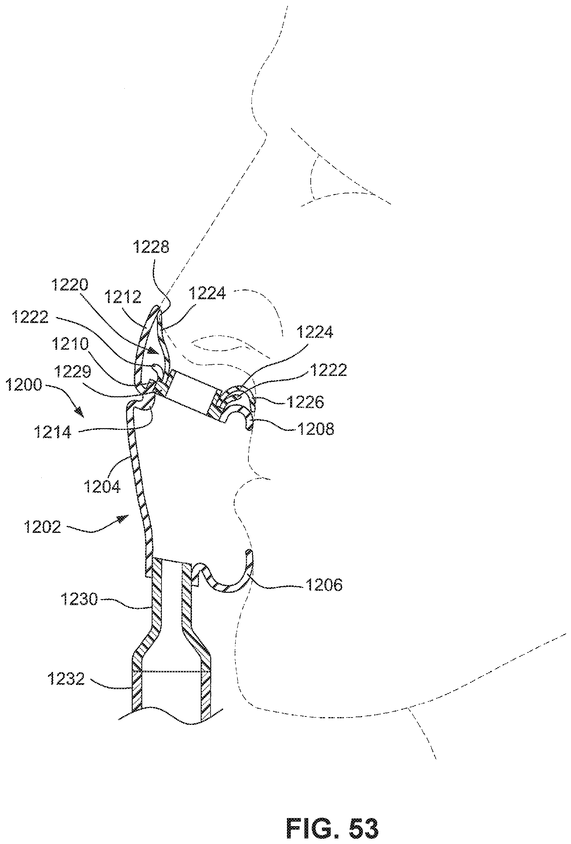

Fig. 53 illustrates a schematic cross-section view of the patient interface system 1200 utilized in a nares and mouth mode, with mouth portion 1202 and a nasal portion 1220. The nasal portion 1220 may be removable from the patient interface system 1200 and replaceable with a plug, so that the patient interface system 1200 may be used in a mouth only mode and the nasal portion may be used in a nares only mode.

The mouth portion 1202 includes a front portion 1204 or fascia, a lower lip engagement portion 1206, an upper lip engagement portion 1208, a nasal portion tab 1210, vanity flap or covering flap 1212. and a decoupling portion 1214. The front portion or fascia 1204 may be a large clear window allowing visibility of the patient's mouth region. The nasal portion tab 1210 may be adapted to receive and sealingly engage with the nasal portion 1220.

The nasal portion 1220 includes an attachment ring 1229 adapted to sealingly engage with the mouth portion 1202, a supporting portion 1222, and a sealing portion 1224. The attachment ring 1229 may be co-molded, welded, permanently snapped or removably attached with the rest of the nasal portion 1220. The sealing portion 1224 includes a nose tip engagement portion 1228 adapted to form a seal with the patient's nose tip, and an upper lip engagement portion 1226 adapted to form a seal just above the patient's upper lip.

The covering flap 1212 is located in close proximity with the nose tip engagement portion 1228 of the nasal portion 1220. Thus, if the covering flap 1212 moves, the nose tip engagement portion 1228 may also move. Accordingly, the decoupling portion 1214 is included on the mouth portion 1202 at a position below the covering flap 1212. The decoupling portion 1214 serves to decouple forces applied to the mouth portion 1202 (such as could be created by forces applied by movement of the air hose 1232) from being transmitted to the nasal portion 1220.

The mouth portion 1202 includes a lower aperture adapted to receive elbow 1230, e.g., gusset elbow, which is connected to air delivery tube 1232. The gusset elbow 1230 may include an anti-asphyxia valve and/or one or more vent holes for venting exhaled gases. The tube may connect to the nose cushion or pillows when in the nose-only mode.

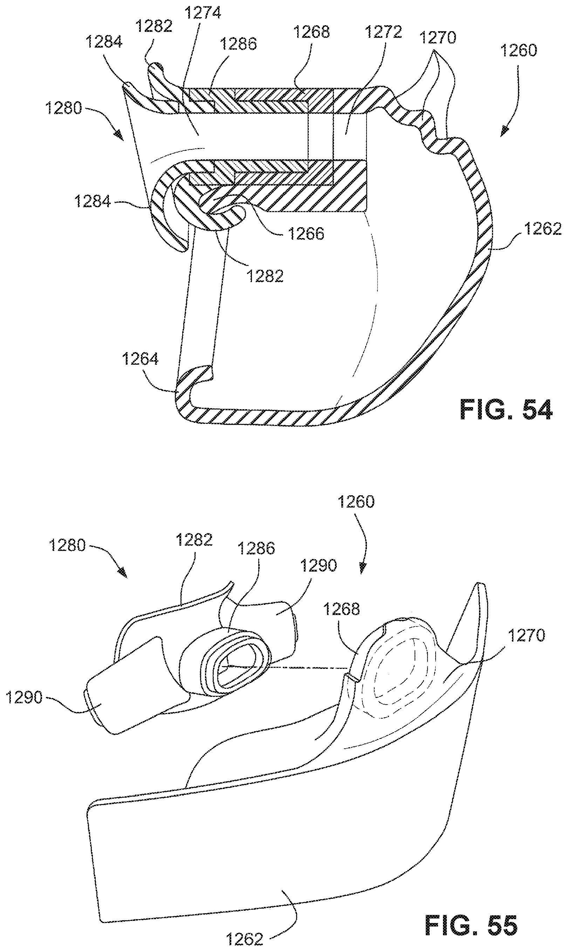

Figs. 54 and 55 illustrate a modular patient interface system 1260, which includes a mouth portion 1262 and a nasal portion 1280. The patient interface system 1260 is adapted to be used in a mouth only mode, a nares only mode or a mouth and nares mode.

The mouth portion 1262 includes a lower lip engagement portion 1264, an upper lip engagement portion 1266 adapted to push nasal seal into engagement with the face, a decoupling (concertina) portion 1270 and aperture 1272. An aperture (not shown) on the front or bottom of the mouth portion may be provided to connect a supply of air, such as air hose connected by an elbow or the like. The flexible portion 1270 could be air actuated or could be a thinner wall section, e.g., less stiff, and forms a decoupling portion that decouples forces applied to the mouth portion 1262 from being applied to the nasal portion 1280. When used in a mouth only mode, the aperture 1272 may be plugged to provide an airtight seal.

The nasal portion 1280 includes a supporting portion 1282, a sealing portion 1284 and headgear connectors 1290. The sealing portion 1284 is adapted to seal with the patient's upper lip and nose tip. When used in a nares only mode, the aperture 1274 can be connected to a source of air or plugged if air is alternatively supplied. When used in a nares and mouth mode, a nares clip 1286 may be connected, preferably permanently connected or otherwise form into the supporting portion 1282, and a mouth clip 1268 may be connected, preferably permanently connected or otherwise form into the cushion of the mouth portion 1262. The nares clip 1286 and the mouth clip 1268 are adapted to sealingly connect, such as through notches or the like formed in the clips, so that the mouth portion 1262 may be connected to the nasal portion 1280 in a nares and mouth mode.

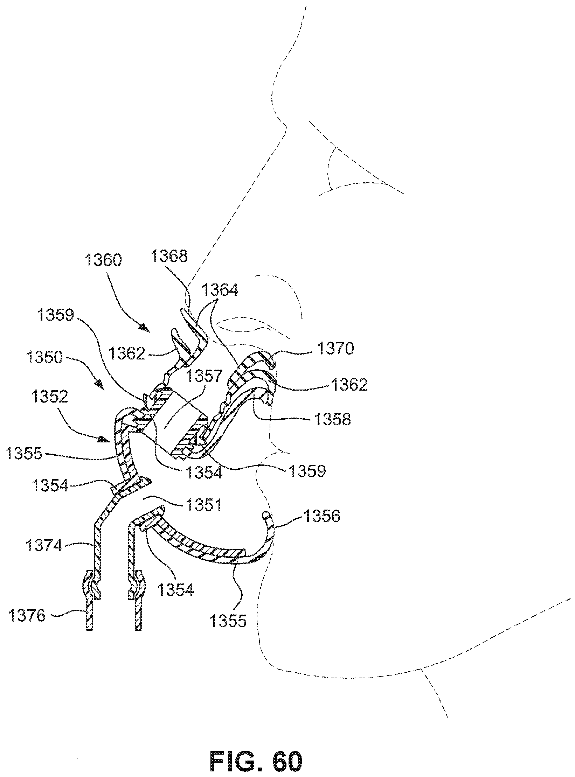

Fig. 60 illustrates a cross section view of modular patient interface system 1350, which includes a mouth portion 1352 and a nasal portion 1360. The nasal portion 1360 may be removable from the patient interface system 1350 and replaceable with a plug, so that the patient interface system 1350 may be used in a mouth only mode, or the nasal portion 1360 may be used in a nares only mode.

The mouth portion includes a frame 1354 and a mouth seal 1355. A first aperture 1351 is positioned in the frame 1354 that is adapted to receive elbow 1374 for delivery via swivel 1376 from an air hose. A second aperture 1357 is formed in the frame 1354 that may be plugged when the patient interface system is to be used in a mouth only mode, and connected to the nasal portion 1360 when the patient interface system 1350 is used in a nares and mouth mode.

The frame 1354 may be a rigid material, e.g., polycarbonate or a semi-rigid material. The mouth seal 1355 may be a flexible material, e.g., silicone. The mouth seal 1355 includes a lower lip engagement portion 1356 adapted to form a seal below a patient's lower lip, and an upper lip engagement portion 1358 adapted to form a seal just above the patient's upper lip (when used in a mouth only mode).

The nasal portion 1360 includes supporting portion 1362 and sealing portion 1364. The sealing portion 1364 includes nose tip engagement portion 1368 adapted to form a seal with the patient's nose tip, and upper lip engagement portion 1370 adapted to form a seal just above the patient's upper lip. The supporting portion 1362 may also form a seal just above the patient's upper lip. A ring 1359 may be used to connect the nasal portion 1360 to the mouth portion 1352.

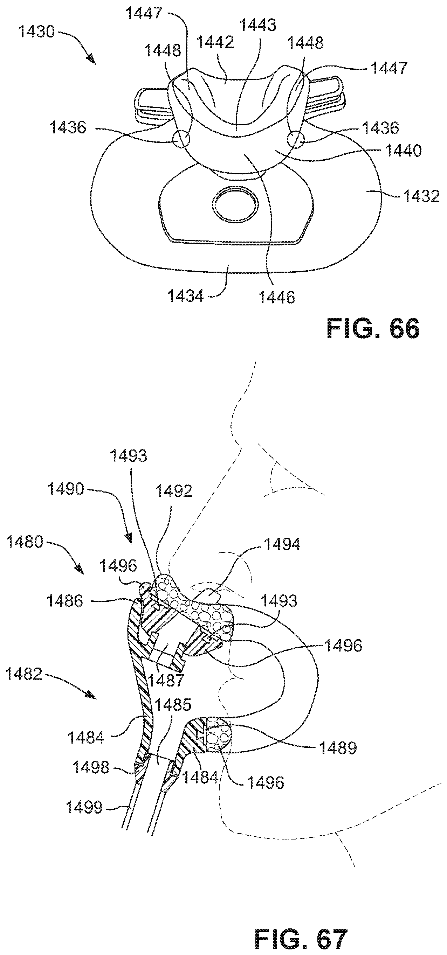

Fig. 66 illustrates a modular patient interface system 1430 that includes a mouth portion 1432 and a nasal portion 1440. The mouth portion 1432 includes a cushion portion 1434 adapted to form a seal around the patient's mouth.

The nasal portion 1440 includes a supporting portion 1446 and a sealing portion 1442. The sealing portion is adapted to form a seal with the patient's nares, and includes corners of the nose regions 1447 and upper lip engagement portion 1443.

Magnets 1436, 1448 may be placed on the mouth portion 1432 and the nasal portion 1440 to ensure that the nasal portion 1440 is oriented correctly relative to the mouth portion 1432. Either of the pairs of magnets 1436, 1448 could be replaced by a metal portion. The magnets 1448 in the nasal portion 1440 may be placed in the corners of the nose regions 1447 to ensure sufficient tension of the nares seal over the patient's top lip and corners of the nose.

Fig. 67 illustrates a cross section view of modular patient interface system 1480, which includes a mouth portion 1482 and a nasal portion 1490. The nasal portion 1490 may be removable from the patient interface system 1480 and replaceable with a plug, so that the patient interface system 1480 may be used in a mouth only mode, or the nasal portion 1490 may be used in a nares only mode. Alternatively, the system may be used in nares and mouth mode. In an example, separate foam components may be provided for mouth and nose seal portions so that the patient interface system may be used in mouth only or nares only modes.

The mouth portion 1482 includes a frame 1484 and a vanity flap or covering flap 1486, a lower aperture 1485 for connection of swivel or cuff 1498 and air hose 1499, and an upper aperture 1487 for connection of nasal portion 1490. The frame 1484 may be a rigid material, e.g., polycarbonate, or a semi-rigid material, e.g., semi-rigid nylon. A foam portion 1492 may have a clip 1493 for attaching to the frame 1484.

The nasal portion 1490 may include a flexible portion 1496, a foam portion 1492 connectable to the flexible portion 1496 with one or more clips 1493 and a nozzle portion 1494. The covering portion 1486 of the mouth portion 1482 may support the nasal portion 1490. Alternatively, a permanent connection may be used, e.g., foam portion directly onto flexible portion.

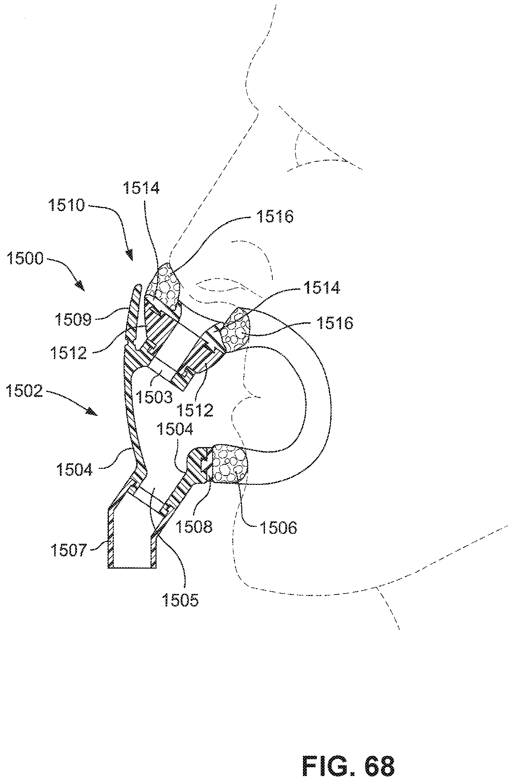

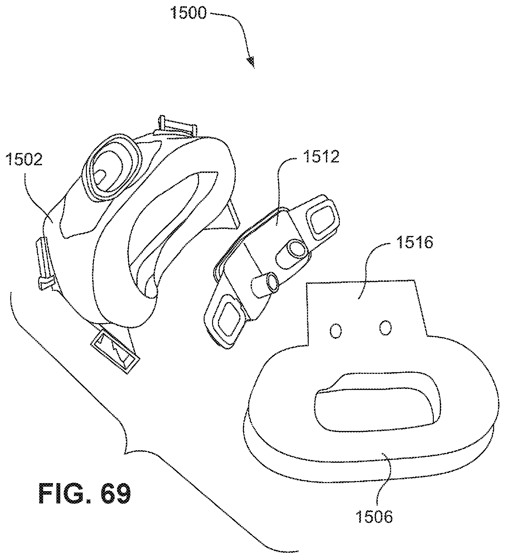

Figs. 67, 68 and 69 illustrate a modular patient interface system 1500, which includes a mouth portion 1502 and a nasal portion 1510. The nasal portion 1510 may be removable from the patient interface system 1500 and replaceable with a plug, so that the patient interface system 1500 may be used in a mouth only mode, or the nasal portion 1510 may be used in a nares only mode.

The mouth portion 1502 includes a frame 1504 and a vanity flap or covering flap 1509, a lower aperture 1505 for connection of elbow 1507, and an upper aperture 1503 for connection of nasal portion 1510. The frame 1504 may be a rigid material, e.g., polycarbonate, semi-rigid or flexible, e.g., nylon, silicone. A foam portion 1506 may have a clip 1508 for attaching to the frame 1504.

The nasal portion 1510 may include a flexible portion 1512, e.g., silicone, and a foam portion 1516 connectable to the flexible portion 1512 with one or more clips 1514. The covering portion 1509 of the mouth portion 1502 may be spaced from the nasal portion 1510. The clips 1508, 1514 may have a surface facing the foam portion that is angled to bias or promote the foam to apply force in certain directions, e.g., toward the patient's lip.

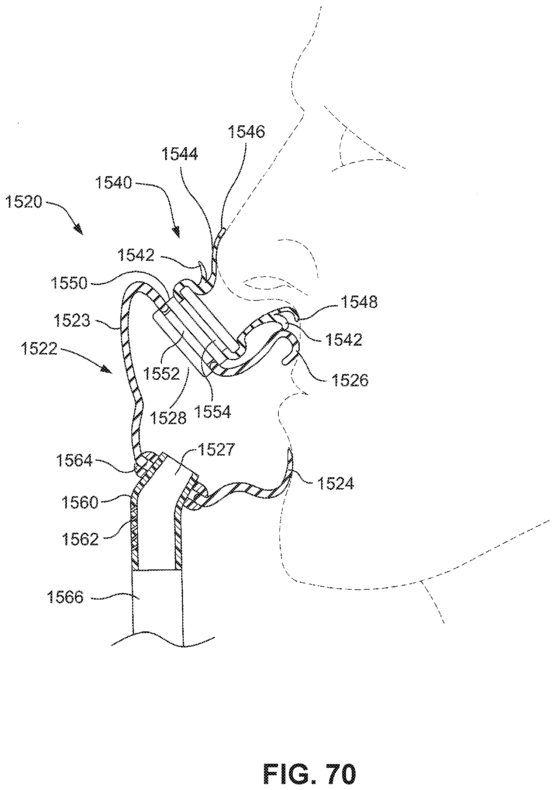

Fig. 70 illustrates a modular patient interface system 1520, which includes a mouth portion 1522 and a nasal portion 1540. The nasal portion 1540 may be removable from the patient interface system 1520 and replaceable with a plug, so that the patient interface system 1520 may be used in a mouth only mode, or the nasal portion 1540 may be used in a nares only mode. Alternatively, the system may be used in a mouth and nose mode.

The mouth portion 1522 includes a frame 1523, a lower aperture 1527 for connection of elbow 1560 (which may include vent holes 1562) and air hose 1566, and an upper aperture 1528 for connection of nasal portion 1540. The frame 1523 may be a rigid material, e.g., polycarbonate, or a more flexible material, e.g., silicone. A lower lip engagement portion 1524 is adapted to form a seal with the patient's lower lip, and an upper lip engagement portion 1526 is adapted to form a seal with the patient's upper lip.

The nasal portion 1540 includes a supporting portion 1542 and a sealing portion 1544. The sealing portion 1544 includes an upper lip engagement portion 1548 adapted to seal just above the patient's upper lip and a nose tip engagement portion 1546 adapted to form a seal with the patient's nose tip or region proximate to the nose tip. In the upper lip region, the supporting portion 1542 may be in contact with the upper lip engagement portion 1526 of the mouth portion 1522. When used in a nares only mode, the aperture 1528 can be connected to a source of air or plugged if air is alternatively supplied.

When used in a nares and mouth mode, a connecting ring 1550 may be utilized. The connecting ring 1550 has a first channel 1552 and a second channel 1554. The first channel 1552 is adapted to receive the mouth portion 1522 and the second channel 1554 is adapted to receive the nasal portion 1540.

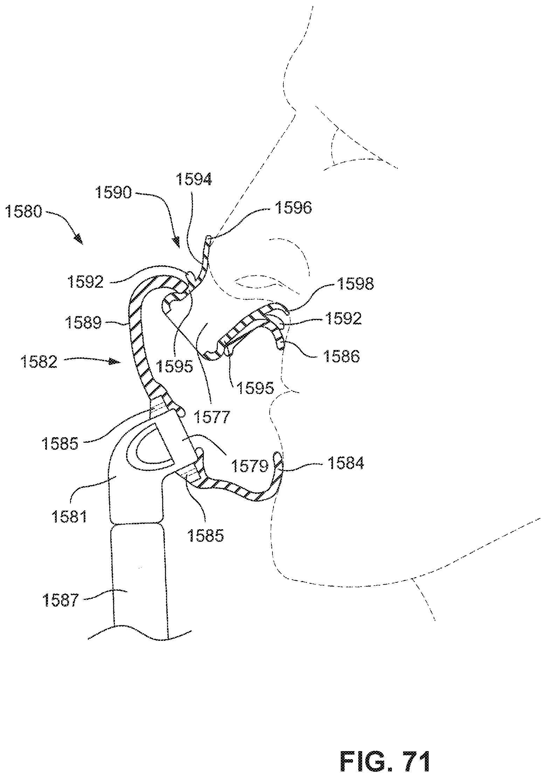

Fig. 71 illustrates a modular patient interface system 1580, which includes a mouth portion 1582 and a nasal portion 1590 that provide a soft to soft connection of nose and mouth parts. The nasal portion 1590 may be removable from the patient interface system 1580 and replaceable with a plug, so that the patient interface system 1580 may be used in a mouth only mode, or the nasal portion 1590 may be used in a nares only mode.

The mouth portion 1582 includes a frame 1589, a lower aperture 1579 for connection of elbow 1581, an air hose 1587, and an upper aperture 1577 for connection of nasal portion 1590. In an example, the elbow may include buttons adapted to permit quick release of the elbow. Vent holes 1585 may be formed in the elbow 1581, e.g., via a vented ring that interconnects the elbow and frame, or alternatively may be formed in the mouth portion 1582. A lower lip engagement portion 1584 is adapted to form a seal with the patient's lower lip, and an upper lip engagement portion 1586 is adapted to form a seal with the patient's upper lip.

The nasal portion 1590 includes a supporting portion 1592 and a sealing portion 1594. The sealing portion 1594 includes an upper lip engagement portion 1598 adapted to form a seal with the patient's upper lip and a nose tip engagement portion 1596 adapted to form a seal with the patient's nose tip.

The nasal portion 1590 may have indentations 1595 adapted to form an interference fit with the mouth portion 1582. The nasal portion 1590 may be resilient and be squeezed inward to accept the mouth portion in the indentations and then snap back into its original shape. The "trumpet" shape on the mouth portion provides a lead-in to guide the nasal portion into position. Also, a thin silicone wall may be provided to seal around the nasal portion.

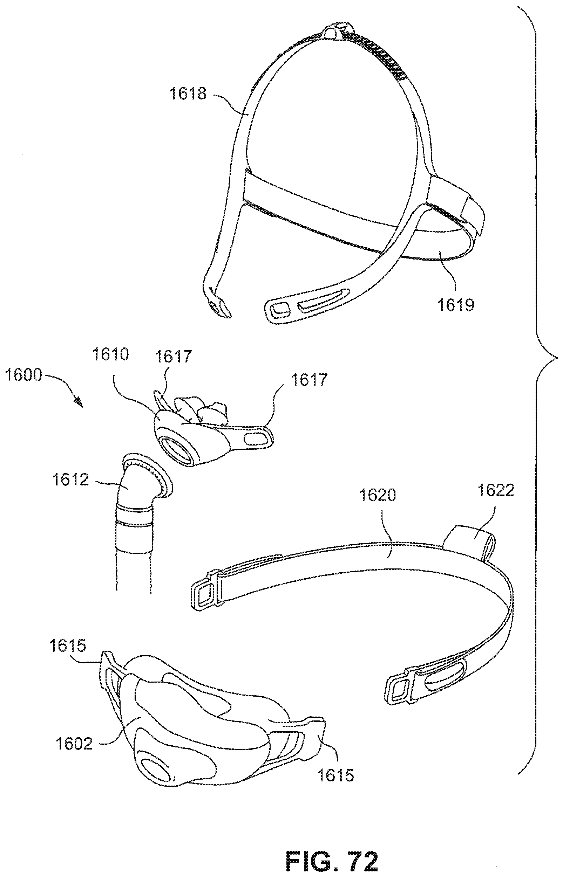

Fig. 72 illustrates a modular patient interface system 1600 including a mouth portion 1602, a nasal portion 1610, elbow and swivel 1612, upper headgear 1618 having a back strap 1619, and lower headgear 1620 having back loop 1622.

The upper headgear 1618 is adapted to connect to nasal portion 1610 via connectors 1617, and the lower headgear 1620 is adapted to connect to mouth portion 1602 via connectors 1615. The back strap 1619 of upper headgear 1618 may be inserted through back loop 1622 of lower headgear 1620. Alternatively, another strap could be connected from back strap 1619 to back loop 1622.

Fig. 73 illustrates a modular patient interface system 1630 including a mouth portion 1632, a nasal portion 1640, an elbow 1644 and an air hose 1646. The mouth portion 1632 may have an aperture 1634 adapted to receive a lug provided on the back of elbow 1644. In this way, the nasal portion 1640 can operate independently of the mouth portion 1632, or can be connected to the mouth portion 1632. In this example, the mouth portion may be connected to the nasal portion without disconnecting the elbow.

### Example Cushion 1

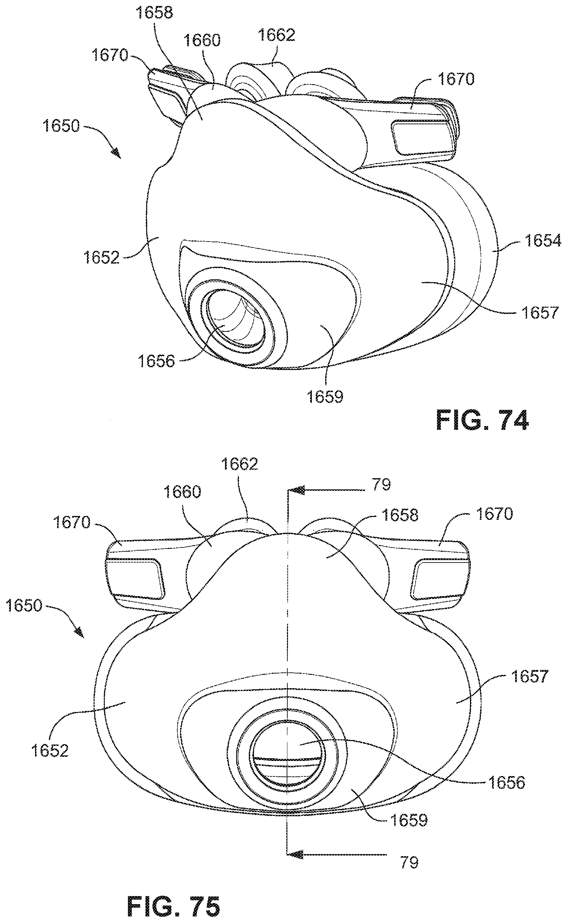

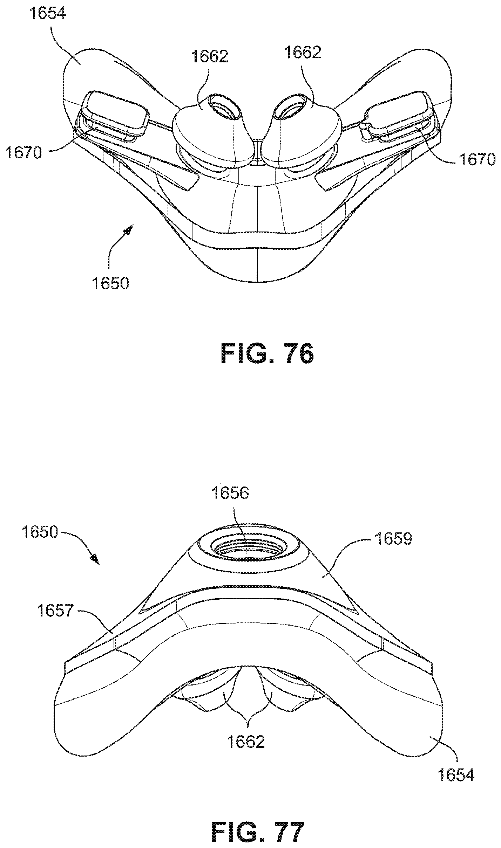

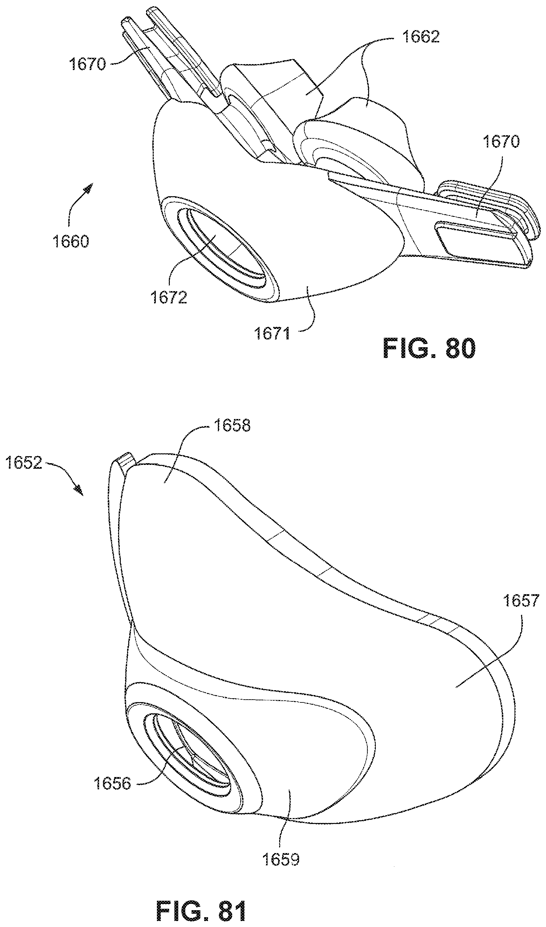

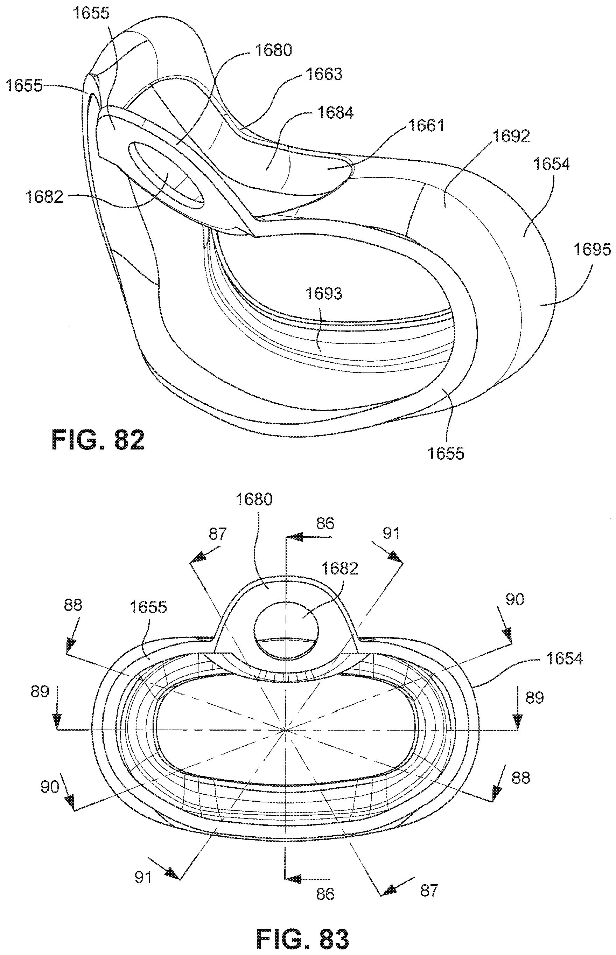

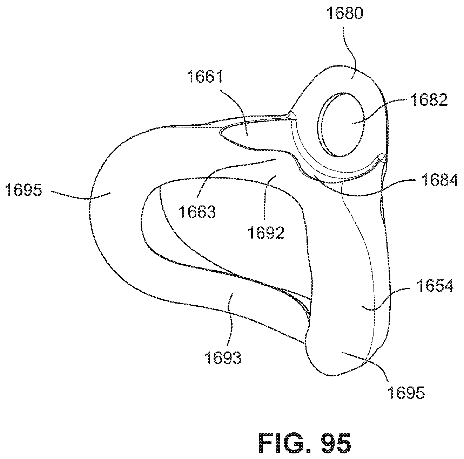

Figs. 74-79 illustrate patient interface system 1650, having a mouth portion 1652 and a nasal portion 1660. Fig. 80 illustrates the nasal portion 1660 and Fig. 81 illustrates the mouth portion, fascia or support structure 1652. Figs. 82 through 95 illustrate the cushion 1654 of the mouth portion 1652.

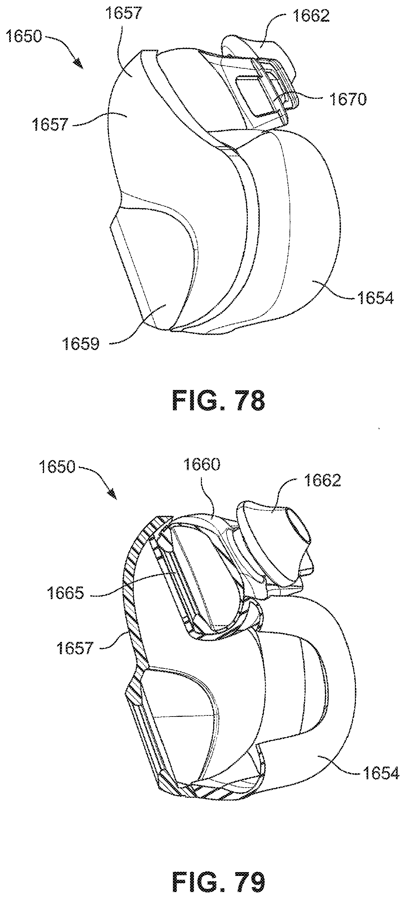

The mouth portion 1652 is removably detachable from the nasal portion 1660, although both portions may be co-molded. Sealing ring 1665 (Fig. 79) may be used to connect the mouth portion 1652 to the nasal portion 1660. An aperture 1656 is adapted to connect to a supply of air to deliver air to the patient interface system 1650. The mouth portion includes cushion fascia portion 1657 (which may be transparent or semi-opaque), decoupling portion 1659, which may be a thinned portion compared to the remainder of the front of the mouth portion, to decouple forces applied by movement of the elbow/air hose connected to aperture 1656 from the rest of the patient interface system 1650.

Vanity flap or covering flap 1658 covers/supports the patient's nares region. Preferably, vanity flap covers all or portion of the nares seal portion 1660. Such an arrangement may be less visually obtrusive to the patient and may aid in stabilizing the nares seal portion 1660.

Cushion 1654 connects to the cushion fascia portion 1657 and is adapted to form a seal around a mouth of a patient. The connection of the cushion 1654 to the cushion fascia portion 1658 may be by an interference fit e.g. tongue and groove.

Lower headgear connectors (not shown) may be placed on the front of the mouth portion 1652 in a manner such as illustrated in Fig. 30. The cushion 1654 and the cushion fascia portion 1657 may both have a hardness of 40 Shore A, or similar. Alternatively, the cushion fascia portion 1657 may have a relatively higher hardness compared to cushion 1654. Cushion fascia portion 1657 may be constructed of a semi-rigid polymer such as nylon, polycarbonate, polypropylene. Cushion fascia portion 1657 may have a hardness of 40-80 Shore A. Cushion 1654 may be constructed of a conformable material for example silicone, foam, gel, TPE. Cushion 1654 may be constructed of a material having a hardness of 5-40 Shore A, for example 5-25 Shore A.

Cushion 1654 may be constructed of a combination of materials, for example a first portion proximate the cushion fascia portion having a first hardness and a second portion proximate a patient contacting portion having a second hardness. Preferably the first hardness is greater than the second hardness, to provide structural support to the patient contacting portion. For example, the first portion may have a hardness of 25-60 Shore A, 35-50 Shore A. The second portion may have a hardness of 5-25 Shore A, 5-15 Shore A. The first portion may be thicker than the second portion, for example the first portion may be 1-3mm, 1-2mm, and the second portion may be 0.1-2mm, 0.1-1mm or about 0.8mm. The combination of a thin wall section and soft material at the second portion may permit the cushion to be more compliant and flexible, thereby shaping to the patient's face. The thicker first portion provides structure to provide stabilization. Together, they allow a single wall cushion that substantially emulates a dual wall cushion.

Preferably, cushion 1654 may be flexible and/or may have a lower hardness (e.g. 5-15 Shore A) in the chin region 1693 so that when the cushion is placed on the user's face, the cushion stretches over the patient's chin. The stretching of the cushion in the chin region 1693 may cause the cushion to be in tension over the patient's chin, which may form a robust seal with the patient's chin as well as ensuring that the cushion may fit a wider range of chin geometries.

The cushion 1654 includes a cushion fascia connecting portion 1655 (Fig. 82) adapted to connect to the cushion fascia 1657, a ring connecting portion 1680, and a pocket 1684. In an alternative, the cushion fascia and cushion may be formed in one piece. The ring connecting portion 1680 includes an aperture 1682 adapted to receive connecting ring (to connect the mouth portion 1652 to the nasal portion 1660). In an alternative, the ring connecting portion and connecting ring may be formed in one piece. In a further alternative, the cushion may be formed with a first connecting ring and the nasal portion may be formed with a second connecting ring, first connecting ring being received by the second connecting ring (or vice versa).

The pocket 1684 is adapted and shaped to receive the gusset 1671 (Fig. 80) of the nasal portion 1660, or other portion of nasal portion 1660 for example the headgear connector portion of nasal portion 1660. A different shaped pocket may be used if a different nasal portion is used. The pocket 1684 is sunken in the cushion 1654 so as not to disturb the seal of the cushion 1654 when the nasal portion 1660 is in the pocket 1684. That is, pocket 1684 may be disposed or offset under or below the uppermost surface of the mouth cushion 1654.

Pocket 1684 may also be a sunken portion, groove, trench, hollow, pouch, alcove, indentation, dip, pit, valley or sinkage - i.e. a region of the cushion that is adapted to receive a nasal portion, the region having an alternate or abstract geometry when compared to the rest of the mouth cushion. The pocket may not be a hole, cut out or aperture. May be the pocket is air tight. May be the pocket is formed by a soft, conformable material such as the mouth cushion, rather than the frame. Such an arrangement is preferable as a conformable pocket may permit adjustment of the position of the nasal portion, thereby fitting more patients. May be, the pocket permits movement of the nasal portion in the superior-inferior direction, to accommodate varying patient nose heights. May be the pocket may increase in stiffness when air pressure is applied in the mouth cushion for example. The pocket may stabilize the nare portion in position in the medial-lateral direction. This may be achieved by the shape of the pocket, adapted to match the shape of the nasal portion. Such an arrangement may ensure that the nasal portion maintains sealing engagement with the patient's nares in use when tube drag or other forces are applied to the side of the patient interface.

The nasal seal portion may reside or rest in the pocket. The patient contacting portion of the nasal seal portion may be positioned outside, above or higher than the pocket. Such an arrangement may ensure that the patient can see the nasal seal portion contacting their nose to ensure alignment and seal.

In one form the nasal seal portion may not be in air flow communication with the mouth portion via the pocket.

The pocket may have a lower stiffness than surrounding portions of the mouth cushion. The pocket may have a comparably thinner wall section than other regions of the mouth cushion. The pocket may be formed from a different material to the rest of the mouth cushion.

May be the pocket is continuous with or formed in one piece with the top lip contacting region of the mouth cushion and is thus in sealing engagement with the patient's top lip.

The mouth cushion may comprise a top lip region adapted to interface with the patient's top lip. The mouth cushion may further comprise a cheek region adapted to interface with the patient's cheek and/or side of mouth. The mouth cushion defines a wall, the wall located between a patient contacting portion of the mouth cushion and a frame contacting portion of the mouth cushion. The wall may comprise different portions, for example a top lip wall portion, a chin wall portion and two cheek wall portions, each corresponding to the respective top lip, chin and cheek regions of the patient's face. In one form, the cheek wall portion may be comparably stiffer than the top lip wall portion. In another form, the chin wall portion may be comparably stiffer than the top lip wall portion. In some forms the top lip portion may be less stiff than the cheek wall portions. In such an arrangement the cheek contacting portion may act in compression on the patient's cheeks, such that the top lip and chin regions may be in tension.

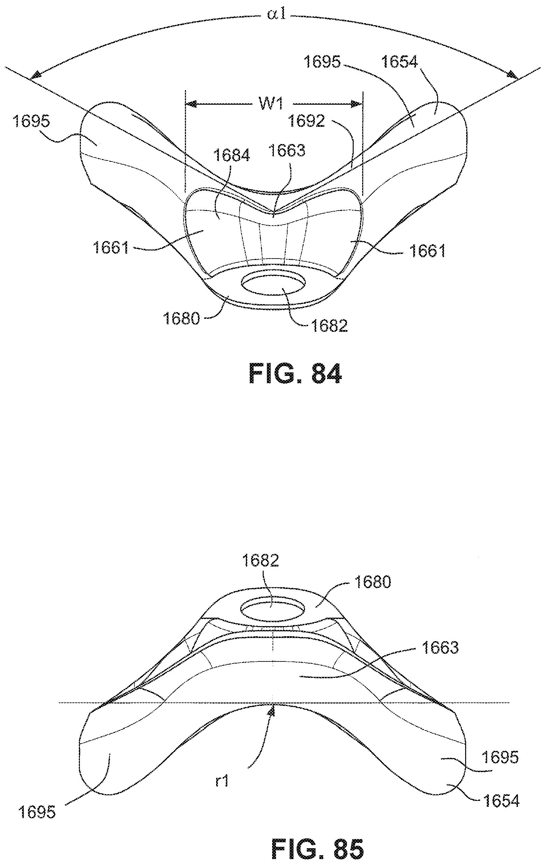

Pocket 1684 in the present example may comprise a curved groove having lobes or regions 1661 as shown on Fig 84. Lobes 1661 may be shaped to correspond to gusset 1671 as shown on Fig 80. The lobes may be separated by a ridge or indentation 1663 adapted to indicate the alignment or positioning of the nasal portion 1660.

Pocket 1684 may have a maximum width w1 of approximately 30-60mm. In some forms, pocket 1684 may have a maximum width w1 of approximately 35-45mm. May be, pocket 1684 may have a maximum width w1 of approximately 40-45mm.

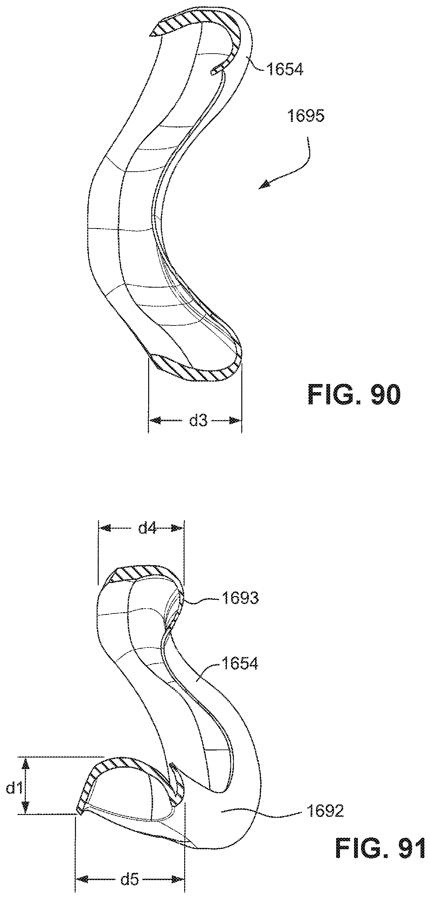

Pocket 1684 may have a length d5, as shown on Fig 91, of about 15-40mm. May be, pocket 1684 may have a length d5 of about 20-30mm. This length may be the approximate length from the fascia contacting side of the pocket to the outer most edge of lobe 1661.

Lobes 1661 of pocket 1684 may be offset or angled with respect of one another to position the nasal seal to be positioned at the angular region of the maxilla (patient's top lip). Lobes 1661 of pocket 1684 may be angled with respect of one another by angle α1 of approximately 90-180°. Lobes 1661 of pocket 1684 may be angled with respect of one another by angle α1 of approximately 90-150°. Lobes 1661 of pocket 1684 may be angled with respect of one another by angle α1 of approximately 90-180°. Lobes 1661 of pocket 1684 may be angled with respect of one another by angle α1 of approximately 90-150°.

The chin or lower seal region 1693 of mouth cushion 1654 may be rounded or curved to conform to a patient's chin. Radius of curvature r1 may preferably be 20-50mm. Radius of curvature r1 may be 30-40mm. Radius of curvature r1 may be 35-45mm.

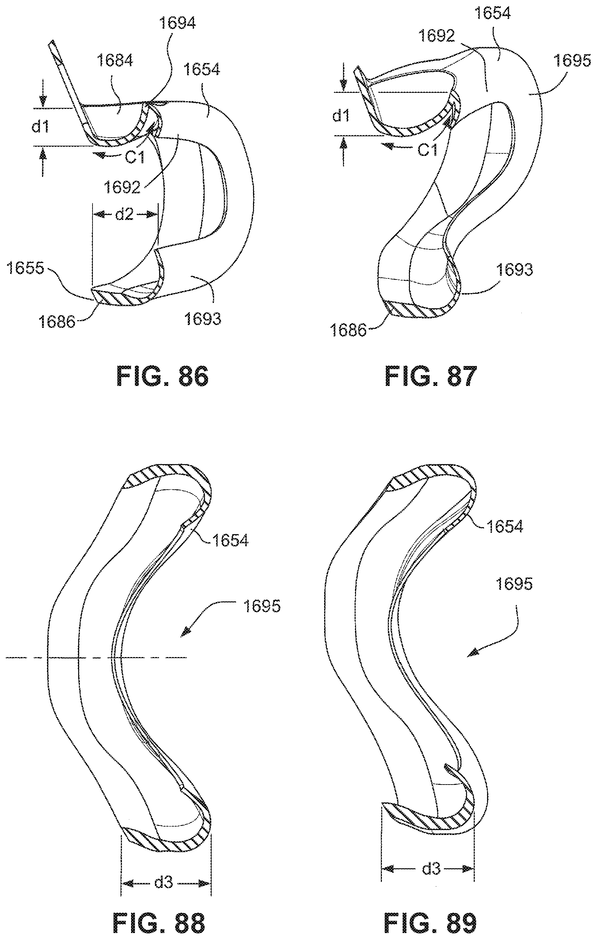

The nasal portion may be secured to the cushion 1654 by connection to the connecting ring. The cushion may also have a thickened portion 1686 (Figs. 86 and 87) to support connection to cushion fascia portion 1657. Also, the thickened portion 1686 supports the sealing portion in position and prevents collapse of the sealing portion, particularly if the sealing portion is constructed of a low hardness, low thickness material, i.e., it will lack structural rigidity.

The nasal portion 1660 includes a sealing portion 1662 adapted to form a seal with the nares of the patient, and headgear connectors 1670 for connecting to headgear. The sealing portion 1662 may be in the form of nozzles, although other sealing portions may be used, such as pillows, prongs, etc. Exemplary sealing portions are disclosed in WO 2009/052560 A1.

The cross sections illustrated in Figs. 88-90 are shaped to accommodate flat faces and more pointy faces of patients due to the question mark like shape of the cross section. Fig 89 shows the cross section at the horizontal plane that may be positioned generally on the cheeks or the lateral extents of the patient's mouth. In this region, the cross section of the cushion shows that the membrane is comparably longer than at the bony upper or lower jaw regions (shown in Figs 88 and 90). This may be to reduce the force of the cushion on the patient's cheeks compared to the bony upper or lower jaw regions, as the longer membrane may absorb more force than the shorter membrane. This may be because the patient's cheeks are more sensitive than the bony regions of the face. The shape of the cushion is shaped to match a shape of a face, as illustrated in Fig. 85, e.g., a C-shape. As illustrated, the sealing membrane includes a varying thickness along its length, e.g., from thick to thin. This varying thickness may be so that a patient contacting portion is supported and positioned by the thicker region, and the patient contacting region may be comfortable and conformable due to the thinner region. The patient contacting portion may be approximately 0.2-2mm. In some forms, patient contacting portion may be approximately 0.8-1.2mm. Thicker region 1686 may be approximately 0.5-4mm. In some forms the thicker region 1686 may be approximately 2-3mm.

In a further example, the load imparted by the cushion on the user's face at the top lip region 1692 may be greater than the load imparted by the cushion on the user's face at the chin region 1693. This may be achieved by having a comparably greater thickness at the top lip region 1692 compared to the thickness at the chin region 1693. Such an arrangement may assist in anchoring the cushion on the patient's face, while ensuring that the force on the lower jaw is low. High forces on the lower jaw may force the upper airway to collapse and thereby exacerbate obstructive sleep apnea, so it is desirable to have a lower force on the patient's lower jaw and hence chin region 1693.

The pocket 1684 is structured to accommodate the nasal portion, and air inside the nasal portion ensures a seal across the top lip. The nesting nasal portion and cushion make the patient interface less obtrusive. Pocket 1684 may have a depth d1 of approximately 5-30mm. In some forms, pocket 1684 may have a depth d1 of approximately 5-20mm. In some forms, pocket 1684 may have a depth d1 of approximately 5-10mm. The depth of pocket d1 can be seen in Figs 86-87.

As best shown on Fig 86, pocket 1684 may be adjacent or connect to a flap seal portion at the top lip region 1692. A ridge or apex 1694 may be formed between the pocket 1684 and the top lip region 1692. Ridge 1694 may act to divide the pocket from the top lip region, and may also provide a hinge or spring point for the top lip region.

As best shown at Figs 86 to 87, pocket 1684 may be generally curved or rounded in a cup like shape as indicated at c1.

At chin region 1693, as best shown on Fig 86, the cushion may have a depth d2 i.e. the distance from the sealing region or patient contacting portion of the chin region to the connecting region 1655, of approximately 0.5-3mm. Depth d2 may be approximately 1-2mm, In some forms depth d2 may be approximately 1.5-2mm. This depth may be desired so as to ensure different shaped chins may be accommodated by the mouth cushion 1654. Chin lip region 1693 as shown in Fig 91 demonstrates depth d4. Depth d4 may be substantially similar to depth d1.

Cushion 1654 may have a cheek region 1695, and at the cheek region 1695 the cushion may have a depth d2, shown on Figs. 88. 89 and 90. Depth d2 may be larger than d1, d4. Such an arrangement may be preferable so that cushion 1654 may flex and deform at the cheeks to a greater extent than at the top lip, so as to anchor the cushion on the top lip and permit variations in the facial geometry of patients. For example, some patients may have relatively flat faces while others may have relatively pointy faces, so the cushion should be able to conform to each of these facial profiles. For example, depth d2 may be approximately 10-50mm. In some forms, depth d2 may be approximately 20-35mm. In some forms, depth d2 may be approximately 20-30mm.

The curvature of the top lip region 1692 of the cushion ensures tension across the patient's top lip and therefore seal. The spring shape cross-section at the top lip lowers force displacement at the patient's top lip (sensitive region).

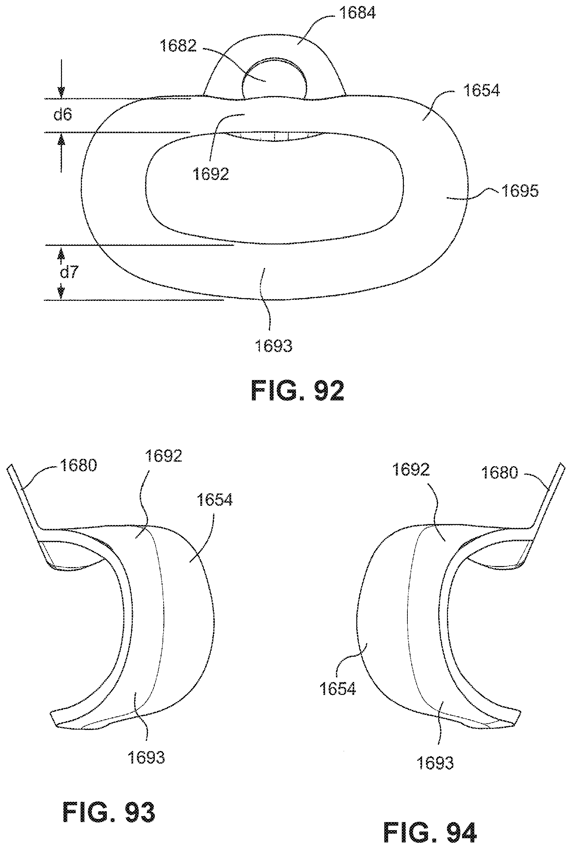

Figure 92 shows the mouth cushion 1654 as viewed from the patient contacting side. The top lip region 1692 has a height d6, which may be less than height d7 of chin region 1693. This may be because typically a person's face has a smaller distance between the top lip and bottom of the nose when compared to the distance between bottom lip and the tip of the chin. For example, height d6 may be 5-15mm. In some forms, height d6 may be approximately 8-10mm. Height d7 may be approximately 10-25 mm. In some forms, height d7 may be approximately 12-16mm.

### Example Cushion 2

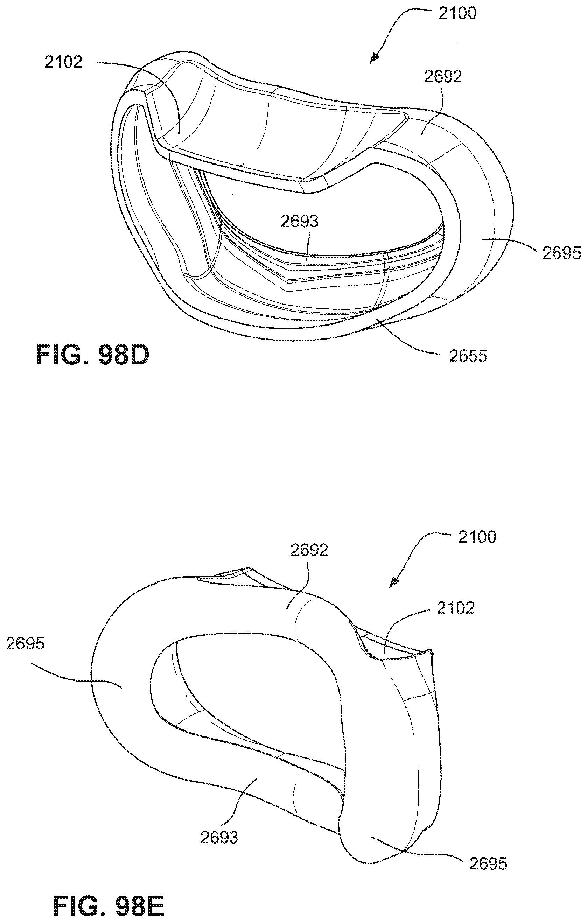

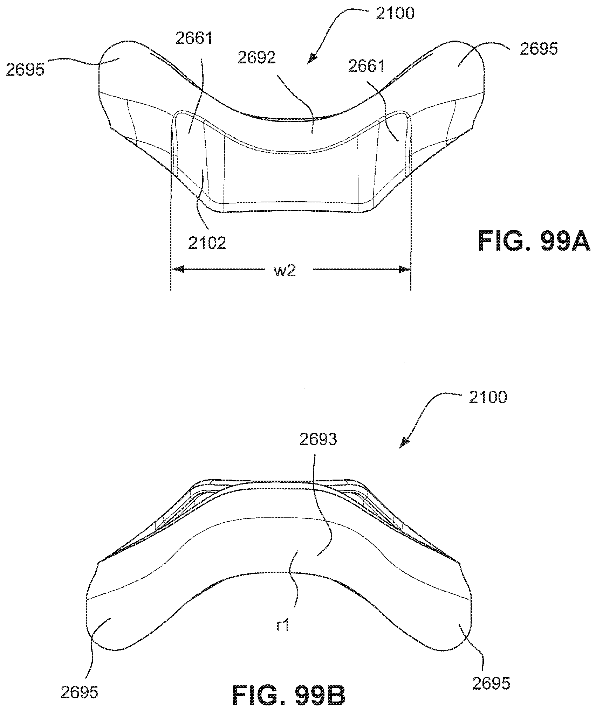

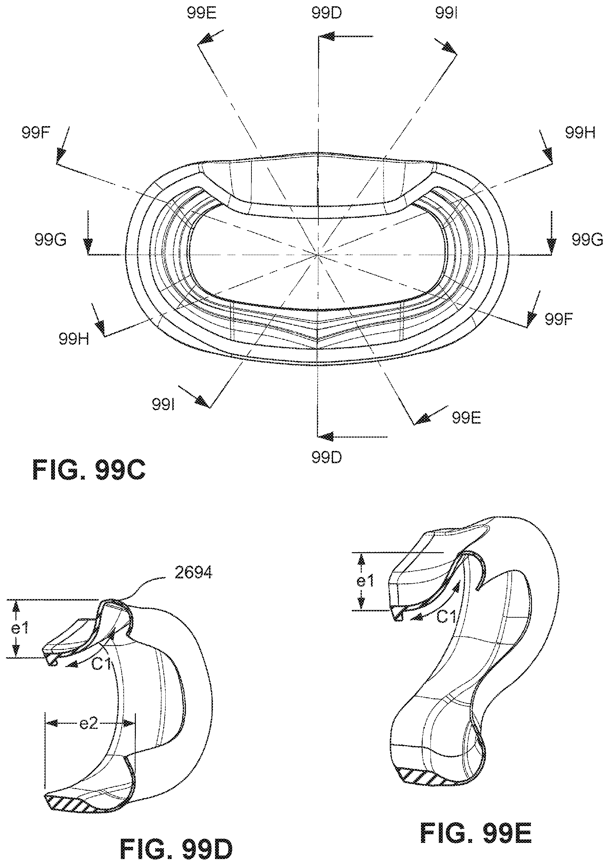

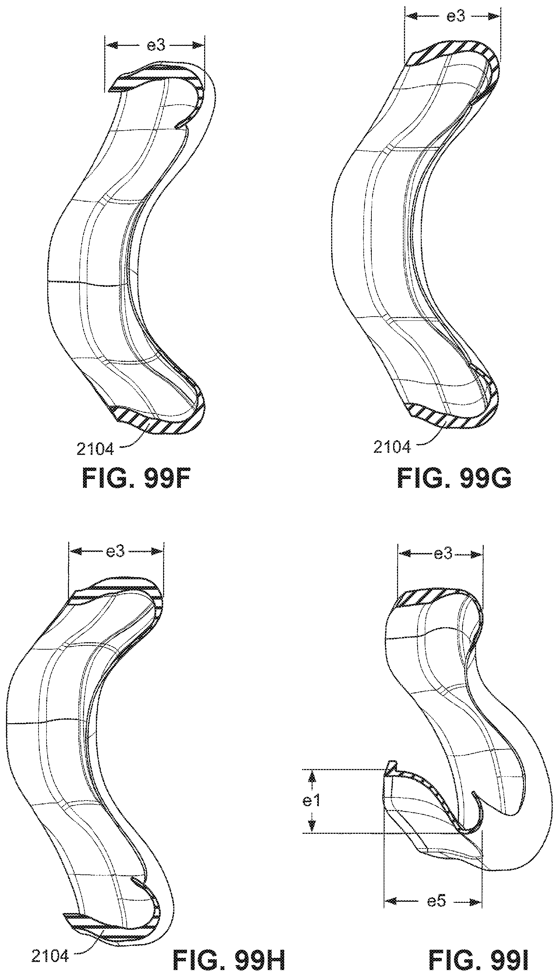

Figures 98A-E and 99A-I illustrate a variant of a mouth cushion 2100 which is similar to that shown in Figures 82-95, and share common features such as a pocket adapted to receive a nasal seal portion and a single side wall 2104 that is thicker at its base where it connects to the fascia and thinner at its distal end where it seals with the patient. The side wall has a sickle or question mark type shape.

Mouth cushion 2100 may have a top lip region 2692, chin region 2693 and cheek regions 2695. Mouth cushion may further include connecting region 2655 for interfacing the mouth cushion with a clip, frame or other portion of the patient interafce assembly.

The top lip region 2692 and chin region 2693 of the seal portion are formed generally flat such that the seal is stretched (by tension) on the patient's face to enhance the seal. This is beneficial since the headgear, even if tightened, would not be able to significantly change the sealing forces in this area.

The pocket 2102 is thinner compared to the pocket 1684 in Figure 82. This allows the nasal seal to more directly add some component of sealing force - i.e. as the nasal seal is pressurized the nasal seal may become stiffer and thereby impart force on the top lip seal portion. The reduction in thickness may also reduce weight and/or material.

In addition, cushion 2100 does not have a ring connecting portion 1680 as in Figs 92-95 (for example). Thus, air flow to the nasal seal portion is not transmitted through the mouth cushion. Instead, a clip may be provided that is adapted to receive both the mouth cushion and the nasal cushion, and may provide the pathway to transmit air to the nasal cushion.

Pocket 2102 may not be a cup like shape, rather it may have an open back and curve upwards (see C1 shown on Figs 99D and 99E) to ridge 2694 to then adjoin top lip portion 2692.

Pocket 2102 may have a similar depth to pocket 1684, as indicated at depth e1 on Figs 99D and 99E. Depth e3 may also be similar to depth d3, depth e4 may be similar to depth e4 and length e5 may be similar to length e5.

Depth e2 as indicated on Fig 99D shows the distance from the edge or connecting region to the patient contacting portion. Depth e2 may be about 10-30mm, preferably 15-25mm. Radius of curvature r2 may be larger than the radius of curvature r1 from Example 1. Such a depth and radius of curvature r2 may be to ensure that the depth at the chin region 2693 is similar to that at cheek region 2695. This may flatten out the cushion in the chin region, so that when the patient positions their chin against chin region 2693, the membrane stretches over their chin. This may ensure a more stable seal and a larger fit range.

Width w2 as shown on Fig 99A may be larger than width w 1. This may be to accommodate a longer nasal seal portion or to move the same nasal seal portion from Example 1 further down into mouth seal portion 2100 (i.e. position the nasal seal further from the top surface or deeper in the mouth cushion 2100). Width w2 may be about 50-70mm. In some forms, width w2 may be about 50-60mm.

### Example Cushion 3

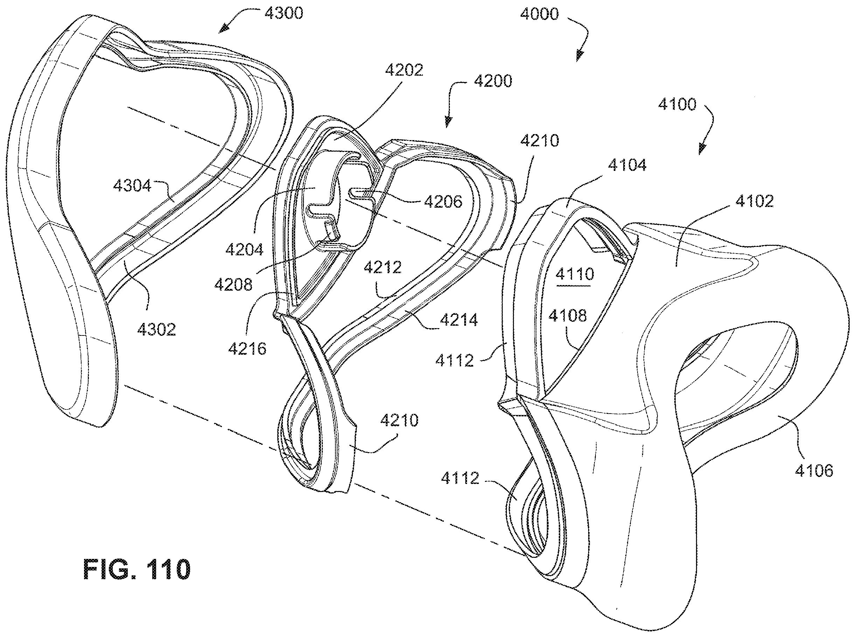

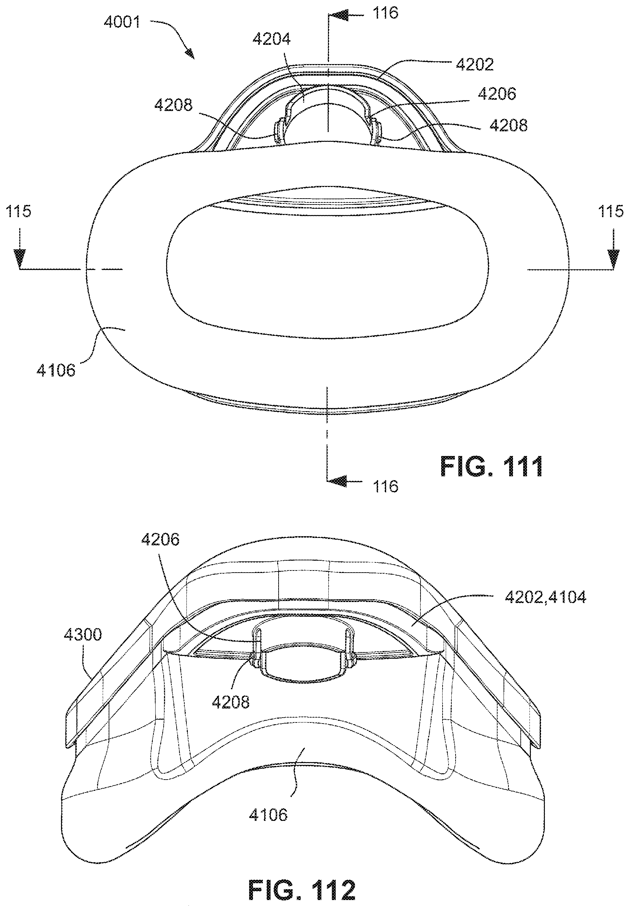

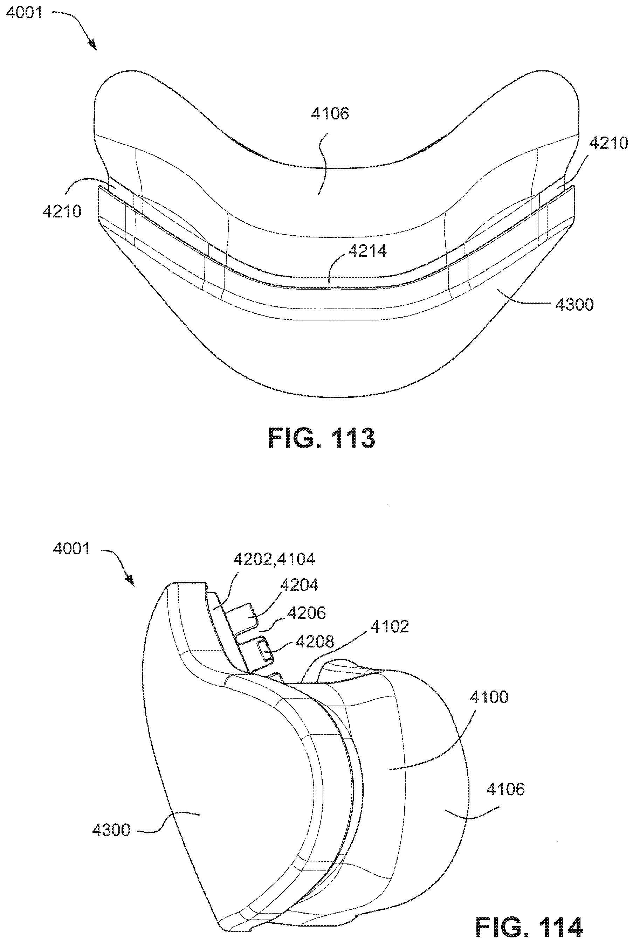

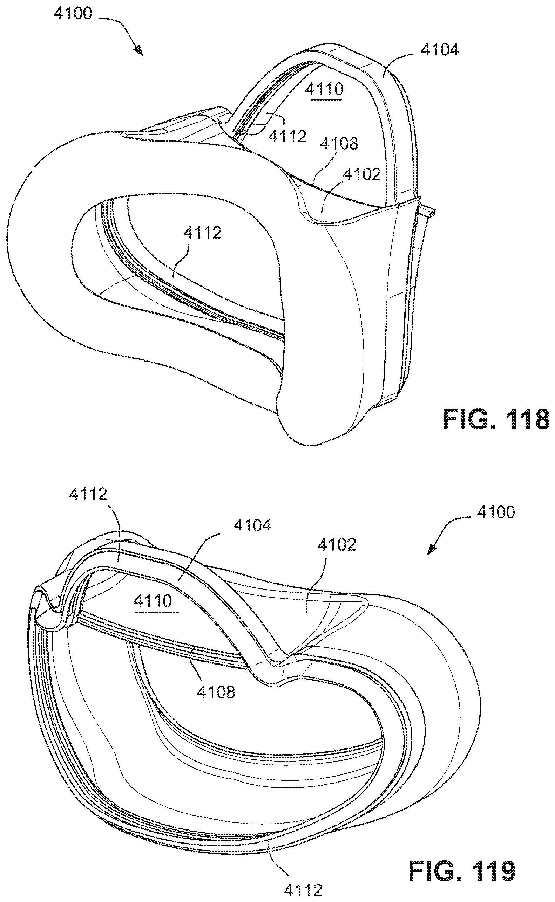

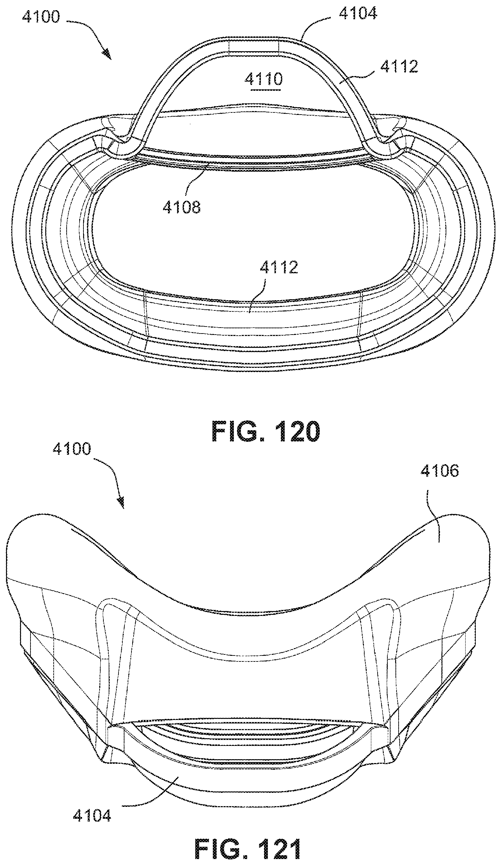

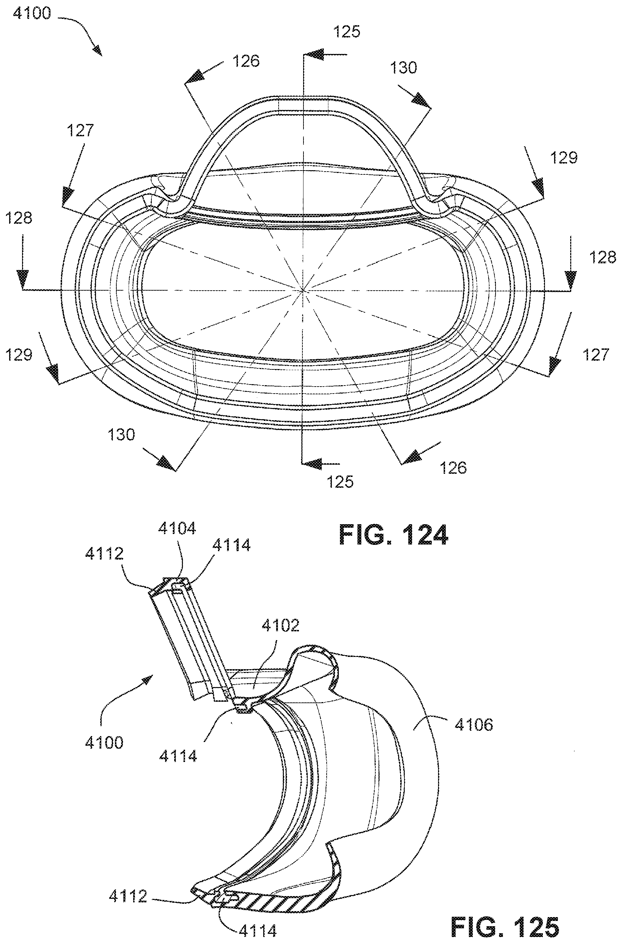

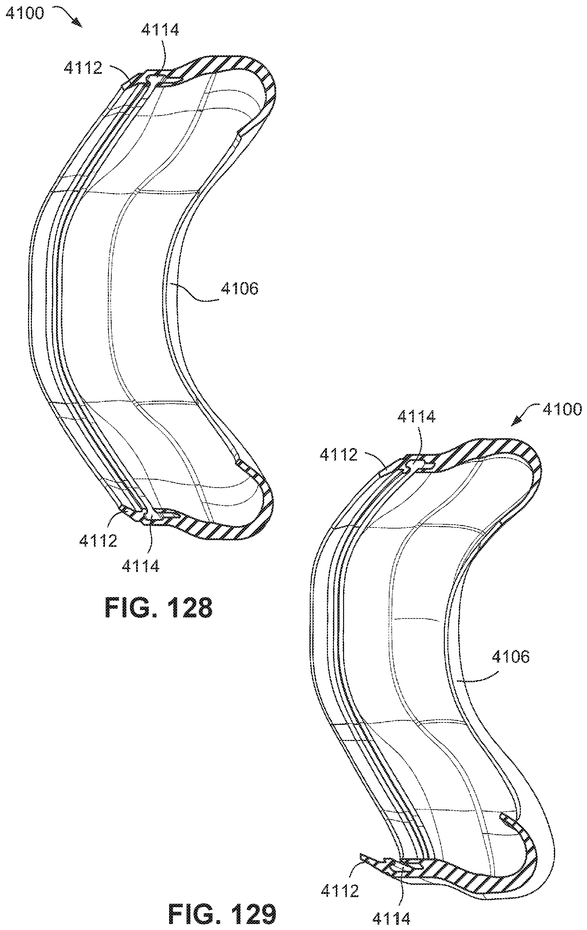

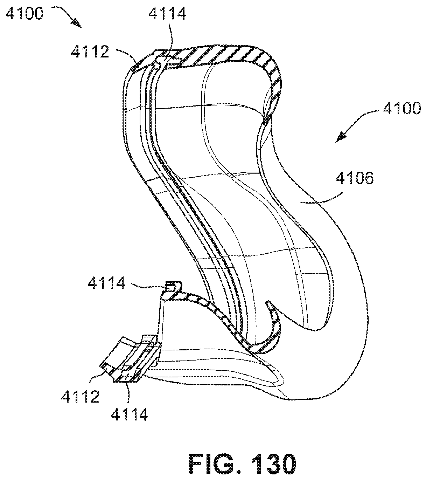

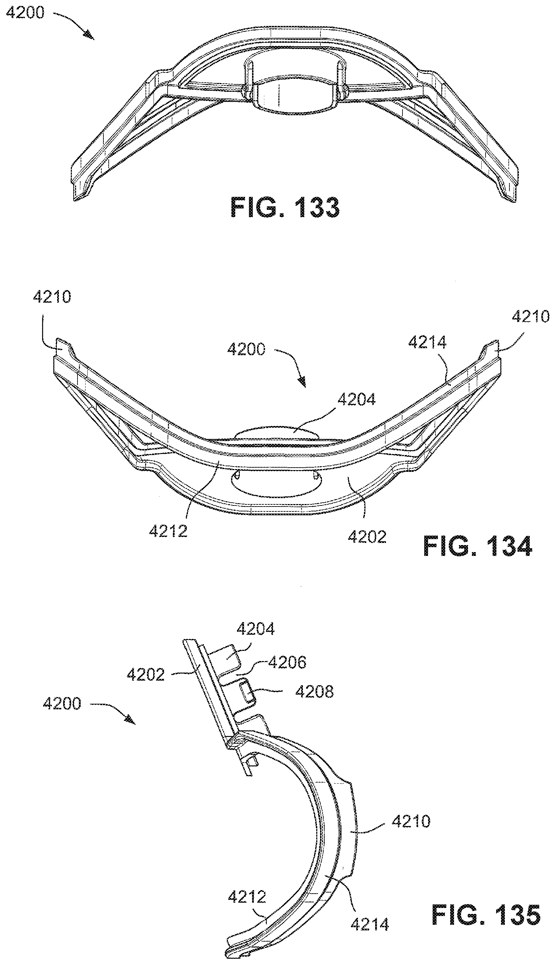

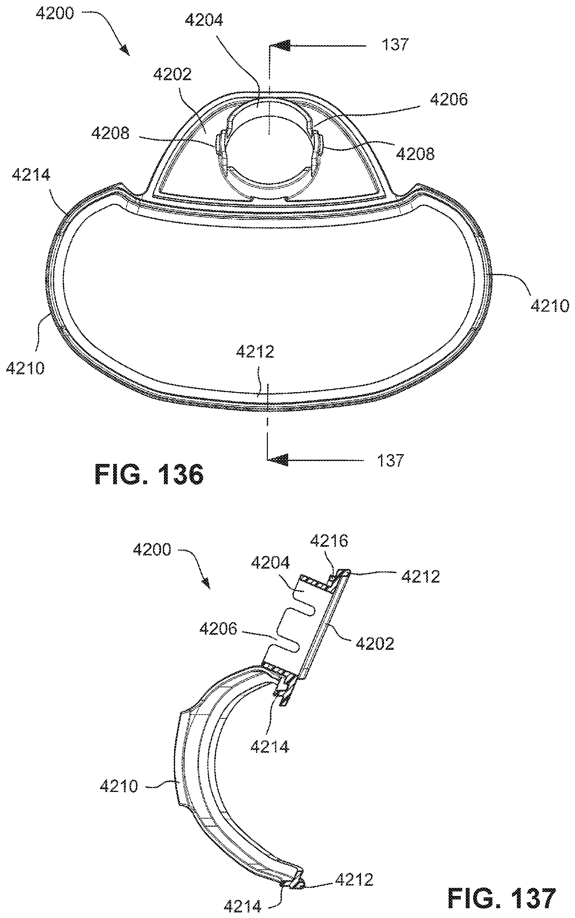

Referring to Figs. 110-137, a patient interface system 4001 according to an embodiment of the present technology comprises a mouth cushion 4100, a cushion clip 4200 and a fascia 4300. The mouth cushion 4100 shares some common features with Example Cushion 2, such as a pocket 4102 adapted to receive a nasal seal portion, and a sealing portion 4106 configured to form a seal with the patient's face. Other common features may be evident from the following description of the patient interface system 4001. The mouth cushion 4100 may be formed of, for example, silicone. The cushion clip 4200 may be formed of, for example, thermoplastic. The fascia 4300 may be formed of, for example, thermoplastic.

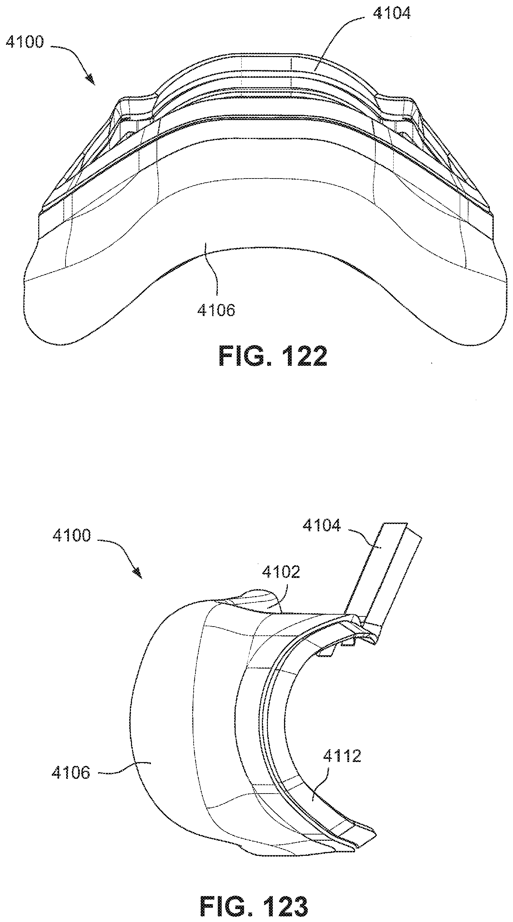

The mouth cushion 4100 may be over-moulded to the cushion clip 4200. It should be appreciated that the mouth cushion 4100 may be detachably connected to the cushion clip 4200 in a manner described below. The mouth cushion 4100 comprises the pocket 4102, an upper connecting portion 4104 adapted to receive a nasal seal connecting portion 4202 of the cushion clip 4200 and retain the mouth cushion 4100 to the fascia 4300. Referring to, for example, Fig. 123, the upper connecting portion 4104 is tilted or offset away from the sealing portion 4106 so as to allow the nasal seal portion (not shown) to orient toward the patient's nasolabial angle. Referring to, for example, Figs. 110, 118 and 119, a rear edge 4108 of the pocket 4102 and the upper connecting portion 4104 form an aperture 4110 that receives the nasal seal connecting portion 4202 of the cushion clip 4200. The nasal seal connecting portion 4202 of the cushion clip 4200 fits within the aperture 4110 of the upper connecting portion 4104 and the rear edge 4108 of the pocket 4102.

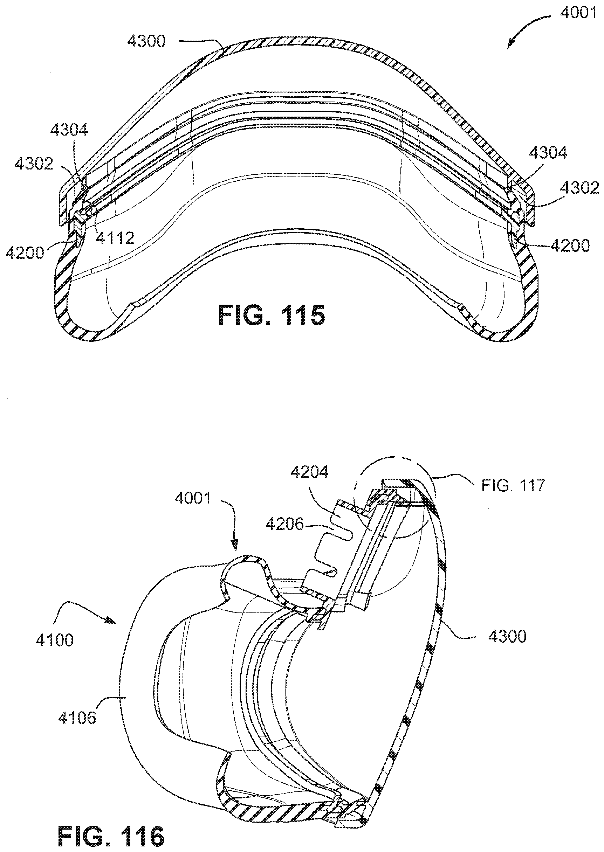

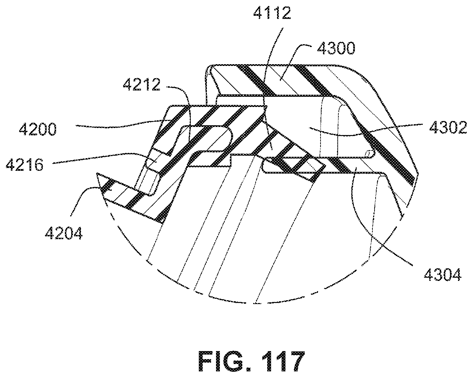

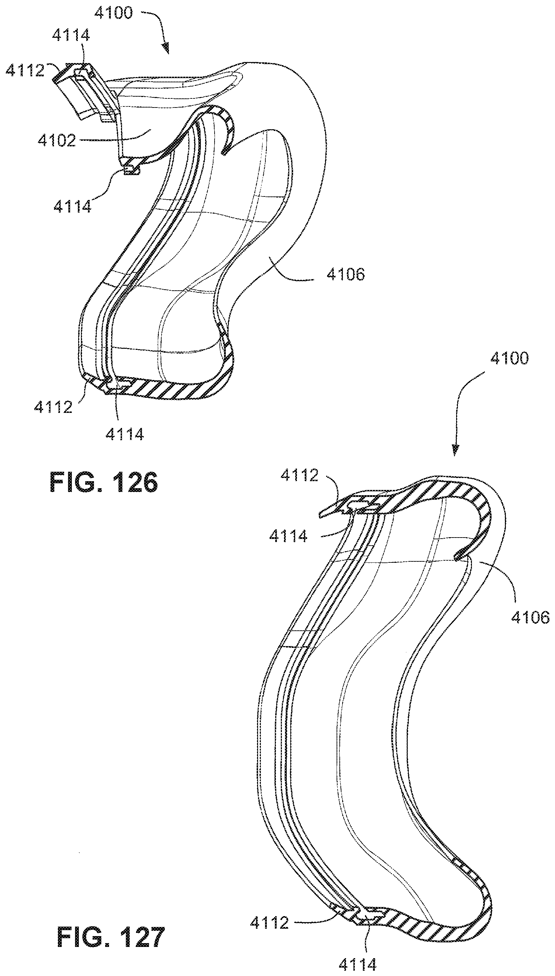

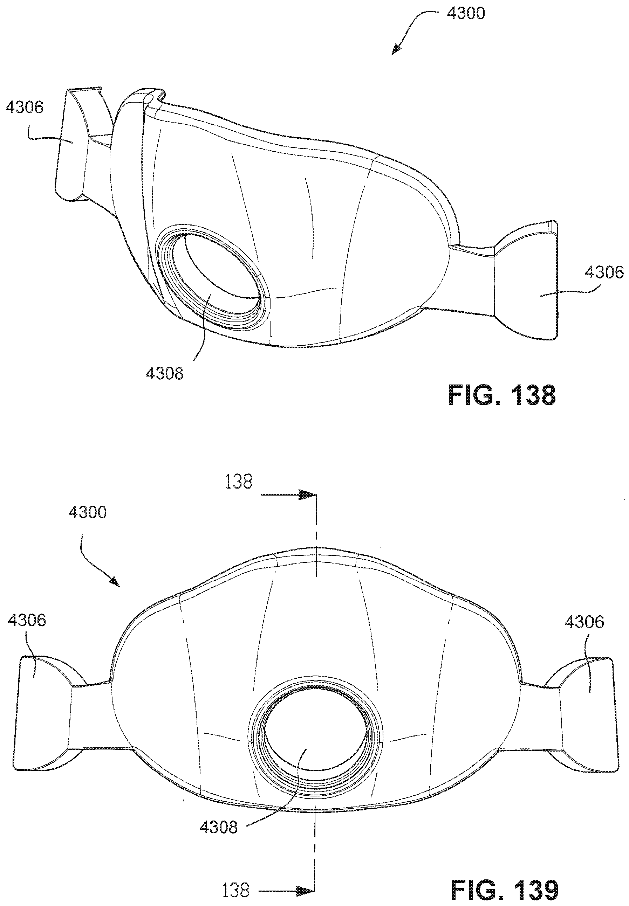

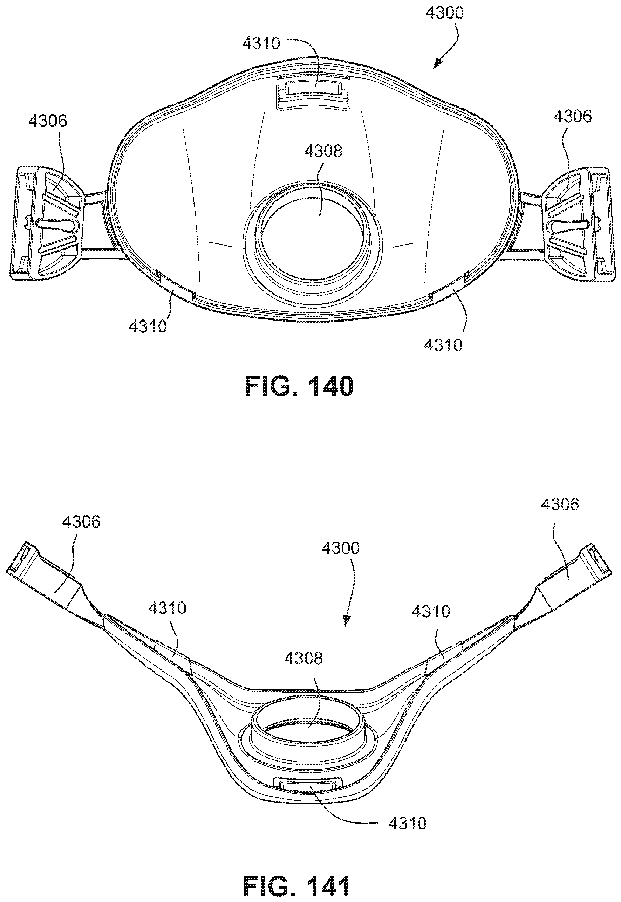

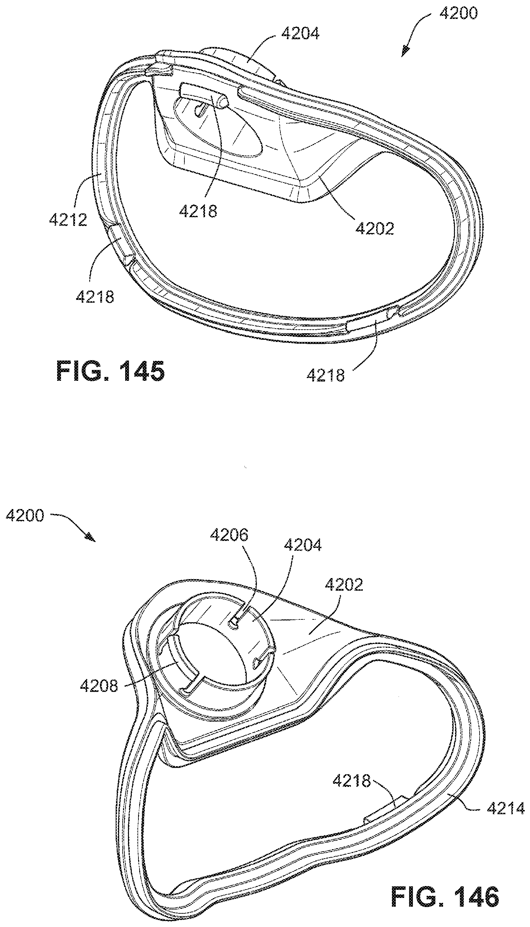

Referring to, for example, Figs. 126-130, the mouth cushion 4100 includes a groove 4114 that is configured to receive the cushion clip 4200. The groove 4114 almost completely encloses the cushion clip 4200. A small gap is provided in the groove 4114 to allow tabs 4218 (Figs. 139-145) of the cushion clip 4200 to extend outwards and engage with and interlock with slots 4310 (Figs. 140, 141 and 144) of the fascia 4300. The mouth cushion 4100 further comprises a lip seal 4112 to interface with a groove 4302 (Figs. 110, 115-117 and 144) of the fascia 4300 formed by a tab 4304. As shown, for example, in Figs. 116 and 117, the lip seal 4112 is shown as overlapping with the tab 4304 of the groove 4302 of the fascia 4300. However it should be appreciated that in the assembled state, the relatively flexible lip seal 4112 will deform and bend into the groove 4302 and be retained by the tab 4304 of the fascia 4300.

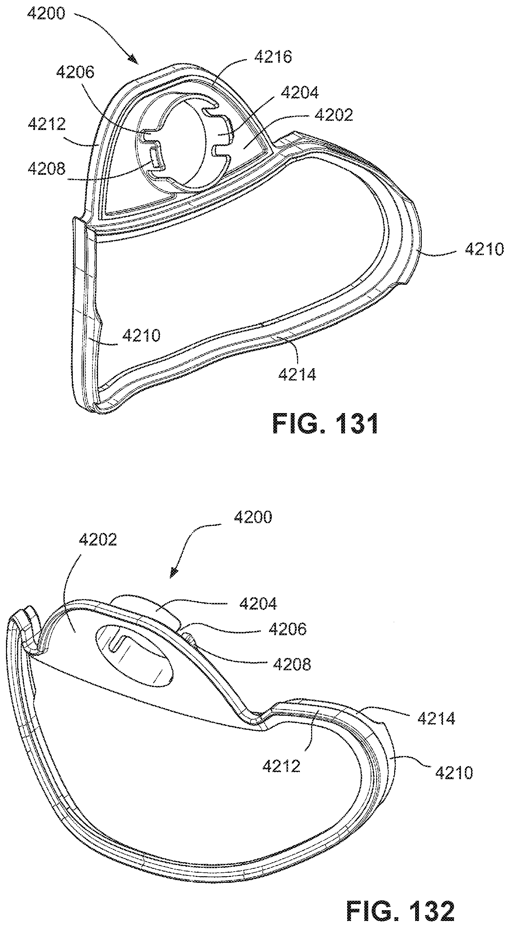

Referring to Figs. 131-137, the cushion clip 4200 comprises a fitting 4204 to receive a ring of the nasal seal portion (not shown). The fitting 4204 includes reliefs 4206 to permit flexing of the fitting 4204 and tabs 4208 to retain the ring of the nasal seal portion. The cushion clip 4200 further comprises supporting regions or portions 4210 on lower corners of the clip 4200 to increase stability and force on the cushion 4100 in the patient's cheek regions to provide a more stable seal on the soft areas of the patient's face. Supporting regions 4210 on lower corners of the clip 4200 may be thicker or stiffer than adjacent portions of the cushion clip 4200. A fascia contacting lip 4212 is provided to engage the groove 4302 of the fascia 4300. A cushion contacting lip 4214 is provided to engage the groove 4114 of the mouth cushion 4100.

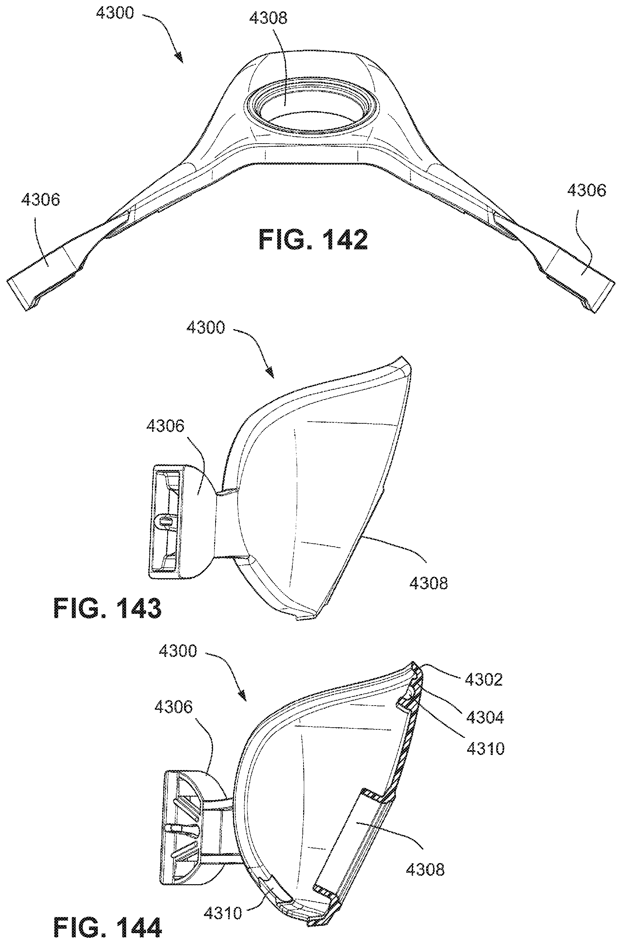

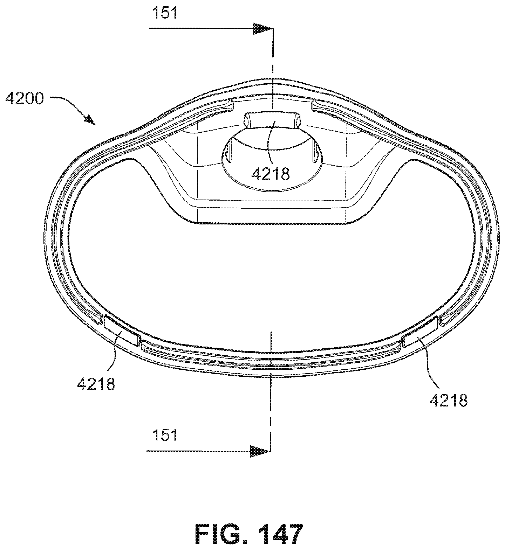

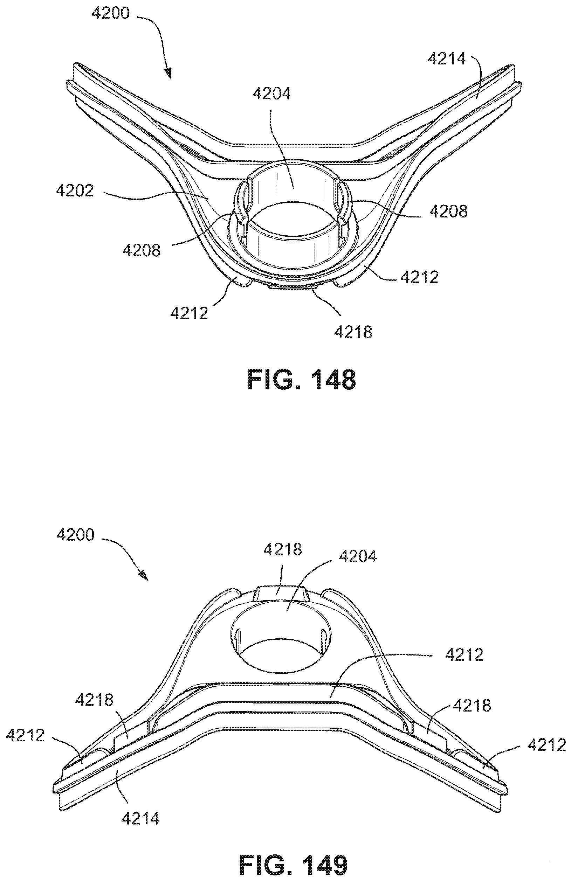

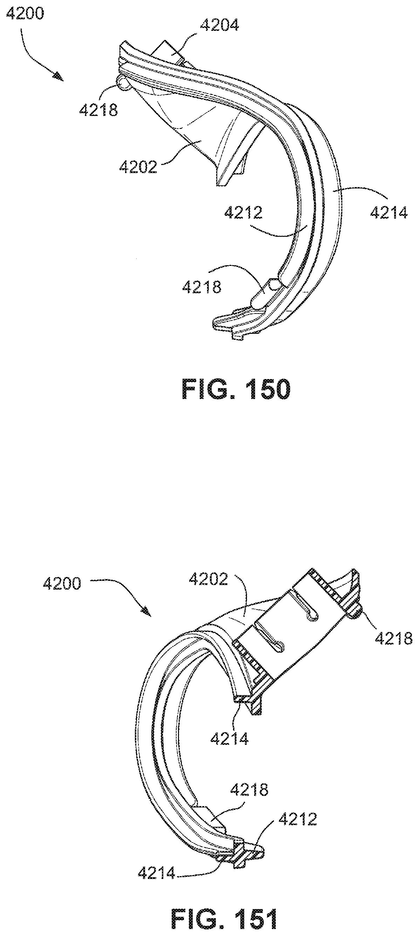

Referring to Figs. 138-151, the fascia 4300 comprises lower headgear connectors 4306 for connecting to the headgear and an elbow connector 4308 for connecting to an elbow adapted to be connected to a gas delivery tube or conduit. The rear surface of the fascia 4300 includes the groove 4302 (Fig. 144) adapted to engage flexible lip seal 4112 of the mouth cushion 4100. The rear surface also includes slots 4310 that are configured to receive and interlock with tabs 4218 of the cushion clip 4200. As shown in Figs. 144 and 151, the tabs 4218 are rounded to smoothly guide the tabs 4218 into the slots 4310 of the fascia 4300. Tabs 4218 may also be rounded so that the upper tab adjacent fitting 4204 may be connected first and in a first position, then the cushion clip can be rotated to position upper tab in a second position, this second position permitting lower labs adjacent lower headgear connectors 4310 (in use) to engage with fascia 4300.

### 5.3.8 Cushion-to-fascia interface

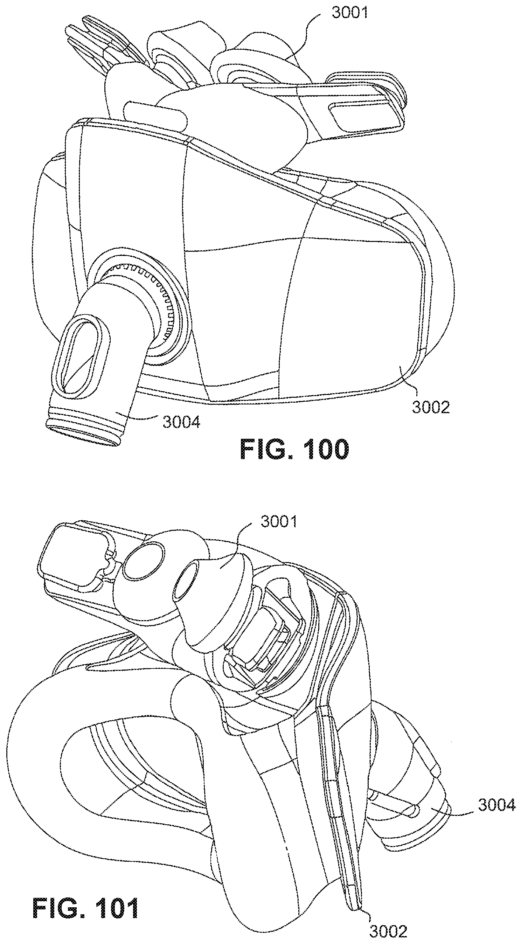

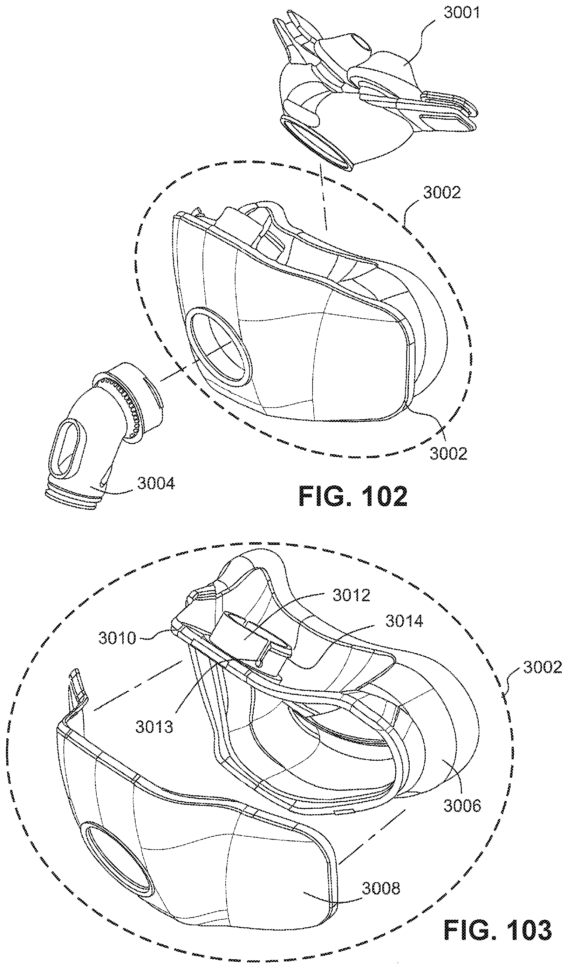

Figures 100-108 illustrate an example of a cushion to fascia interface. Figures 100 and 101 illustrate the patient interface assembly in its assembled condition, including pillows 3001, mouth cushion subassembly 3002 and elbow 3004. Figure 102 illustrates an exploded view thereof.

Figure 103 is an exploded view of subassembly 3002, including cushion 3006 and fascia 3008. Cushion 3006 includes a clip 3010 that may be overmolded on to the cushion. Cushion 3006 may be removably connected to clip 3010.

Clip 3010 may have a fitting 3012 for attachment to the pillows 3001. Fitting 3012 may comprise reliefs 3013 to permit fitting 3012 to flex inwards and thereby accept a nasal seal portion. Fitting 3012 may also comprise tabs 3014 adapted to lock or maintain the position of the nasal seal portion once it is connected. Fitting 3012 may be angled in such a way so as to present the nasal seal portion directly towards the patient's nares. For example, Figs 100 and 101 shows the nasal seal portion 3001 oriented to approximate the patient's nasolabial angle.

Clip may be more rigid than cushion, and may be made of Hytrel, polypropylene, nylon, injection molded plastic, etc.

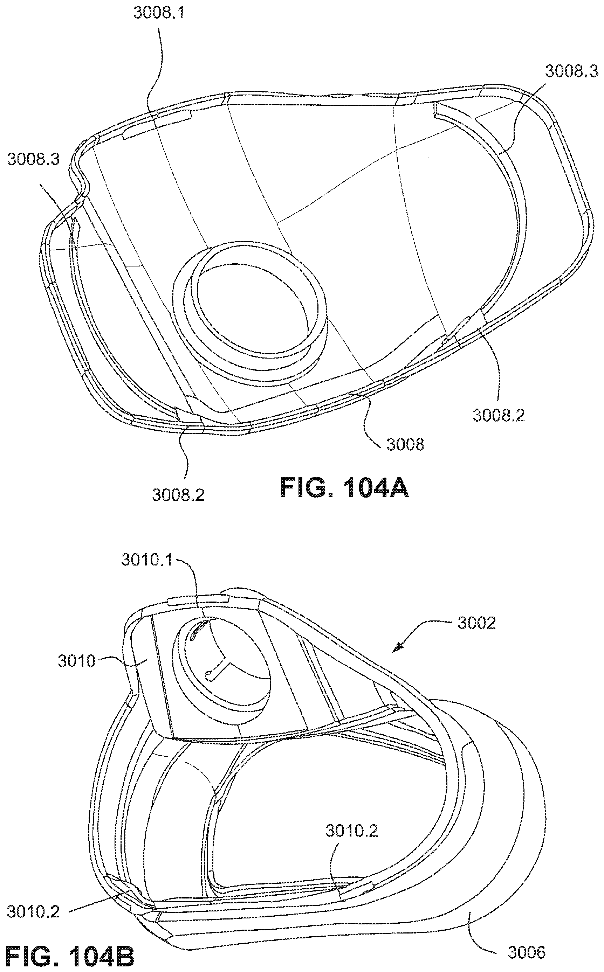

Fig. 104A shows the patient side of the fascia 3008. Fascia 3008 may include recess 3008.1 at the top of the fascia and recesses 3008.2 at the lower side corners of the fascia. Fascia 3008 may further include lugs or ridges 3008.3 adapted to align and/or position the mouth seal portion and/or clip. The outer side or non-patient contacting side of fascia 3008 may be curved or stepped such that the middle portion is raised compared to the side portions. This may aid a patient in picking up the fascia as they can grip on to the curved regions. These curved regions may further include gripping portions (not shown) such as dimples or overmolded buttons to aid the patient in gripping the fascia.

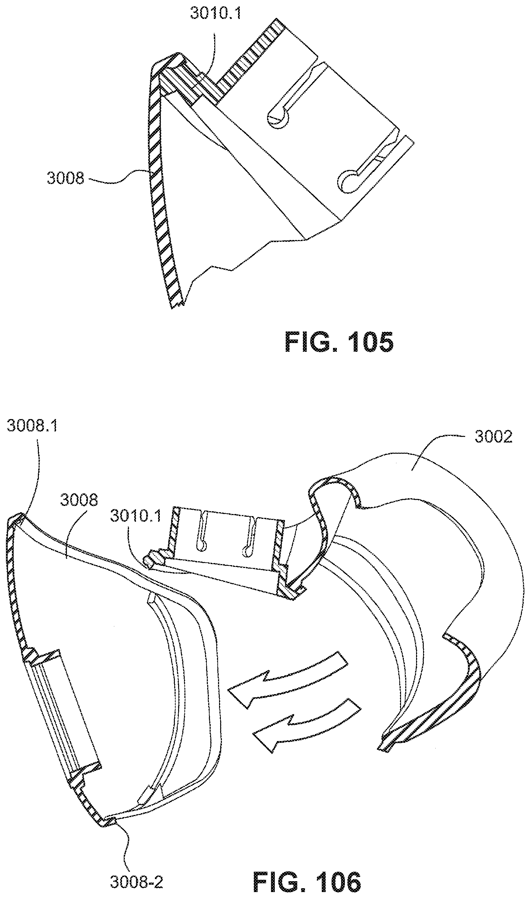

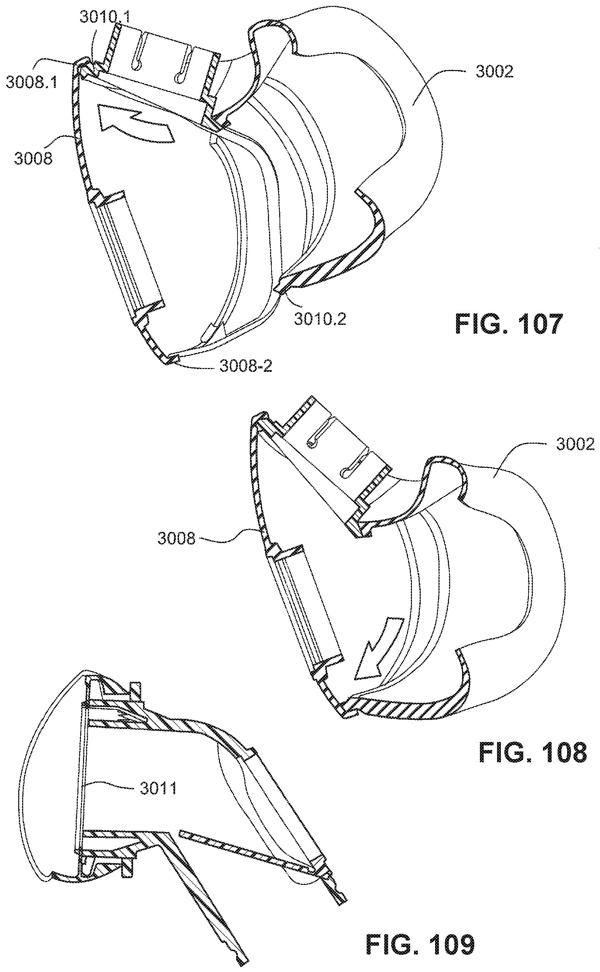

Fig. 103 is a further exploded view showing that the fascia 3008 and set clip 3010 may be clipped together at a number of locations, e.g., two or more, and in this case three locations. As shown in Figs. 104A, 104B and 105, fascia 3008 may have a recess 3008.1 to receive a projection 3010.1 of clip 3010. Figs. 106-108 show a sequence of an exemplary fitting assembly between the subassembly 3002. Fig. 106 shows initial engagement between the projection 3010.1 and recess 3008.1 at the top of the patient interface, after which the interface is pivoted in Figure 107. Once the lower end of clip readies to the lower part of fascia 3008, the clip snaps home for complete assembly.

The clip may include an integrated (silicone) lip seal to prevent leak between the clip and the fascia 3008. This would make the assembly more robust and less dependent on tolerances to prevent leak. The lip seal may be located in close proximity to the projections 3010.1 or 3010.2. Fig. 109 shows an example of a lip seal 3011 that may be used in such assembly.

### 5.3.9 Integrated patient interface System

Figs. 20 and 21 illustrate another patient interface system 101, which is an integrated patient interface system. The patient interface system 101 includes a nasal portion 120, a mouth portion 140 and a flexible tube 105 connected to the mouth portion 140 via an elbow 102.

The nasal portion 120 may utilize a nares sealing portion 122, which may be in the form of nasal pillows, prongs, a membrane seal such as a nasal cradle, and/or a nasal chamber. The nares sealing portion 122 may be in the form of the nares sealing portion 222 illustrated in Figs. 25 and 26. The nasal portion 120 may further include decoupling portion 125 and headgear connectors 121 adapted to connect to headgear 160.

The mouth portion 140 may include a structural portion 147, a mouth sealing portion 142, decoupling portion 145, headgear connectors 141, and vent 103, which may include one or more vent holes or slots for venting gas exhaled by the patient. A nasal portion connector 143 adapted to connect to the nasal portion 120 may be utilized when the nasal portion 120 and the mouth portion 140 are not formed as a unitary element. The patient interface system 101 could alternatively be formed as a modular patient interface system, where the nasal portion 120 and the mouth portion 140 are separable and may be used individually, and the nasal portion 120 is connectable to the flexible tube 105 via elbow 102.

Figs. 22-24 illustrate another patient interface system 201, which includes a nasal portion 220 and a mouth portion 240. The nasal portion 220 may include a nares sealing portion 222, an orifice 226 for receiving pressurized, breathable gas. The patient interface system 201 is illustrated with a nares sealing portion 222 such as illustrated in Figs. 25 and 26, although other nares sealing portions could be utilized, such as pillows, prongs, a nasal chamber, etc.

The mouth portion 240 includes a mouth seal portion 242 which may be in the form of a cushion for sealing with the mouth of the patient, a frame 247, a mouth orifice 246 for receiving the pressurized, breathable air, a decoupling portion 245, and a nares connecting portion 243 for connecting with the nasal portion. Fig. 22 is illustrated with the frame 247, the decoupling portion 245 and the connecting portion 243 removed. Although not illustrated, the mouth portion 240 may include connection to a flexible tube for delivery of the pressurized, breathable gas, such as the swivel connector, elbow and flexible tube illustrated in other embodiments.

The patient interface system 201 could alternatively be formed as a modular patient interface system, where the nasal portion 220 and the mouth portion 240 are separable and may be used individually, and the nasal portion 220 is connectable to the flexible tube 105 via elbow 202.

Fig. 29 illustrates a patient interface system 370, which may include a nasal portion 372, a mouth portion 380, and headgear 360. In this embodiment, the patient interface system 370 may have the nasal portion 372 and the mouth portion 380 integrated. The lower headgear strap 379 passes under the patient's ears and provides a normal headgear vector force or sealing force on the patient's face. The headgear strap 360 provides a supporting force to aide positioning and stabilization of the mouth portion 380 on the patient's face. The headgear strap 360 does not connect near the mouth of the patient to aid in keeping obtrusiveness of the patient interface to a minimum.

The nasal portion 372 may include nares sealing portion 374 and mouth sealing portion 375. Decoupling portions may be included.

Fig. 33 illustrates a schematic cross-section view of an integrated patient interface system 201 where the nasal portion 220 and the mouth portion 240 are formed as a unitary element. The decoupling portion 245 is formed in the mouth portion 240, and may decouple forces applied to the patient interface system (such as from a flexible supply tube) from the mouth portion 240. The nares sealing portion 222 is a nares sealing portion such as illustrated in Figs. 25 and 26. The nose tip engagement portion 254 seals with the patients nose tip and the upper lip engagement portion seals with the patient's upper lip. This schematic cross-sectional view omits the connection to the flexible tube.

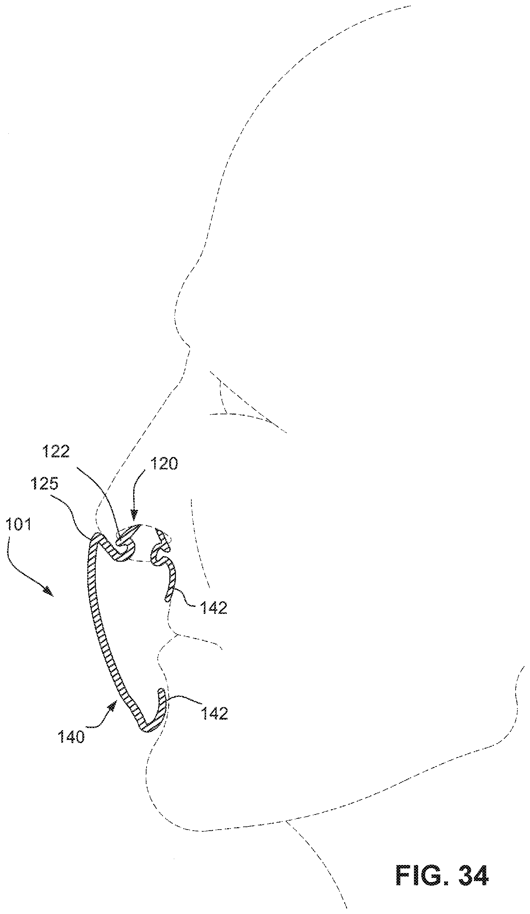

Fig. 34 illustrates a schematic cross-sectional view of an integrated patient interface system 101, where the nasal portion 120 and the mouth portion 140 are formed as a unitary element. This schematic cross-sectional view omits the connection to the flexible tube 105. In this embodiment, the mouth sealing portion 142 extends from the mouth portion 140 where the mouth sealing portion 142 seals with the area between the patient's lower lip and the patient's chin, and extends from the nasal portion 120 where the mouth sealing portion 142 seals with the patient's upper lip area. A decoupling portion may be formed in the mouth portion 140, and may decouple forces applied to the patient interface system (such as from a flexible supply tube) from the mouth portion 140.

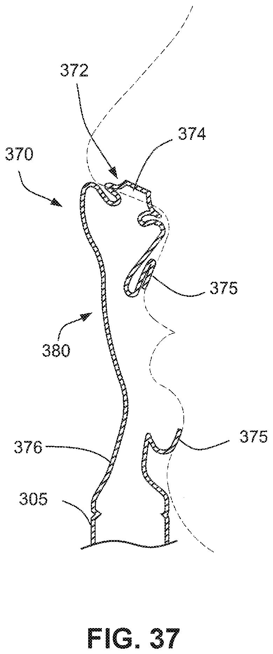

Fig. 37 illustrates a schematic cross-sectional view of an integrated patient interface system 370, where the nasal portion 372 and the mouth portion 380 are formed as a unitary element. The nasal portion 372 includes a nares sealing portion 374 that forms a seal with the patient's nares, and a mouth sealing portion 375 that forms a seal with the patient's mouth. A gusset 376 may be provided to connect the mouth portion 380 to flexible tube 305. The gusset 376 may function to decouple movement of flexible tube 305 from disrupting the seal of the mouth portion 380 and/or the nasal portion 372.

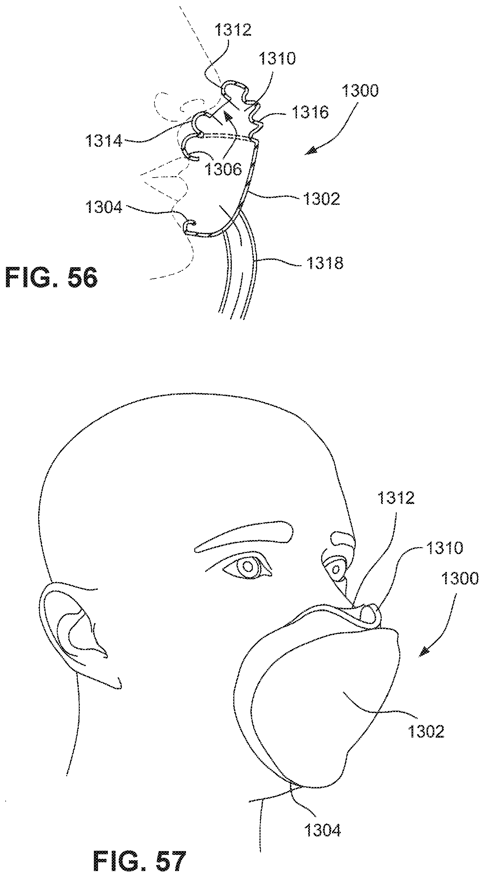

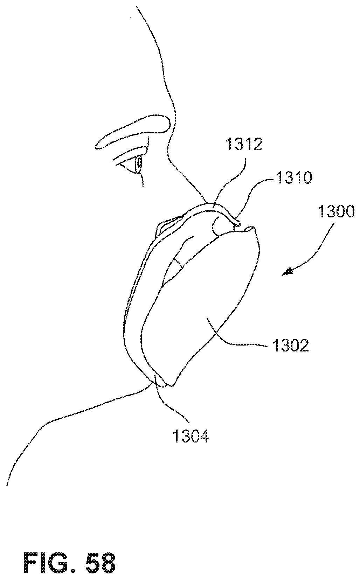

Figs. 56-58 illustrate an integrated patient interface system 1300. The patient interface system 1300 includes mouth portion 1302 and nasal portion 1310. The mouth seal of the mouth portion 1302 is oriented generally horizontally, while the nasal portion 1310 is oriented generally vertically to anatomically match the mouth and nares of the patient. A gas washout vent may also be included.

The mouth portion 1302 includes a lower lip engagement portion 1304 that is adapted to form a seal with a patient's lower lip, an upper lip engagement portion 1306 adapted to form a seal with the patient's upper lip, and an aperture adapted to receive pressurized air from air supply hose 1318. An elbow and/or swivel may be used in conjunction with the air supply hose 1318.

The nasal portion 1310 includes a nares engagement portion 1314 adapted to form a seal with the patient's upper lip, a nose tip engagement portion 1312 adapted to form a seal with the patient's nose tip, and a concertina or decoupling portion 1316 (e.g., see Fig. 56). The concertina portion 1316 decouples forces applied to the mouth portion 1302 from being applied to the nasal portion 1310. The concertina portion may also fill with air, and utilize air pressure to increase the force and thereby provide a more robust seal.

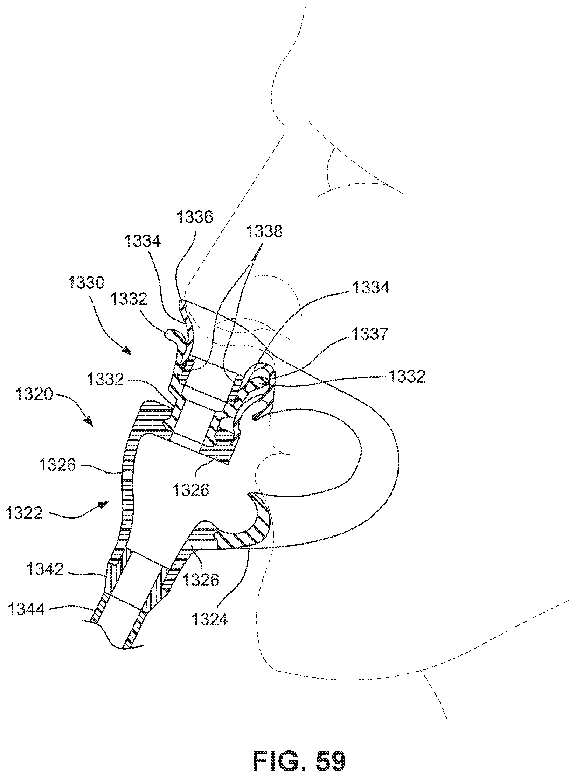

Fig. 59 illustrates an integrated patient interface system 1320. The patient interface system 1320 includes a mouth portion 1322 and a nasal portion 1330.

The mouth portion 1322 includes a lower lip engagement portion 1324 adapted to form a seal with the patient's lower lip, and a frame 1326. The frame 1326 may be a rigid material, e.g., polycarbonate. The lower lip engagement portion 1324 may be co-molded with the frame 1326.

The nasal portion 1330 may include a supporting portion 1332, a sealing portion 1334 and a clip portion 1338 adapted to engage the sealing portion 1334 with the supporting portion 1332. The supporting portion 1332 may be a semi-rigid material, e.g., nylon. The clip portion 1338 may be a semi-rigid material, e.g., nylon or polypropylene.

The sealing portion 1334 includes an upper lip engagement portion 1337 adapted to form a seal with the patient's upper lip, and a nose tip engagement portion 1336 adapted to form a seal with the patient's nose tip. The sealing portion 1334 may be a flexible material, e.g., silicone. The sealing portion 1334 may be co-molded as one piece with the frame 1326.

An air supply hose 1344 may be connected to the mouth portion 1322 by a swivel 1342. The swivel 1342 may be a rigid material, e.g., polycarbonate.

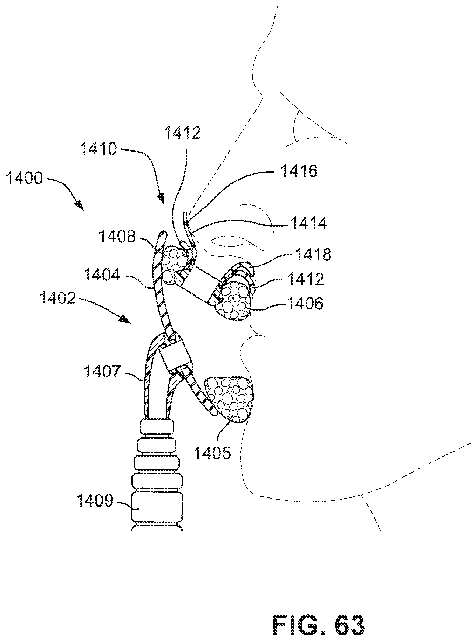

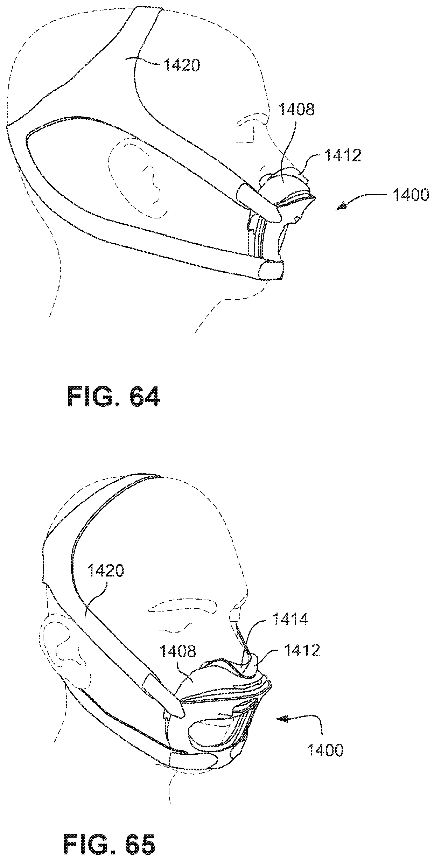

Fig. 63 illustrates a modular patient interface system 1400 and Figs. 64 and 65 illustrate a related integrated patient interface system (which could be modular). The patient interface system 1400 includes mouth portion 1402 and nasal portion 1410. An elbow 1407 connects an air supply hose 1409 to the mouth portion 1402 to deliver air to the patient interface system 1400. Headgear 1420 connects to the patient interface system 1400 to secure the patient interface system 1400 to the patient's head.

The mouth portion 1402 includes a front portion 1404 and a lower foam portion 1405. The lower foam portion 1405 is positioned to form a seal between the patient's lower lip/chin and the mouth portion 1402. The front portion 1404 may be a clear portion allowing visibility of the patient's mouth region. The front portion 1404 may be silicone, for example.

The nasal portion 1410 may include a supporting portion 1412 and a sealing portion 1414. The sealing portion 1414 may include a nose tip engagement portion 1416 adapted to form a seal with the patient's nose tip, and an upper lip engagement portion 1418 adapted to form a seal with the patient's upper lip. The supporting portion 1412 may also form a seal with the patient's upper lip. An upper foam portion 1406 may be disposed between the nasal portion 1410 and the patient's upper lip to provide additional comfort. An outer foam portion 1408 may be disposed between the front portion 1404 and the nasal portion 1410. The upper foam portion 1406 and the outer foam portion 1408 may be one foam piece, such as a ring shaped piece of foam, with a hole formed in the foam for insertion of the nasal portion 1410.

### 5.3.10 Kits

Various components of the disclosed patient interface systems may be utilized in one or more kits that may be provided. For example, Fig. 39 illustrates a nares only kit 805 that may be provided according to embodiments of the present technology. The nares only kit 805 may include a nasal portion 820 adapted to seal with and provide respiratory therapy to a patient's nares, and nasal portion headgear 812. The nares only kit may optionally include an elbow or other connector 802 that may connect the nasal portion 820 to a supply of pressurized air, although such an elbow or other connector 802 may be provided as part of the nasal portion 820. The nasal portion 820 may include plug 35 in the embodiment of Figs. 7-1 to 10. However the nares only kit 805 may be provided with a commercially available patient interface, e.g., the Swift FX patient interface. Plug 35 may be removed if utilized with the mouth portion 840.

The nares only kit 805 is adapted to provide a patient with respiratory therapy to a patient's nares. This form of respiratory therapy, provided to the nares only, is preferred because it is less obtrusive to wear, and more comfortable for a patient, and many patients only need nares therapy. However, some patients may need mouth therapy, either mouth only therapy or mouth and nares therapy. For example, some patients who may be mouth breathers need a mouth portion to provide a mouth seal so that effective therapy may be applied to the nares. Other patients may need mouth only therapy or mouth and nares therapy.

Accordingly, mouth kit 810 may be provided to a patient that needs mouth only therapy, or needs mouth and nares therapy but already has a nares only device or nares only kit 805. The mouth kit 810 may include a mouth portion 840 that is adapted to seal with and provide respiratory therapy to a patient's mouth, and mouth portion headgear 850. The mouth portion 840 may be adapted to provide respiratory therapy only to a patient's mouth. The mouth kit 810 may optionally include an elbow or other connector 802 that may connect the mouth portion 840 to a supply of pressurized air, if such a connector is not incorporated in the mouth portion 840.

The mouth portion 840 may optionally be adapted to connect pneumatically to nasal portion 820 or another nares only CPAP device. For example, the mouth portion 840 may include the aperture 46 illustrated in Fig. 7-5 adapted to connect to a nasal portion, in which case plug 35 may also be provided in the mouth kit 810 for plugging aperture 46. The mouth portion 840 may also optionally include the valve 55 for selectively closing the aperture 46. Further, the mouth kit 810 may be optionally provided with plug 803, which may be used to plug an aperture in the front of the mouth portion 840, and may optionally be provided with double elbow 855, that may be used to connect both the mouth portion 840 and the nasal portion 820 to a supply of pressurized air. The mouth portion 840 may function as a docking station, where the mouth portion 840 is adapted to dock with the nasal portion 820.

The mouth kit 810 may also be optionally provided with nares and mouth portion headgear 860, which includes a connection region in the back portion, such as connection region 67 illustrated in Fig. 13. The nares and mouth portion headgear 860 may be used instead of the nasal portion headgear 812 and the mouth portion headgear 850. Further, the mouth kit 810 may also be optionally provided with connected nares and mouth portion headgear 870, which includes a connector 856 for connecting the nares only headgear and the mouth only headgear at a back portion.

The nares only kit 805 may be utilized as a retrofit kit to a patient having a mouth portion kit 810 or other mouth portion, who wants to convert his mouth only device into a mouth and nares device. In this case, the nares only kit may optionally be provided with the double elbow connector 855, with the plug 803, with the nares and mouth portion headgear 860, or with the connected nares and mouth portion headgear 870. Likewise, the mouth kit 810 may be provided as a retrofit kit to a patient having a nares only kit 805 or other nasal portion, who wants to convert his nares only device into a nares and mouth device.

The patient may utilize the nares only kit 805 and/or the mouth kit 810 to convert the nares only CPAP device into a nares and mouth CPAP device in several ways. The patient may remove the elbow or other connector from the nasal portion 820, and connect the double elbow connector 855 to both the nasal portion 820 and to the mouth portion 840, so that air is delivered through the double elbow connector 855 to both the nasal portion 820 and the mouth portion 840. The patient may utilize the original nasal portion headgear 812 on the nasal portion along with mouth portion headgear 850 on the mouth portion 840. The connector 856 may optionally be utilized to connect the nasal portion headgear 812 to the mouth portion headgear 850. Alternatively, nares and mouth portion headgear 860 could be provided to connect to the nasal portion 820 to the mouth portion 840. The nares and mouth portion headgear 860 may be connected in a back portion such as through using a connection region 67 or a unified strap as illustrated in Fig. 13. If the nasal portion 820 is provided with the structure adapted to connect to a mouth portion, such as the connector 30 illustrated in Fig. 7-5, then the mouth portion 840 provided in the mouth kit 810 may include structure to connect the nasal portion 820 to the mouth portion 840, such as opening 46 illustrated in Fig. 11-1. The valve 55 and knob 54 may also be provided.

In embodiments including the structure allowing pneumatic connection between the nasal portion 820 and the mouth portion 840, such as the connector 30 and opening 46, the patient may connect the connector 30 to the opening 46, and utilize the elbow or other connector 802 on the nasal portion 820 to deliver the air to the patient's nares and to deliver the air to the patient's mouth through the connector 30 and opening 46 to the mouth portion 840. In this embodiment, the plug 803 is connected to the opening on the front of the mouth portion 840. Structure for plugging the connection between the nasal portion 820 and the mouth portion 840 could be used, such as a plug 35 or the valve 55, and the mouth portion 840 could then be used as a mouth seal.

Alternatively, in embodiments including the structure allowing pneumatic connection between the nasal portion 820 and the mouth portion 840, such as the connector 30 and opening 46, the patient may connect the connector 30 to the opening 46, and utilize the plug 803 to plug the opening in the nasal portion 820. The elbow or other connector 802 may then be connected to the opening in the front of the mouth portion 840 to deliver the pressurized air to the mouth portion 840, and through the connector 30 and opening 46 to the nasal portion 820. Structure for plugging the connection between the nasal portion 820 and the mouth portion 840 could be used, such as a plug 35 or the valve 55, and the nasal portion 820 could then be used as a nares seal.

The nares and mouth kit 801 may include the components of the nares only kit 805 and the mouth kit 810. The nares and mouth kit 801 may allow a patient selectively switch between using the nasal portion 820 alone, to using the mouth portion 840 alone, or to use the nasal portion 820 and the mouth portion 840 together. This allows the patient to periodically change how the devices feel on the patients face. For example, if the patient's nares area becomes irritated (e.g., by air jetting and/or concentrated sealing forces), the patient may switch to using the nasal portion 820 and the mouth portion 840, or the mouth portion 840 alone. The patient may switch on a periodic basis, e.g., nightly, weekly, monthly, etc.

Fig. 43 illustrates a prior art nares only CPAP device 901 that provides CPAP therapy to a patients nares. The CPAP device 901 may include a nasal portion 910 forming a seal with the patient's nares, an elbow connector 912 connecting the nasal portion 910 to a tube 915 supplying pressurized gas to the nasal portion 910, where the tube extends along the patient's nose, between the patient's eyes and over the patients head, where it is connected to a supply of the pressurized gas. The elbow connector may include a vent 914 for venting gas exhaled by the patient, with the vent having a removable insert. The nasal portion 910 may include a nares sealing portion such as nasal pillows, nasal prongs, a nasal membrane, or a nasal chamber. Headgear 918 may secure the nasal portion 910 to the patient's head.

Figure 44 illustrates a side view of how the prior art nasal only device 901 may be converted to a nares and mouth patient interface system 940 according to an embodiment of the present technology. The nasal portion 910 and the elbow 912 may be removed and the patient interface system 940 including may be connected to the tube 915, such as by a connector 924. The prior art nasal only device may be a "Breeze^{®} Sleepgear^{®}" device, for example.

The patient interface system 940 may include a mouth portion 942 for forming a seal with the patient's mouth and a nasal portion 952 for forming a seal with the patient's nares. The mouth portion 942 may include headgear connectors 946 for connecting to headgear 954, and the nasal portion 952 may include headgear connectors 944 for connecting to headgear 950. The patient interface system 940 may be a modular patient interface system or an integrated patient interface system, and is capable of delivering pressurized air to the patient's nares and/or mouth.

The patient interface system 940 may alternatively utilize the nasal portion 910, and add a mouth portion, where the patient interface system 940 is a mouth portion. In this embodiment, the vent 914 of the elbow 912 is used for connection to the mouth portion 942, where the insert in the vent 914 is removed, and the mouth portion 942 is adapted to connect to the vent pneumatically. In this embodiment, air may flow out of the vent 914 to the mouth portion 942, and any original headgear, such as headgear 918 may be retained.

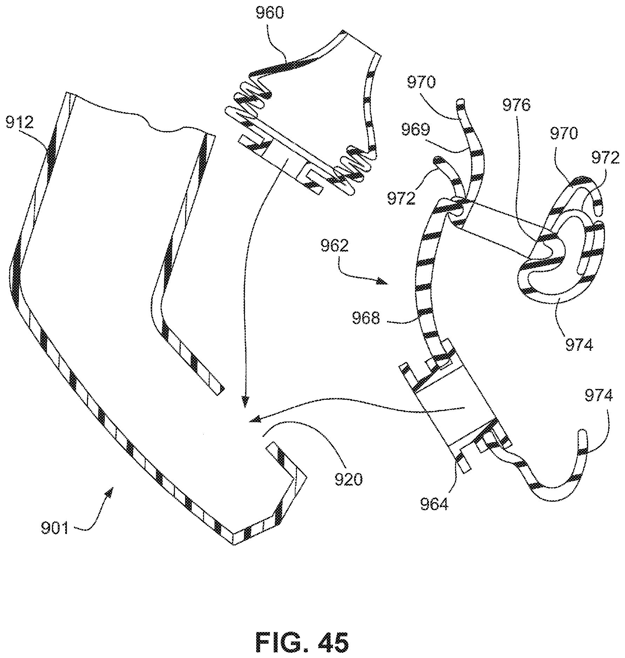

Figure 45 illustrates a schematic side view of another modification of the nares only device 901. In this embodiment, the nasal pillows 960 are removed from the openings 920 in the nares only device 901, and a patient interface system 962 is connected to the nares only device 901 in place of the nasal pillows 960. The patient interface system 962 has a pair of connectors 964 adapted to connect to the openings.

The patient interface system 962 includes a mouth portion 968 and a nasal portion 969. The mouth portion 968 forms a seal with a patient's mouth at mouth seal 974 and receives the pressurized air through the connectors 964. The mouth portion 968 is connected to the nasal portion 969 with a connector 976. The nasal portion 969 may include nares engagement portion 970 adapted to form a seal with the patient's nares and support portion 972. The nasal portion 969 may alternatively be another type of nasal portion, such as nasal pillows or prongs, or a nasal chamber.

The patient interface systems 940 and 962 may be used to convert a nares only device for delivering respiratory therapy to a patient into a device for delivering nasal and/or mouth respiratory therapy to a patient.

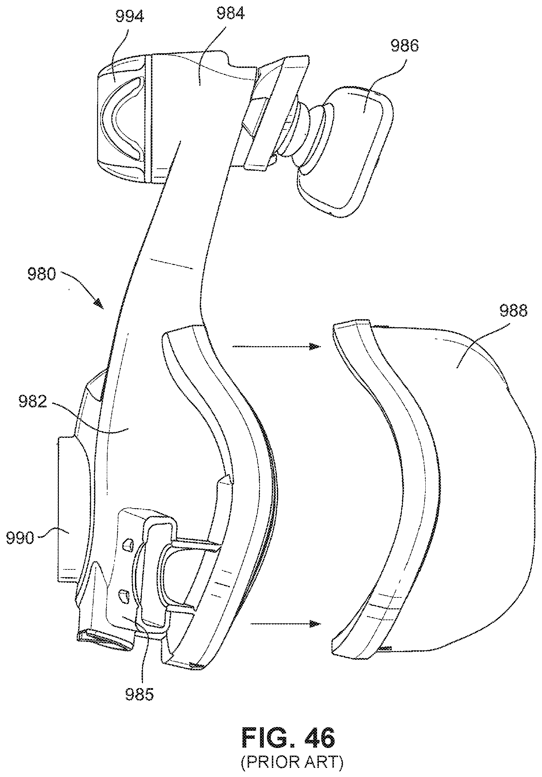

Fig. 46 illustrates a prior art nares only CPAP device 980 that provides CPAP therapy to a patient's nares. The CPAP device 980 may include a nasal portion 982 that includes a cushion 988 forming a seal with the patient's nares, a connector 990 for connecting to a source of pressurized gas, headgear connectors 985 for connecting to headgear and a forehead portion 984. The forehead portion 984 includes a forehead support pad 986 and dial 994 for adjusting a position of the forehead support pad 986. The cushion 988 may be removed by pulling it in the direction of the arrows.

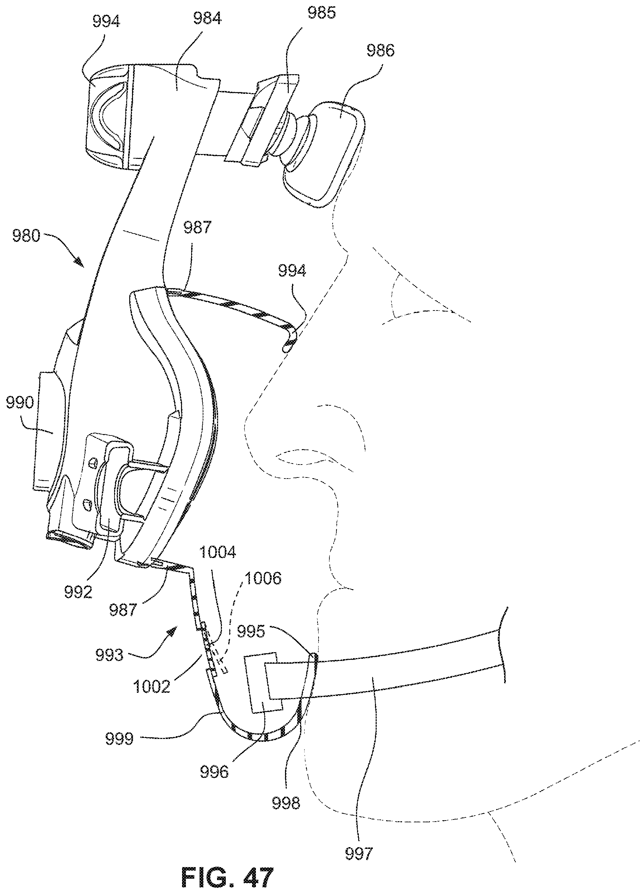

Figure 47 illustrates a side view of how the prior art nasal only device 980 may be converted to a nares and mouth patient interface system 993 according to an embodiment of the present technology. The cushion 988 may be removed and the nares and mouth patient interface system 993 may be connected to the nasal only device in place of the cushion 988.

The nares and mouth patient interface system 993 may be adapted to connect to the frame of the nasal only device 980 in place of the cushion by being formed with an interface 987 in the same shape as the cushion 988. The nares and mouth patient interface system 993 includes a cushion 998 adapted to seal with a face of the patient, and may include a nose engagement portion 994 and a lower lip engagement portion 995. A headgear connector 996 may connect to headgear 997. The nares and mouth patient interface system 993 may thus be utilized to convert the nares only device 980 into a nares and mouth patient interface system 993. The front portion 999 of the patient interface system 993 may be formed with a rigidizer to provide additional rigidity.

The nares and mouth patient interface system 993 may include an AAV (anti-asphyxia valve), which may have and opening 1002 and a flap 1004. When pressure is applied to the patient interface system 993 such as delivered from an air delivery tube, the flap 1004 is in the closed position. When no pressure is applied, the flap 1004 moves to the open position 1006, allowing a user to breathe through the opening 1002. While not shown, the embodiment of Fig. 45 may also include such an AAV.

The patient interface systems described herein may be used to provide a method of providing respiratory therapy to one of a patient's nares only, a patient's mouth only, or to both a patient's nares and mouth, and periodically changing the respiratory therapy to another of the patient's nares only, the patient's mouth only, or to both the patient's nares and mouth. For example, the respiratory therapy may be initially provided to the patient's nares only, and then periodically changed from being applied to the patient's nares only to being applied to the patient's nares and mouth.

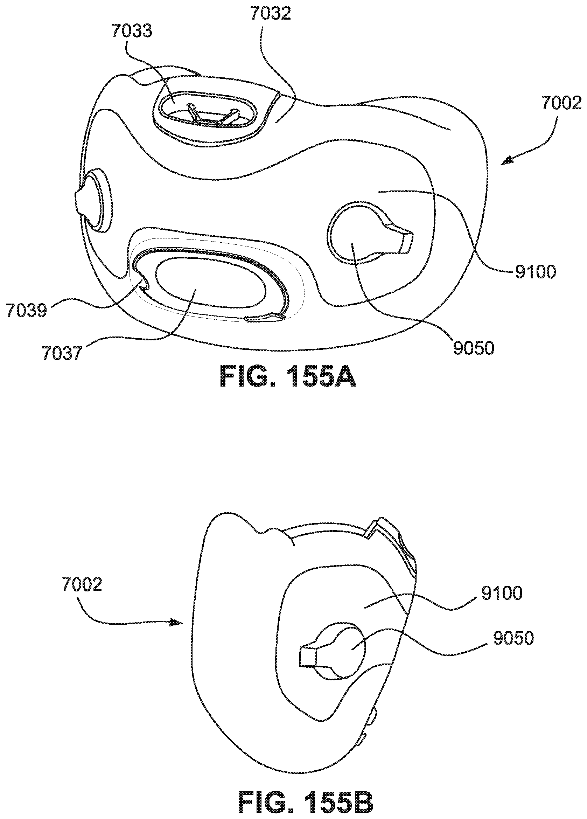

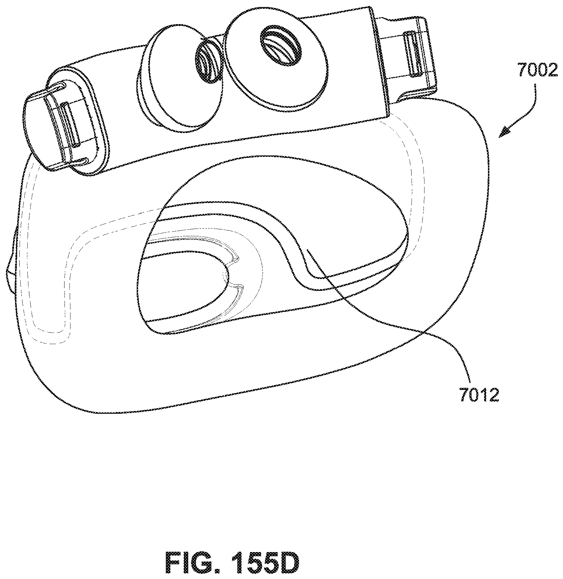

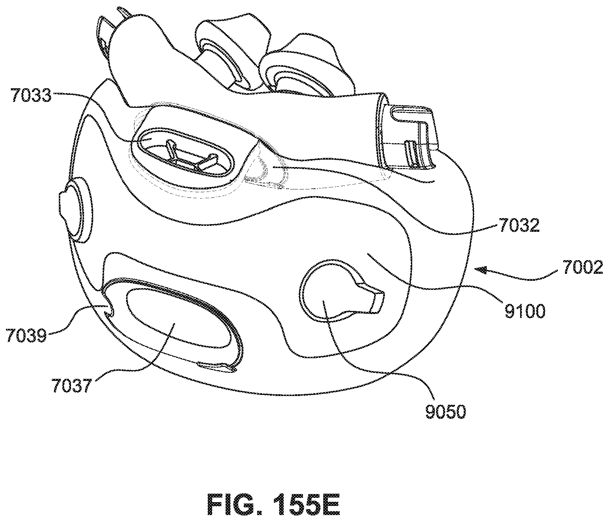

It may be advantageous for patients if the nasal cradle (e.g. pillows) cushion were able to be used in a modular manner. For example, it may be configured to be used as an oro-nasal mask comprising a separable nasal cradle and oral cushion. This system allows interchangeable nasal portions for patient preference. The patient can be sent a system with each nasal cradle, nasal pillows and mouth cushion components and select which configuration to use to suit their particular needs or preferences. If the patient is a mouth breather, they can connect the mouth cushion to a nasal cushion and wear the system as an oro-nasal. If the patient is a nose breather, they can elect to use a nasal cushion only and place a vent module in the nasal connection opening. Examples of the present technology provide connector geometry such that different nasal portions (e.g., either a nasal cradle version shown in Fig. 155D or a nasal pillows version shown in Fig. 155E) are able to be attached to the same mouth cushion or to different mouth cushions, using different headgears and/or the same gear, which may be configurable.

It may beneficial if the nasal portion (e.g. nasal cushion) is able to be used in a modular manner. For example, if the nasal portion is configured as a nasal only patient interface and to be used as an oro-nasal mask comprising a separable nasal cushion and oral cushion.

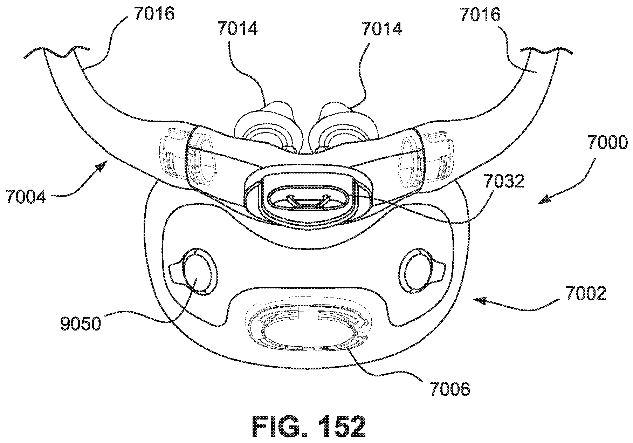

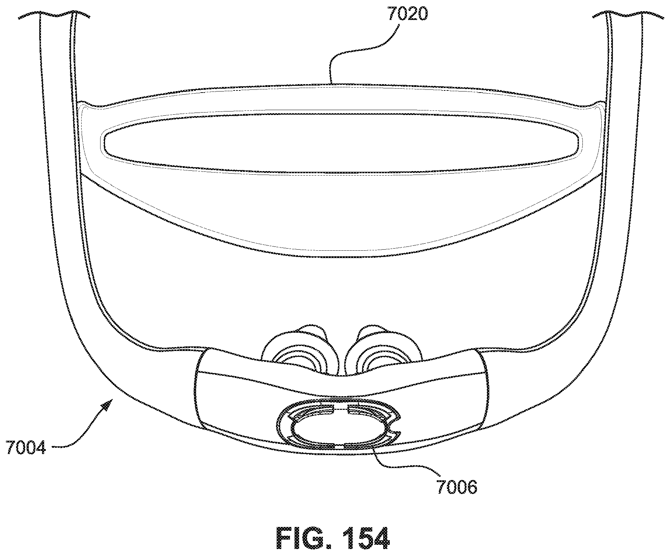

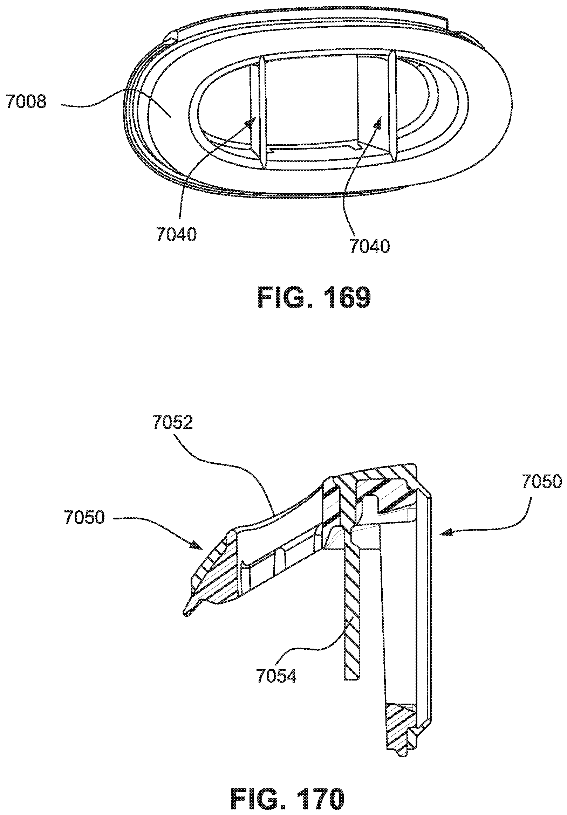

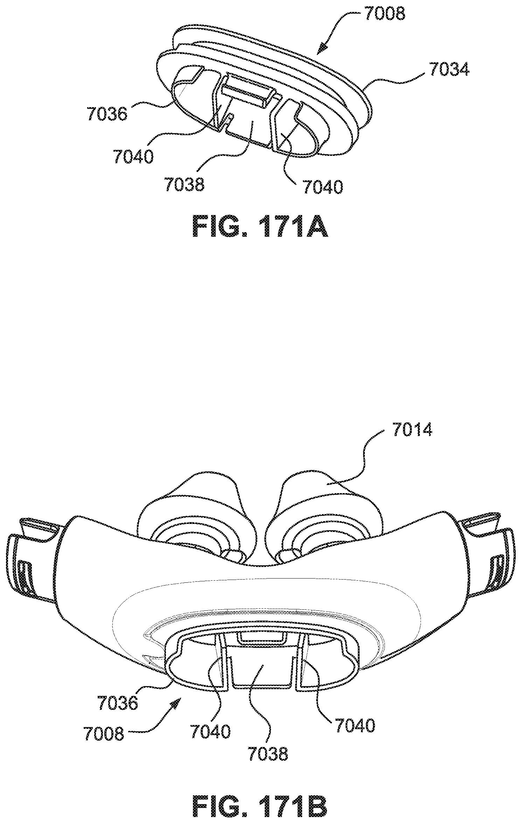

In an example embodiment illustrated in Figs. 152 to 154, which illustrates an exemplary embodiment of the present invention, a patient interface 7000 comprises a mouth or oral portion 7002 (e.g., an oral cushion) that is configured as a removable component from the nasal portion 7004 (e.g., nasal cradle), whereby the two effectively may perform as individual chambers during therapy. In order to connect the oral portion 7002 to the nasal portion 7004, a removable vent 7006 of the nasal portion 7004 may be removed and replaced with a connector 7008 (see examples of connector 7008 in Figs. 169 and 171). The vent 7006 allows for gas exhaled by the patient to be exhausted from the nasal chamber whereas the connector 7008 is designed to form part of a fluid coupling between the nasal and mouth portions so as to allow air to flow from the nasal portion chamber 7010 into the oral chamber 7012 of the mouth portion 7002 so as to enable therapy via the patient's oral interface.

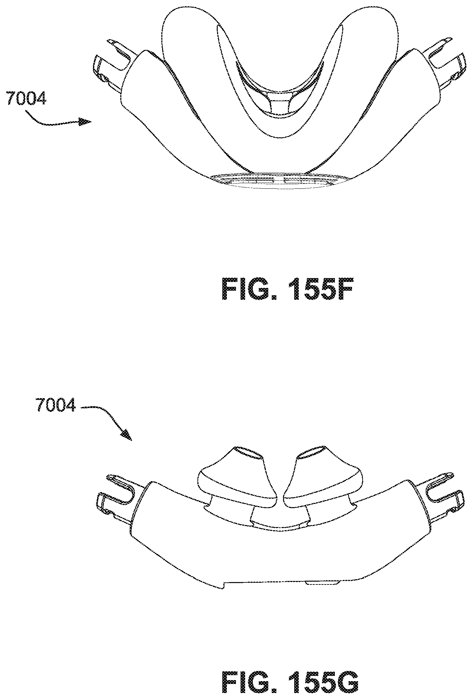

In the illustrated form, the nasal portion 7004 is in the form of a nasal cushion. Whilst in the illustrated form, the nasal cushion is a pillow cushion including nasal pillows or prongs 7014 (each nasal pillow being constructed and arranged to form a seal with a respective naris of the nose of a patient), the nasal portion may be similarly formed as a cradle cushion. Indeed, a feature of the patient interface 7000 with its modular design is that one type of nasal cushion may be swapped out with another type to suit requirements or user preference. Fig. 155F illustrates an example of a nasal portion including a pillow cushion. Fig. 155G illustrates an example of a nasal portion including a prongs.

The nasal portion 7004 includes inlet conduits 7016 to introduce the pressurized, breathable gas into the nasal chamber 7010. The inlet conduits or tubes 7016 may form a part of a hoop that passes over the top of the patient's head. The hoop incorporates the tubes 7016 and the nasal portion 7004 (e.g., cushion). In use, the tubes extend across the patient's cheek regions and the hoop is preferably arranged so that portions of the tubes contact regions of the patient's head superior to an otobasion superior of the patient's head. In the illustrated form, the conduits are joined at junction 7018 which is arranged in use to be disposed to the crown region of the patient's head. The junction is arranged to be connected to a source of pressurised air. In addition, the conduits are flexible and may be extensible along their length to a limited extent (such as by being concertined along a portion of their length) to improve patient comfort and fit. It is to be appreciated that in alternative embodiments of the technology, the pressurised air may also be delivered to the nasal chamber 7010 in other configurations of air circuits as described.

Headgear strap 7020 may be connected to the conduits 7016, in the illustrated form through eyelets 7022, to support and stabilise the patient interface. Due to the modularity of the patient interface to operate in both a nasal only and an oro-nasal mode, the headgear strap 7020 may include complementary patient interface connectors adapted to connect the headgear strap to either or both the nasal portion and the mouth portion depending on the mode of use of the patient interface 7000.

The nasal portion chamber 7010 includes an opening 7024 that allows for part of a fluid coupling between the nasal portion and mouth portion. A coupling component (given general reference 7026) forms part of the fluid coupling and is used to connect components to the nasal portion 7004, such as vent 7006, connector 7008, or directly to the mouth portion 7002. In an embodiment of the patient interface, nasal portion and mouth portion are in direct contact (without use of an intermediate connector 7008) such that there is direct connection between the mouth portion and nasal portion when the patient interface is in an oro-nasal configuration. The coupling component 7026 may take various forms but typically surrounds the exterior of the opening 7024 so as to form part of a sealed connection for fluidly connecting the nasal chamber 7010 to the other components.

Figs. 155a to 155c, illustrate an embodiment of the mouth portion 7002 in more detail. A feature of the mouth portion is that it includes a complementary opening 7028 formed in the mouth portion 7002 of cushion that forms part of the mouth chamber 7012. In the illustrated form, the opening 7028 is formed within a depression of the mouth portion 7002 (e.g., mouth cushion) extending along an upper region of the mouth portion 7002 (e.g., mouth cushion). When in oro-nasal mode, the nasal portion 7004 locates at least partially within the depression to provide a more compact design. For example, the oral chamber chassis has a depression to partially swallow/nest the nasal cradle when they are connected. This may achieve a sleeker overall profile. Further, the length of the connector 7008 allows for close cushion to cushion contact between the nasal and mouth portions.

A coupling component (given general reference 7030) is associated with mouth opening 7028 to form part of the fluid coupling between the mouth portion 7002 and nasal portion 7004. Whilst the coupling component 7030 make take various forms, it typically surrounds the exterior of the opening 7028 so as to form part of a sealed connection for fluidly connecting the mouth chamber 7012 to the nasal portion 7004.

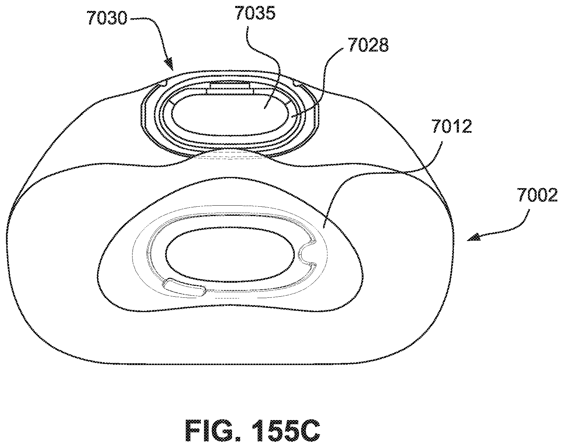

In one form, as illustrated the mouth portion coupling component 7030 is integrated as part of an AAV (anti-asphyxia valve) 7032, which may have an opening 7033 and a flap 7035. When air pressure is applied to the mouth chamber 7012 such as delivered through the fluid coupling from the nasal portion 7004, the flap 7035 is in the closed position and seals off the opening 7033. When no pressure is applied, the flap 7035 moves to the open position (as shown in Fig. 155c), allowing a user to breathe through the opening 7033.

In some forms, the AAV 7032 forms part of the mouth chamber coupling component 7030. In a particular form, the coupling component 7030 and the AAV 7032 is formed as an overmoulding incorporating a silicone overlay and a rigid backing (underlay). Typically, the backing is plastic and allows for the coupling component 7030 to provide a hard snap when connecting to the nasal portion 7004 via the connector 7008 or directly. The AAV may be part of the same silicone shot as the coupling component 7030 overlay. The mouth portion 7002 (e.g., mouth cushion) may be completed in two shots of silicone and bonds together such that the AAV is integrated with the cushion and has not gaps or crevices for bacteria to grow. With this arrangement, the connector and/or the AAV may be formed as a unitary piece. In some examples, the connector may be formed from multiple components.

The mouth portion 7002 further comprises a vent opening 7037, that incorporates an associated coupling component 7039. The opening 7037 and coupling component 7030 are arranged to be complementary to the opening 7024 in the nasal chamber 7010. With this arrangement the vent opening 7037 is arranged to receive the vent 7006. In this way, the removable vent module 7006 is adapted to be attached to the nasal chamber 7010 to vent gas exhaled by the patient and is arranged to be releasably connectable with the nasal chamber coupling component such that the patient interface system is configurable in a first mode where the vent is fluidly coupled to the nasal chamber to enable the patient interface system to operate as a nasal only patient interface and a second mode where the vent is removed to enable the nasal portion to be fluidly coupled to a mouth portion to communicate a portion of the pressurized, breathable gas through the nasal chamber opening to the mouth portion. Further when in this second (oro-nasal) mode, the vent module 7006, removed from the nasal chamber 7010, is fitted to the vent opening 7037 in the mouth portion to allow for venting of gas exhaled by the patient from the mouth chamber 7012.

As best shown in Figs. 171 and 152, the connector 7008 in some forms is adapted to form part of the fluid coupling in the patient interface and has a nasal portion end 7034 adapted to be attachable to the nasal chamber coupling component 7026, an opposite mouth portion end 7036 attachable to the mouth portion coupling component 7030, and a passage 7038 extending between the nasal portion end and the mouth portion end. The connector 7008 is typically formed from a rigid material, such as a hard plastic but may also be formed from a more malleable material to allow for connection in the fluid coupling. In some forms, the connector 7008 is dimensioned so the distance between the ends 7034, 7036 allows the mouth portion 7002 and nasal portion 7004 to be in close proximity when in the patient interface is in oro-nasal mode.

One or more guide vanes 7040 may be incorporated with the patient interface 7000, particularly as part of the nasal portion 7004, to assist in directing flow of gas within the interface. This can assist the performance of the patient interface including patient comfort during therapy, improving the acoustics of the interface, as well as the operation of the components of the patient interface such as the AAV. In use the one or more guide vanes are positioned to provide an obstruction within the gas flow to allow for the one or more vanes 7040 to direct the flow.

In some forms of the present technology, a vent (e.g. a cushion air vent 7006 provided in the mouth portion 7002 or the nasal portion 7004), for example when used with a headgear conduit system cradle (shown in Figs. 152-154), can comprise one or more guide vanes 7040 within a hollow portion of the vent 7006 so as to improve the air flow. The vanes 7040 may guide the air inside the cradle through the vent whereby turbulence within the cradle may advantageously be reduced. This may also balance the velocity of the air within the cradle such that it impacts an AAV flap 7054 evenly whereby the flap 7054 can be optimally activated. The vanes 7040 may improve performance as the AAV needs to have an even air flow at the flap so that the flap does not open at an angle that may partially obscure the vent opening. The vanes 7040 may also improve the acoustic output of the vent 7006.

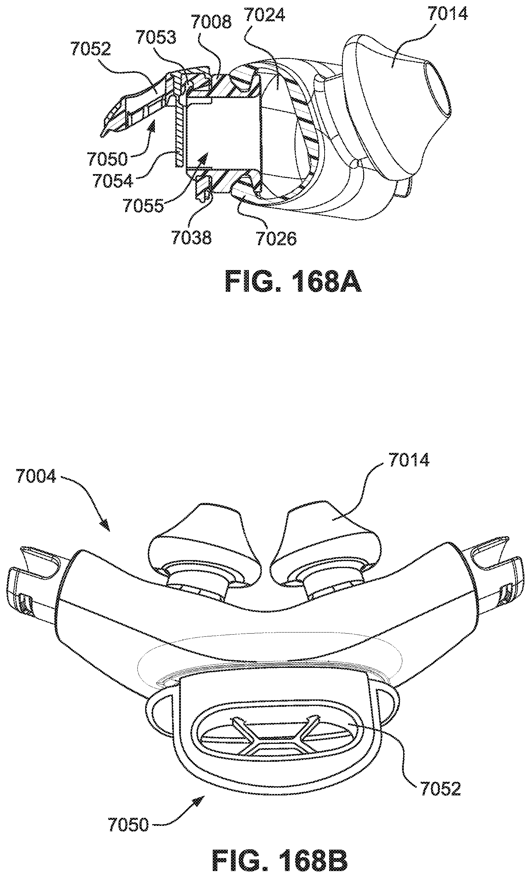

In some forms of the present technology, the one or more guide vanes 7040 are located within the connector passage 7038 (as best illustrated in Figs. 168 and 171) of the connector 7008. In this position, the guide vanes 7040 may assist the flow of pressurised gas into the mouth chamber 7012 when the patient interface is in the oro-nasal mode. This can improve patient comfort during therapy and to stop undue turbulence in the nasal chamber. It can also improve the function of the AAV 7032 disposed in the mouth portion.

In particular, the guide vanes may guide the air inside the nasal chamber through the vent opening whereby turbulence within the chamber may advantageously be reduced. This may also balance the velocity of the air within the nasal portion such that it impacts the AAV flap evenly whereby the flap can be optimally activated. The AAV needs to have an even air flow at the flap so that the flap does not open at an angle that may partially obscure the vent opening. As such the flow of air contacting the flap 7035 under direction from the one or more guide vanes may be more laminar which reduces the likelihood of inadvertent activation of the AAV which may be detrimental to therapy, and it can also provide a physical stop to the flap 7035 to prevent it from over hinging into the passage. This may also improve the acoustic output of the vent. In use the guide vanes 7040 may prevent swing-back of AAV into the opening 7033.

A plurality of guide vanes 7040 may be provided in an opening (e.g., a round opening) of the connector 7008. In some examples, sides of the plurality of guide vanes 7040 may be disposed parallel to each other. In other examples, the sides of the guide vanes 7040 may be provided at an angle to each other. For example, the guide vanes 7040 may be displaced from the centre of the connector 7008 an equal distance (as shown in Fig. 169) and/or may be angled such that the surfaces provide an acute angle directed towards the mouth portion 7002 or the nasal portion 7004. In some examples, the connector 7008 or other components coupling the nasal portion to the mouth portion may provide a latch connection.

In an alternative form, as shown in Figs. 169 to 170, a AAV 7050 may be integrated directly with the connector 7008. The AAV 7050 includes an opening 7052, and flap 7054. In the illustrated form, the AAV is formed separately to the connector typically clipped together with hard to hard interface 7053 and incorporating a robust seal 7055. In another form, the AAV may be integrally formed with the connector 7008. With this arrangement, the AAV may be incorporated within the passage of the fluid coupling between the nasal portion 7004 and mouth portion 7002, or may be utilised, if required, when the patient interface is used in a nasal only configuration.

In some forms, the nasal portion 7004 or mouth portion 7002 may be integrally formed with the connector 7008 rather than be an additional component that connects to both the nasal cushion and the mouth cushion.

In some forms, at least some of the components that enable the releasable fluid coupling (nasal portion coupling component 7026, mouth portion coupling component 7030, connector 7008) may be manufactured utilising an over-molding process. This may be advantageous in improving the surface finish of the component (e.g., the connector 7008) whereby the component may be easier to clean and may thus assist the patient in preventing bacteria build up at fluid coupling.

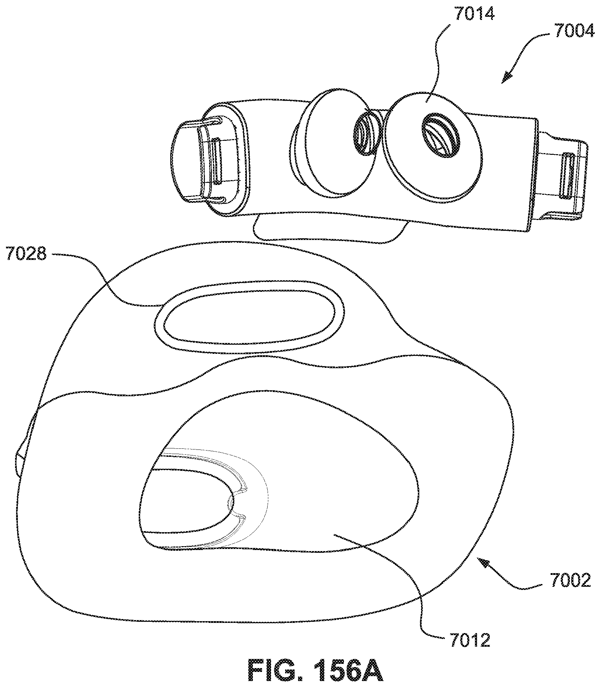

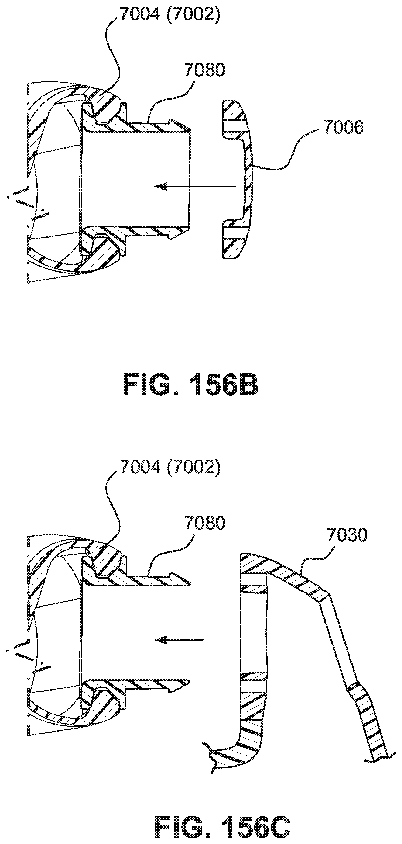

In some forms, the fluid coupling may include a clip mechanism (various examples shown in Figs. 156A to 159) to interconnect the components (nasal portion coupling component 7026, mouth portion coupling component 7030, connector 7008). In some examples, the clip mechanism may include a resilient hinge. While Figs. 156A to 159 show coupling features on specific components (e.g., nasal portion coupling component 7026, mouth portion coupling component 7030, connector 7008, nasal portion 7004, or mouth portion 7002) examples of the present technology are not so limited and the coupling features may be provided on other components disclosed in this application. The clip mechanism 7080 may include a coupling component that projects from one or both of the nasal and oral cushions (see Figs. 156B to 156C). In some forms, at least one of the coupling component of the clip may be substantially rigid and integrally formed with at least one of the nasal cushion and the oral cushion and be arranged to engage with a complementary coupling component to form the clip mechanism.

In some forms the clip may comprise hinged components that can be resiliently moved towards one another so as to engage each other.

In some forms, the coupling arrangement of the fluid coupling may include one component (nasal portion coupling component 7026, mouth portion coupling component 7030, connector 7008) that is resiliently malleable, whereby at least a portion of that component can be depressed so as to interfit with another component of the coupling. For example, an end of the connector 7008 may be able to be depressed to locate within the opening 7024 of the nasal cushion that would normally house the nasal vent. For example, in some forms, the mouth portion coupling component 7030 may include an upper projecting portion that may be squeezed so as to fit within the nasal opening 7024, however, once the squeezing pressure is removed the connector provides an interference fit sufficient to be retained within the vent opening.

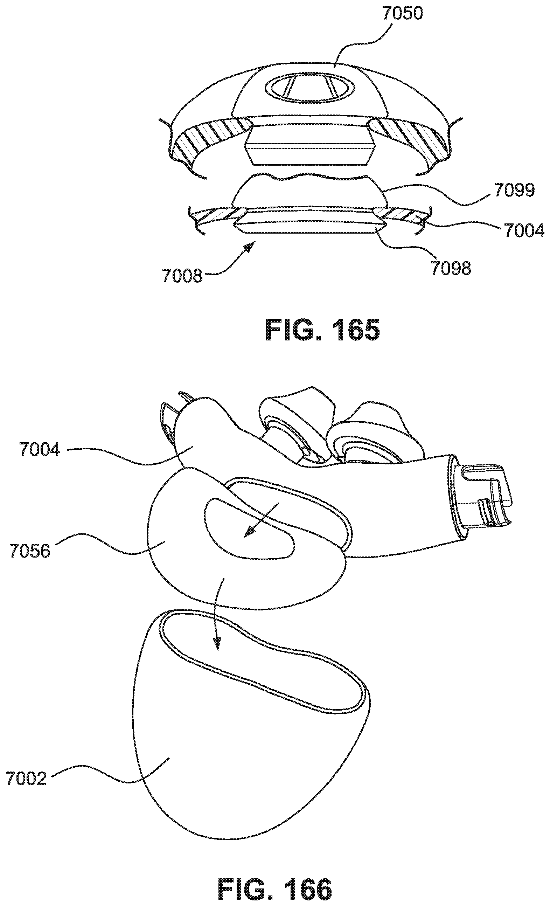

In some forms, as illustrated in Fig. 166 the connector 7008 may be configured so as to form an additional (third) chamber 7056 that is in fluid connection between the vent of the nasal portion 7004 and the mouth portion 7002. The chamber 7069 may include a first opening on one side configured to couple (directly or via a connector) to the opening 7024 in the nasal portion 7004 and a second opening on a different side configured to couple (directly or via a connector) to the opening 7024 in the mouth portion 7002.

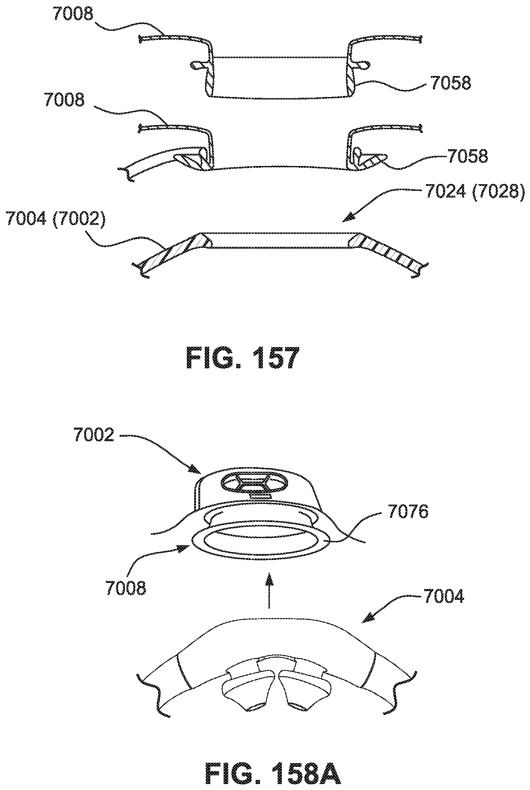

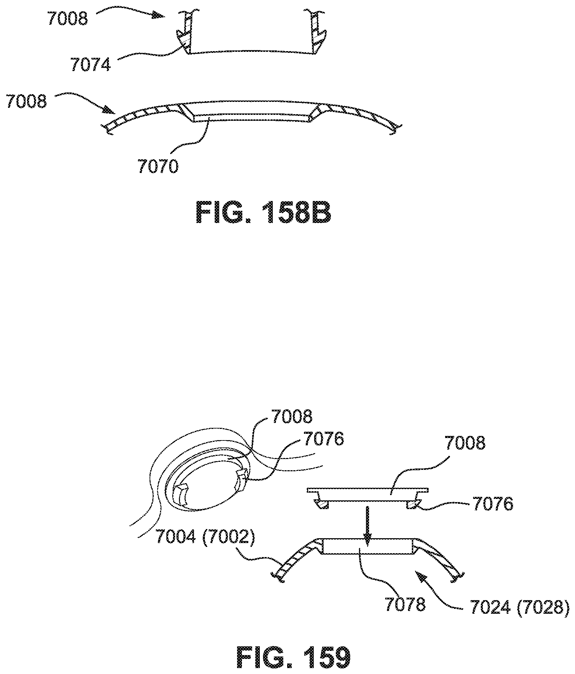

In some forms, the coupling arrangement of the fluid coupling may include one component (nasal portion coupling component 7026, mouth portion coupling component 7030, connector 7008) having a flange 7058 (see Fig. 157) that projects laterally from the connector wall so as to engage the rim of another coupling component such as the vent opening of the nasal portion 7004. In some forms, where the connector 7008 is used, a similar flange on an opposing end of the connector may be configured to engage an opening 7028 at the upper portion of the mouth portion 7002. In some forms, the flange may be formed as an "open flange". The open flange may be located along the connector wall (i.e. between the body of the connector and the distal end of the connector wall). In some forms, the flange 7072 may be formed as a "closed flange". The closed flange may be located at the distal end of the connector wall. In some forms, at least one of the vent opening 7024 and the opening 7028 at the upper portion of the mouth cushion may also comprise a laterally extending flange. In some forms, one or both of the connector and aperture flanges may comprise a raked or lead-in surface 7070 to assist in guiding the connector into the aperture (as shown in Figs. 157, 158A, 158B). The connector 7008 may include a slightly more aggressive retention barb 7074 at the distal end of the outside connector wall configured to engage features in the vent opening 7024 or 7028.

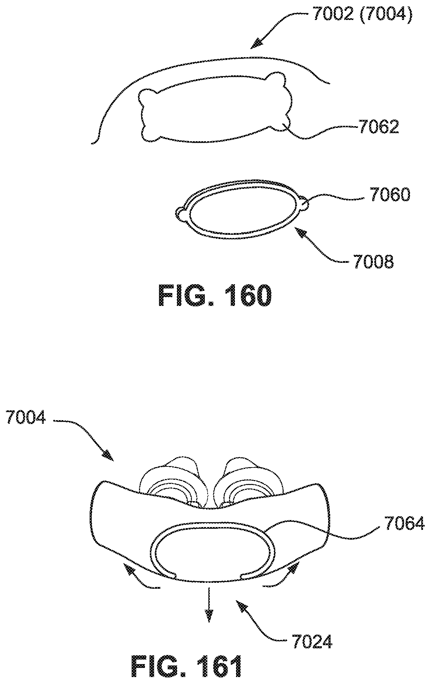

In some forms, the coupling arrangement of the fluid coupling may include one component (nasal portion coupling component 7026, mouth portion coupling component 7030, connector 7008) comprising a plurality of clipping flanges at its distal end. In some forms, the angled clipping flanges 7076 may be spaced on opposing sides of the connector (see Figs 159, 160). The connector facing walls of the nasal portion coupling component 7026, and/or mouth portion coupling component 7030 may be adapted with fins 7078 that extend laterally around the rim of the aperture to assist the connector in effecting an air-pressure seal at non-interference fit regions. In some forms, one or both of the connector and nasal portion coupling component 7026, and/or mouth portion coupling component 7030 may comprise a lead-in surface7070 to assist in guiding the connector into the aperture. In some forms, one or both of the clipping and aperture flanges may comprise a lead-in surface to assist in guiding the components into engagement.

In some forms, the coupling components may be formed, whereby one or more of the clipping flanges 7060 may substantially correspond to one or more matching grooves 7062 along the perimeter of the flange aperture (see Fig. 160). In such forms, in use, once the connector has been inserted, the connector may be rotated slightly so as to effectively lock the clipping flange against the flange of the aperture.

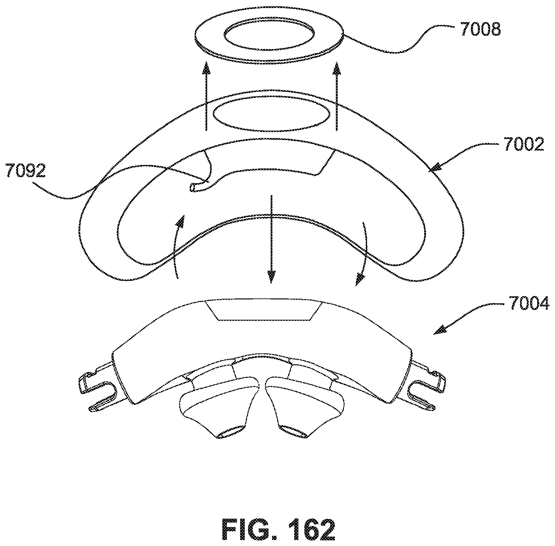

In some forms, the one of the coupling components (e.g., connector 7008) may comprise a horn (see Fig. 162), which may be an asymmetrical horn. This may advantageously assist with levering the components into the nasal vent or oral cushion opening, or connecter end.

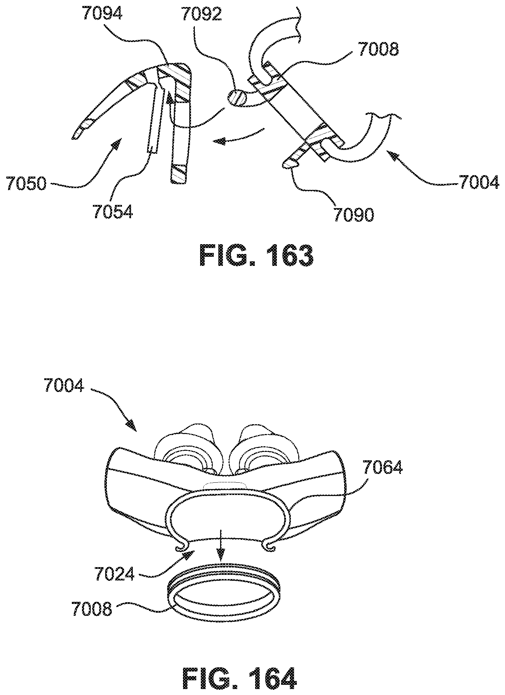

In some forms, the coupling arrangement of the fluid coupling may include one component (nasal portion coupling component 7026, mouth portion coupling component 7030, connector 7008) where one component has as a protruding latch portion 7090, whilst the other may act as a hook portion 7092, whereby the hook portion 7092 may slide over, and snap engage, around the latch portion 7094 (Fig. 163). For example, in some forms the mouth cushion may have a protruding portion having a laterally projecting flange formed proximal the distal end of said portion, the protruding portion surrounding the opening at the upper portion of the oral cushion. The nasal portion may then have a corresponding protruding portion having that partially surrounds the vent opening, whereby it may be slid into engagement around the protruding portion of the oral cushion.

In some forms, where the connector 7008 is provided as an additional component, each end of the component may utilise a different connection means (for example of those listed above). For example, in one form, the connector may have one distal end formed as a resiliently malleable flange (e.g. a lip seal) that surrounds partially or completely the opening at a first distal end, and a plurality of clipping flanges configured on the opposing second distal end.

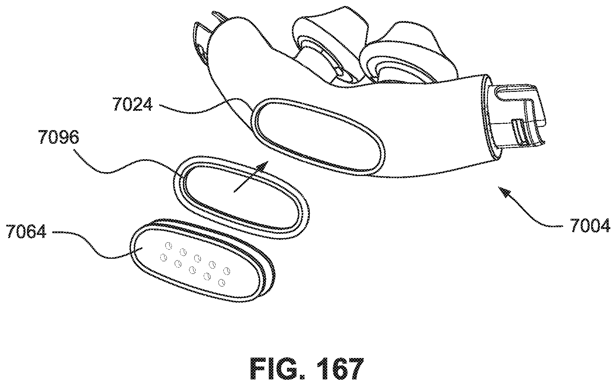

In some forms, where the connector is an additional component, the connector may actually be formed as an assembly of multiple components (see Fig. 167). For example, in one form, the connector assembly may comprise a rigid insert 7096 that can be configured to line the inner rim of the vent opening 7024, and a retainer 7064 that clips into the rigid insert so as to provide a means to engage the opening at the upper portion of the mouth cushion (Figs. 161, 164, 167). In some forms, the peripheral rim forming one of the coupling components may be closed or open thereby forming a partial rim arrangement (see Fig. 164). The partial rim arrangement may provide a slide in vertical assembly. In some examples, the peripheral rim may include at least one of projections or recesses that allow for an interlocking connection and/or a raked surface to provide a lead in surface to a resilient connection.

In some examples, the peripheral rim may be provided at the mouth chamber opening and may include one or more projections and/or recesses that allow for an interlocking connection, includes a raked surface to provide a lead in surface to a resilient connection.

In some forms, the connector 7008 may be able to be passed through a respective opening 7024, 7028 and then locked. In some forms, the portion that has passed through the aperture may be depressed (like a corrugated flap 7098) so as to form a locking flange (see Fig. 165). The opposite end of the connector may include a lip 7099 to engage a portion of the AAV 7050 or the mouth portion 7002. As shown in Fig. 165, the portion of the AAV 7050 may include a corrugated flap to engage the lip 7099 of the connector 7008.

The patient interface systems described may also be utilized to provide a method of respiratory therapy treatment, where a first patient interface with a first footprint is applied to a patient for a period of time, then the first patient interface is removed, and a second patient interface with a second footprint different from the first footprint is applied to the patient for a second period of time. For example, the first footprint may be one of a nares only patient interface, a nares and mouth patient interface, and a mouth only patient interface, while the second footprint is another one of the nares only patient interface, the nares and mouth patient interface, and the mouth only patient interface.

While the disclosure has been described in connection with what are presently considered to be the most practical and preferred embodiments, it is to be understood that the disclosure is not to be limited to the disclosed embodiments, but on the contrary, is intended to cover various modifications and equivalent arrangements included within the scope of the invention. Also, the various embodiments described above may be implemented in conjunction with other embodiments, e.g., aspects of one embodiment may be combined with aspects of another embodiment to realize yet other embodiments. Further, each independent feature or component of any given assembly may constitute an additional embodiment. In addition, while the disclosure has particular application to patients who suffer from OSA, it is to be appreciated that patients who suffer from other illnesses (e.g., congestive heart failure, diabetes, morbid obesity, stroke, bariatric surgery, etc.) can derive benefit from the above teachings. Moreover, the above teachings have applicability with patients and non-patients alike in non-medical applications.

### 5.3.11 Headgear Magnetic Clip with Reverse Hinge

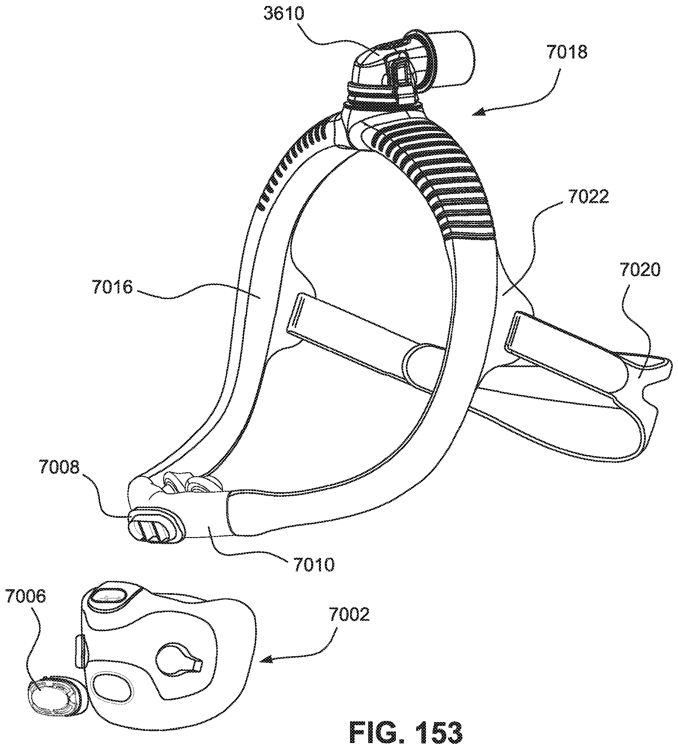

In order to simplify the interface and design, it may be advantageous to for at least a portion of the headgear strap (e.g., mouth portion headgear 850, nares and mouth portion headgear 860, or nares and mouth portion headgear 870) to have an active "hinge" with a magnet thereon to removable connect to a patient interface. In some examples, the headgear strap 7020 (shown in Fig. 153) may include a lower strap configured to removably connect to the mouth portion 7002 using an active hinge. The active hinge connector 9000 may include a headgear connector 9030 for coupling to the strap and a patient interface engagement portion 9040 for coupling to the patient interface (e.g., the mouth portion 7002).

The connector 9000 may include a hinge portion 9020 extending, in use, from a headgear eyelet towards a distal end, with a headgear hinge magnet 9010 disposed on the interior face thereof so as to engage a corresponding chassis magnet 9060 on a chassis 9100 of the mouth portion 7002 (e.g., the oral chamber of the mouth portion 7002). The headgear connector 9030 may include an opening for receiving the headgear eyelet. As shown in Figs. 155A-155C, 180 and 181, a plurality of patient interface connectors 9050 are provided near opposite end of and on a common surface of the chassis 9100. The chassis 9100 may provide a shell supporting and/or defining at least a portion of the plenum chamber and/or supporting a seal forming structure. While the patient interface connectors 9050 are illustrated being provided on the chassis 9100, in some examples of the present technology the patient interface connectors 9050 may be provided directly on the cushion surface of the mouth portion 7002.

The forward portion of each of the chassis magnet 9060 and the headgear hinge magnet 9010 is formed so as to hook into one another in use.

To support the stability and retention of the hook engagement, the in-use rearward portion of the chassis 9100 comprises support pads 9070 configured to retain the connector 9000 with respect to the patient interface. The support pads 9070 are configured to elevate and angle the hinge portion 9020 of the connector 9000 (e.g., patient interface engagement portion 9040) thus improving the engagement of the hooks, whereby the magnets are harder to pry apart from one another. Thus, the support pads 9070 can reduce the risk of the headgear being disengaged and separated from the patient interface (e.g., the oral chassis 9100) during use.

The connector 9000 and the patient interface connectors 9050 are configured to removably engage one another through a plurality of different retention arrangements. The retention arrangements may include a mechanical arrangement and/or magnetic arrangement. In some examples, complementary protrusions may be adapted to retain a retaining portion in the headgear connector 9000 with respect to the patient interface connectors 9050 in a direction along a surface of the patient interface. One of the arrangements may retain the retaining portion with respect to the patient interface in a direction away from the patient interface (e.g., to the side of and/or towards the back of a patient's head).

In some examples, the patient interface connectors 9050 may project at least partially from an outside surface of the patient interface (e.g., see Fig. 155A). The patient interface connectors 9050 may project at least partially from an outside surface of the chassis 9100.

In some examples, the hinged portion 9020 of the connector 9000 may be provided intermediate the headgear connector 9030 and the patient interface engagement portion 9040, such that they are hinged with respect to each other. In some examples, the hinged portion 9020 may comprise a living hinge. The living hinge may be of the same material as portions of the headgear connector 9030 and the patient interface engagement portion 9040. The portions of the headgear connector 9030 and the patient interface engagement portion 9040 connected to the living hinge may be rigid.

In some examples of the present technology, extra space between the retention portion (e.g., the magnet 9010) and sides of the connector 9000 may allow for the connector 9000 to rotate when coupled to the patient interface. As shown in Figs. 179-181, the sides of the connector 9000 may at least partially correspond to the shape of the support pads 9070 and be configured to surround and enclose the support pads 9070. In this example, the support pads 9070 may be fixed in position on the chassis 9100. In another example, the pad including the magnet 9010 and/or the support pads 9070 may be configured to rotate relative to the connector 9000 or the chassis 9100. The rotation of the connector 9000 relative to the chassis 9100 may allow different positioning of the headgear straps.

As illustrated in Figs. 155A-155E, 180 and 181, the chassis 9100 (containing the magnets 9060 therein) is over-moulded onto the cushion. This may advantageously be easier to clean, and may thus assist the patient in preventing bacteria build up at the connector.

In some examples of the present technology, the chassis 9100 (containing the magnets 9060 therein) may be removable from the cushion of the patient interface. In some forms, the chassis 9100 may be retained in the cushion using the magnets 9060 or alternative magnets provided for the purpose of engaging the cushion. This may advantageously be easier to clean, and may thus assist the patient in preventing bacteria build up at the connector.

In some examples of the present technology, the chassis 9100 of the oral chamber may be formed to provide rigid support adjacent to the middle of the oral chamber, whilst providing adequate resilient/deformable spring in the regions adjacent the sides of the oral chamber.

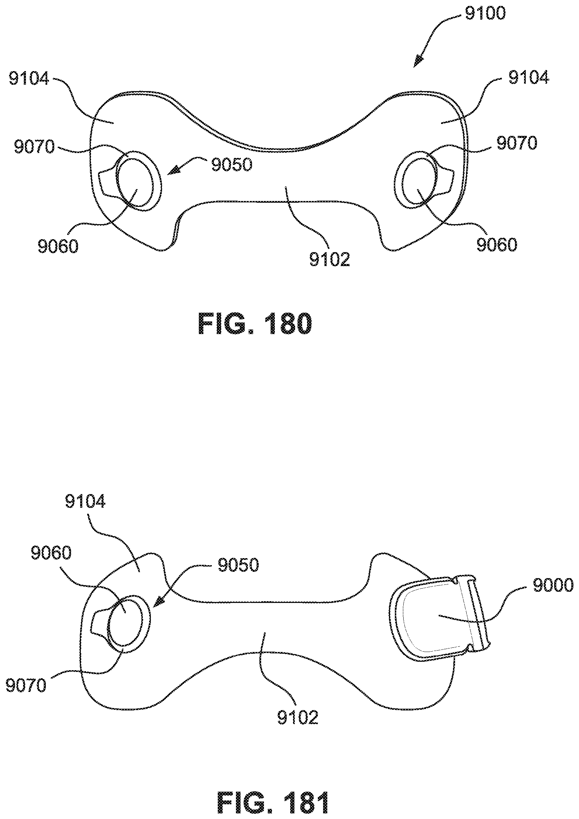

In some forms, the chassis 9100 may be formed to have a varying thickness, whereby a central region 9102 adjacent the middle of the oral chamber is thicker than the side regions 9104 adjacent the sides of the oral chamber. As shown in Figs. 180 and 181, each of the side regions 9104 includes a respective support pad 9110.

In some forms, the chassis 9100 may be formed to have substantially larger, yet thinner, regions 9104 adjacent the sides of the oral chamber, whilst having a smaller, yet thicker, region 9102 adjacent the middle portion of the oral chamber (i.e. somewhat like a "figure 8" that is thicker in the middle and thins towards the sides). In this example, the increased rigidity in region 9102 may provide sufficient support in the middle portion of the oral chamber and provide flexibility in regions 9102 such that the oral chamber is more easily adapted to a patient facial features and/or placement of the headgear strap due to differences in positioning of the headgear.

In some forms (see Figs. 155A-155E), the chassis 9100 and cushion may be formed so as to provide sufficient flexible silicone around the cushion air vent 7037, whereby the air vent 7006 is removable. The flexibility of the cushion around the air vent 7037 may provide for easier installation and removal of the vent, while the increased rigidity of the chassis 9100 in region 9102 support the shape of the cushion providing the plenum chamber.

### 5.3.12 Plenum chamber

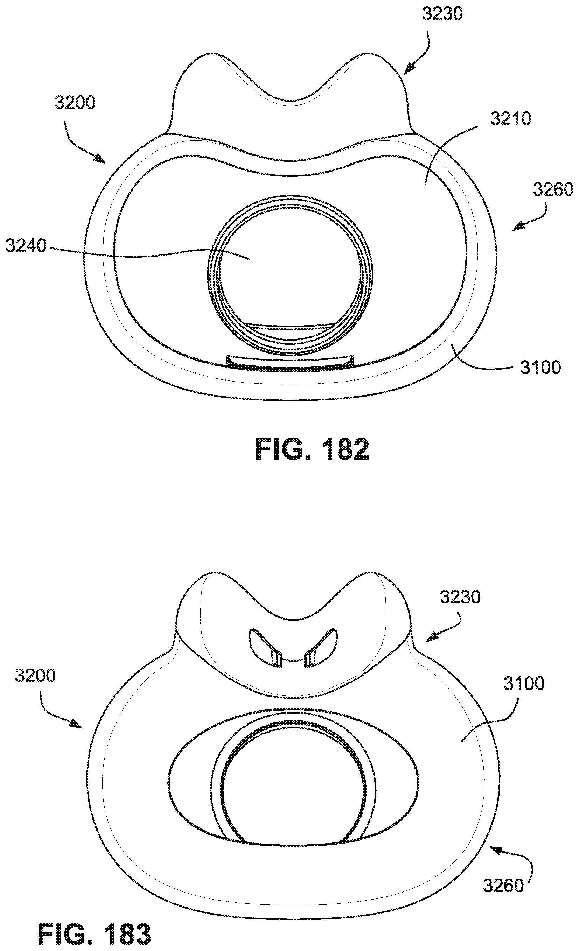

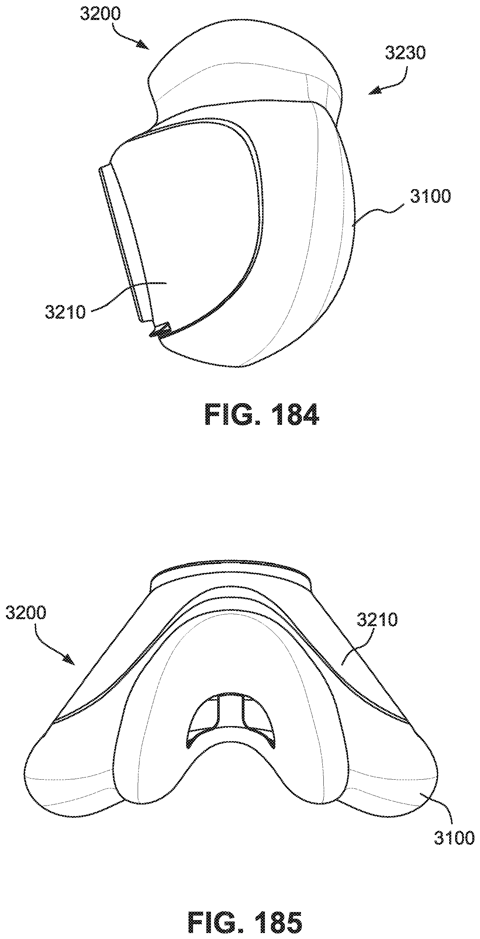

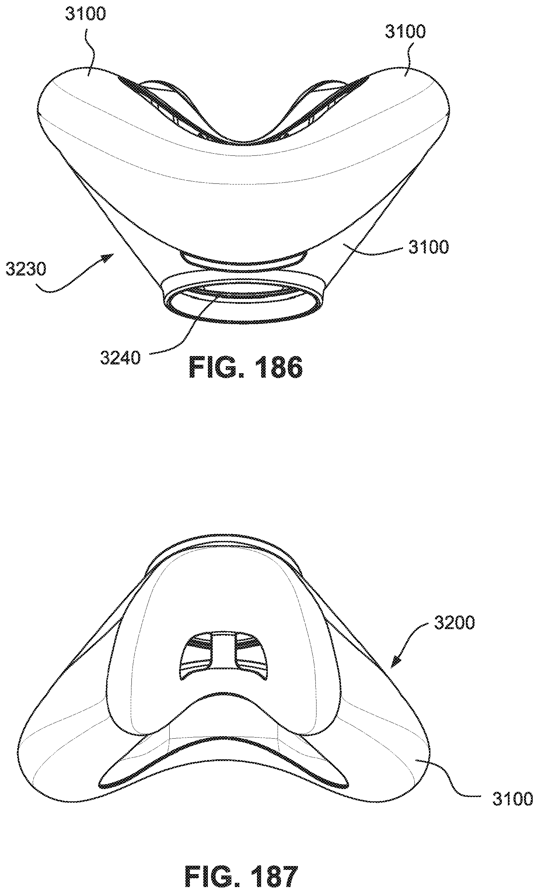

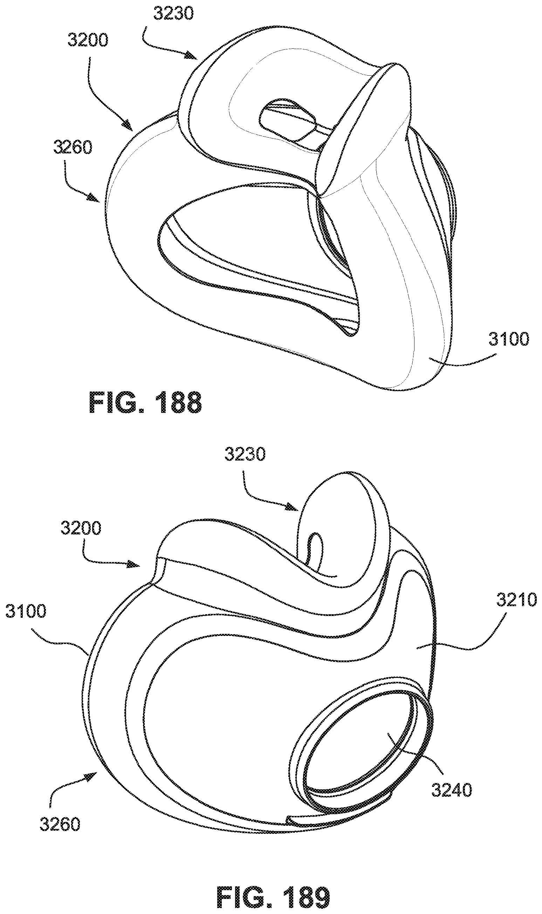

The plenum chamber 3200 has a perimeter that is shaped to be complementary to the surface contour of the face of an average person in the region where a seal will form in use. In the examples shown in Figs. 182-189, the plenum chamber comprises a shell 3210 and a seal-forming structure 3100. Other examples of the plenum chamber comprises a shell 3210 and a seal-forming structure 3100 are described in International Publication No. WO 2019/183680 A1. In these examples a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 and the seal-forming structure 3100 are formed from a single homogeneous piece of material.

In certain forms of the present technology, the plenum chamber 3200 does not cover the eyes of the patient in use. In other words, the eyes are outside the pressurised volume defined by the plenum chamber. Such forms tend to be less obtrusive and/or more comfortable for the wearer, which can improve compliance with therapy.

In certain forms of the present technology, a portion of the plenum chamber 3200 is constructed from a transparent material, e.g., a transparent polycarbonate. In the examples shown in Figs. 182-189, the shell 3210 is constructed from transparent polycarbonate. The use of a transparent material can reduce the obtrusiveness of the patient interface, and help improve compliance with therapy. The use of a transparent material can aid a clinician to observe how the patient interface is located and functioning.

In certain forms of the present technology, the plenum chamber 3200 is constructed from a translucent material. The use of a translucent material can reduce the obtrusiveness of the patient interface, and help improve compliance with therapy.

Figs. 182-189show plenum chambers 3200 according to examples of the present technology which are formed partially by a shell 3210. Additionally, the plenum chamber 3200 is formed partially by the seal-forming structure 3100. In some examples the seal-forming structure 3100 is overmoulded to the shell 3210. The seal-forming structure 3100 may alternatively be formed separately from the shell 3210 and be configured to permanently or removably connect to the shell 3210. The seal-forming structure 3100 and shell 3210 may be integrally formed.

In the examples shown in Figs. 182-189, the shell 3210 is formed from polycarbonate and the seal-forming structure 3100 is formed from silicone. The silicone may have a Shore A hardness of 30 or 40 Durometer. A silicone or similar material with this hardness is advantageous for comfort and flexibility to conform and seal to the patient's face. The use of polycarbonate (or other stiffer material), with its higher hardness and stiffness than silicone is advantageous in providing portions of higher resistance to deformation with less material than would be required to provide the same resistance with silicone. Using less material can be advantageous in keeping overall bulk and weight down, which may reduce obtrusiveness to the user. In alternative examples the shell 3210 may be formed from nylon or polypropylene. The seal-forming structure 3100 may alternatively be formed from a suitable foam or any suitable thermoplastic elastomer.

As described in International Publication No. WO 2019/183680 A1, the shell 3210 may have posteriorly protruding portions at superior lateral corners of the shell 3210. The posteriorly protruding portions may reinforce a base of the nasal portion and may help provide stability to the seal-forming structure 3100. The posteriorly protruding portions also help reinforce the locations of the seal-forming structure proximate posterior corners which also support the nasal portion of the plenum chamber on the patient's face, and also advantageously fit into the patient's nasolabial sulci. The posterior protruding portions may protrude from the shell 3210 towards the location of the patient's nasolabial sulcus or the patient's cheeks alongside the nasolabial sulcus. The posterior protruding portions may be provided inferior to lateral support portions.

### 5.3.12.1 Lateral Support Portions

In some examples of the present technology, the plenum chamber 3200 comprises lateral support portions on the anterior side of the nasal portion 3230 of the plenum chamber 3200, see examples of lateral support portions described in International Publication No. WO 2019/183680 A1, among others. The lateral support portions may have a higher resistance to deformation than one or more adjacent portions of the seal-forming structure 3100. The lateral support portions may be stiffer than regions of the plenum chamber 3200 superior to lateral support portions. Additionally, or alternatively, the lateral support portions may be stiffer than a central region of the plenum chamber 3200. The regions of relatively greater stiffness may be in the form of fins configured to provide areas of relatively high rigidity in comparison to surrounding areas of the plenum chamber.

Additionally, some flexibility in the overall structure of the seal-forming structure 3100 can be advantageous for accommodating long and/or narrow noses. When the seal-forming structure 3100 is moved upwards into contact with the underside of a narrow nose, an amount of flexibility in the overall structure of the nasal portion 3230 is desirable as it enables the lateral sides of the seal-forming structure 3100 to be drawn inwards as a result of downwards force applied to the centre of the seal-forming structure 3100 by the patient's nose. When the seal-forming structure 3100 in the nasal portion 3230 is brought into contact with the patient's nose, the outwardly-facing lateral sides of the seal-forming structure 3100 are pulled inwardly to assist the inwardly-facing lateral sides of the seal-forming structure 3100 to conform to the lower periphery of the patient's nose. If there is insufficient flexibility in the lateral sides of the seal-forming structure 3100, the seal-forming structure 3100 may be less accommodating to narrow noses and/or may be uncomfortable if the patient is required to tighten the headgear to compensate. Excess flexibility may result in the seal-forming structure 3100 being incapable of holding its shape and maintaining an effective seal.

### 5.3.12.1.1 Lateral support portions formed by seal-forming structure

The plenum chamber 3200 shown in some examples comprises lateral support portions provided by the seal-forming structure 3100. The lateral support portions are provided to the anterior side of the plenum chamber 3200 and on each lateral side of the nasal portion 3230. In this example, the lateral support portions are provided at inferior regions of each lateral side of the nasal portion 3230. See examples of lateral support portions described in International Publication No. WO 2019/183680 A1.

At least a majority of the nasal portion 3230 is formed by the seal-forming structure 3100 in this example. The majority of the nasal portion 3230 of the plenum chamber 3200 is formed from a soft, flexible resilient material. The majority of the nasal portion 3230 is formed from silicone in this example. In some, substantially the entire nasal portion is formed by the seal-forming structure 3100. In some examples, portions of the nasal portion are formed by the shell 3210.

In this example, the lateral support portions are regions of the seal-forming structure 3100 having relatively greater stiffness in comparison to one or more adjacent regions of the seal-forming structure 3100. In particular, the lateral support portions have a greater material thickness than regions of the seal-forming structure 3100 superior to the lateral support portions. This greater material thickness provides an amount of stiffness or structural rigidity to the structure of the seal-forming structure 3100 and in particular the nasal portion 3230. Much of the posterior side of the nasal portion 3230 has a low wall thickness for comfort and ability to seal against the patient's face, which may not provide significant structural rigidity to the shape of the nasal portion 3230. The lateral support portions compensate for the lack of structural rigidity provided by the patient-contacting walls and increase the overall structural rigidity to the nasal portion 3230.

### 5.3.12.1.2 Lateral support portions formed by shell

The plenum chamber 3200 in some examples comprises lateral support portions provided by the shell 3210 of the plenum chamber 3200. The lateral support portions in this example comprise superior portions of the shell 3210. The lateral support portions extend in a superior direction into the nasal portion 3230 of the plenum chamber 3200. The lateral support portions comprise portions of the shell 3210 provided to the nasal portion 3230 in this example. See examples of lateral support portions described in International Publication No. WO 2019/183680 A1.

### 5.3.12.2 Plenum chamber inlet port

The shell 3210 may comprise one or more plenum chamber inlet ports 3240. The one or more plenum chamber inlet ports 3240 may allow for a connection to other components, such as a frame, decoupling structure, vent arrangement, heat and moisture exchanger (HMX), constant-flow vent (CFV), anti-asphyxia valve (AAV) and/or connection port to a conduit in various examples.

In some examples, the plenum chamber 3200 comprises a single inlet port 3240. The inlet port 3240 is provided substantially centrally in the shell 3210. The inlet port 3240 in this example may be configured to connect to a frame to which headgear or other positioning and stabilising structure components can be connected. The inlet port 3240 is substantially circular in this exemplary form of the technology.

In some examples, the plenum chamber comprises two inlet ports 3240. The inlet ports 3240 are provided to lateral sides of the shell 3210. The inlet ports 3240 in this example are configured to connect to conduits which connect to a decoupling component located above the patient's head where the conduits are connected to the air circuit. The conduits may form part of the positioning and stabilising structure 3300, i.e., they may be "headgear conduits". In some examples the inlet ports 3240 may receive combined headgear and conduit connection assemblies, in order to provide multiple functions such as venting, supply of the flow of air and headgear attachment points. The combined headgear and conduit connection assemblies may also comprise an AAV. The inlet ports 3240 in this example are approximately elliptically shaped, e.g., oval. Specific examples are described in International Publication No. WO 2019/183680 A1.

In some examples, the plenum chamber 3200 may be provided with one or two inlet ports 3240 on the lateral sides of the shell 3210 to connect with conduit headgear. It would be understood that any of the features described herein of seal-forming structures 3100 (including that of the plenum chamber 3200) may be incorporated in a patient interface including conduit headgear.

### 5.3.12.3 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs - the actual sealing surface - may change within a given treatment session, from day to day, and from patient to patient, depending on a range of factors including for example, where the patient interface was placed on the face, tension in the positioning and stabilising structure and the shape of a patient's face.

In one form the target seal-forming region is located on an outside surface of the seal-forming structure 3100.

In certain forms of the present technology, the seal-forming structure 3100 is constructed from a biocompatible material, e.g., liquid silicone rubber (LSR).

A seal-forming structure 3100 in accordance with the present technology may be constructed from a soft, flexible, resilient material such as liquid silicone rubber (LSR).

In certain forms of the present technology, a system is provided comprising more than one seal-forming structure 3100, each being configured to correspond to a different size and/or shape range. For example the system may comprise one form of a seal-forming structure 3100 suitable for a large sized head, but not a small sized head and another suitable for a small sized head, but not a large sized head.

### 5.3.13 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled CO₂ by the patient while maintaining the therapeutic pressure in the plenum chamber in use.

One form of vent 3400 in accordance with the present technology comprises a plurality of holes, for example, about 20 to about 80 holes, or about 40 to about 60 holes, or about 45 to about 55 holes.

The vent 3400 may be located in the plenum chamber 3200. Alternatively, the vent 3400 is located in a decoupling structure, e.g., a swivel.

In some examples the patient interface 3000 comprises a vent 3400. The vent 3400 may be provided in passages within the frame of the patient interface 3000 (e.g., conduits 7016) and/or swivel elbow assembly 3610 (see e.g., connector 44, elbow and swivel 1612 and elbow 3004) through which air can flow from the interior of the plenum chamber 3200 to atmosphere. Other features of the vent and positioning of the vent are described in International Publication No. WO 2019/183680 A1. The swivel elbow assembly 3610 may be substantially as described in International Publication No. WO 2017/049357 A1.

### 5.3.14 Ports

In one form of the present technology, a patient interface 3000 includes one or more ports that allow access to the volume within the plenum chamber 3200. In one form this allows a clinician to supply supplemental oxygen. In one form, this allows for the direct measurement of a property of gases within the plenum chamber 3200, such as the pressure.

### 5.4 RPT DEVICE

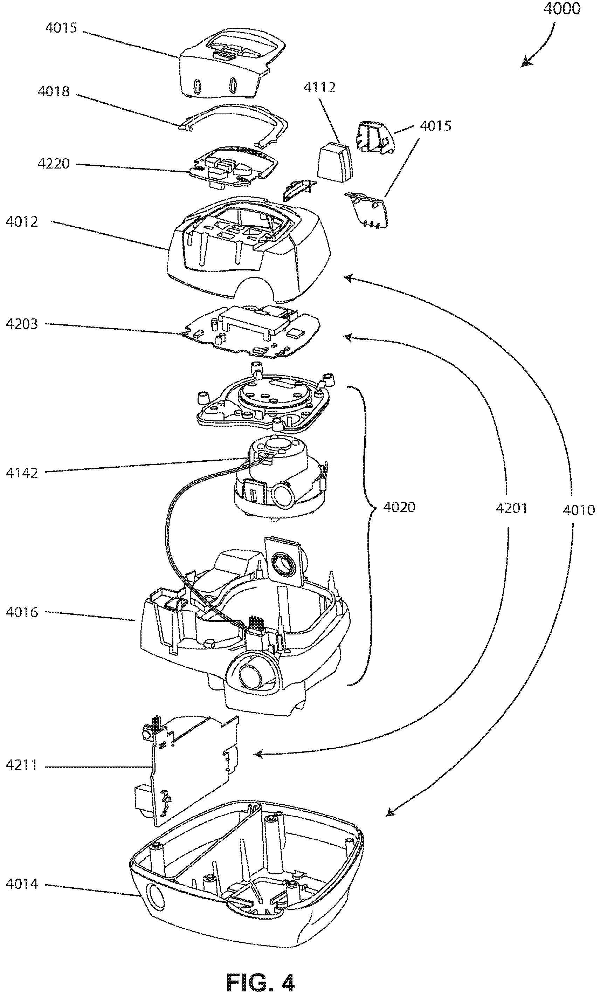

An RPT device 4000 (as shown in Fig. 4) in accordance with one aspect of the present technology comprises mechanical, pneumatic, and/or electrical components and is configured to execute one or more algorithms, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH₂O, or at least 10cmH₂O, or at least 20 cmH₂O.RPT device algorithms

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., an inlet air filter 4113, an inlet muffler 4122, a pressure generator 4140 capable of supplying air at positive pressure (e.g., a blower 4142), an outlet muffler 4124 and one or more transducers 4270, such as pressure sensors and flow rate sensors.

As mentioned above, in some forms of the present technology, the central controller may be configured to implement one or more algorithms expressed as computer programs stored in a non-transitory computer readable storage medium, such as memory. The algorithms are generally grouped into groups referred to as modules.

The RPT device 4000 may have an electrical power supply 4211, one or more input devices 4220, a central controller, a therapy device controller, a pressure generator 4140, one or more protection circuits, memory, transducers 4270, data communication interface and one or more output devices 4290. Electrical components 4201 may be mounted on a single Printed Circuit Board Assembly (PCBA) 4202. In an alternative form, the RPT device 4000 may include more than one PCBA 4203.

An RPT device in accordance with one form of the present technology may include an air filter 4111, or a plurality of air filters 4111.

In one form, an outlet air filter 4115, for example an antibacterial filter, is located between an outlet of the pneumatic block 4020 and a patient interface 3000.

An RPT device in accordance with one form of the present technology may include a muffler 4120, or a plurality of mufflers 4120.

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 5.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller. One example of an air circuit 4170 comprising a heated wire circuit is described in United States Patent 8,733,349.

### 5.5.1 Oxygen delivery

In one form of the present technology, supplemental oxygen 4180 is delivered to one or more points in the pneumatic path, such as upstream of the pneumatic block 4020, to the air circuit 4170 and/or to the patient interface 3000.

### 5.6 BREATHING WAVEFORMS

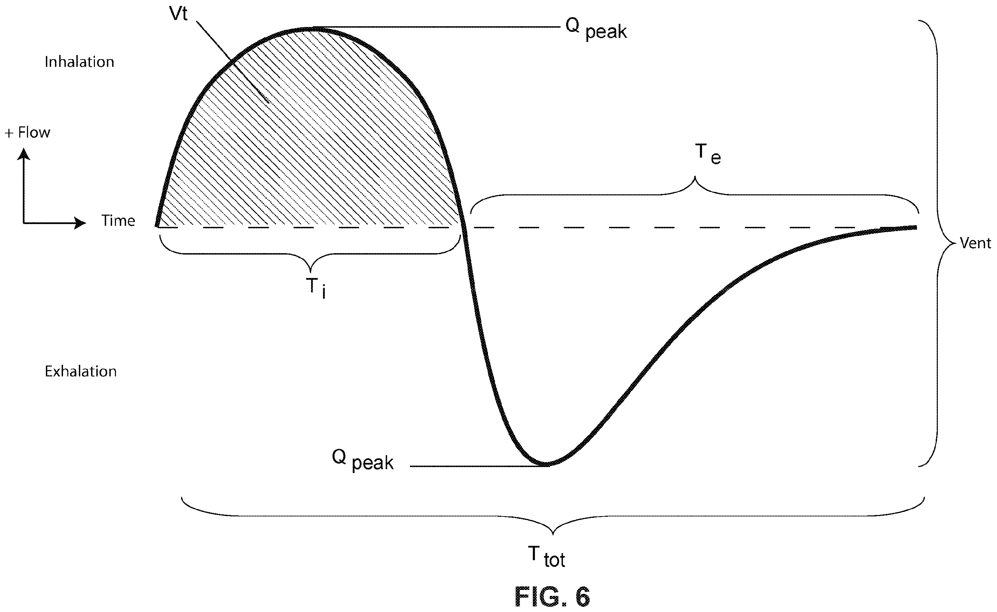

Fig. 6 shows a model typical breath waveform of a person while sleeping. The horizontal axis is time, and the vertical axis is respiratory flow rate. While the parameter values may vary, a typical breath may have the following approximate values: tidal volume *Vt* 0.5L, inhalation time *Ti* 1.6s, peak inspiratory flow rate *Qpeak* 0.4 L/s, exhalation time *Te* 2.4s, peak expiratory flow rate *Qpeak* -0.5 L/s. The total duration of the breath, *Ttot,* is about 4s. The person typically breathes at a rate of about 15 breaths per minute (BPM), with Ventilation *Vent* about 7.5 L/min. A typical duty cycle, the ratio of *Ti* to *Ttot,* is about 40%.

### 5.7 RESPIRATORY PRESSURE THERAPY MODES

Various respiratory pressure therapy modes may be implemented by the RPT device 4000 depending on the values of the parameters A and *P*₀ in the treatment pressure equation used by the therapy parameter determination algorithm in one form of the present technology.

### 6 GLOSSARY

For the purposes of the present technology disclosure, in certain forms of the present technology, one or more of the following definitions may apply. In other forms of the present technology, alternative definitions may apply.

### 6.1.1 General

*Air:* In certain forms of the present technology, air may be taken to mean atmospheric air, and in other forms of the present technology air may be taken to mean some other combination of breathable gases, e.g. atmospheric air enriched with oxygen.

*Ambient*: In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol *Q*. 'Flow rate' is sometimes shortened to simply 'flow' or 'airflow'.

*Humidifier:* The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Leak:* The word leak will be taken to be an unintended flow of air. In one example, leak may occur as the result of an incomplete seal between a mask or patient interface and a patient's face. In another example leak may occur in a swivel elbow to the ambient.

*Noise, conducted (acoustic):* Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic):* Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Patient:* A person, whether or not they are suffering from a respiratory condition.

*Pressure:* Force per unit area. Pressure may be expressed in a range of units, including cmH₂O, g-f/cm² and hectopascal. 1 cmH₂O is equal to 1 g-f/cm² and is approximately 0.98 hectopascal. In this specification, unless otherwise stated, pressure is given in units of cmH₂O.

The pressure in the patient interface is given the symbol *Pm,* while the treatment pressure, which represents a target value to be achieved by the mask or patient interface pressure *Pm* at the current instant of time, is given the symbol *Pt.*

*Respiratory Pressure Therapy (RPT):* The application of a supply of air to an entrance to the airways at a treatment pressure that is typically positive with respect to atmosphere.

*Ventilator:* A mechanical device that provides pressure support to a patient to perform some or all of the work of breathing.

### 6.1.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate:* a thermoplastic polymer of Bisphenol-A Carbonate.

### 6.1.1.2 Mechanical properties

*Resilience:* Ability of a material to absorb energy when deformed elastically and to release the energy upon unloading.

*Resilient:* Will release substantially all of the energy when unloaded. Includes e.g. certain silicones, and thermoplastic elastomers.

*Hardness:* The ability of a material *per se* to resist deformation (e.g. described by a Young's Modulus, or an indentation *hardness* scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component:* The ability of the structure or component to resist deformation in response to an applied load. The load may be a force or a moment, e.g. compression, tension, bending or torsion. The structure or component may offer different resistances in different directions.

*Floppy structure or component:* A structure or component that will change shape, e.g. bend, when caused to support its own weight, within a relatively short period of time such as 1 second.

*Rigid structure or component:* A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH₂O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 6.1.2 Respiratory cycle

*Apnea:* According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate:* The rate of spontaneous respiration of a patient, usually measured in breaths per minute.

*Duty cycle:* The ratio of inhalation time, *Ti* to total breath time, *Trot.*

*Effort (breathing):* The work done by a spontaneously breathing person attempting to breathe.

*Expiratory portion of a breathing cycle:* The period from the start of expiratory flow to the start of inspiratory flow.

*Flow limitation:* Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

*Hypopnea:* According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:
(i) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

*Hyperpnea:* An increase in flow to a level higher than normal.

*Inspiratory portion of a breathing cycle:* The period from the start of inspiratory flow to the start of expiratory flow will be taken to be the inspiratory portion of a breathing cycle.

*Patency (airway):* The degree of the airway being open, or the extent to which the airway is open. A *patent* airway is open. Airway patency may be quantified, for example with a value of one (1) being patent, and a value of zero (0), being closed (obstructed).

*Positive End-Expiratory Pressure (PEEP):* The pressure above atmosphere in the lungs that exists at the end of expiration.

*Peak flow rate (Qpeak):* The maximum value of flow rate during the inspiratory portion of the respiratory flow waveform.

*Respiratory flow rate,* patient *airflow rate, respiratory airflow rate (Qr):* These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt):* The volume of air inhaled or exhaled during normal breathing, when extra effort is not applied. In principle the inspiratory volume *Vi* (the volume of air inhaled) is equal to the expiratory volume Ve (the volume of air exhaled), and therefore a single tidal volume *Vt* may be defined as equal to either quantity. In practice the tidal volume *Vt* is estimated as some combination, e.g. the mean, of the inspiratory volume *Vi* and the expiratory volume *Ve.*

*(inhalation) Time (Ti):* The duration of the inspiratory portion of the respiratory flow rate waveform.

*(exhalation) Time (Te):* The duration of the expiratory portion of the respiratory flow rate waveform.

*(total) Time (Ttot):* The total duration between the start of one inspiratory portion of a respiratory flow rate waveform and the start of the following inspiratory portion of the respiratory flow rate waveform.

*Typical recent ventilation:* The value of ventilation around which recent values of ventilation *Vent* over some predetermined timescale tend to cluster, that is, a measure of the central tendency of the recent values of ventilation.

*Upper airway obstruction (UAO):* includes both partial and total upper airway obstruction. This may be associated with a state of flow limitation, in which the flow rate increases only slightly or may even decrease as the pressure difference across the upper airway increases (Starling resistor behaviour).

*Ventilation (Vent):* A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 6.1.3 Anatomy

### 6.1.3.1 Anatomy of the face

*Ala:* the external outer wall or "wing" of each nostril (plural: alar)
*Alare:* The most lateral point on the nasal *ala.*

*Alar curvature (or alar crest) point:* The most posterior point in the curved base line of each *ala,* found in the crease formed by the union of the *ala* with the cheek.

*Auricle:* The whole external visible part of the ear.

*(nose) Bony framework:* The bony framework of the nose comprises the nasal bones, the frontal process of the maxillae and the nasal part of the frontal bone.

*(nose) Cartilaginous framework:* The cartilaginous framework of the nose comprises the septal, lateral, major and minor cartilages.

*Columella:* the strip of skin that separates the nares and which runs from the pronasale to the upper lip.

*Columella angle:* The angle between the line drawn through the midpoint of the nostril aperture and a line drawn perpendicular to the Frankfort horizontal while intersecting subnasale.

*Frankfort horizontal plane:* A line extending from the most inferior point of the orbital margin to the left tragion. The tragion is the deepest point in the notch superior to the tragus of the auricle.

*Glabella:* Located on the soft tissue, the most prominent point in the midsagittal plane of the forehead.

*Lateral nasal cartilage:* A generally triangular plate of cartilage. Its superior margin is attached to the nasal bone and frontal process of the maxilla, and its inferior margin is connected to the greater alar cartilage.

### Lip, lower (labrale inferius):

### Lip, upper (labrale superius):

*Greater alar cartilage:* A plate of cartilage lying below the lateral nasal cartilage. It is curved around the anterior part of the naris. Its posterior end is connected to the frontal process of the maxilla by a tough fibrous membrane containing three or four minor cartilages of the *ala.*

*Nares (Nostrils):* Approximately ellipsoidal apertures forming the entrance to the nasal cavity. The singular form of nares is naris (nostril). The nares are separated by the nasal septum.

*Naso-labial sulcus or Naso-labial fold:* The skin fold or groove that runs from each side of the nose to the corners of the mouth, separating the cheeks from the upper lip.

*Naso-labial angle:* The angle between the columella and the upper lip, while intersecting subnasale.

*Otobasion inferior:* The lowest point of attachment of the auricle to the skin of the face.

*Otobasion superior:* The highest point of attachment of the auricle to the skin of the face.

*Pronasale:* the most protruded point or tip of the nose, which can be identified in lateral view of the rest of the portion of the head.

*Philtrum:* the midline groove that runs from lower border of the nasal septum to the top of the lip in the upper lip region.

*Pogonion:* Located on the soft tissue, the most anterior midpoint of the chin.

*Ridge (nasal):* The nasal ridge is the midline prominence of the nose, extending from the Sellion to the Pronasale.

*Sagittal plane:* A vertical plane that passes from anterior (front) to posterior (rear). The midsagittal plane is a sagittal plane that divides the body into right and left halves.

*Sellion:* Located on the soft tissue, the most concave point overlying the area of the frontonasal suture.

*Septal cartilage (nasal):* The nasal septal cartilage forms part of the septum and divides the front part of the nasal cavity.

*Subalare:* The point at the lower margin of the alar base, where the alar base joins with the skin of the superior (upper) lip.

*Subnasal point:* Located on the soft tissue, the point at which the columella merges with the upper lip in the midsagittal plane.

*Supramenton:* The point of greatest concavity in the midline of the lower lip between labrale inferius and soft tissue pogonion

### 6.1.3.2 Anatomy of the skull

*Frontal bone:* The frontal bone includes a large vertical portion, the *squama frontalis,* corresponding to the region known as the forehead.

*Mandible:* The mandible forms the lower jaw. The mental protuberance is the bony protuberance of the jaw that forms the chin.

*Maxilla:* The maxilla forms the upper jaw and is located above the mandible and below the orbits. The *frontal process of the maxilla* projects upwards by the side of the nose, and forms part of its lateral boundary.

*Nasal bones:* The nasal bones are two small oblong bones, varying in size and form in different individuals; they are placed side by side at the middle and upper part of the face, and form, by their junction, the "bridge" of the nose.

*Nasion:* The intersection of the frontal bone and the two nasal bones, a depressed area directly between the eyes and superior to the bridge of the nose.

*Occipital bone:* The occipital bone is situated at the back and lower part of the cranium. It includes an oval aperture, the *foramen magnum,* through which the cranial cavity communicates with the vertebral canal. The curved plate behind the foramen magnum is the *squama occipitalis.*

*Orbit:* The bony cavity in the skull to contain the eyeball.

*Parietal bones:* The parietal bones are the bones that, when joined together, form the roof and sides of the cranium.

*Temporal bones:* The temporal bones are situated on the bases and sides of the skull, and support that part of the face known as the temple.

*Zygomatic bones:* The face includes two zygomatic bones, located in the upper and lateral parts of the face and forming the prominence of the cheek.

### 6.1.3.3 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx:* The larynx, or voice box houses the vocal folds and connects the inferior part of the pharynx (hypopharynx) with the trachea.

*Lungs:* The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity:* The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 6.1.4 Patient interface

*Anti-asphyxia valve (AAV):* The component or sub-assembly of a mask or patient interface system that, by opening to atmosphere in a failsafe manner, reduces the risk of excessive CO₂ rebreathing by a patient.

*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

*Frame:* Frame will be taken to mean a mask or patient interface structure that bears the load of tension between two or more points of connection with a headgear. A mask or patient interface frame may be a non-airtight load bearing structure in the mask or patient interface. However, some forms of mask or patient interface frame may also be air-tight.

*Headgear:* Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane:* Membrane will be taken to mean a typically thin element that has, preferably, substantially no resistance to bending, but has resistance to being stretched.

*Plenum chamber:* a mask or patient interface plenum chamber will be taken to mean a portion of a patient interface having walls at least partially enclosing a volume of space, the volume having air therein pressurised above atmospheric pressure in use. A shell may form part of the walls of a mask or patient interface plenum chamber.

*Seal:* May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*

*Shell:* A shell will be taken to mean a curved, relatively thin structure having bending, tensile and compressive stiffness. For example, a curved structural wall of a mask or patient interface may be a shell. In some forms, a shell may be faceted. In some forms a shell may be airtight. In some forms a shell may not be airtight.

*Stiffener:* A stiffener will be taken to mean a structural component designed to increase the bending resistance of another component in at least one direction.

*Strut:* A strut will be taken to be a structural component designed to increase the compression resistance of another component in at least one direction.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

*Tie* (noun): A structure designed to resist tension.

*Vent:* (noun): A structure that allows a flow of *air* from an interior of the mask or patient interface, or conduit, to ambient air for clinically effective washout of exhaled gases. For example, a clinically effective washout may involve a flow rate of about 10 litres per minute to about 100 litres per minute, depending on the mask or patient interface design and treatment pressure.

### 6.1.5 Shape of structures

Products in accordance with the present technology may comprise one or more three-dimensional mechanical structures, for example a mask or patient interface cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

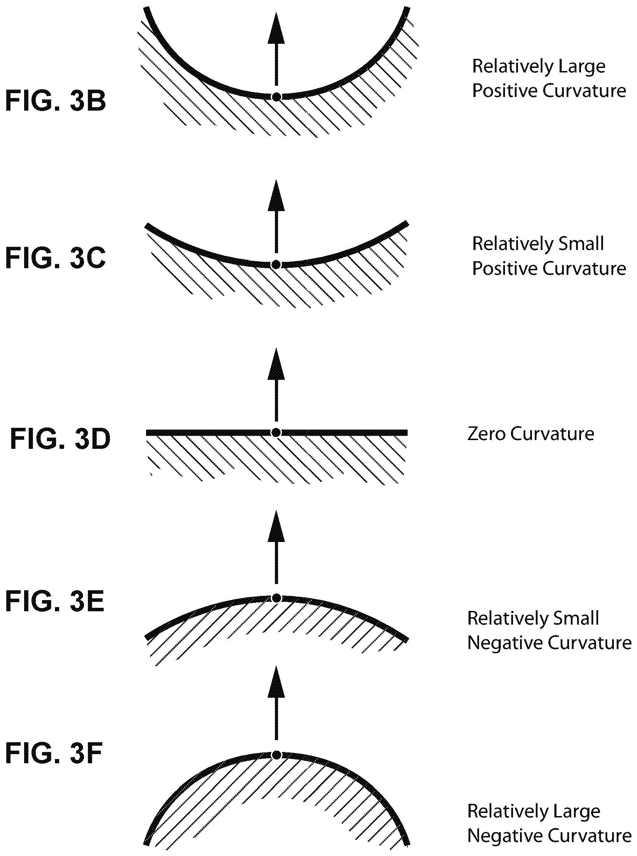

To facilitate describing the shape of the three-dimensional structures and the surfaces, we first consider a cross-section through a surface of the structure at a point, *p.* See Fig. 3B to Fig. 3F, which illustrate examples of cross-sections at point *p* on a surface, and the resulting plane curves. Figs. 3B to 3F also illustrate an outward normal vector at *p.* The outward normal vector at *p* points away from the surface. In some examples we describe the surface from the point of view of an imaginary small person standing upright on the surface.

### 6.1.5.1 Curvature in one dimension

The curvature of a plane curve at *p* may be described as having a sign (e.g. positive, negative) and a magnitude (e.g. 1/radius of a circle that just touches the curve at *p*)*.*

*Positive curvature:* If the curve at *p* turns towards the outward normal, the curvature at that point will be taken to be positive (if the imaginary small person leaves the point *p* they must walk uphill). See Fig. 3B (relatively large positive curvature compared to Fig. 3C) and Fig. 3C (relatively small positive curvature compared to Fig. 3B). Such curves are often referred to as concave.

*Zero curvature:* If the curve at *p* is a straight line, the curvature will be taken to be zero (if the imaginary small person leaves the point *p*, they can walk on a level, neither up nor down). See Fig. 3D.

*Negative curvature:* If the curve at *p* turns away from the outward normal, the curvature in that direction at that point will be taken to be negative (if the imaginary small person leaves the point *p* they must walk downhill). See Fig. 3E (relatively small negative curvature compared to Fig. 3F) and Fig. 3F (relatively large negative curvature compared to Fig. 3E). Such curves are often referred to as convex.

### 6.1.5.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

*Principal curvatures and directions:* The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at *p* are the curvatures in the principal directions.

*Region of a surface:* A connected set of points on a surface. The set of points in a region may have similar characteristics, e.g. curvatures or signs.

*Saddle region:* A region where at each point, the principal curvatures have opposite signs, that is, one is positive, and the other is negative (depending on the direction to which the imaginary person turns, they may walk uphill or downhill).

*Dome region:* A region where at each point the principal curvatures have the same sign, e.g. both positive (a "concave dome") or both negative (a "convex dome").

*Cylindrical region:* A region where one principal curvature is zero (or, for example, zero within manufacturing tolerances) and the other principal curvature is non-zero.

*Planar region:* A region of a surface where both of the principal curvatures are zero (or, for example, zero within manufacturing tolerances).

*Edge of a surface:* A boundary or limit of a surface or region.

*Path:* In certain forms of the present technology, 'path' will be taken to mean a path in the mathematical - topological sense, e.g. a continuous space curve from f(0) to f(1) on a surface. In certain forms of the present technology, a 'path' may be described as a route or course, including e.g. a set of points on a surface. (The path for the imaginary person is where they walk on the surface, and is analogous to a garden path).

*Path length:* In certain forms of the present technology, 'path length' will be taken to mean the distance along the surface from f(0) to f(1), that is, the distance along the path on the surface. There may be more than one path between two points on a surface and such paths may have different path lengths. (The path length for the imaginary person would be the distance they have to walk on the surface along the path).

*Straight-line distance:* The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 6.1.5.3 Space curves

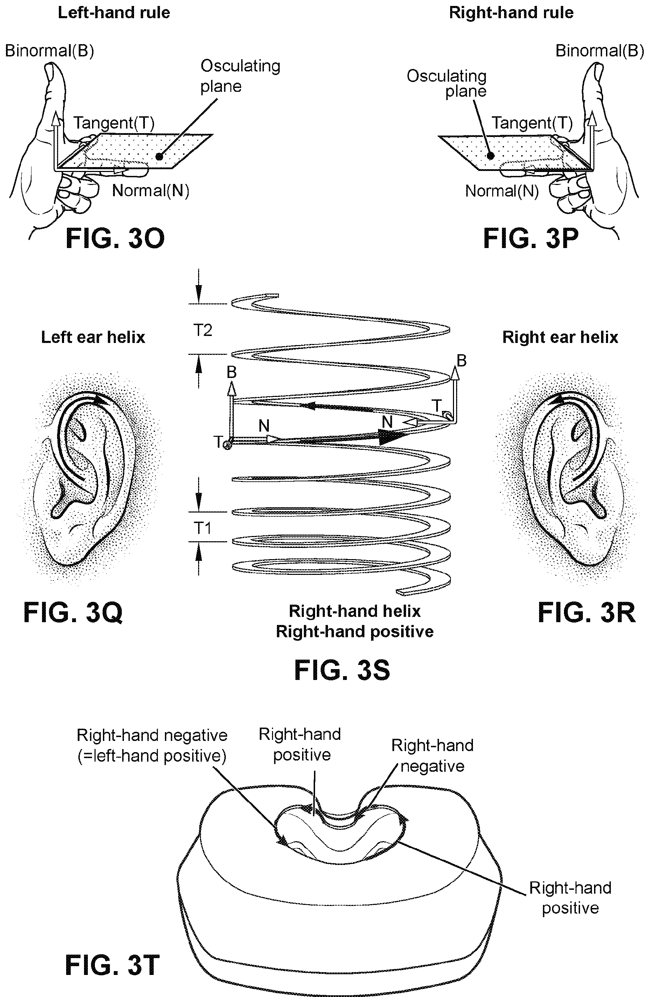

*Space curves:* Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

*Tangent unit vector (or unit tangent vector):* For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

*Unit normal vector:* As the imaginary person moves along the curve, this tangent vector itself changes. The unit vector pointing in the same direction that the tangent vector is changing is called the unit principal normal vector. It is perpendicular to the tangent vector.

*Binormal unit vector:* The binormal unit vector is perpendicular to both the tangent vector and the principal normal vector. Its direction may be determined by a right-hand rule (see e.g. Fig. 3P), or alternatively by a left-hand rule (Fig. 3O).

*Osculating plane:* The plane containing the unit tangent vector and the unit principal normal vector. See Figures 3O and 3P.

*Torsion of a space curve:* The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

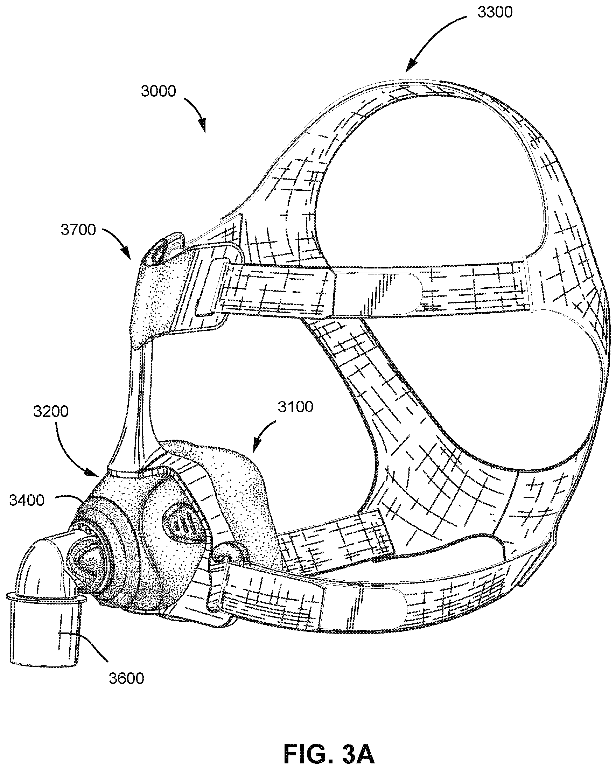

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 6.1.5.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

### 7 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology.

### 4 REFERENCE SIGNS LIST

| | |
|---|---|
| patient interface system | 1 |
| elbow | 2 |
| plug | 3 |
| swivel connector | 4 |
| tube | 5 |
| vent | 6 |
| gusset | 8 |
| asphyxia valve adaptor | 10 |
| nasal portion | 20 |
| headgear connectors | 21 |
| nares seal portion | 22 |
| decoupling portion | 25 |
| connector | 30 |
| headgear tabs | 31 |
| plug | 35 |
| mouth portion | 40 |
| headgear connector | 41 |
| sealing portion | 42 |
| nasal portion connector | 43 |
| connector | 44 |
| decoupling portion | 45 |
| opening | 46 |
| aperture | 46 |
| frame | 47 |
| chamber | 49 |
| rear opening | 50 |
| sealing ring | 51 |
| front aperture | 52 |
| knob | 54 |
| valve | 55 |
| docking station | 56 |
| headgear | 60 |
| headgear strap | 61 |
| side headgear connectors | 62 |
| lower headgear strap | 63 |
| locking device | 64 |
| headgear rear portion | 65 |
| crown headgear portion | 66 |
| connection region | 67 |
| strap loop | 68 |
| adjustment mechanism | 69 |
| apertures | 70 |
| bottom portion | 79 |
| side portions | 81 |
| top portion | 85 |
| neck portion | 87 |
| patient interface system | 101 |
| elbow | 102 |
| vent | 103 |
| flexible tube | 105 |
| nasal portion | 120 |
| headgear connectors | 121 |
| nares sealing portion | 122 |
| decoupling portion | 125 |
| mouth portion | 140 |
| headgear connectors | 141 |
| mouth sealing portion | 142 |
| nasal portion connector | 143 |
| decoupling portion | 145 |
| structural portion | 147 |
| headgear | 160 |
| patient interface system | 201 |
| elbow | 202 |
| nasal portion | 220 |
| wall | 221 |
| nares sealing portion | 222 |
| orifice | 226 |
| headgear connectors | 230 |
| headgear tabs | 231 |
| mouth portion | 240 |
| mouth seal portion | 242 |
| nares connecting portion | 243 |
| decoupling portion | 245 |
| mouth orifice | 246 |
| frame | 247 |
| support membrane | 252 |
| portion | 253 |
| nose tip engagement portion | 254 |
| upper lip engagement portion | 256 |
| front anchor points | 257 |
| corner regions | 258 |
| rear anchor points | 259 |
| thickened portions | 260 |
| rear thickened portions | 262 |
| upper portion | 264 |
| portion | 266 |
| curved portion | 268 |
| portion | 270 |
| patient interface system | 301 |
| flexible tube | 305 |
| connector | 306 |
| optional swivel connector | 308 |
| nasal portion | 320 |
| headgear connectors | 321 |
| nares sealing portion | 322 |
| decoupling portion | 325 |
| opening | 326 |
| mouth portion | 340 |
| decoupling portion | 345 |
| opening | 348 |
| connector | 349 |
| headgear strap | 360 |
| patient interface system | 370 |
| nasal portion | 372 |
| nares sealing portion | 374 |
| mouth sealing portion | 375 |
| gusset | 376 |
| air delivery tube | 379 |
| mouth portion | 380 |
| patient interface system | 401 |
| elbow | 402 |
| flexible tube | 405 |
| thickened regions | 409 |
| nasal portion | 420 |
| headgear connectors | 421 |
| nares sealing portion | 422 |
| decoupling portion | 425 |
| nares flare engagement portion | 427 |
| mouth portion | 440 |
| headgear connectors | 441 |
| mouth sealing portion | 442 |
| flexible portion | 443 |
| swivel connector | 444 |
| decoupling portion | 445 |
| upper lip engagement portion | 448 |
| nose tip engagement portion | 454 |
| upper lip engagement portion | 456 |
| support portion | 458 |
| headgear | 460 |
| nasal sealing area | 470 |
| sealing ring | 472 |
| patient interface system | 501 |
| elbow | 502 |
| flexible hose | 505 |
| nasal portion | 520 |
| headgear connectors | 521 |
| nares sealing portion | 522 |
| decoupling portion | 525 |
| mouth portion | 540 |
| headgear connectors | 541 |
| mouth sealing portion | 542 |
| swivel connector | 544 |
| optional decoupling portion | 545 |
| nose tip engagement portion | 554 |
| upper lip engagement portion | 556 |
| portion | 558 |
| headgear | 560 |
| foam patient contacting | 569 |
| patient contacting portion | 569 |
| sealing ring | 572 |
| cushion rigidizer | 573 |
| patient interface system | 701 |
| tube | 705 |
| connector | 710 |
| elbow | 712 |
| nasal portion | 720 |
| headgear connectors | 721 |
| sealing portion | 722 |
| decoupling portion | 725 |
| mouth portion | 740 |
| headgear connectors | 741 |
| mouth sealing portion | 742 |
| connector | 744 |
| decoupling portion | 745 |
| headgear | 760 |
| mouth kit | 801 |
| connector | 802 |
| plug | 803 |
| kit | 805 |
| mouth kit | 810 |
| nasal portion headgear | 812 |
| nares sealing portion | 820 |
| dial | 822 |
| rigid elements | 823 |
| opening | 826 |
| mouth portion | 840 |
| mouth portion headgear | 850 |
| elbow connector | 855 |
| connector | 856 |
| mouth portion headgear | 860 |
| mouth portion headgear | 870 |
| CPAP device | 901 |
| nasal portion | 910 |
| elbow | 912 |
| vent | 914 |
| tube | 915 |
| headgear | 918 |
| openings | 920 |
| connector | 924 |
| patient interface system | 940 |
| mouth portion | 942 |
| headgear connectors | 944 |
| headgear connectors | 946 |
| headgear | 950 |
| nasal portion | 952 |
| headgear | 954 |
| nasal pillows | 960 |
| patient interface system | 962 |
| connectors | 964 |
| mouth portion | 968 |
| nasal portion | 969 |
| nares engagement portion | 970 |
| support portion | 972 |
| connector | 976 |
| CPAP device | 980 |
| nasal portion | 982 |
| forehead portion | 984 |
| headgear connectors | 985 |
| forehead support pad | 986 |
| interface | 987 |
| cushion | 988 |
| patient interface system | 993 |
| nose engagement portion | 994 |
| lip engagement portion | 995 |
| headgear connector | 996 |
| headgear | 997 |
| cushion | 998 |
| front portion | 999 |
| patient | 1000 |
| opening | 1002 |
| flap | 1004 |
| open position | 1006 |
| patient interface system | 1100 |
| swivel | 1105 |
| nasal portion | 1120 |
| nozzles | 1122 |
| mouth portion | 1130 |
| upper lip engagement portion | 1132 |
| lip engagement portion | 1134 |
| elbow | 1140 |
| lower end | 1141 |
| first elbow ring | 1142 |
| barbed end portions | 1143 |
| second elbow ring | 1144 |
| first branch | 1146 |
| second branch | 1148 |
| nasal portion | 1150 |
| nozzles | 1152 |
| portion | 1153 |
| foam portion | 1154 |
| nasal portion | 1160 |
| portion | 1162 |
| sealing portion | 1164 |
| sealing portions | 1164 |
| end portions | 1165 |
| nasal portion | 1170 |
| portion | 1172 |
| sealing portion | 1174 |
| nose tip engagement portion | 1176 |
| upper lip engagement portion | 1178 |
| patient interface system | 1180 |
| decoupling portion | 1181 |
| mouth portion | 1182 |
| flap | 1183 |
| lip engagement portion | 1184 |
| air hose | 1185 |
| upper lip engagement portion | 1186 |
| swivel | 1187 |
| elbow | 1188 |
| sealing ring | 1189 |
| nasal portion | 1190 |
| portion | 1192 |
| sealing portion | 1193 |
| nose tip engagement portion | 1194 |
| upper lip engagement portion | 1196 |
| mouth clip | 1198 |
| patient interface system | 1200 |
| mouth portion | 1202 |
| front portion | 1204 |
| lip engagement portion | 1206 |
| upper lip engagement portion | 1208 |
| nasal portion tab | 1210 |
| flap | 1212 |
| decoupling portion | 1214 |
| nasal portion | 1220 |
| portion | 1222 |
| sealing portion | 1224 |
| upper lip engagement portion | 1226 |
| nose tip engagement portion | 1228 |
| attachment ring | 1229 |
| elbow | 1230 |
| air delivery tube | 1232 |
| patient interface system | 1260 |
| mouth portion | 1262 |
| lip engagement portion | 1264 |
| upper lip engagement portion | 1266 |
| mouth clip | 1268 |
| flexible portion | 1270 |
| aperture | 1272 |
| aperture | 1274 |
| nasal portion | 1280 |
| portion | 1282 |
| sealing portion | 1284 |
| nares clip | 1286 |
| headgear connectors | 1290 |
| patient interface system | 1300 |
| mouth portion | 1302 |
| lip engagement portion | 1304 |
| upper lip engagement portion | 1306 |
| nasal portion | 1310 |
| nose tip engagement portion | 1312 |
| nares engagement portion | 1314 |
| decoupling portion | 1316 |
| air supply hose | 1318 |
| patient interface system | 1320 |
| mouth portion | 1322 |
| lip engagement portion | 1324 |
| frame | 1326 |
| nasal portion | 1330 |
| portion | 1332 |
| sealing portion | 1334 |
| nose tip engagement portion | 1336 |
| upper lip engagement portion | 1337 |
| clip portion | 1338 |
| swivel | 1342 |
| air supply hose | 1344 |
| patient interface system | 1350 |
| modular patient interface system | 1350 |
| first aperture | 1351 |
| mouth portion | 1352 |
| frame | 1354 |
| mouth seal | 1355 |
| lip engagement portion | 1356 |
| second aperture | 1357 |
| upper lip engagement portion | 1358 |
| ring | 1359 |
| nasal portion | 1360 |
| portion | 1362 |
| sealing portion | 1364 |
| nose tip engagement portion | 1368 |
| upper lip engagement portion | 1370 |
| elbow | 1374 |
| swivel | 1376 |
| nasal portions | 1380 |
| foam portion | 1382 |
| holes | 1384 |
| nasal portions | 1390 |
| foam portion | 1392 |
| holes | 1394 |
| patient interface system | 1400 |
| mouth portion | 1402 |
| front portion | 1404 |
| foam portion | 1405 |
| upper foam portion | 1406 |
| elbow | 1407 |
| outer foam portion | 1408 |
| air supply hose | 1409 |
| nasal portion | 1410 |
| portion | 1412 |
| sealing portion | 1414 |
| nose tip engagement portion | 1416 |
| upper lip engagement portion | 1418 |
| headgear | 1420 |
| modular patient interface system | 1430 |
| mouth portion | 1432 |
| cushion portion | 1434 |
| magnets | 1436 |
| nasal portion | 1440 |
| sealing portion | 1442 |
| upper lip engagement portion | 1443 |
| portion | 1446 |
| nose regions | 1447 |
| magnets | 1448 |
| patient interface system | 1480 |
| mouth portion | 1482 |
| frame | 1484 |
| aperture | 1485 |
| flap | 1486 |
| upper aperture | 1487 |
| nasal portion | 1490 |
| foam portion | 1492 |
| clip | 1493 |
| nozzle portion | 1494 |
| flexible portion | 1496 |
| cuff | 1498 |
| air hose | 1499 |
| patient interface system | 1500 |
| mouth portion | 1502 |
| upper aperture | 1503 |
| frame | 1504 |
| aperture | 1505 |
| foam portion | 1506 |
| elbow | 1507 |
| clip | 1508 |
| flap | 1509 |
| nasal portion | 1510 |
| flexible portion | 1512 |
| clips | 1514 |
| foam portion | 1516 |
| patient interface system | 1520 |
| mouth portion | 1522 |
| frame | 1523 |
| lip engagement portion | 1524 |
| upper lip engagement portion | 1526 |
| aperture | 1527 |
| aperture | 1528 |
| nasal portion | 1540 |
| portion | 1542 |
| sealing portion | 1544 |
| nose tip engagement portion | 1546 |
| upper lip engagement portion | 1548 |
| ring | 1550 |
| first channel | 1552 |
| second channel | 1554 |
| elbow | 1560 |
| vent holes | 1562 |
| air hose | 1566 |
| upper aperture | 1577 |
| aperture | 1579 |
| patient interface system | 1580 |
| elbow | 1581 |
| mouth portion | 1582 |
| lip engagement portion | 1584 |
| vent holes | 1585 |
| upper lip engagement portion | 1586 |
| air hose | 1587 |
| frame | 1589 |
| nasal portion | 1590 |
| portion | 1592 |
| sealing portion | 1594 |
| indentations | 1595 |
| nose tip engagement portion | 1596 |
| upper lip engagement portion | 1598 |
| modular patient interface system | 1600 |
| mouth portion | 1602 |
| nasal portion | 1610 |
| swivel | 1612 |
| connectors | 1615 |
| connectors | 1617 |
| upper headgear | 1618 |
| strap | 1619 |
| lower headgear | 1620 |
| back loop | 1622 |
| modular patient interface system | 1630 |
| mouth portion | 1632 |
| aperture | 1634 |
| nasal portion | 1640 |
| elbow | 1644 |
| air hose | 1646 |
| patient interface system | 1650 |
| mouth portion | 1652 |
| support structure | 1652 |
| mouth cushion | 1654 |
| connecting portion | 1655 |
| aperture | 1656 |
| cushion fascia portion | 1657 |
| cushion fascia portion | 1658 |
| decoupling portion | 1659 |
| nasal portion | 1660 |
| lobe | 1661 |
| sealing portion | 1662 |
| indentation | 1663 |
| sealing ring | 1665 |
| headgear connectors | 1670 |
| gusset | 1671 |
| ring connecting portion | 1680 |
| aperture | 1682 |
| pocket | 1684 |
| thickened portion | 1686 |
| top lip region | 1692 |
| seal region | 1693 |
| ridge | 1694 |
| cheek region | 1695 |
| headgear | 2000 |
| point headgear | 2001 |
| first rear strap | 2002 |
| second rear strap | 2006 |
| 9th edition published | 2012 |
| mouth cushion | 2100 |
| pocket | 2102 |
| single side wall | 2104 |
| region | 2655 |
| top lip region | 2692 |
| adjoin top lip portion | 2692 |
| chin region | 2693 |
| ridge | 2694 |
| cheek region | 2695 |
| patient interface | 3000 |
| pillows | 3001 |
| mouth cushion subassembly | 3002 |
| elbow | 3004 |
| cushion | 3006 |
| fascia | 3008 |
| clip | 3010 |
| lip seal | 3011 |
| fitting | 3012 |
| reliefs | 3013 |
| tabs | 3014 |
| seal - forming structure | 3100 |
| plenum chamber | 3200 |
| chord | 3209 |
| shell | 3210 |
| superior point | 3220 |
| inferior point | 3229 |
| nasal portion | 3230 |
| inlet port | 3240 |
| plenum chamber inlet ports | 3240 |
| structure | 3300 |
| vent | 3400 |
| swivel elbow assembly | 3610 |
| ISO | 3744 |
| RPT device | 4000 |
| patient interface system | 4001 |
| external housing | 4010 |
| upper portion | 4012 |
| portion | 4014 |
| panel s | 4015 |
| chassis | 4016 |
| handle | 4018 |
| pneumatic block | 4020 |
| mouth cushion | 4100 |
| pocket | 4102 |
| upper connecting portion | 4104 |
| sealing portion | 4106 |
| rear edge | 4108 |
| aperture | 4110 |
| air filter | 4111 |
| lip seal | 4112 |
| inlet air filter | 4113 |
| groove | 4114 |
| outlet air filter | 4115 |
| muffler | 4120 |
| inlet muffler | 4122 |
| outlet muffler | 4124 |
| pressure generator | 4140 |
| blower | 4142 |
| motor | 4144 |
| anti - spill back valve | 4160 |
| air circuit | 4170 |
| supplemental oxygen | 4180 |
| cushion clip | 4200 |
| electrical components | 4201 |
| nasal seal connecting portion | 4202 |
| PCBA | 4203 |
| fitting | 4204 |
| reliefs | 4206 |
| tabs | 4208 |
| portions | 4210 |
| electrical power supply | 4211 |
| fascia contacting lip | 4212 |
| cushion contacting lip | 4214 |
| tabs | 4218 |
| input devices | 4220 |
| transducers | 4270 |
| output devices | 4290 |
| fascia | 4300 |
| groove | 4302 |
| tab | 4304 |
| lower headgear connectors | 4306 |
| elbow connector | 4308 |
| slots | 4310 |
| humidifier | 5000 |
| patient interface | 7000 |
| mouth portion | 7002 |
| nasal portion | 7004 |
| air vent | 7006 |
| connector | 7008 |
| nasal chamber | 7010 |
| mouth chamber | 7012 |
| prongs | 7014 |
| conduits | 7016 |
| junction | 7018 |
| headgear strap | 7020 |
| eyelets | 7022 |
| opening | 7024 |
| nasal chamber coupling component | 7026 |
| opening | 7028 |
| coupling component | 7030 |
| AAV | 7032 |
| opening | 7033 |
| nasal portion end | 7034 |
| flap | 7035 |
| ends | 7036 |
| air vent | 7037 |
| passage | 7038 |
| coupling component | 7039 |
| guide vanes | 7040 |
| AAV | 7050 |
| opening | 7052 |
| hard interface | 7053 |
| flap | 7054 |
| robust seal | 7055 |
| additional third chamber | 7056 |
| flange | 7058 |
| flanges | 7060 |
| matching grooves | 7062 |
| retainer | 7064 |
| chamber | 7069 |
| lead - in surface | 7070 |
| flange | 7072 |
| aggressive retention barb | 7074 |
| flanges | 7076 |
| fins | 7078 |
| clip mechanism | 7080 |
| latch portion | 7090 |
| hook portion | 7092 |
| latch portion | 7094 |
| rigid insert | 7096 |
| corrugated flap | 7098 |
| lip | 7099 |
| nasal portion headgear | 8812 |
| slit | 8814 |
| mouth portion headgear | 8850 |
| U - shaped segment | 8852 |
| connector | 8856 |
| tabs | 8857 |
| latches | 8858 |
| alignment guide | 8860 |
| flap | 8862 |
| connector | 9000 |
| headgear hinge magnet | 9010 |
| hinge portion | 9020 |
| headgear connector | 9030 |
| patient interface engagement portion | 9040 |
| patient interface connectors | 9050 |
| magnets | 9060 |
| support pads | 9070 |
| chassis | 9100 |
| region | 9102 |
| central region | 9102 |
| side regions | 9104 |
| respective support pad | 9110 |
| connector | 8856' |
| connectors | 8856' |
| support walls | 1162 - 1 |

## Claims

1. A patient interface system (7000) for delivery of respiratory therapy to a patient, comprising:
a nasal portion (7004) including a nasal chamber (7010) and a nasal sealing portion adapted to form a seal with the patient's face surrounding the entrance to a patient's nares, the nasal chamber (7010) including an opening (7024) allowing for part of a fluid coupling between the nasal portion (7004) and a mouth portion (7002);
inlet conduits (7016) connected to the nasal portion (7004) to deliver the pressurized, breathable gas to the nasal chamber (7010), the inlet conduits (7016) forming a part of a hoop that passes over the top of the patient's head, the hoop incorporating the inlet conduits (7016) and the nasal portion (7004), wherein in use the inlet conduits (7016) extend across the patient's cheek regions and the hoop is arranged so that portions of the inlet tubes contact regions of the patient's head superior to an otobasion superior of the patient's head;
a nasal chamber coupling component (7026) adapted to form part of a releasable fluid coupling to enable the nasal portion (7004) to be fluidly coupled to the mouth portion (7002) to communicate a portion of the pressurized, breathable gas through the nasal chamber (7010) opening to the mouth portion (7002), the nasal chamber coupling component (7026) surrounding an exterior of the opening (7024) so as to form part of a sealed connection for fluidly connecting the nasal chamber (7010) to the mouth portion (7002) or to a vent (7006) or to a connector (7008),
with the nasal chamber coupling component (7026) being manufactured utilising an over-molding process, and/or being resiliently malleable, and/or comprising a plurality of clipping flanges at a distal end, and/or comprising a lead-in surface, and/or having a protruding latch portion; and
the mouth portion (7002).

2. A patient interface system (7000) according to claim 1, wherein the nasal portion (7004) comprises a nasal cushion that forms at least part of the nasal chamber (7010) and wherein the nasal chamber opening (7024) and the coupling component (7026) are formed in the nasal cushion.

3. A patient interface system (7000) according to claim 2, wherein the nasal cushion (7004) is a pillow cushion or a cradle cushion.

4. A patient interface system (7000) according to any preceding claim, wherein the coupling component (7026) comprises a clip mechanism that extends about the nasal chamber opening (7024) and arranged to form part of a resilient clip connection.

5. A patient interface system (7000) according to claim 4, wherein the clip mechanism includes a resilient hinge.

6. A patient interface system (7000) according to any one of claims 1 to 3, wherein the coupling component (7026) comprises an at least partial peripheral rim extending about the nasal chamber opening 7024).

7. A patient interface system (7000) according to claim 6, wherein the peripheral rim includes at least one of projections or recesses that allow for an interlocking connection.

8. A patient interface system (7000) according to claim 6 or 7, wherein the peripheral rim includes a raked surface to provide a lead in surface to a resilient connection.

9. A patient interface system (7000) according to any one of claims 1 to 3, wherein the coupling component forms part of a latch connection.

10. A patient interface system (7000) according to any preceding claim, further comprising a removable vent module (7006) adapted to be attached to the nasal chamber (7010) to vent gas exhaled by the patient; the vent module (7006) being arranged to be releasable connectable with the nasal chamber coupling component (7026) such that the patient interface system is configurable in a first mode where the vent module is fluidly coupled to the nasal chamber (7010) to enable the patient interface system to operate as a nasal only patient interface and a second mode where the vent module (7006) is removed to enable the nasal portion (7004) to be fluidly coupled to a mouth portion (7002) to communicate a portion of the pressurized, breathable gas through the nasal chamber (7010) opening to the mouth portion (7002).

11. A patient interface system (7000) according to claim 10, further comprising at least one guide vane (7040) providing an obstruction to the fluid coupling to direct the follow of gas from the nasal chamber (7010) through the vent module.

12. A patient interface system (7000) according to claim 11, wherein the at least one guide vane (7040) forms part of the vent module (7006).

13. A patient interface system (7000) according to any one of claims 10 to 12, wherein the vent module (7050) is an anti-asphyxiation vent (AAV).

14. A patient interface system (7000) as claimed in claim 13, wherein the AAV (7050) includes a flap (7054) and the at least one guide vane (7040) is arranged to direct gas from the nasal chamber onto the flap (7054).

15. A patient interface system (7000) according to any preceding claim, further comprising a mouth portion (7002) including a mouth chamber (7012) and a mouth sealing portion adapted to form a seal with the patient's mouth, the mouth chamber (7012) including an opening (7028) adapted to receive pressurized, breathable gas, and wherein when fluidly coupled, the nasal portion (7004) is adapted to communicate the portion of the pressurized, breathable gas to the mouth portion (7002) through the nasal chamber opening (7024) and the mouth chamber opening (7028).

## Patentansprüche

1. Patientenschnittstellensystem (7000) zur Abgabe von Atemtherapie an einen Patienten, das aufweist:
einen Nasenabschnitt (7004) mit einer Nasenkammer (7010) und einem Nasendichtungsabschnitt, der geeignet ist, eine Dichtung mit dem Patientengesicht zu bilden, die den Eingang zu den Nasenlöchern eines Patienten umgibt, wobei die Nasenkammer (7010) eine Öffnung (7024) aufweist, die einen Teil einer Fluidkopplung zwischen dem Nasenabschnitt (7004) und einem Mundabschnitt (7002) ermöglicht;
Einlassleitungen (7016), die mit dem Nasenabschnitt (7004) verbunden sind, um das Druckatemgas zur Nasenkammer (7010) abzugeben, wobei die Einlassleitungen (7016) einen Teil eines Bügels bilden, der über der Oberseite des Patientenkopfs verläuft, und der Bügel die Einlassleitungen (7016) und den Nasenabschnitt (7004) beinhaltet, wobei sich im Gebrauch die Einlassleitungen (7016) über die Wangenregionen des Patienten erstrecken und der Bügel so angeordnet ist, dass Abschnitte der Einlassschläuche Regionen des Patientenkopfs kontaktieren, die superior zu einem Otobasion superior des Patientenkopfs sind;
eine Nasenkammer-Koppelkomponente (7026), die geeignet ist, Teil einer lösbaren Fluidkopplung zu bilden, damit der Nasenabschnitt (7004) mit dem Mundabschnitt (7002) fluidgekoppelt sein kann, um einen Anteil des Druckatemgases durch die Nasenkammer- (7010) Öffnung hindurch zum Mundabschnitt (7002) zu leiten, wobei die Nasenkammer-Koppelkomponente (7026) eine Außenseite der Öffnung (7024) umgibt, um Teil einer abgedichteten Verbindung zum Fluidverbinden der Nasenkammer (7010) mit dem Mundabschnitt (7002) oder mit einer Entlüftung (7006) oder mit einem Verbinder (7008) zu bilden,
wobei die Nasenkammer-Koppelkomponente (7026) mit Hilfe eines Überformungsverfahrens hergestellt ist und/oder elastisch verformbar ist und/oder mehrere Clipflansche an einem distalen Ende aufweist und/oder eine Einführfläche aufweist und/oder einen vorstehenden Riegelabschnitt hat; und
den Mundabschnitt (7002).

2. Patientenschnittstellensystem (7000) nach Anspruch 1, wobei der Nasenabschnitt (7004) ein Nasenpolster aufweist, das mindestens Teil der Nasenkammer (7010) bildet, und wobei die Nasenkammeröffnung (7024) und die Koppelkomponente (7026) im Nasenpolster ausgebildet sind.

3. Patientenschnittstellensystem (7000) nach Anspruch 2, wobei das Nasenpolster (7004) ein Kissenpolster oder ein Wiegen-Polster ist.

4. Patientenschnittstellensystem (7000) nach einem der vorstehenden Ansprüche, wobei die Koppelkomponente (7026) einen Clipmechanismus aufweist, der sich um die Nasenkammeröffnung (7024) erstreckt und so angeordnet ist, dass er Teil einer elastischen Clipverbindung bildet.

5. Patientenschnittstellensystem (7000) nach Anspruch 4, wobei der Clipmechanismus ein elastisches Scharnier aufweist.

6. Patientenschnittstellensystem (7000) nach einem der Ansprüche 1 bis 3, wobei die Koppelkomponente (7026) einen mindestens teilweisen Umfangsrand aufweist, der sich um die Nasenkammeröffnung (7024) erstreckt.

7. Patientenschnittstellensystem (7000) nach Anspruch 6, wobei der Umfangsrand Vorsprünge und/oder Aussparungen aufweist, die eine ineinandergreifende Verbindung ermöglichen.

8. Patientenschnittstellensystem (7000) nach Anspruch 6 oder 7, wobei der Umfangsrand eine Schrägfläche aufweist, um eine Einführfläche für eine elastische Verbindung bereitzustellen.

9. Patientenschnittstellensystem (7000) nach einem der Ansprüche 1 bis 3, wobei die Koppelkomponente Teil einer Riegelverbindung bildet.

10. Patientenschnittstellensystem (7000) nach einem der vorstehenden Ansprüche, ferner mit einem entfernbaren Entlüftungsmodul (7006), das geeignet ist, an der Nasenkammer (7010) angebracht zu sein, um vom Patienten ausgeatmetes Gas zu entlüften; wobei das Entlüftungsmodul (7006) so angeordnet ist, dass es mit der Nasenkammer-Koppelkomponente (7026) lösbar verbindbar ist, so dass das Patientenschnittstellensystem konfigurierbar ist in einem ersten Modus, in dem das Entlüftungsmodul mit der Nasenkammer (7010) fluidgekoppelt ist, damit das Patientenschnittstellensystem als ausschließliche Nasen-Patientenschnittstelle arbeiten kann, und einem zweiten Modus, in dem das Entlüftungsmodul (7006) entfernt ist, damit der Nasenabschnitt (7004) mit einem Mundabschnitt (7002) fluidgekoppelt sein kann, um einen Anteil des Druckatemgases durch die Nasenkammer- (7010) Öffnung hindurch zum Mundabschnitt (7002) zu leiten.

11. Patientenschnittstellensystem (7000) nach Anspruch 10, ferner mit mindestens einer Leitschaufel (7040), die ein Hindernis für die Fluidkopplung darstellt, um die Gasströmung aus der Nasenkammer (7010) durch das Entlüftungsmodul hindurch zu richten.

12. Patientenschnittstellensystem (7000) nach Anspruch 11, wobei die mindestens eine Leitschaufel (7040) Teil des Entlüftungsmoduls (7006) bildet.

13. Patientenschnittstellensystem (7000) nach einem der Ansprüche 10 bis 12, wobei das Entlüftungsmodul (7050) eine Anti-Asphyxie-Entlüftung (AAV) ist.

14. Patientenschnittstellensystem (7000) nach Anspruch 13, wobei die AAV (7050) eine Klappe (7054) aufweist und die mindestens eine Leitschaufel (7040) so angeordnet ist, dass sie Gas aus der Nasenkammer auf die Klappe (7054) richtet.

15. Patientenschnittstellensystem (7000) nach einem der vorstehenden Ansprüche, ferner mit einem Mundabschnitt (7002), der eine Mundkammer (7012) und einen Munddichtungsabschnitt aufweist, der geeignet ist, eine Dichtung mit dem Patientenmund zu bilden, wobei die Mundkammer (7012) eine Öffnung (7028) aufweist, die geeignet ist, Druckatemgas aufzunehmen, und wobei bei Fluidkopplung der Nasenabschnitt (7004) geeignet ist, den Anteil des Druckatemgases zum Mundabschnitt (7002) durch die Nasenkammeröffnung (7024) und die Mundkammeröffnung (7028) hindurch zu leiten.

## Revendications

1. Système d'interface patient (7000) d'administration d'une thérapie respiratoire à un patient, comprenant :
une partie nasale (7004) comprenant une chambre nasale (7010) et une partie d'étanchéité nasale conçue pour former une étanchéité avec le visage du patient entourant l'entrée des narines du patient, la chambre nasale (7010) comprenant une ouverture (7024) permettant une partie d'un accouplement fluidique entre la partie nasale (7004) et une partie buccale (7002) ;
des conduits d'entrée (7016) raccordés à la partie nasale (7004) pour délivrer le gaz respirable mis sous pression à la chambre nasale (7010), les conduits d'entrée (7016) faisant partie d'un cerceau qui passe sur le sommet de la tête du patient, le cerceau incorporant les conduits d'entrée (7016) et la partie nasale (7004), dans lequel, en utilisation, les conduits d'entrée (7016) s'étendent d'un bout à l'autre des régions de joue du patient et le cerceau est disposé de sorte que des parties des tubes d'entrée contactent des régions de la tête du patient supérieures à un otobasion supérieur de la tête du patient ;
un composant d'accouplement de chambre nasale (7026) conçu pour former une partie d'un accouplement fluidique libérable servant à accoupler en communication fluidique la partie nasale (7004) à la partie buccale (7002) pour communiquer une partie du gaz respirable mis sous pression par l'intermédiaire de l'ouverture de chambre nasale (7010) à la partie buccale (7002), le composant d'accouplement de chambre nasale (7026) entourant l'extérieur de l'ouverture (7024) de façon à former une partie d'une connexion étanche permettant de mettre en communication fluidique la chambre nasale (7010) avec la partie buccale (7002) ou avec un évent (7006) ou avec un connecteur (7008),
le composant d'accouplement de chambre nasale (7026) étant fabriqué en utilisant un processus de surmoulage, et/ou étant malléable élastiquement, et/ou comprenant une pluralité de brides de serrage au niveau d'une extrémité distale, et/ou comprenant une surface de raccordement, et/ou comportant une partie de verrouillage en saillie ; et
la partie buccale (7002).

2. Système d'interface patient (7000) selon la revendication 1, dans lequel la partie nasale (7004) comprend un coussin nasal qui forme au moins une partie de la chambre nasale (7010), et dans lequel l'ouverture de chambre nasale (7024) et le composant d'accouplement (7026) sont formés dans le coussin nasal.

3. Système d'interface patient (7000) selon la revendication 2, dans lequel le coussin nasal (7004) est un coussin en forme d'oreiller ou un coussin en forme de berceau.

4. Système d'interface patient (7000) selon l'une quelconque des revendications précédentes, dans lequel le composant d'accouplement (7026) comprend un mécanisme d'attache qui s'étend autour de l'ouverture de chambre nasale (7024) et qui est conçu pour former une partie d'une connexion d'attache élastique.

5. Système d'interface patient (7000) selon la revendication 4, dans lequel le mécanisme d'attache comprend une charnière élastique.

6. Système d'interface patient (7000) selon l'une quelconque des revendications 1 à 3, dans lequel le composant d'accouplement (7026) comprend un rebord périphérique au moins partiel s'étendant autour de l'ouverture de chambre nasale (7024).

7. Système d'interface patient (7000) selon la revendication 6, dans lequel le rebord périphérique comprend des saillies et/ou des évidements qui permettent une connexion de verrouillage mutuel.

8. Système d'interface patient (7000) selon la revendication 6 ou la revendication 7, dans lequel le rebord périphérique comprend une surface inclinée qui fournit une surface de raccordement à une connexion élastique.

9. Système d'interface patient (7000) selon l'une quelconque des revendications 1 à 3, dans lequel le composant d'accouplement fait partie d'une connexion à verrouillage.

10. Système d'interface patient (7000) selon l'une quelconque des revendications précédentes, comprenant en outre un module d'évent amovible (7006) conçu pour être fixé à la chambre nasale (7010) pour mettre à l'air libre un gaz exhalé par le patient ; le module d'évent (7006) étant conçu pour pouvoir être connecté de manière libérable au composant d'accouplement de chambre nasale (7026) de sorte que le système d'interface patient puisse être configuré dans un premier mode dans lequel le module d'évent est accouplé en communication fluidique avec la chambre nasale (7010) pour permettre au système d'interface patient de fonctionner en tant qu'interface patient uniquement nasale et dans un second mode dans lequel le module d'évent (7006) est retiré pour permettre à la partie nasale (7004) d'être accouplée en communication fluidique avec une partie buccale (7002) de façon à communiquer une partie du gaz respirable mis sous pression par l'intermédiaire de l'ouverture de chambre nasale (7010) à la partie buccale (7002).

11. Système d'interface patient (7000) selon la revendication 10, comprenant en outre au moins une aube directrice (7040) fournissant une obstruction à l'accouplement fluidique pour diriger le flux de gaz de la chambre nasale (7010) à travers le module d'évent.

12. Système d'interface patient (7000) selon la revendication 11, dans lequel l'au moins une aube directrice (7040) fait partie du module d'évent (7006).

13. Système d'interface patient (7000) selon l'une quelconque des revendications 10 à 12, dans lequel le module d'évent (7050) est un évent anti-asphyxie (AAV).

14. Système d'interface patient (7000) selon la revendication 13, dans lequel l'AAV (7050) comprend un volet (7054) et l'au moins une aube directrice (7040) est conçue pour diriger du gaz de la chambre nasale sur le volet (7054).

15. Système d'interface patient (7000) selon l'une quelconque des revendications précédentes, comprenant en outre une partie buccale (7002) comprenant une chambre buccale (7012) et une partie d'étanchéité buccale conçue pour former une étanchéité avec la bouche du patient, la chambre buccale (7012) comprenant une ouverture (7028) conçue pour recevoir un gaz respirable mis sous pression, et dans lequel, en accouplement fluidique, la partie nasale (7004) est conçue pour communiquer la partie du gaz respirable mis sous pression à la partie buccale (7002) par l'intermédiaire de l'ouverture de chambre nasale (7024) et de l'ouverture de chambre buccale (7028).
